(19) Europäisches Patentamt
European Patent Office
Office européen des brevets

(11) EP 3 728 239 B1

(12) **EUROPEAN PATENT SPECIFICATION**

(45) Date of publication and mention of the grant of the patent:
**20.05.2026 Bulletin 2026/21**

(21) Application number: **18830743.3**

(22) Date of filing: **10.12.2018**

(51) International Patent Classification (IPC):
***C07D 491/048*** *(2006.01)* ***C07D 495/04*** *(2006.01)*
***C09K 11/07*** *(2006.01)* ***H05B 33/00*** *(2006.01)*

(52) Cooperative Patent Classification (CPC):
**C07D 491/048; C07D 405/14; C07D 495/04; C09K 11/06; H10K 85/654; H10K 85/6572; H10K 85/6574;** H10K 50/11; Y02E 10/549

(86) International application number:
**PCT/EP2018/084191**

(87) International publication number:
**WO 2019/121112 (27.06.2019 Gazette 2019/26)**

(54) **ORGANIC MOLECULES FOR USE IN OPTOELECTRONIC DEVICES**

ORGANISCHE MOLEKÜLE ZUR VERWENDUNG IN OPTOELEKTRONISCHEN VORRICHTUNGEN

MOLÉCULES ORGANIQUES DESTINÉES À ÊTRE UTILISÉES DANS DES DISPOSITIFS OPTOÉLECTRONIQUES

(84) Designated Contracting States:
**AL AT BE BG CH CY CZ DE DK EE ES FI FR GB GR HR HU IE IS IT LI LT LU LV MC MK MT NL NO PL PT RO RS SE SI SK SM TR**

(30) Priority: **21.12.2017 DE 102017130982**

(43) Date of publication of application:
**28.10.2020 Bulletin 2020/44**

(73) Proprietor: **Samsung Display Co., Ltd.**
**Gyeonggi-do 17113 (KR)**

(72) Inventor: **SZAFRANOWSKA, Barbara**
**64625 Bensheim (DE)**

(74) Representative: **Dr. Weitzel & Partner**
**Patent- und Rechtsanwälte mbB**
**Friedenstrasse 10**
**89522 Heidenheim (DE)**

(56) References cited:
**EP-A1- 3 130 591**
**WO-A1-2016/089080**
**WO-A1-2017/005699**

Note: Within nine months of the publication of the mention of the grant of the European patent in the European Patent Bulletin, any person may give notice to the European Patent Office of opposition to that patent, in accordance with the Implementing Regulations. Notice of opposition shall not be deemed to have been filed until the opposition fee has been paid. (Art. 99(1) European Patent Convention).

EP 3 728 239 B1

## Description

The invention relates to light-emitting organic molecules and their use in organic light-emitting diodes (OLEDs) and in other optoelectronic devices.

## Prior art

WO 2016/089080 A1 discloses an organic luminescent compound represented by chemical formula 1 and an organic electroluminescent device comprising such compounds. Chemical formula 1 is as follows:

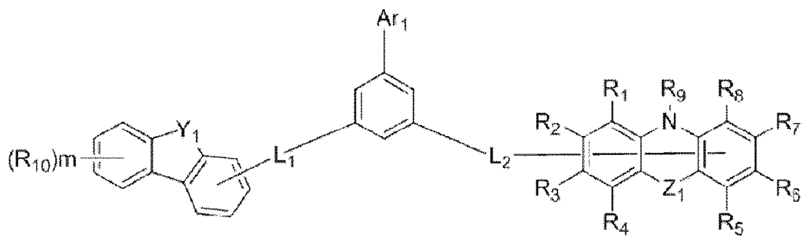

,

wherein $Ar_1$, $L_1$, $L_2$, $Y_1$, $R_1$ to $R_{10}$, $Z_1$, and m are defined as disclosed in WO 2016/089080 A1.

## Description

The object of the present invention is to provide molecules which are suitable for use in optoelectronic devices.

This object is achieved by the invention which provides a new class of organic molecules.

According to the invention the organic molecules are purely organic molecules, i.e. they do not contain any metal ions in contrast to metal complexes known for use in optoelectronic devices.

According to the present invention, the organic molecules exhibit emission maxima in the blue, sky-blue or green spectral range. The organic molecules exhibit in particular emission maxima between 420 nm and 520 nm, preferably between 440 nm and 495 nm, more preferably between 450 nm and 470 nm. The photoluminescence quantum yields of the organic molecules according to the invention are, in particular, 50 % or more. The molecules according to the invention exhibit in particular thermally activated delayed fluorescence (TADF). The use of the molecules according to the invention in an optoelectronic device, for example an organic light-emitting diode (OLED), leads to higher efficiencies of the device. Corresponding OLEDs have a higher stability than OLEDs with known emitter materials and comparable color.

The organic light-emitting molecules of the invention comprise or consist of one first chemical moiety comprising or consisting of a structure of Formula I,

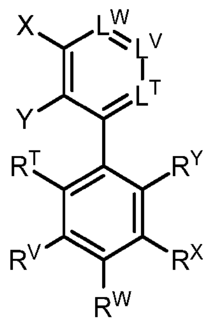

Formula I

- one second chemical moiety comprising or consisting of a structure of Formula II,

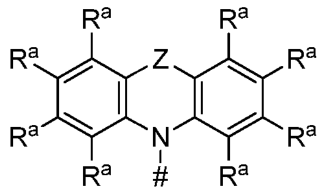

Formula II

wherein the first chemical moiety is linked to the second chemical moiety via a single bond, and

- one third chemical moiety comprising or consisting of a structure of Formula III,

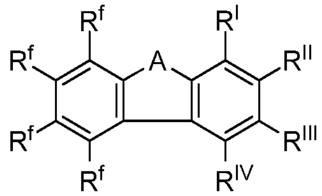

Formula III

wherein the first chemical moiety is linked to the third chemical moiety via a single bond.
$L^T$ is N or C-T.
$L^V$ is N or C-V.
$L^W$ is N or C-W.
T is the binding site of a single bond linking the first chemical moiety to the third chemical moiety or is $R^1$.
V is the binding site of a single bond linking the first chemical moiety to the third chemical moiety or is $R^1$.
W is the binding site of a single bond linking the first chemical moiety to the third chemical moiety or is $R^1$.
X is the binding site of a single bond linking the first chemical moiety to the third chemical moiety or is $R^1$.
Y is the binding site of a single bond linking the first chemical moiety to the third chemical moiety or is $R^1$.
$R^T$ is selected from the group consisting of $R^A$ and $R^2$.
$R^V$ is selected from the group consisting of $R^A$ and $R^2$.
$R^W$ is the binding site of a single bond linking the first chemical moiety to the second chemical moiety or is selected from the group consisting of $R^A$ and $R^2$.
$R^X$ is selected from the group consisting of $R^A$ and $R^2$.
$R^Y$ is the binding site of a single bond linking the first chemical moiety to the second chemical moiety or is selected from the group consisting of $R^A$ and $R^2$.
# represents the binding site of a single bond linking the second chemical moiety to the first chemical moiety;
Z is at each occurrence independently from another selected from the group consisting of a direct bond, $CR^3R^4$, $C=CR^3R^4$, C=O, $C=NR^3$, $NR^3$, O, $SiR^3R^4$, S, S(O) and $S(O)_2$;
A is selected from the group consisting of O, S and N-Ph.
$R^I$, $R^{II}$, $R^{III}$, and $R^{IV}$ is at each occurrence independently from another selected from the group consisting of $ and $R^d$.
$ represents the binding site of a single bond linking the first chemical moiety to the third chemical moiety.
$R^d$ is at each occurrence independently from another selected from the group consisting of hydrogen, deuterium, $C_6$-$C_{18}$-aryl,
which is optionally substituted with one or more substituents independently from another selected from the group consisting of

$C_1$-$C_5$-alkyl,
wherein optionally one or more hydrogen atoms are independently from each other substituted by deuterium, CN, $CF_3$, or F;
and
$C_6$-$C_{18}$-aryl,
which is optionally substituted with one or more $C_1$-$C_5$-alkyl substituents.

$R^A$ comprises or consists a structure of Formula Tz:

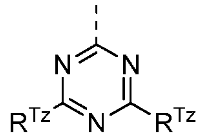

Formula Tz

wherein the dotted bond represents the binding site of $R^A$ to the single bond linking the first chemical moiety and $R^A$.

R' is at each occurrence independently from another selected from the group consisting of hydrogen, deuterium,
$C_1$-$C_5$-alkyl,
wherein one or more hydrogen atoms are optionally substituted by deuterium;
$C_2$-$C_8$-alkenyl,
wherein one or more hydrogen atoms are optionally substituted by deuterium;
$C_2$-$C_8$-alkynyl,
wherein one or more hydrogen atoms are optionally substituted by deuterium; and
$C_6$-$C_{18}$-aryl,
which is optionally substituted with one or more substituents $R^6$.

$R^2$ is at each occurrence independently from another selected from the group consisting of hydrogen, deuterium,
$C_1$-$C_5$-alkyl,
wherein one or more hydrogen atoms are optionally substituted by deuterium;
$C_2$-$C_8$-alkenyl,
wherein one or more hydrogen atoms are optionally substituted by deuterium;
$C_2$-$C_8$-alkynyl,
wherein one or more hydrogen atoms are optionally substituted by deuterium; and
$C_6$-$C_{18}$-aryl,
which is optionally substituted with one or more substituents $R^6$.

$R^{Tz}$ is at each occurrence independently from another selected from the group consisting of: hydrogen, deuterium,
$C_1$-$C_5$-alkyl,
wherein one or more hydrogen atoms are optionally substituted by deuterium;
$C_6$-$C_{18}$-aryl,
which is optionally substituted with one or more substituents $R^6$, and
$C_3$-$C_{17}$-heteroaryl,
which is optionally substituted with one or more substituents $R^6$.

$R^a$, $R^3$ and $R^4$ is at each occurrence independently from another selected from the group consisting of hydrogen, deuterium, $N(R^5)_2$, $OR^5$, $Si(R^5)_3$, $B(OR^5)_2$, $OSO_2R^5$, $CF_3$, CN, F, Br, I,
$C_1$-$C_{40}$-alkyl,

which is optionally substituted with one or more substituents $R^5$ and
wherein one or more non-adjacent $CH_2$-groups are optionally substituted by $R^5C=CR^5$, C≡C, $Si(R^5)_2$, $Ge(R^5)_2$, $Sn(R^5)_2$, C=O, C=S, C=Se, $C=NR^5$, $P(=O)(R^5)$, SO, $SO_2$, $NR^5$, O, S or $CONR^5$;

$C_1$-$C_{40}$-alkoxy,

which is optionally substituted with one or more substituents $R^5$ and
wherein one or more non-adjacent $CH_2$-groups are optionally substituted by $R^5C=CR^5$, C≡C, $Si(R^5)_2$, $Ge(R^5)_2$, $Sn(R^5)_2$, C=O, C=S, C=Se, $C=NR^5$, $P(=O)(R^5)$, SO, $SO_2$, $NR^5$, O, S or $CONR^5$;

$C_1$-$C_{40}$-thioalkoxy,

which is optionally substituted with one or more substituents $R^5$ and
wherein one or more non-adjacent $CH_2$-groups are optionally substituted by $R^5C=CR^5$, C≡C, $Si(R^5)_2$, $Ge(R^5)_2$, $Sn(R^5)_2$, C=O, C=S, C=Se, $C=NR^5$, $P(=O)(R^5)$, SO, $SO_2$, $NR^5$, O, S or $CONR^5$;

$C_2$-$C_{40}$-alkenyl,

which is optionally substituted with one or more substituents $R^5$ and wherein one or more non-adjacent $CH_2$-groups are optionally substituted by $R^5C=CR^5$, $C\equiv C$, $Si(R^5)_2$, $Ge(R^5)_2$, $Sn(R^5)_2$, C=O, C=S, C=Se, $C=NR^5$, $P(=O)(R^5)$, SO, $SO_2$, $NR^5$, O, S or $CONR^5$;

$C_2$-$C_{40}$-alkynyl,

which is optionally substituted with one or more substituents $R^5$ and wherein one or more non-adjacent $CH_2$-groups are optionally substituted by $R^5C=CR^5$, $C\equiv C$, $Si(R^5)_2$, $Ge(R^5)_2$, $Sn(R^5)_2$, C=O, C=S, C=Se, $C=NR^5$, $P(=O)(R^5)$, SO, $SO_2$, $NR^5$, O, S or $CONR^5$;

$C_6$-$C_{60}$-aryl,
which is optionally substituted with one or more substituents $R^5$; and
$C_3$-$C_{57}$-heteroaryl,
which is optionally substituted with one or more substituents $R^5$.
$R^5$ is at each occurrence independently from another selected from the group consisting of hydrogen, deuterium, $N(R^6)_2$, $OR^6$, $Si(R^6)_3$, $B(OR^6)_2$, $OSO_2R^6$, $CF_3$, CN, F, Br, I,
$C_1$-$C_{40}$-alkyl,

which is optionally substituted with one or more substituents $R^6$ and wherein one or more non-adjacent $CH_2$-groups are optionally substituted by $R^6C=CR^6$, $C\equiv C$, $Si(R^6)_2$, $Ge(R^6)_2$, $Sn(R^6)_2$, C=O, C=S, C=Se, $C=NR^6$, $P(=O)(R^6)$, SO, $SO_2$, $NR^6$, O, S or $CONR^6$;

$C_1$-$C_{40}$-alkoxy,

which is optionally substituted with one or more substituents $R^6$ and wherein one or more non-adjacent $CH_2$-groups are optionally substituted by $R^6C=CR^6$, $C\equiv C$, $Si(R^6)_2$, $Ge(R^6)_2$, $Sn(R^6)_2$, C=O, C=S, C=Se, $C=NR^6$, $P(=O)(R^6)$, SO, $SO_2$, $NR^6$, O, S or $CONR^6$;

$C_1$-$C_{40}$-thioalkoxy,

which is optionally substituted with one or more substituents $R^6$ and wherein one or more non-adjacent $CH_2$-groups are optionally substituted by $R^6C=CR^6$, $C\equiv C$, $Si(R^6)_2$, $Ge(R^6)_2$, $Sn(R^6)_2$, C=O, C=S, C=Se, $C=NR^6$, $P(=O)(R^6)$, SO, $SO_2$, $NR^6$, O, S or $CONR^6$;

$C_2$-$C_{40}$-alkenyl,

which is optionally substituted with one or more substituents $R^6$ and wherein one or more non-adjacent $CH_2$-groups are optionally substituted by $R^6C=CR^6$, $C\equiv C$, $Si(R^6)_2$, $Ge(R^6)_2$, $Sn(R^6)_2$, C=O, C=S, C=Se, $C=NR^6$, $P(=O)(R^6)$, SO, $SO_2$, $NR^6$, O, S or $CONR^6$;

$C_2$-$C_{40}$-alkynyl,

which is optionally substituted with one or more substituents $R^6$ and wherein one or more non-adjacent $CH_2$-groups are optionally substituted by $R^6C=CR^6$, $C\equiv C$, $Si(R^6)_2$, $Ge(R^6)_2$, $Sn(R^6)_2$, C=O, C=S, C=Se, $C=NR^6$, $P(=O)(R^6)$, SO, $SO_2$, $NR^6$, O, S or $CONR^6$;

$C_6$-$C_{60}$-aryl,
which is optionally substituted with one or more substituents $R^6$; and
$C_3$-$C_{57}$-heteroaryl,
which is optionally substituted with one or more substituents $R^6$.
$R^f$ is at each occurrence independently from another selected from the group consisting of hydrogen, deuterium,

$N(R^{5f})_2$,

$OR^{5f}$,

$Si(R^{5f})_3$,

$B(OR^{5f})_2$,

$OSO_2R^{5f}$,

$CF_3$,

CN,

F,

Br,

I,

$C_1$-$C_{40}$-alkyl,

which is optionally substituted with one or more substituents $R^{5f}$ and wherein one or more non-adjacent $CH_2$-groups are optionally substituted by $R^{5f}C=CR^{5f}$, C≡C, $Si(R^{5f})_2$, $Ge(R^{5f})_2$, $Sn(R^{5f})_2$, C=O, C=S, C=Se, $C=NR^{5f}$, $P(=O)(R^{5f})$, SO, $SO_2$, $NR^{5f}$, O, S or $CONR^{5f}$;

$C_1$-$C_{40}$-alkoxy,

which is optionally substituted with one or more substituents $R^{5f}$ and wherein one or more non-adjacent $CH_2$-groups are optionally substituted by $R^{5f}C=CR^{5f}$, C≡C, $Si(R^{5f})_2$, $Ge(R^{5f})_2$, $Sn(R^{5f})_2$, C=O, C=S, C=Se, $C=NR^{5f}$, $P(=O)(R^{5f})$, SO, $SO_2$, $NR^{5f}$, O, S or $CONR^{5f}$;

$C_1$-$C_{40}$-thioalkoxy,

which is optionally substituted with one or more substituents $R^{5f}$ and wherein one or more non-adjacent $CH_2$-groups are optionally substituted by $R^{5f}C=CR^{5f}$, C≡C, $Si(R^{5f})_2$, $Ge(R^{5f})_2$, $Sn(R^{5f})_2$, C=O, C=S, C=Se, $C=NR^{5f}$, $P(=O)(R^{5f})$, SO, $SO_2$, $NR^{5f}$, O, S or $CONR^{5f}$;

$C_2$-$C_{40}$-alkenyl,

which is optionally substituted with one or more substituents $R^{5f}$ and wherein one or more non-adjacent $CH_2$-groups are optionally substituted by $R^{5f}C=CR^{5f}$, C≡C, $Si(R^{5f})_2$, $Ge(R^{5f})_2$, $Sn(R^{5f})_2$, C=O, C=S, C=Se, $C=NR^{5f}$, $P(=O)(R^{5f})$, SO, $SO_2$, $NR^{5f}$, O, S or $CONR^{5f}$;

$C_2$-$C_{40}$-alkynyl,

which is optionally substituted with one or more substituents $R^{5f}$ and wherein one or more non-adjacent $CH_2$-groups are optionally substituted by $R^{5f}C=CR^{5f}$, C≡C, $Si(R^{5f})_2$, $Ge(R^{5f})_2$, $Sn(R^{5f})_2$, C=O, C=S, C=Se, $C=NR^{5f}$, $P(=O)(R^{5f})$, SO, $SO_2$, $NR^{5f}$, O, S or $CONR^{5f}$;

$C_6$-$C_{60}$-aryl,
which is optionally substituted with one or more substituents $R^{5f}$; and
$C_3$-$C_{57}$-heteroaryl,
which is optionally substituted with one or more substituents $R^{5f}$.
$R^{5f}$ is at each occurrence independently from another selected from the group consisting of hydrogen, deuterium,

$N(R^6)_2$,

$OR^6$,

$Si(R^6)_3$,

$B(OR^6)_2$,

$OSO_2R^6$,

$CF_3$,

CN,

F,

Br,

I,

$C_1$-$C_{40}$-alkyl,

which is optionally substituted with one or more substituents $R^6$ and
wherein one or more non-adjacent $CH_2$-groups are optionally substituted by $R^6C=CR^6$, C≡C, $Si(R^6)_2$, $Ge(R^6)_2$, $Sn(R^6)_2$, C=O, C=S, C=Se, $C=NR^6$, $P(=O)(R^6)$, SO, $SO_2$, $NR^6$, O, S or $CONR^6$;

$C_1$-$C_{40}$-alkoxy,

which is optionally substituted with one or more substituents $R^6$ and
wherein one or more non-adjacent $CH_2$-groups are optionally substituted by $R^6C=CR^6$, C≡C, $Si(R^6)_2$, $Ge(R^6)_2$, $Sn(R^6)_2$, C=O, C=S, C=Se, $C=NR^6$, $P(=O)(R^6)$, SO, $SO_2$, $NR^6$, O, S or $CONR^6$;

$C_1$-$C_{40}$-thioalkoxy,

which is optionally substituted with one or more substituents $R^6$ and
wherein one or more non-adjacent $CH_2$-groups are optionally substituted by $R^6C=CR^6$, C≡C, $Si(R^6)_2$, $Ge(R^6)_2$, $Sn(R^6)_2$, C=O, C=S, C=Se, $C=NR^6$, $P(=O)(R^6)$, SO, $SO_2$, $NR^6$, O, S or $CONR^6$;

$C_2$-$C_{40}$-alkenyl,

which is optionally substituted with one or more substituents $R^6$ and
wherein one or more non-adjacent $CH_2$-groups are optionally substituted by $R^6C=CR^6$, C≡C, $Si(R^6)_2$, $Ge(R^6)_2$, $Sn(R^6)_2$, C=O, C=S, C=Se, $C=NR^6$, $P(=O)(R^6)$, SO, $SO_2$, $NR^6$, O, S or $CONR^6$;

$C_2$-$C_{40}$-alkynyl,

which is optionally substituted with one or more substituents $R^6$ and
wherein one or more non-adjacent $CH_2$-groups are optionally substituted by $R^6C=CR^6$, C≡C, $Si(R^6)_2$, $Ge(R^6)_2$, $Sn(R^6)_2$, C=O, C=S, C=Se, $C=NR^6$, $P(=O)(R^6)$, SO, $SO_2$, $NR^6$, O, S or $CONR^6$;

$C_6$-$C_{60}$-aryl,

which is optionally substituted with one or more substituents $R^6$; and $C_3$-$C_{57}$-heteroaryl,
which is optionally substituted with one or more substituents $R^6$.

$R^6$ is at each occurrence independently from another selected from the group consisting of hydrogen, deuterium, OPh, $CF_3$, CN, F,
$C_1$-$C_5$-alkyl,
wherein optionally one or more hydrogen atoms are independently from each other substituted by deuterium, CN, $CF_3$, or F;
$C_1$-$C_5$-alkoxy,
wherein optionally one or more hydrogen atoms are independently from each other substituted by deuterium, CN,

$CF_3$, or F;
$C_1$-$C_5$-thioalkoxy,
wherein optionally one or more hydrogen atoms are independently from each other substituted by deuterium, CN, $CF_3$, or F;
$C_2$-$C_5$-alkenyl,
wherein optionally one or more hydrogen atoms are independently from each other substituted by deuterium, CN, $CF_3$, or F;
$C_2$-$C_5$-alkynyl,
wherein optionally one or more hydrogen atoms are independently from each other substituted by deuterium, CN, $CF_3$, or F;
$C_6$-$C_{18}$-aryl,

which is optionally substituted with one or more $C_1$-$C_5$-alkyl substituents; $C_3$-$C_{17}$-heteroaryl,
which is optionally substituted with one or more $C_1$-$C_5$-alkyl substituents; N($C_6$-$C_{18}$-aryl)$_2$,

N($C_3$-$C_{17}$-heteroaryl)$_2$; and
N($C_3$-$C_{17}$-heteroaryl)($C_6$-$C_{18}$-aryl).

The substituents $R^a$, $R^3$, $R^4$ or $R^5$ independently from each other can optionally form a mono- or polycyclic, aliphatic, aromatic and/or benzo-fused ring system with one or more substituents $R^a$, $R^3$, $R^4$ or $R^5$.

Optionally, one or more of the substituents $R^f$ or $R^{5f}$ independently from each other form a mono- or polycyclic, aliphatic, aromatic and/or benzo-fused ring system with one or more other substituents $R^f$ or $R^{5f}$.

According to the invention, exactly one (one and only one) ring member (atom or group) selected from the group consisting of $L^T$, $L^V$ and $L^W$ is N;

exactly one substituent selected from the group consisting of $R^T$, $R^V$, $R^W$, $R^X$ and $R^Y$ is $R^A$; exactly one substituent selected from the group consisting of T, V, W, X and Y is the binding site of a single bond linking the first chemical moiety to the third chemical moiety; and
exactly one substituent selected from the group consisting of $R^W$ and $R^Y$ represents the binding site of a single bond linking the first chemical moiety and the second chemical moiety.

In one embodiment of the invention, first chemical moiety comprises or consists of a structure of Formula Ia:

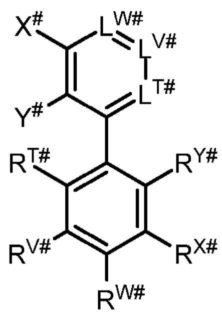

Formula Ia

wherein $R^1$, $R^2$, $R^I$ and $R^A$ are defined as above;
$L^{T\#}$ is N or C-$R^1$;
$L^{V\#}$ is N or C- $R^1$,
$L^{W\#}$ is N or C-$W^\#$;
$W^\#$ is the binding site of a single bond linking the first chemical moiety to the third chemical moiety or is $R^1$;
$X^\#$ is the binding site of a single bond linking the first chemical moiety to the third chemical moiety or is $R^1$;
$Y^\#$ is the binding site of a single bond linking the first chemical moiety to the third chemical moiety or is $R^1$;
$R^{T\#}$ is selected from the group consisting of $R^A$ and $R^2$;
$R^{V\#}$ is selected from the group consisting of $R^A$ and $R^2$;
$R^{W\#}$ is the binding site of a single bond linking the first chemical moiety to the second chemical moiety or is selected from the group consisting of $R^A$ and $R^2$;

$R^{X\#}$ is $R^2$;
$R^{Y\#}$ is the binding site of a single bond linking the first chemical moiety to the second chemical moiety or is $R^2$;
wherein
exactly one ring member (atom or group) selected from the group consisting of $L^{T\#}$, $L^{V\#}$ and $L^{W\#}$ is N;
exactly one substituent selected from the group consisting of $R^{T\#}$, $R^{V\#}$ and $R^{W\#}$ is $R^A$, exactly one substituent selected from the group consisting of $W^\#$, $X^\#$ and $Y^\#$ is the binding site of a single bond linking the first chemical moiety to the third chemical moiety
and exactly one substituent selected from the group consisting of $R^{W\#}$ and $R^{Y\#}$ represents the binding site of a single bond linking the first chemical moiety and the second chemical moiety.

In one embodiment of the invention, first chemical moiety comprises or consists of a structure of Formula Iaa:

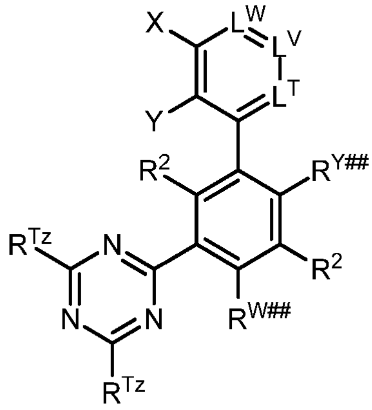

Formula Iaa

wherein $L^T$, $L^V$, $L^W$, X, Y, $R^1$, $R^2$ and $R^{Tz}$ are defined as above;
$R^{Y\#\#}$ is the binding site of a single bond linking the first chemical moiety to the second chemical moiety or is $R^2$;
$R^{W\#\#}$ is the binding site of a single bond linking the first chemical moiety to the second chemical moiety or is $R^2$;
wherein
exactly one ring member (atom or group) selected from the group consisting of $L^T$, $L^Y$ and $L^W$ is N;
exactly one substituent selected from the group consisting of T, V, W, X and Y is the binding site of a single bond linking the first chemical moiety to the third chemical moiety;
exactly one substituent selected from the group consisting of $R^{W\#\#}$ and $R^{Y\#\#}$ represents the binding site of a single bond linking the first chemical moiety and the second chemical moiety.

In one embodiment of the invention, first chemical moiety comprises or consists of a structure of Formula Iaaa:

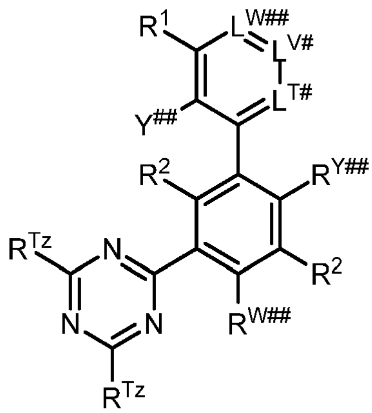

Formula Iaaa

wherein

$L^{T\#}$ is N or C-$R^1$;
$L^{V\#}$ is N or C- $R^1$,
$L^{W\#\#}$ is N or C- $R^1$;
$R^1$, $R^2$ and $R^{Tz}$ are defined as above;
$R^{Y\#\#}$ is the binding site of a single bond linking the first chemical moiety to the second chemical moiety or is $R^2$;
$R^{W\#\#}$ is the binding site of a single bond linking the first chemical moiety to the second chemical moiety or is $R^2$;
$Y^{\#\#}$ is the binding site of a single bond linking the first chemical moiety to the third chemical moiety;
wherein
exactly one ring member (atom or group) selected from the group consisting of $L^{T\#}$, $L^{V\#}$ and $L^{W\#\#}$ is N;
exactly one substituent selected from the group consisting of $R^{W\#\#}$ and $R^{Y\#\#}$ represents the binding site of a single bond linking the first chemical moiety and the second chemical moiety.

In one embodiment of the invention, first chemical moiety comprises or consists of a structure of Formula Iaaaa:

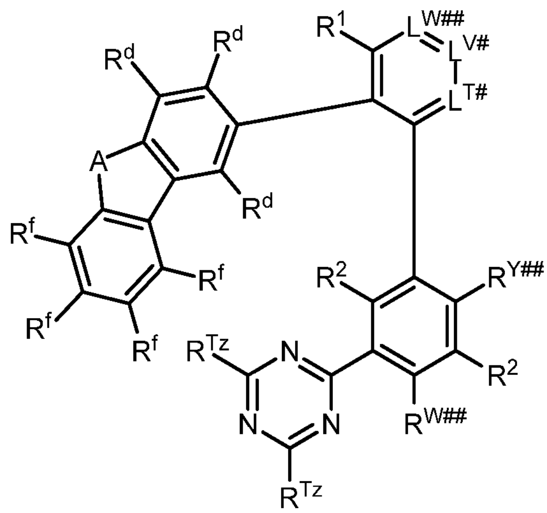

Formula Iaaaa

wherein

$L^{T\#}$ is N or C-$R^1$;
$L^{V\#}$ is N or C- $R^1$,
$L^{W\#\#}$ is N or C- $R^1$;
A, $R^d$, $R^f$, $R^1$, $R^2$ and $R^{Tz}$ are defined as above;
$R^{Y\#\#}$ is the binding site of a single bond linking the first chemical moiety to the second chemical moiety or is $R^2$;
$R^{W\#\#}$ is the binding site of a single bond linking the first chemical moiety to the second chemical moiety or is $R^2$;
$Y^{\#\#}$ is the binding site of a single bond linking the first chemical moiety to the third chemical moiety;
wherein
exactly one ring member (atom or group) selected from the group consisting of $L^{T\#}$, $L^{V\#}$ and $L^{W\#\#}$ is N;
exactly one substituent selected from the group consisting of $R^{W\#\#}$ and $R^{Y\#\#}$ represents the binding site of a single bond linking the first chemical moiety and the second chemical moiety;

In one embodiment, $R^1$ and $R^2$ is at each occurrence independently from another selected from the group consisting of hydrogen (H), methyl, mesityl, tolyl and phenyl. The term tolyl comprises 2-tolyl, 3-tolyl and 4-tolyl.
In one embodiment, $R^1$ and $R^2$ is at each occurrence independently from another selected from the group consisting of hydrogen (H), methyl, and phenyl.
In one embodiment, $R^1$ and $R^2$ is at each occurrence hydrogen (H).
In one embodiment, $R^V$ is $R^A$.
In one embodiment, $R^W$ is $R^A$.
In one embodiment, $R^T$ is $R^A$.
In one embodiment, $R^X$ is $R^A$.
In one embodiment, $R^Y$ is $R^A$.
In one embodiment, $L^V$ is N.

In one embodiment, $L^W$ is N.
In one embodiment, $L^T$ is N.
In one embodiment, $R^V$ is $R^A$, and $L^V$ is N.
In one embodiment, $R^V$ is $R^A$, and $L^W$ is N.
In one embodiment, $R^V$ is $R^A$, and $L^T$ is N.
In one embodiment, $R^W$ is $R^A$, and $L^W$ is N.
In one embodiment, $R^W$ is $R^A$, and $L^T$ is N.
In one embodiment, $R^W$ is $R^A$, and $L^V$ is N.
In one embodiment, $R^T$ is $R^A$, and $L^W$ is N.
In one embodiment, $R^T$ is $R^A$, and $L^T$ is N.
In one embodiment, $R^T$ is $R^A$, and $L^V$ is N.
In a further embodiment of the invention $R^{Tz}$ is independently from each other selected from the group consisting of H, methyl,

phenyl, which is optionally substituted with one or more substituents $R^6$,
1,3,5-triazinyl, which is optionally substituted with one or more substituents $R^6$, and
pyrimidinyl, which is optionally substituted with one or more substituents $R^6$.

In a further embodiment of the invention $R^{Tz}$ is independently from each other selected from the group consisting of H, methyl and phenyl.
In a further embodiment of the invention $R^{Tz}$ is phenyl at each occurrence.
In one embodiment, $R^f$ is H.
In one embodiment, $R^d$ is H.
In one embodiment, exactly one substituent selected from the group consisting of $R^I$ and $R^{III}$ is the binding site of a single bond linking the first chemical moiety to the third chemical moiety (represented by $).
In one embodiment, $R^{III}$ is the binding site of a single bond linking the first chemical moiety to the third chemical moiety (represented by $).
In one embodiment, $R^I$ is the binding site of a single bond linking the first chemical moiety to the third chemical moiety (represented by $)
In a further embodiment of the invention, the second chemical moiety comprises or consists of a structure of Formula IIa:

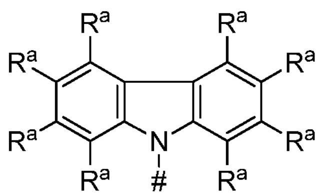

Formula IIa

wherein # and $R^a$ are defined as above.
In a further embodiment of the invention, $R^a$ is at each occurrence independently from another selected from the group consisting of H,

Me,
$^i$Pr,
$^t$Bu,
CN,
$CF_3$,
Ph, which is optionally substituted with one or more substituents independently from each other selected from the group consisting of Me, $^i$Pr, $^t$Bu, CN, $CF_3$, and Ph,
pyridinyl, which is optionally substituted with one or more substituents independently from each other selected from the group consisting of Me, $^i$Pr, $^t$Bu, CN, $CF_3$, and Ph,
pyrimidinyl, which is optionally substituted with one or more substituents independently from each other selected from the group consisting of Me, $^i$Pr, $^t$Bu, CN, $CF_3$, and Ph,
carbazolyl, which is optionally substituted with one or more substituents independently from each other selected from the group consisting of Me, $^i$Pr, $^t$Bu, CN, $CF_3$, and Ph,

triazinyl, which is optionally substituted with one or more substituents independently from each other selected from the group consisting of Me, $^{i}$Pr, $^{t}$Bu, CN, $CF_3$, and Ph,
and $N(Ph)_2$.

In a further embodiment of the invention, $R^a$ is at each occurrence independently from another selected from the group consisting of H,

Me,
$^{i}$Pr,
$^{t}$Bu,
CN,
$CF_3$,
Ph, which is optionally substituted with one or more substituents independently from each other selected from the group consisting of Me, $^{i}$Pr, $^{t}$Bu, CN, $CF_3$, and Ph,
pyridinyl, which is optionally substituted with one or more substituents independently from each other selected from the group consisting of Me, $^{i}$Pr, $^{t}$Bu, CN, $CF_3$, and Ph,
pyrimidinyl, which is optionally substituted with one or more substituents independently from each other selected from the group consisting of Me, $^{i}$Pr, $^{t}$Bu, CN, $CF_3$, and Ph, and
triazinyl, which is optionally substituted with one or more substituents independently from each other selected from the group consisting of Me, $^{i}$Pr, $^{t}$Bu, CN, $CF_3$, and Ph.

In a further embodiment of the invention, $R^a$ is at each occurrence independently from another selected from the group consisting of H,

Me,
$^{t}$Bu,
Ph, which is optionally substituted with one or more substituents independently from each other selected from the group consisting of Me, $^{i}$Pr, $^{t}$Bu, CN, $CF_3$, and Ph,
triazinyl, which is optionally substituted with one or more substituents independently from each other selected from the group consisting of Me, $^{i}$Pr, $^{t}$Bu, CN, $CF_3$, and Ph.

In a further embodiment of the invention, $R^a$ is H at each occurrence

In a further embodiment of the invention, the second chemical moiety comprises or consists of a structure of Formula IIb, a structure of Formula IIb-2, a structure of Formula IIb-3 or a structure of Formula IIb-4:

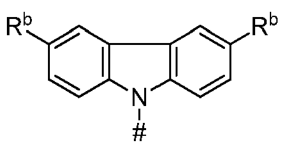
Formula IIb

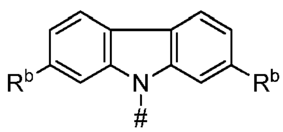
Formula IIb-2

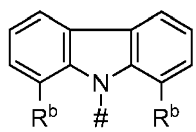
Formula IIb-3

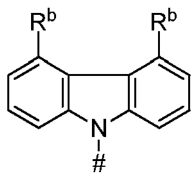
Formula IIb-4

wherein

$R^b$ is at each occurrence independently from another selected from the group consisting of deuterium,

$N(R^5)_2$,

$OR^5$,

$Si(R^5)_3$,

$B(OR^5)_2$,

$OSO_2R^5$,

$CF_3$,

CN,

F,

Br,

I,

$C_1$-$C_{40}$-alkyl,

which is optionally substituted with one or more substituents $R^5$ and
wherein one or more non-adjacent $CH_2$-groups are optionally substituted by $R^5C=CR^5$, C≡C, $Si(R^5)_2$, $Ge(R^5)_2$, $Sn(R^5)_2$, C=O, C=S, C=Se, $C=NR^5$, $P(=O)(R^5)$, SO, $SO_2$, $NR^5$, O, S or $CONR^5$;

$C_1$-$C_{40}$-alkoxy,

which is optionally substituted with one or more substituents $R^5$ and
wherein one or more non-adjacent $CH_2$-groups are optionally substituted by $R^5C=CR^5$, C≡C, $Si(R^5)_2$, $Ge(R^5)_2$, $Sn(R^5)_2$, C=O, C=S, C=Se, $C=NR^5$, $P(=O)(R^5)$, SO, $SO_2$, $NR^5$, O, S or $CONR^5$;

$C_1$-$C_{40}$-thioalkoxy,

which is optionally substituted with one or more substituents $R^5$ and
wherein one or more non-adjacent $CH_2$-groups are optionally substituted by $R^5C=CR^5$, C≡C, $Si(R^5)_2$, $Ge(R^5)_2$, $Sn(R^5)_2$, C=O, C=S, C=Se, $C=NR^5$, $P(=O)(R^5)$, SO, $SO_2$, $NR^5$, O, S or $CONR^5$;

$C_2$-$C_{40}$-alkenyl,

which is optionally substituted with one or more substituents $R^5$ and
wherein one or more non-adjacent $CH_2$-groups are optionally substituted by $R^5C=CR^5$, C≡C, $Si(R^5)_2$, $Ge(R^5)_2$, $Sn(R^5)_2$, C=O, C=S, C=Se, $C=NR^5$, $P(=O)(R^5)$, SO, $SO_2$, $NR^5$, O, S or $CONR^5$;

$C_2$-$C_{40}$-alkynyl,

which is optionally substituted with one or more substituents $R^5$ and
wherein one or more non-adjacent $CH_2$-groups are optionally substituted by $R^5C=CR^5$, C≡C, $Si(R^5)_2$, $Ge(R^5)_2$, $Sn(R^5)_2$, C=O, C=S, C=Se, $C=NR^5$, $P(=O)(R^5)$, SO, $SO_2$, $NR^5$, O, S or $CONR^5$;

$C_6$-$C_{60}$-aryl,
which is optionally substituted with one or more substituents $R^5$; and
$C_3$-$C_{57}$-heteroaryl,
which is optionally substituted with one or more substituents $R^5$.
Apart from that the aforementioned definitions apply.

In one additional embodiment of the invention, the second chemical moiety comprises or consists of a structure of Formula IIc, a structure of Formula IIc-2, a structure of Formula IIc-3 or a structure of Formula IIc-4:

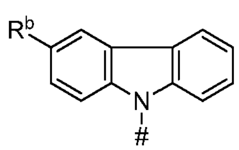

Formula IIc

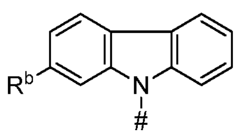

Formula IIc-2

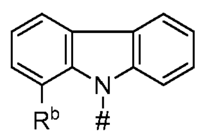

Formula IIc-3

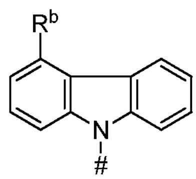

Formula IIc-4

wherein the aforementioned definitions apply.

In a further embodiment of the invention, $R^b$ is at each occurrence independently from another selected from the

group consisting of

Me,
$^{i}$Pr,
$^{t}$Bu,
CN,
$CF_3$,
Ph, which is optionally substituted with one or more substituents independently from each other selected from the group consisting of Me, $^{i}$Pr, $^{t}$Bu, CN, $CF_3$, and Ph,
pyridinyl, which is optionally substituted with one or more substituents independently from each other selected from the group consisting of Me, $^{i}$Pr, $^{t}$Bu, CN, $CF_3$, and Ph,
carbazolyl, which is optionally substituted with one or more substituents independently from each other selected from the group consisting of Me, $^{i}$Pr, $^{t}$Bu, CN, $CF_3$, and Ph,
triazinyl, which is optionally substituted with one or more substituents independently from each other selected from the group consisting of Me, $^{i}$Pr, $^{t}$Bu, CN, $CF_3$, and Ph,
and $N(Ph)_2$.

In a further embodiment of the invention, $R^b$ is at each occurrence independently from another selected from the group consisting of

Me,
$^{i}$Pr,
$^{t}$Bu,
CN,
$CF_3$,
Ph, which is optionally substituted with one or more substituents independently from each other selected from the group consisting of Me, $^{i}$Pr, $^{t}$Bu, CN, $CF_3$, and Ph,
pyridinyl, which is optionally substituted with one or more substituents independently from each other selected from the group consisting of Me, $^{i}$Pr, $^{t}$Bu, CN, $CF_3$, and Ph,
pyrimidinyl, which is optionally substituted with one or more substituents independently from each other selected from the group consisting of Me, $^{i}$Pr, $^{t}$Bu, CN, $CF_3$, and Ph, and
triazinyl, which is optionally substituted with one or more substituents independently from each other selected from the group consisting of Me, $^{i}$Pr, $^{t}$Bu, CN, $CF_3$, and Ph.

In a further embodiment of the invention, $R^b$ is at each occurrence independently from another selected from the group consisting of

Me,
$^{t}$Bu,
Ph, which is optionally substituted with one or more substituents independently from each other selected from the group consisting of Me, $^{i}$Pr, $^{t}$Bu, CN, $CF_3$, and Ph,
triazinyl, which is optionally substituted with one or more substituents independently from each other selected from the group consisting of Me, $^{i}$Pr, $^{t}$Bu, CN, $CF_3$, and Ph.

In the following, exemplary embodiments of the second chemical moiety are shown:

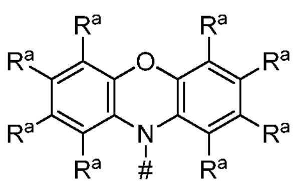 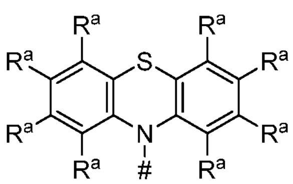 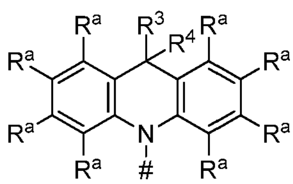 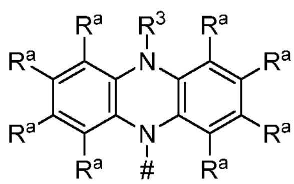

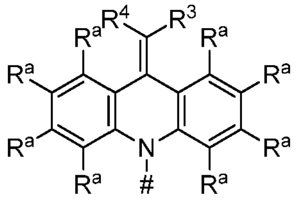
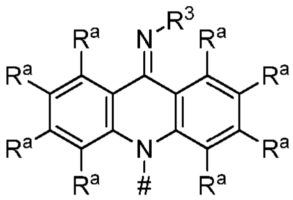
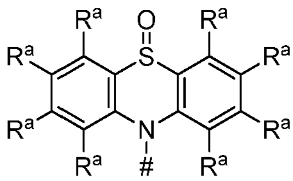

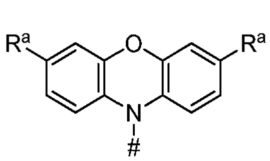
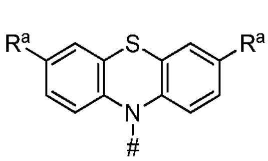
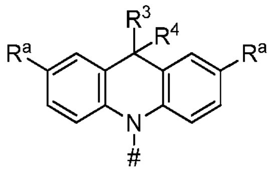
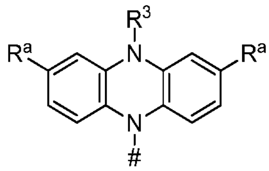

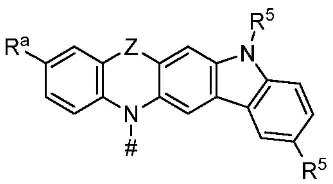
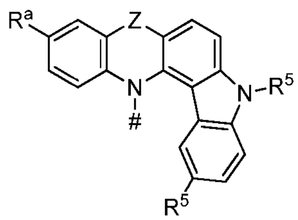
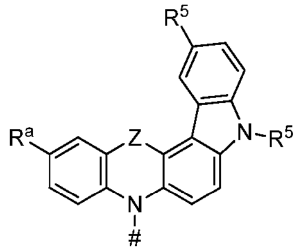

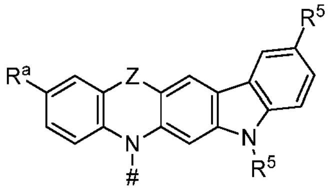
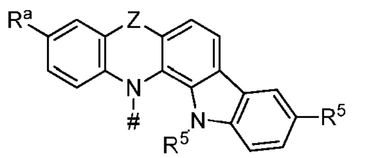
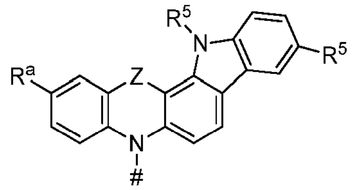

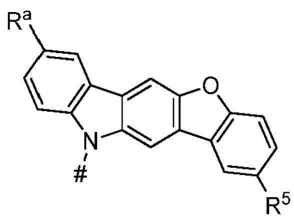
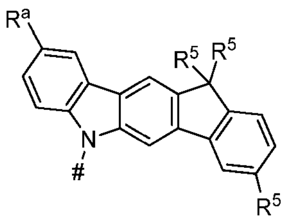
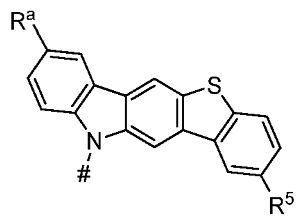

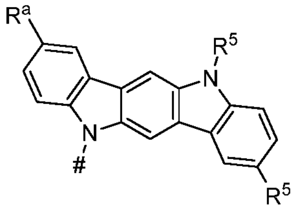

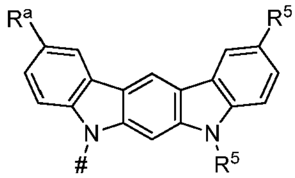
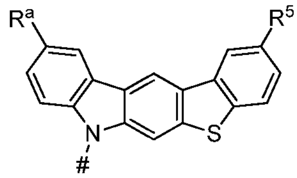
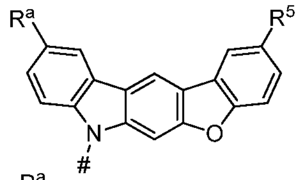
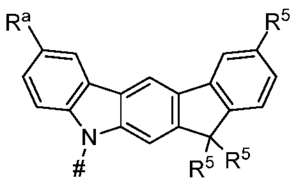
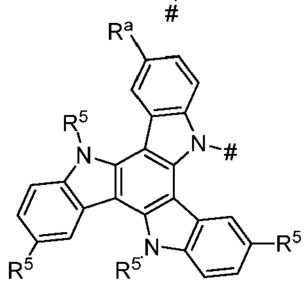
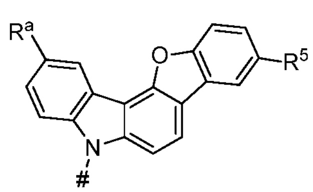
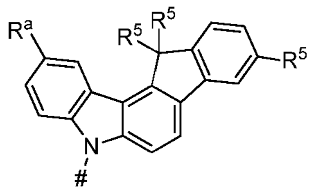
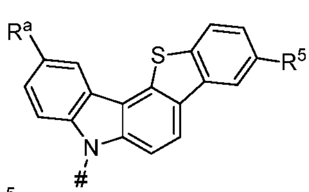
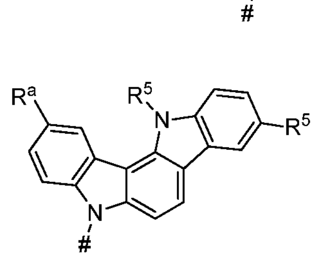
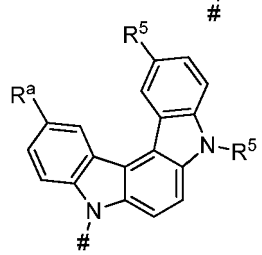
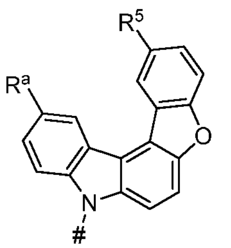
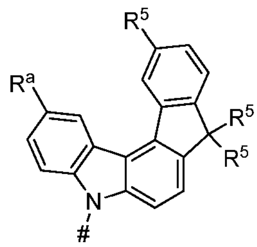
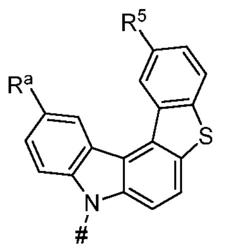
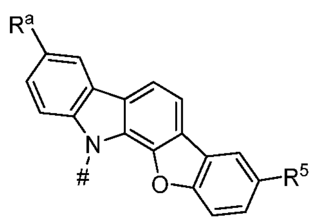
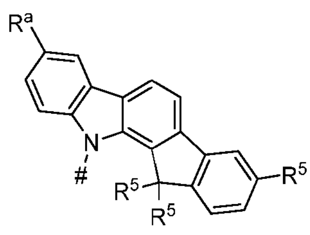
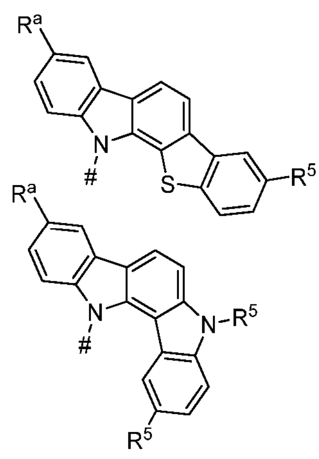
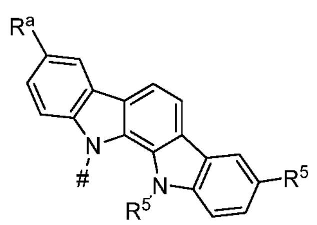

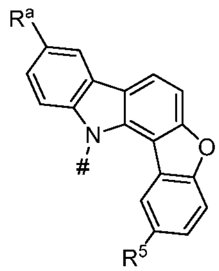

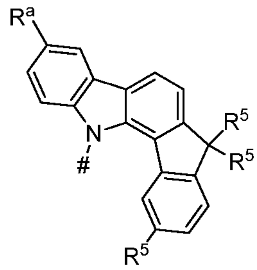

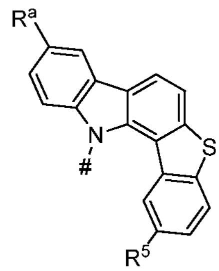

wherein for #, Z, $R^a$, $R^3$, $R^4$ and $R^5$ the aforementioned definitions apply.

In one embodiment, $R^a$ and $R^5$ is at each occurrence independently from another selected from the group consisting of hydrogen (H), methyl (Me), i-propyl ($CH(CH_3)_2$) ($^iPr$), t-butyl ($^tBu$), phenyl (Ph), CN, $CF_3$, and diphenylamine ($NPh_2$).

In one embodiment of the invention, the organic molecules comprise or consist of a structure of Formula IIIA:

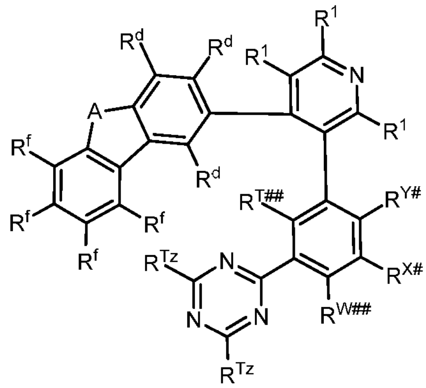

Formula IIIA

wherein

$R^{W\#\#}$ is the binding site of a single bond linking the first chemical moiety to the second chemical moiety or is $R^2$;
$R^{T\#\#}$ is the binding site of a single bond linking the first chemical moiety to the second chemical moiety or is $R^2$;
$R^{X\#}$ is $R^2$;
$R^{Y\#}$ is the binding site of a single bond linking the first chemical moiety to the second chemical moiety or is $R^2$;

and apart from that the aforementioned definitions apply.

In a further embodiment of the invention, the organic molecules comprise or consist of a structure selected from the group of Formula III-1, Formula III-2, and Formula III-4:

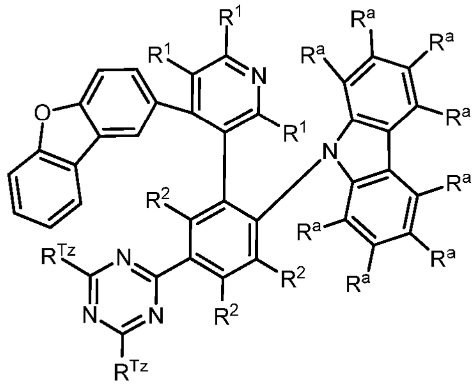

Formula III-1

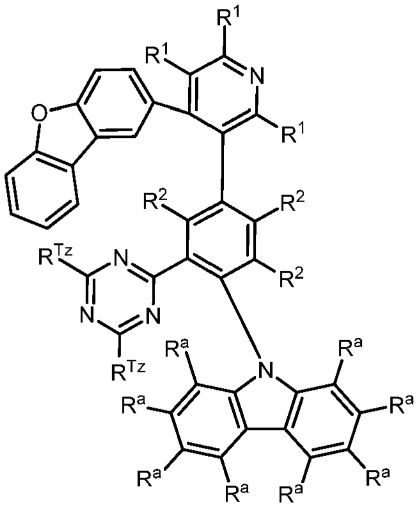

Formula III-2

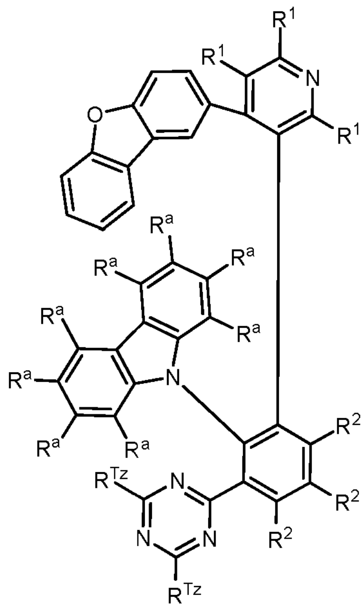

Formula III-4

wherein the aforementioned definitions apply.

In a preferred embodiment, the organic molecules comprise or consist of a structure selected from the group of Formula III-1 and Formula III-2.

In a further embodiment of the invention, the organic molecules comprise or consist of a structure selected from the group of Formula IIIa-1 and Formula IIIa-2:

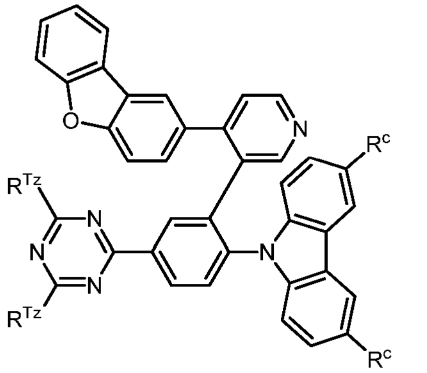

Formula IIIa-1

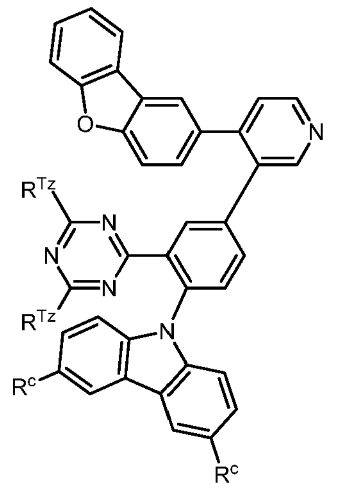

Formula IIIa-2

wherein

$R^c$ is at each occurrence independently from another selected from the group consisting of
Me,
$^iPr$,
$^tBu$,
Ph, which is optionally substituted with one or more substituents independently from each other selected from the group consisting of Me, $^iPr$, $^tBu$, CN, $CF_3$, and Ph,
pyridinyl, which is optionally substituted with one or more substituents independently from each other selected from the group consisting of Me, $^iPr$, $^tBu$, CN, $CF_3$, and Ph,
pyrimidinyl, which is optionally substituted with one or more substituents independently from each other selected from the group consisting of Me, $^iPr$, $^tBu$, CN, $CF_3$, and Ph,
carbazolyl, which is optionally substituted with one or more substituents independently from each other selected from the group consisting of Me, $^iPr$, $^tBu$, CN, $CF_3$, and Ph,
triazinyl, which is optionally substituted with one or more substituents independently from each other selected from the group consisting of Me, $^iPr$, $^tBu$, CN, $CF_3$, and Ph,
and $N(Ph)_2$.

In a further embodiment of the invention, the organic molecules comprise or consist of a structure selected from the group of Formula IIIb-1 and Formula IIIb-2:

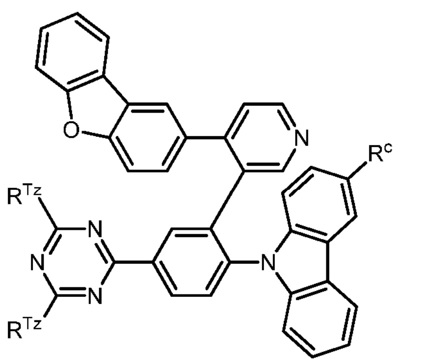

Formula IIIb-1

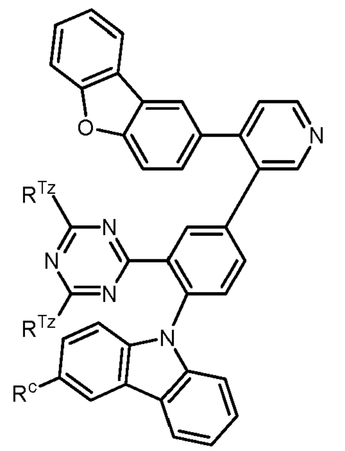

Formula IIIb-2

wherein the aforementioned definitions apply.

In one embodiment of the invention, the organic molecules comprise or consist of a structure of Formula IV:

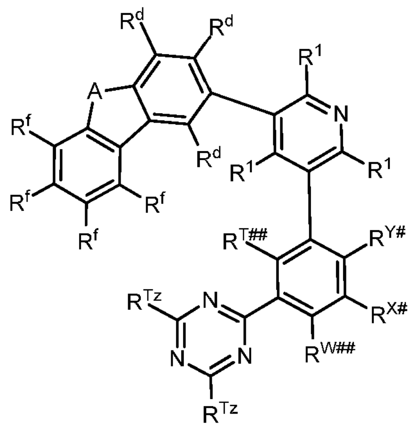

Formula IV

wherein the aforementioned definitions.

In a further embodiment of the invention, the organic molecules comprise or consist of a structure selected from the group of Formula IV-1, Formula IV-2, and Formula IV-4:

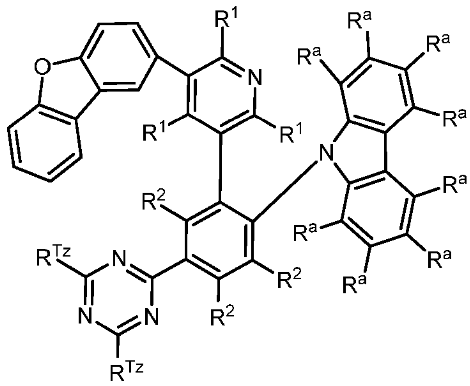

Formula IV-1

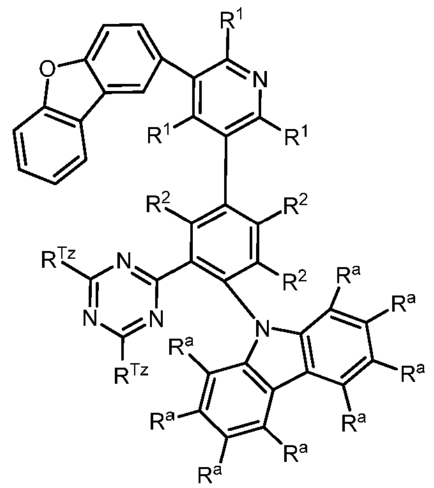

Formula IV-2

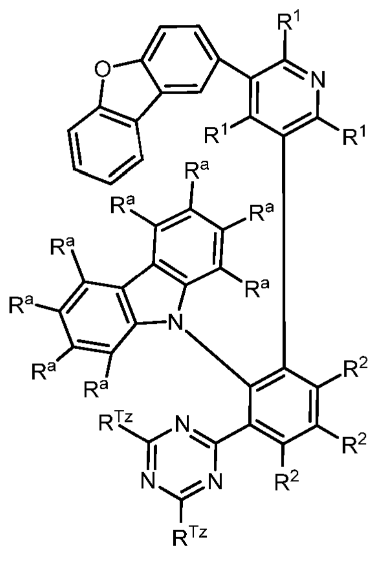

Formula IV-4

wherein the aforementioned definitions apply.

In a preferred embodiment, the organic molecules comprise or consist of a structure selected from the group of Formula IV-1 and Formula IV-2.

In a further embodiment of the invention, the organic molecules comprise or consist of a structure selected from the group of Formula IVa-1 and Formula IVa-2:

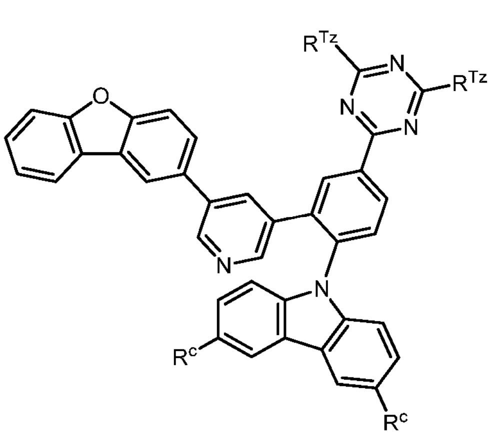

Formula IVa-1

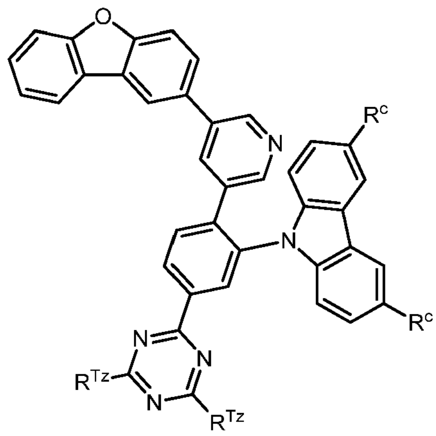

Formula IVa-2

wherein the aforementioned definitions apply.

In a further embodiment of the invention, the organic molecules comprise or consist of a structure selected from the group of Formula IVb-1 and Formula IVb-2:

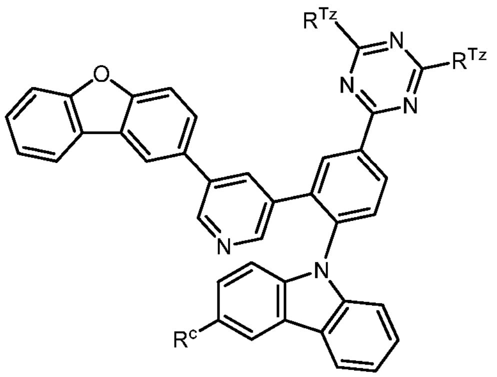

Formula IVb-1

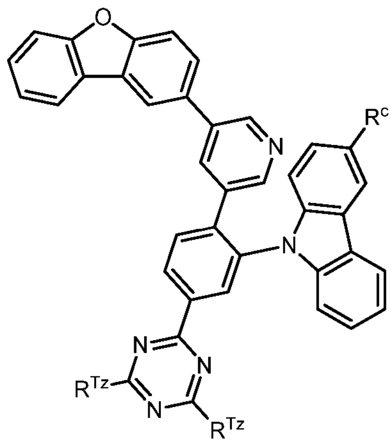

Formula IVb-2

wherein the aforementioned definitions apply.

In one embodiment of the invention, the organic molecules comprise or consist of a structure of Formula V:

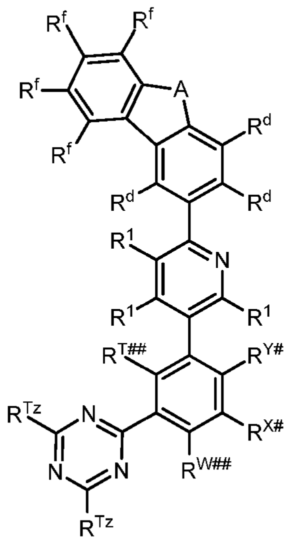

Formula V

wherein the aforementioned definitions apply.

In a further embodiment of the invention, the organic molecules comprise or consist of a structure selected from the group of Formula V-1, Formula V-2, and Formula V-4:

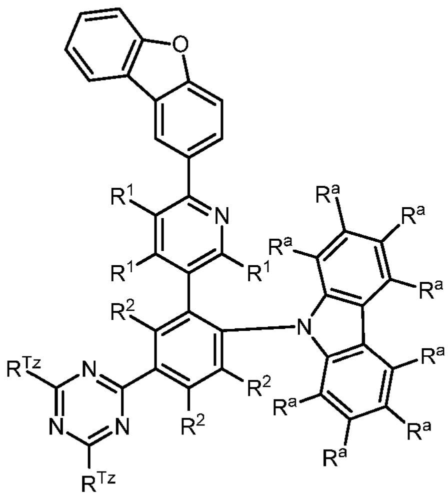

Formula V-1

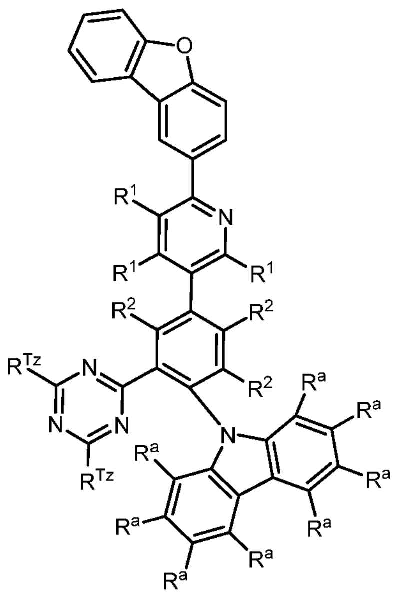

Formula V-2

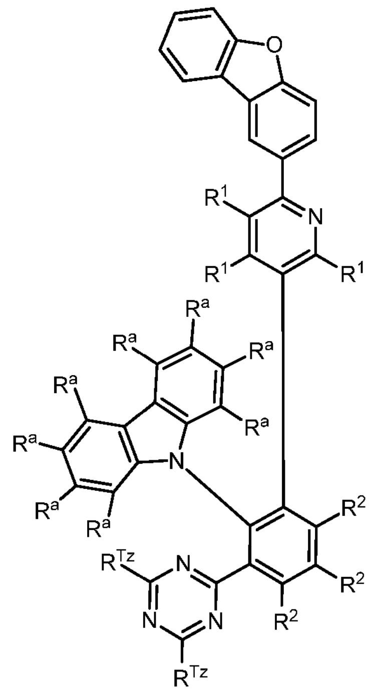

Formula V-4

wherein the aforementioned definitions apply.

In a preferred embodiment, the organic molecules comprise or consist of a structure selected from the group of Formula V-1 and Formula V-2.

In a further embodiment of the invention, the organic molecules comprise or consist of a structure selected from the group of Formula Va-1 and Formula Va-2:

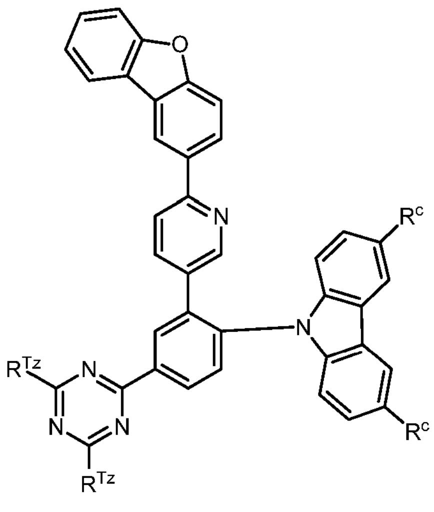

Formula Va-1

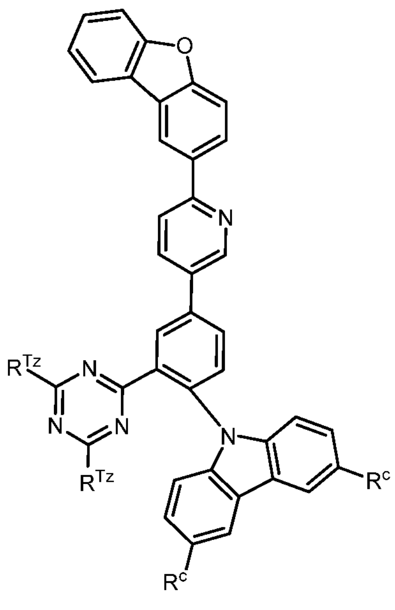

Formula Va-2

wherein the aforementioned definitions apply.

In a further embodiment of the invention, the organic molecules comprise or consist of a structure selected from the group of Formula Vb-1 and Formula Vb-2:

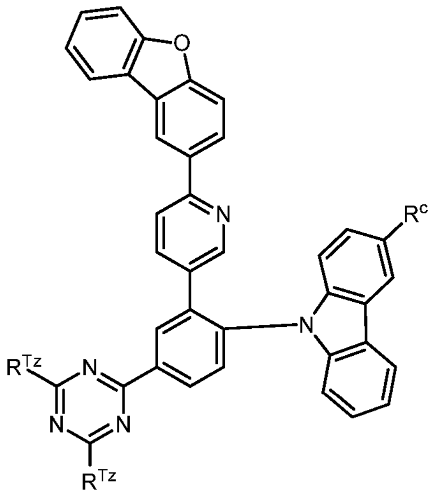

Formula Vb-1

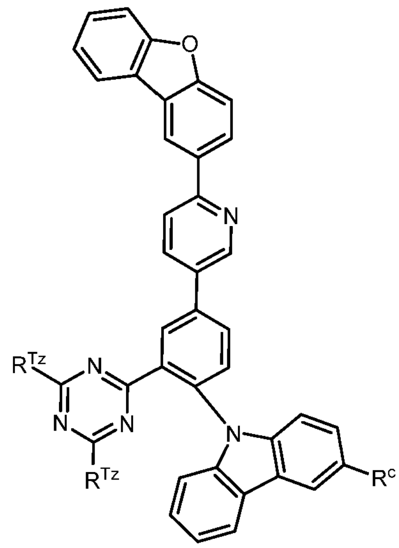

Formula Vb-2

wherein the aforementioned definitions apply.

In one embodiment of the invention, the organic molecules comprise or consist of a structure of Formula VI:

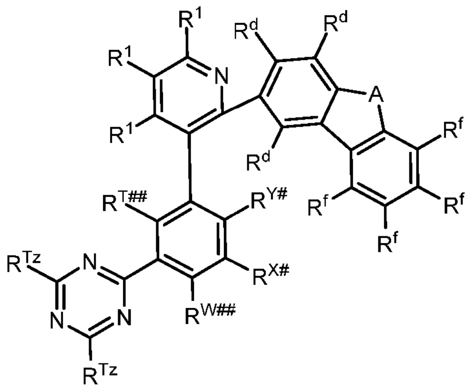

Formula VI

wherein the aforementioned definitions.

In a further embodiment of the invention, the organic molecules comprise or consist of a structure selected from the group of Formula VI-1, Formula VI-2, and Formula VI-4:

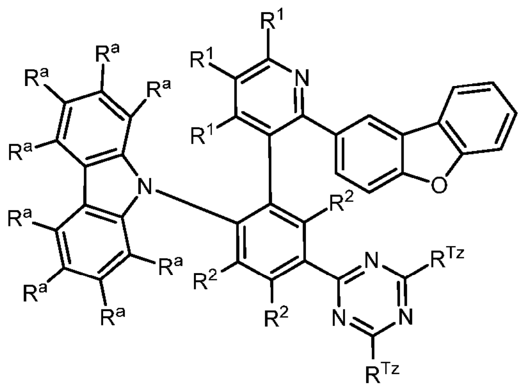

Formula VI-1

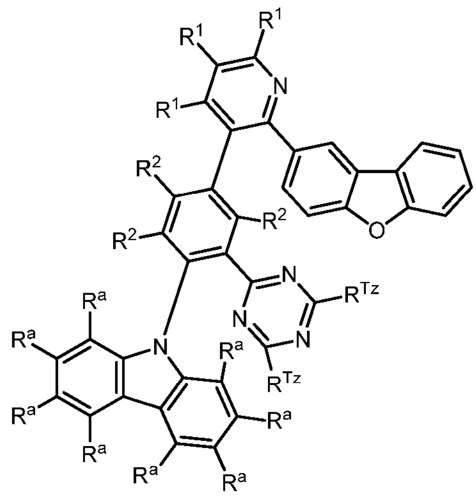

Formula VI-2

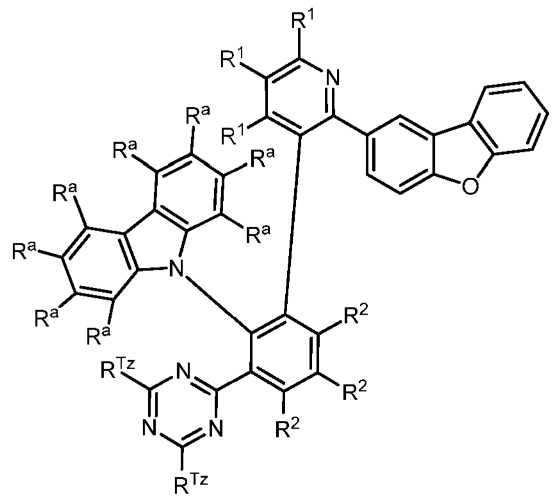

Formula VI-4

wherein the aforementioned definitions apply.

In a preferred embodiment, the organic molecules comprise or consist of a structure selected from the group of Formula VI-1 and Formula VI-2.

In a further embodiment of the invention, the organic molecules comprise or consist of a structure selected from the group of Formula VIa-1 and Formula VIa-2:

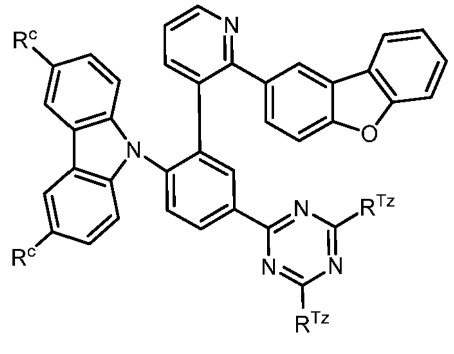

Formula VIa-1

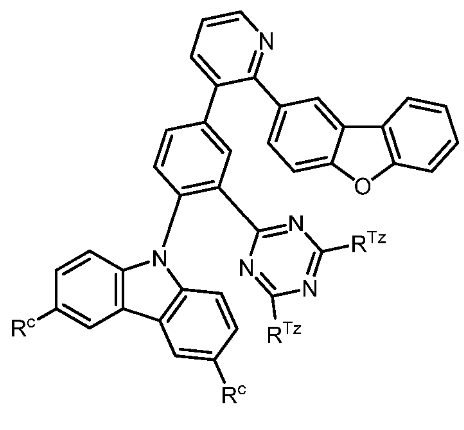

Formula VIa-2

wherein the aforementioned definitions apply.

In a further embodiment of the invention, the organic molecules comprise or consist of a structure selected from the group of Formula VIb-1 and Formula VIb-2:

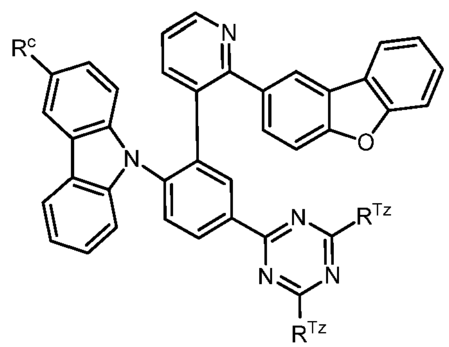

Formula VIb-1

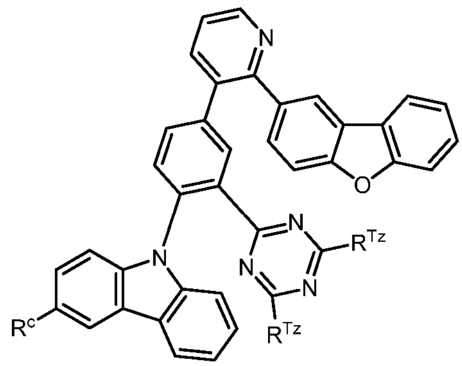

Formula VIb-2

wherein the aforementioned definitions apply.

In one embodiment of the invention, the organic molecules comprise or consist of a structure of Formula VII:

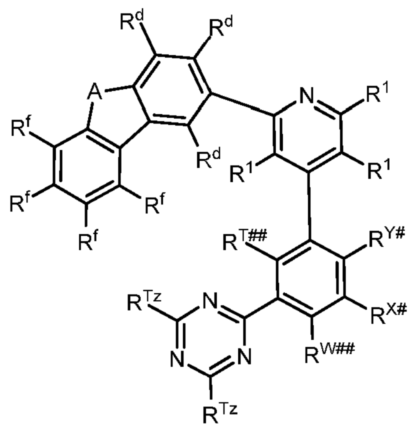

Formula VII

wherein the aforementioned definitions apply.

In a further embodiment of the invention, the organic molecules comprise or consist of a structure selected from the group of Formula VII-1, Formula VII-2, and Formula VII-4:

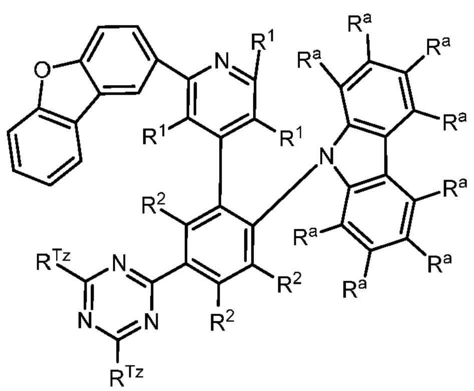

Formula VII-1

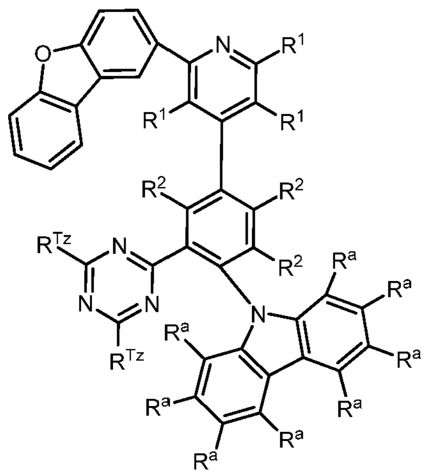

Formula VII-2

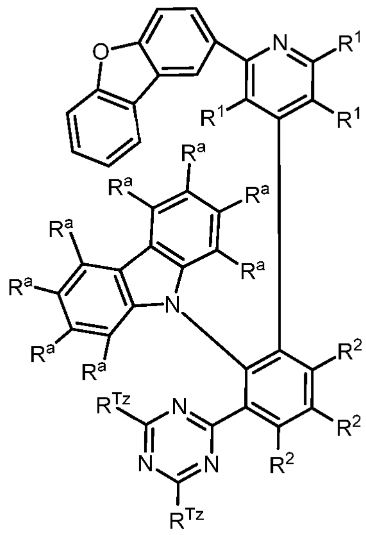

Formula VII-4

wherein the aforementioned definitions apply.

In a preferred embodiment, the organic molecules comprise or consist of a structure selected from the group of Formula VII-1 and Formula VII-2.

In a further embodiment of the invention, the organic molecules comprise or consist of a structure selected from the group of Formula VIIa-1 and Formula VIIa-2:

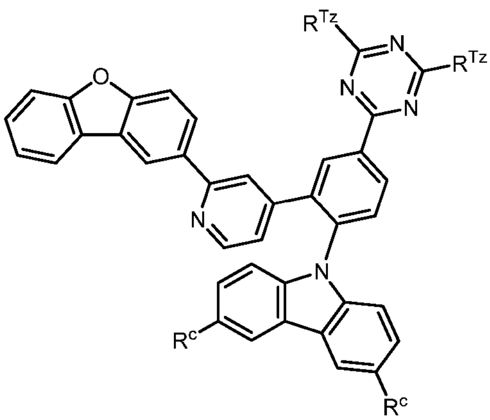

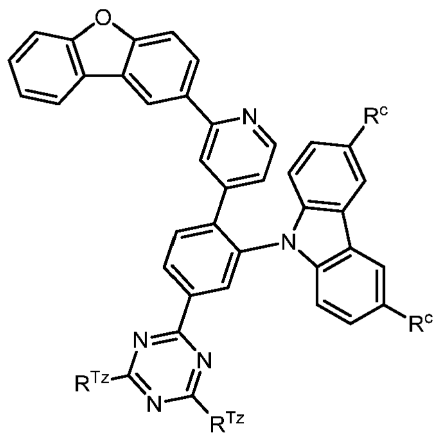

Formula VIIa-1 Formula VIIa-2

wherein the aforementioned definitions apply.

In a further embodiment of the invention, the organic molecules comprise or consist of a structure selected from the group of Formula VIIb-1 and Formula VIIb-2:

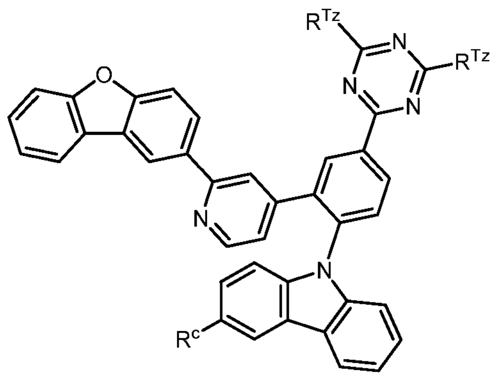

Formula VIIb-1

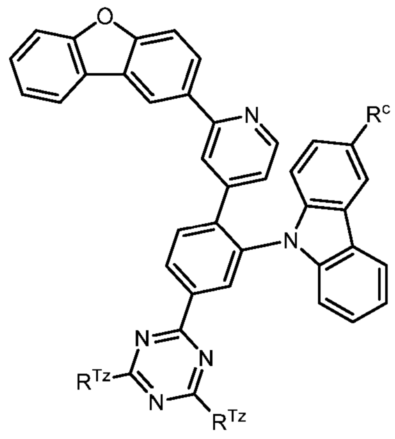

Formula VIIb-2

wherein the aforementioned definitions apply.

In one embodiment of the invention, the organic molecules comprise or consist of a structure of Formula VIII:

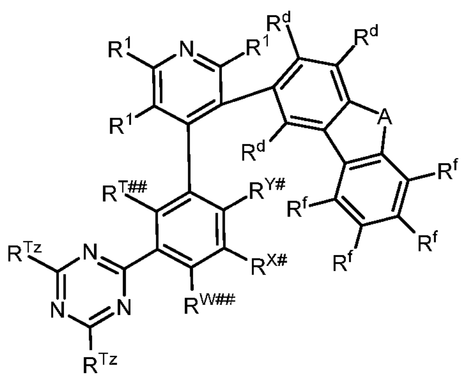

Formula VIII

wherein the aforementioned definitions apply.

In a further embodiment of the invention, the organic molecules comprise or consist of a structure selected from the group of Formula VIII-1, Formula VIII-2, and Formula VIII-4:

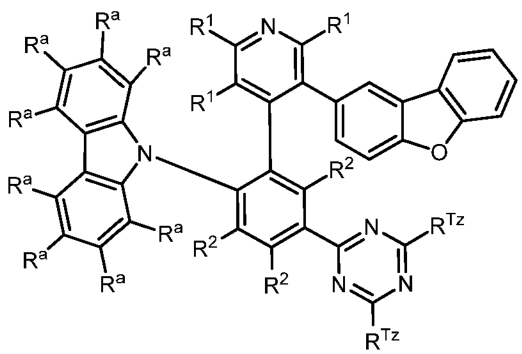

Formula VIII-1

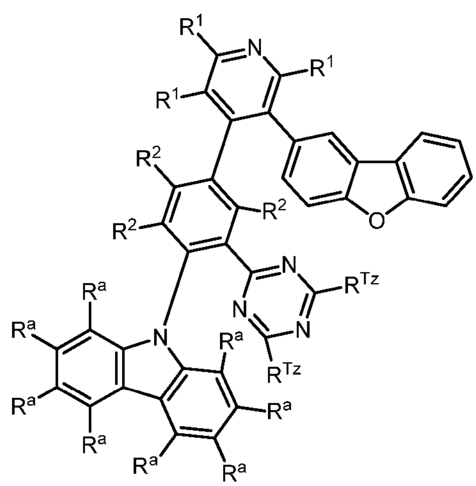

Formula VIII-2

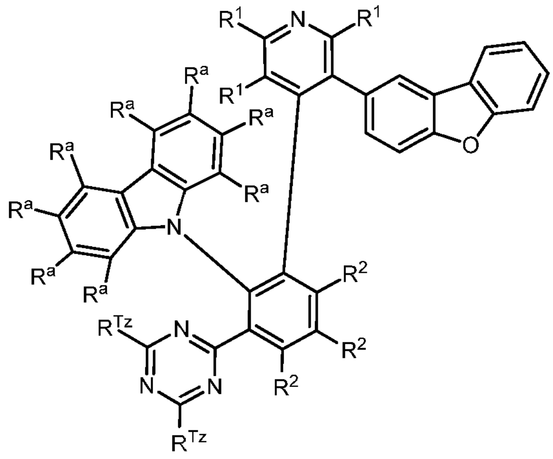

Formula VIII-4

wherein the aforementioned definitions apply.

In a preferred embodiment, the organic molecules comprise or consist of a structure selected from the group of Formula VIII-1 and Formula VIII-2.

In a further embodiment of the invention, the organic molecules comprise or consist of a structure selected from the group of Formula VIIIa-1 and Formula VIIIa-2:

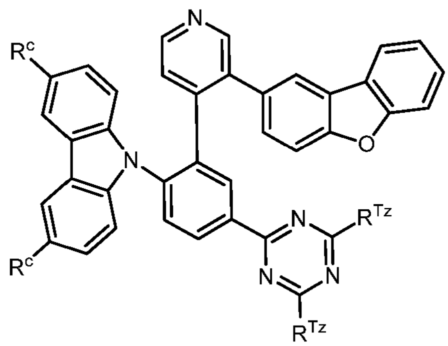

Formula VIIIa-1

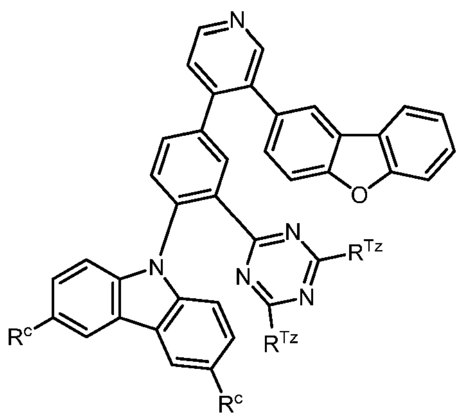

Formula VIIIa-2

wherein the aforementioned definitions apply.

In a further embodiment of the invention, the organic molecules comprise or consist of a structure selected from the group of Formula VIIIb-1 and Formula VIIIb-2:

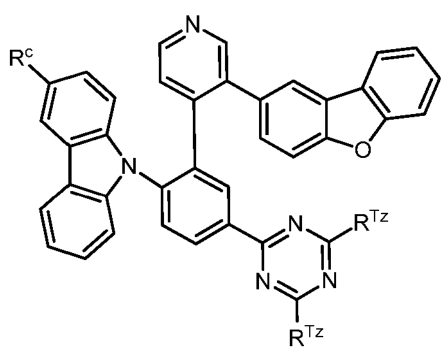

Formula VIIIb-1

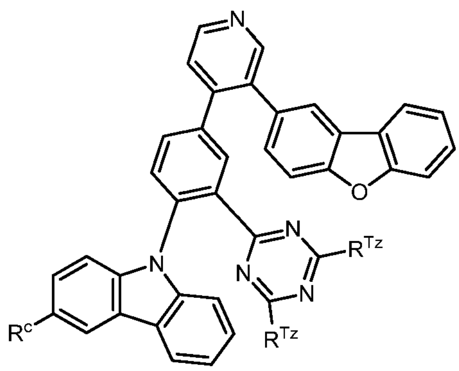

Formula VIIIb-2

wherein the aforementioned definitions apply.

In one embodiment of the invention, the organic molecules comprise or consist of a structure of Formula IX:

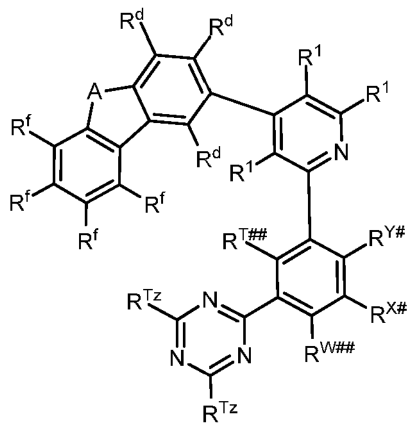

Formula IX

wherein the aforementioned definitions apply.

In a further embodiment of the invention, the organic molecules comprise or consist of a structure selected from the group of Formula IX-1, Formula IX-2, and Formula IX-4:

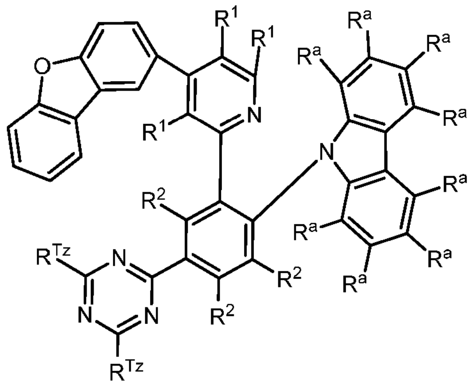

Formula IX-1

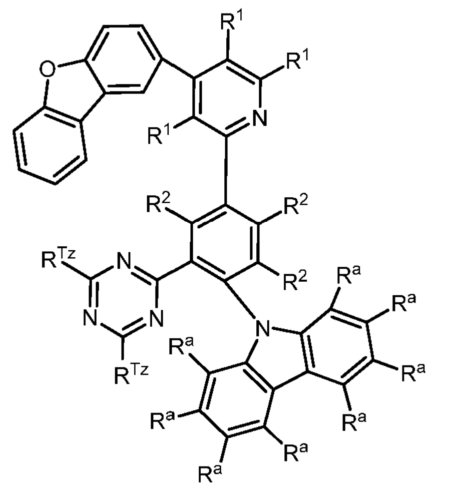

Formula IX-2

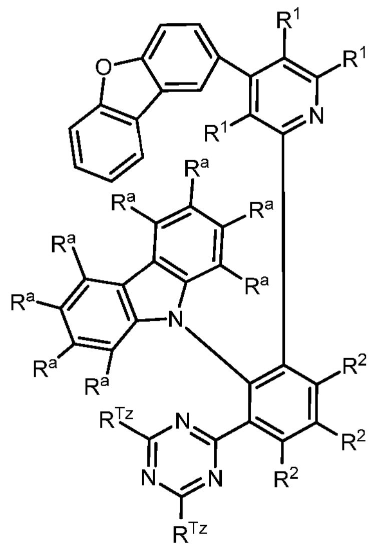

Formula IX-4

wherein the aforementioned definitions apply.

In a preferred embodiment, the organic molecules comprise or consist of a structure selected from the group of Formula IX-1 and Formula IX-2.

In a further embodiment of the invention, the organic molecules comprise or consist of a structure selected from the group of Formula IXa-1 and Formula IXa-2:

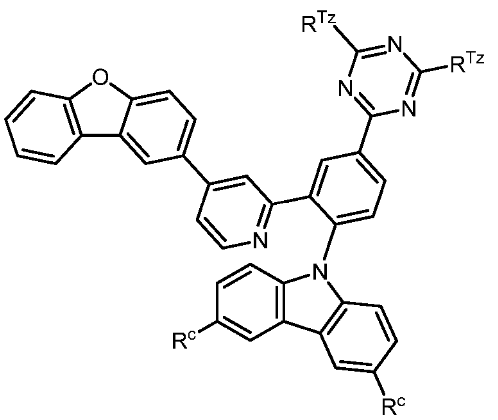

Formula IXa-1

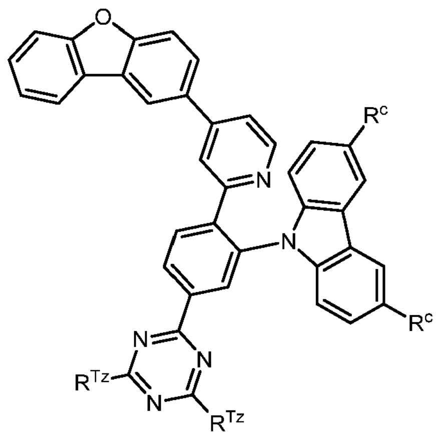

Formula IXa-2

wherein the aforementioned definitions apply.

In a further embodiment of the invention, the organic molecules comprise or consist of a structure selected from the group of Formula IXb-1 and Formula IXb-2:

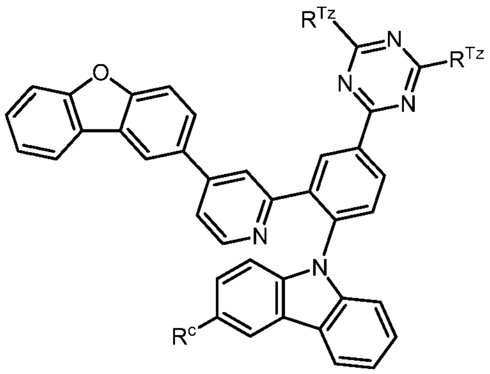

Formula IXb-1

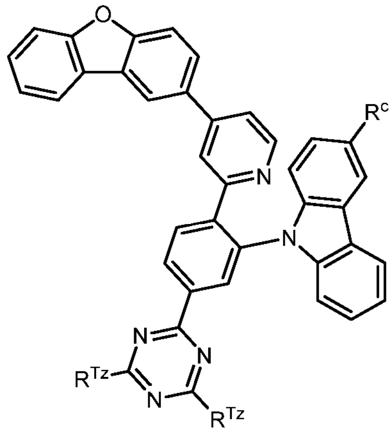

Formula IXb-2

wherein the aforementioned definitions apply.

In one embodiment of the invention, the organic molecules comprise or consist of a structure of Formula X:

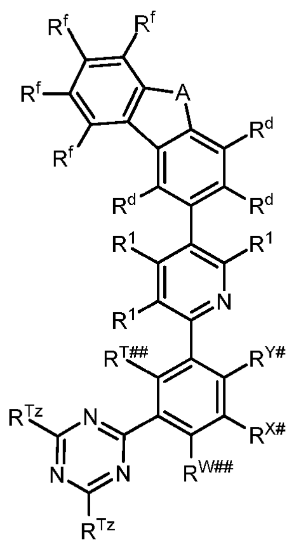

Formula X

wherein the aforementioned definitions apply.

In a further embodiment of the invention, the organic molecules comprise or consist of a structure selected from the group of Formula X-1, Formula X-2, and Formula X-4:

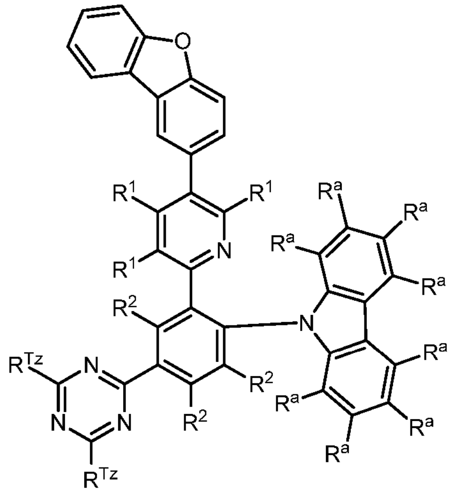

Formula X-1

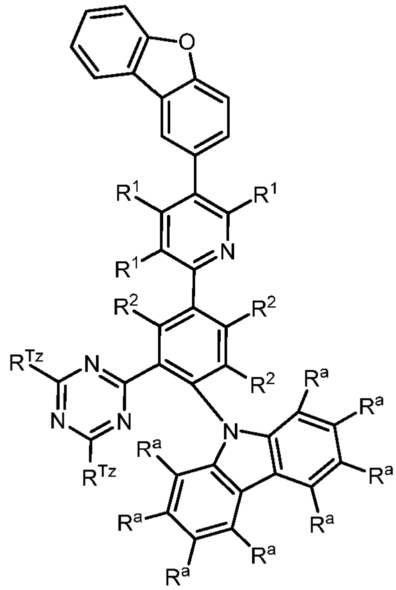

Formula X-2

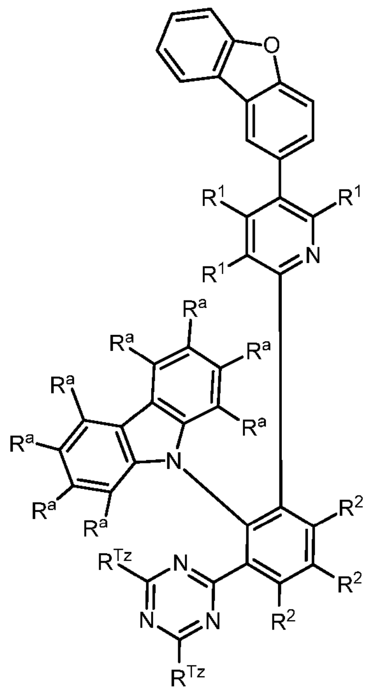

Formula X-4

wherein the aforementioned definitions apply.

In a preferred embodiment, the organic molecules comprise or consist of a structure selected from the group of Formula X-1 and Formula X-2.

In a further embodiment of the invention, the organic molecules comprise or consist of a structure selected from the group of Formula Xa-1 and Formula Xa-2:

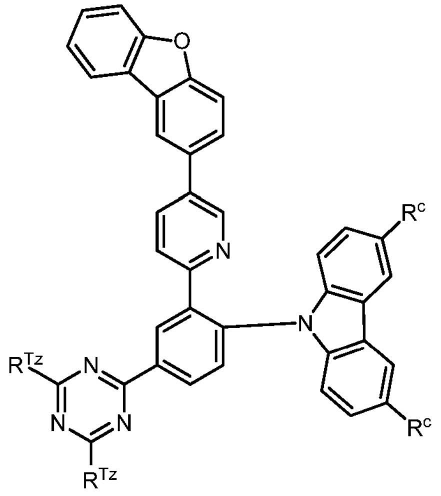

Formula Xa-1

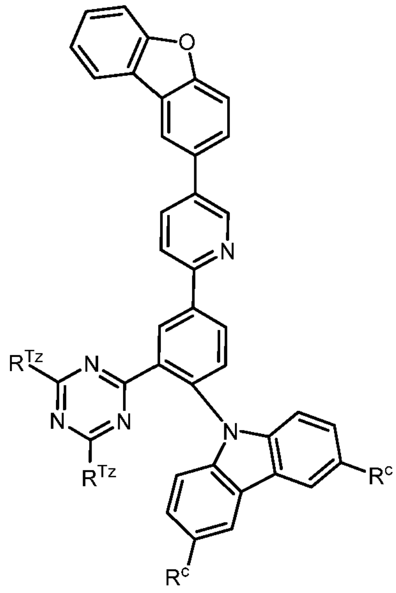

Formula Xa-2

wherein the aforementioned definitions apply.

In a further embodiment of the invention, the organic molecules comprise or consist of a structure selected from the group of Formula Xb-1 and Formula Xb-2:

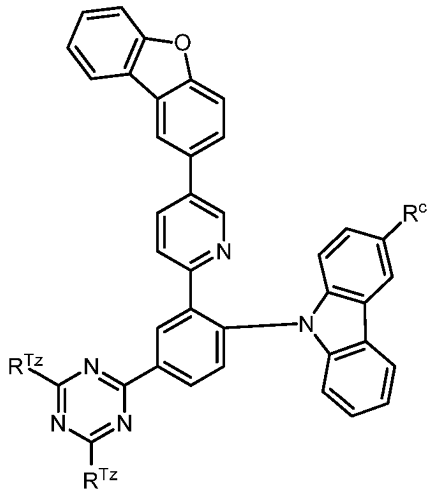

Formula Xb-1

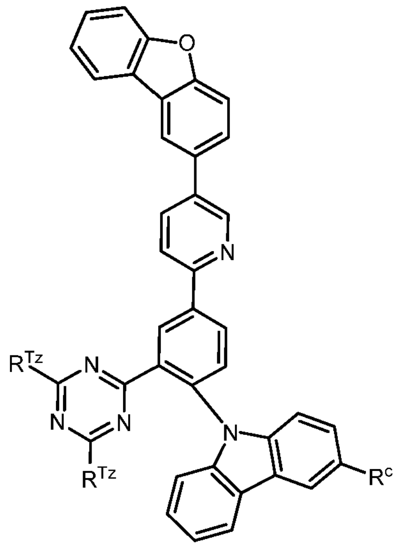

Formula Xb-2

wherein the aforementioned definitions apply.

In one embodiment of the invention $R^c$ is at each occurrence independently from another selected from the group consisting of

Me,
$^i$Pr,
$^t$Bu,
Ph, which is optionally substituted with one or more substituents independently from each other selected from the group consisting of Me, $^i$Pr, $^t$Bu, CN, $CF_3$ and Ph; and
triazinyl, which is optionally substituted with one or more substituents independently from each other selected from the group consisting of Me, $^i$Pr, $^t$Bu, CN, $CF_3$ and Ph.

As used throughout the present application, the terms "aryl" and "aromatic" may be understood in the broadest sense as any mono-, bi- or polycyclic aromatic moieties. Accordingly, an aryl group contains 6 to 60 aromatic ring atoms, and a heteroaryl group contains 5 to 60 aromatic ring atoms, of which at least one is a heteroatom. Notwithstanding, throughout the application the number of aromatic ring atoms may be given as subscripted number in the definition of certain substituents. In particular, the heteroaromatic ring includes one to three heteroatoms. Again, the terms "heteroaryl" and "heteroaromatic" may be understood in the broadest sense as any mono-, bi- or polycyclic hetero-aromatic moieties that include at least one heteroatom. The heteroatoms may at each occurrence be the same or different and be individually selected from the group consisting of N, O and S. Accordingly, the term "arylene" refers to a divalent substituent that bears two binding sites to other molecular structures and thereby serving as a linker structure. In case, a group in the exemplary embodiments is defined differently from the definitions given here, for example, the number of aromatic ring atoms or number of heteroatoms differs from the given definition, the definition in the exemplary embodiments is to be applied. According to the invention, a condensed (annulated) aromatic or heteroaromatic polycycle is built of two or more single aromatic or heteroaromatic cycles, which formed the polycycle via a condensation reaction.

In particular, as used throughout the present application the term aryl group or heteroaryl group comprises groups which can be bound via any position of the aromatic or heteroaromatic group, derived from benzene, naphthaline, anthracene, phenanthrene, pyrene, dihydropyrene, chrysene, perylene, fluoranthene, benzanthracene, benzphenanthrene, tetracene, pentacene, benzpyrene, furan, benzofuran, isobenzofuran, dibenzofuran, thiophene, benzothiophene, isobenzothiophene, dibenzothiophene; pyrrole, indole, isoindole, carbazole, pyridine, quinoline, isoquinoline, acridine, phenanthridine, benzo-5,6-quinoline, benzo-6,7-quinoline, benzo-7,8-quinoline, phenothiazine, phenoxazine, pyrazole, indazole, imidazole, benzimidazole, naphthoimidazole, phenanthroimidazole, pyridoimidazole, pyrazinoimidazole, quinoxalinoimidazole, oxazole, benzoxazole, napthooxazole, anthroxazol, phenanthroxazol, isoxazole, 1,2-thiazole, 1,3-thiazole, benzothiazole, pyridazine, benzopyridazine, pyrimidine, benzopyrimidine, 1,3,5-triazine, quinoxaline, pyrazine, phenazine, naphthyridine, carboline, benzocarboline, phenanthroline, 1,2,3-triazole, 1,2,4-triazole, benzotriazole, 1,2,3-oxadiazole, 1,2,4-oxadiazole, 1,2,5-oxadiazole, 1,2,3,4-tetrazine, purine, pteridine, indolizine and benzothiadiazole or combinations of the abovementioned groups.

As used throughout the present application the term cyclic group may be understood in the broadest sense as any mono-, bi- or polycyclic moieties.

As used throughout the present application the term biphenyl as a substituent may be understood in the broadest sense as ortho-biphenyl, meta-biphenyl, or para-biphenyl, wherein ortho, meta and para is defined in regard to the binding site to another chemical moiety.

As used throughout the present application the term alkyl group may be understood in the broadest sense as any linear, branched, or cyclic alkyl substituent. In particular, the term alkyl comprises the substituents methyl (Me), ethyl (Et), n-propyl ($^n$Pr), i-propyl ($^i$Pr), cyclopropyl, n-butyl ($^n$Bu), i-butyl ($^i$Bu), s-butyl ($^s$Bu), t-butyl ($^t$Bu), cyclobutyl, 2-methylbutyl, n-pentyl, s-pentyl, t-pentyl, 2-pentyl, neo-pentyl, cyclopentyl, n-hexyl, s-hexyl, t-hexyl, 2-hexyl, 3-hexyl, neo-hexyl, cyclohexyl, 1-methylcyclopentyl, 2-methylpentyl, n-heptyl, 2-heptyl, 3-heptyl, 4-heptyl, cycloheptyl, 1-methylcyclohexyl, n-octyl, 2-ethylhexyl, cyclooctyl, 1-bicyclo[2,2,2]octyl, 2-bicyclo[2,2,2]-octyl, 2-(2,6-dimethyl)octyl, 3-(3,7-dimethyl)octyl, adamantyl, 2,2,2-trifluorethyl, 1,1-dimethyl-n-hex-1-yl, 1,1-dimethyl-n-hept-1-yl, 1,1-dimethyl-n-oct-1-yl, 1,1-dimethyl-n-dec-1-yl, 1,1-dimethyl-n-dodec-1-yl, 1,1-dimethyl-n-tetradec-1-yl, 1,1-dimethyl-n-hexadec-1-yl, 1,1-dimethyl-n-octadec-1-yl, 1,1-diethyl-n-hex-1-yl, 1,1-diethyl-n-hept-1-yl, 1,1-diethyl-n-oct-1-yl, 1,1-diethyl-n-dec-1-yl, 1,1-diethyl-n-dodec-1-yl, 1,1-diethyl-n-tetradec-1-yl, 1,1-diethyln-n-hexadec-1-yl, 1,1-diethyl-n-octadec-1-yl, 1-(n-propyl)-cyclohex-1-yl, 1-(n-butyl)-cyclohex-1-yl, 1-(n-hexyl)-cyclohex-1-yl, 1-(n-octyl)-cyclohex-1-yl and 1-(n-decyl)-cyclohex-1-yl.

As used throughout the present application the term alkenyl comprises linear, branched, and cyclic alkenyl substituents. The term alkenyl group exemplarily comprises the substituents ethenyl, propenyl, butenyl, pentenyl, cyclopentenyl, hexenyl, cyclohexenyl, heptenyl, cycloheptenyl, octenyl, cyclooctenyl or cyclooctadienyl.

As used throughout the present application the term alkynyl comprises linear, branched, and cyclic alkynyl substituents. The term alkynyl group exemplarily comprises ethynyl, propynyl, butynyl, pentynyl, hexynyl, heptynyl or octynyl.

As used throughout the present application the term alkoxy comprises linear, branched, and cyclic alkoxy substituents. The term alkoxy group exemplarily comprises methoxy, ethoxy, n-propoxy, i-propoxy, n-butoxy, i-butoxy, s-butoxy, t-butoxy and 2-methylbutoxy.

As used throughout the present application the term thioalkoxy comprises linear, branched, and cyclic thioalkoxy substituents, in which the O of the exemplarily alkoxy groups is replaced by S.

As used throughout the present application, the terms "halogen" and "halo" may be understood in the broadest sense as being preferably fluorine, chlorine, bromine or iodine.

Whenever hydrogen (H) is mentioned herein, it could also be replaced by deuterium at each occurrence.

It is understood that when a molecular fragment is described as being a substituent or otherwise attached to another moiety, its name may be written as if it were a fragment (e.g. naphtyl, dibenzofuryl) or as if it were the whole molecule (e.g. naphthalene, dibenzofuran). As used herein, these different ways of designating a substituent or attached

fragment are considered to be equivalent.

In one embodiment, the organic molecules according to the invention have an excited state lifetime of not more than 150 μs, of not more than 100 μs, in particular of not more than 50 μs, more preferably of not more than 10 μs or not more than 7 μs in a film of poly(methyl methacrylate) (PMMA) with 10 % by weight of organic molecule at room temperature.

In one embodiment of the invention, the organic molecules according to the invention represent thermally-activated delayed fluorescence (TADF) emitters, which exhibit a $\Delta E_{ST}$ value, which corresponds to the energy difference between the first excited singlet state (S1) and the first excited triplet state (T1), of less than 5000 $cm^{-1}$, preferably less than 3000 $cm^{-1}$, more preferably less than 1500 $cm^{-1}$, even more preferably less than 1000 $cm^{-1}$ or even less than 500 $cm^{-1}$.

In a further embodiment of the invention, the organic molecules according to the invention have an emission peak in the visible or nearest ultraviolet range, i.e., in the range of a wavelength of from 380 to 800 nm, with a full width at half maximum of less than 0.50 eV, preferably less than 0.48 eV, more preferably less than 0.45 eV, even more preferably less than 0.43 eV or even less than 0.40 eV in a film of poly(methyl methacrylate) (PMMA) with 10 % by weight of organic molecule at room temperature.

In a further embodiment of the invention, the organic molecules according to the invention have a "blue material index" (BMI), calculated by dividing the photoluminescence quantum yield (PLQY) in % by the CIEy color coordinate of the emitted light, of more than 150, in particular more than 200, preferably more than 250, more preferably of more than 300 or even more than 500.

Orbital and excited state energies can be determined either by means of experimental methods or by calculations employing quantum-chemical methods, in particular density functional theory calculations. The energy of the highest occupied molecular orbital $E^{HOMO}$ is determined by methods known to the person skilled in the art from cyclic voltammetry measurements with an accuracy of 0.1 eV. The energy of the lowest unoccupied molecular orbital $E^{LUMO}$ is calculated as $E^{HOMO} + E^{gap}$, wherein $E^{gap}$ is determined as follows: For host compounds, the onset of the emission spectrum of a film with 10 % by weight of host in poly(methyl methacrylate) (PMMA) is used as $E^{gap}$, unless stated otherwise. For emitter molecules, $E^{gap}$ is determined as the energy at which the excitation and emission spectra of a film with 10 % by weight of emitter in PMMA cross.

The energy of the first excited triplet state T1 is determined from the onset of the emission spectrum at low temperature, typically at 77 K. For host compounds, where the first excited singlet state and the lowest triplet state are energetically separated by > 0.4 eV, the phosphorescence is usually visible in a steady-state spectrum in 2-Me-THF. The triplet energy can thus be determined as the onset of the phosphorescence spectrum. For TADF emitter molecules, the energy of the first excited triplet state T1 is determined from the onset of the delayed emission spectrum at 77 K, if not otherwise stated measured in a film of PMMA with 10 % by weight of emitter. Both for host and emitter compounds, the energy of the first excited singlet state S1 is determined from the onset of the emission spectrum, if not otherwise stated measured in a film of PMMA with 10 % by weight of host or emitter compound.

The onset of an emission spectrum is determined by computing the intersection of the tangent to the emission spectrum with the x-axis. The tangent to the emission spectrum is set at the high-energy side of the emission band and at the point at half maximum of the maximum intensity of the emission spectrum.

A further aspect of the invention relates to a process for preparing organic molecules (with an optional subsequent reaction) according to the invention, wherein a $Hal^a$-fluorophenyl-1,3,5-triazine, in which the central phenyl ring is substituted with three $R^I$-substituents and the triazine unit is substituted with two $R^{Tz}$ in 4- and 6-position, is used as a reactant:

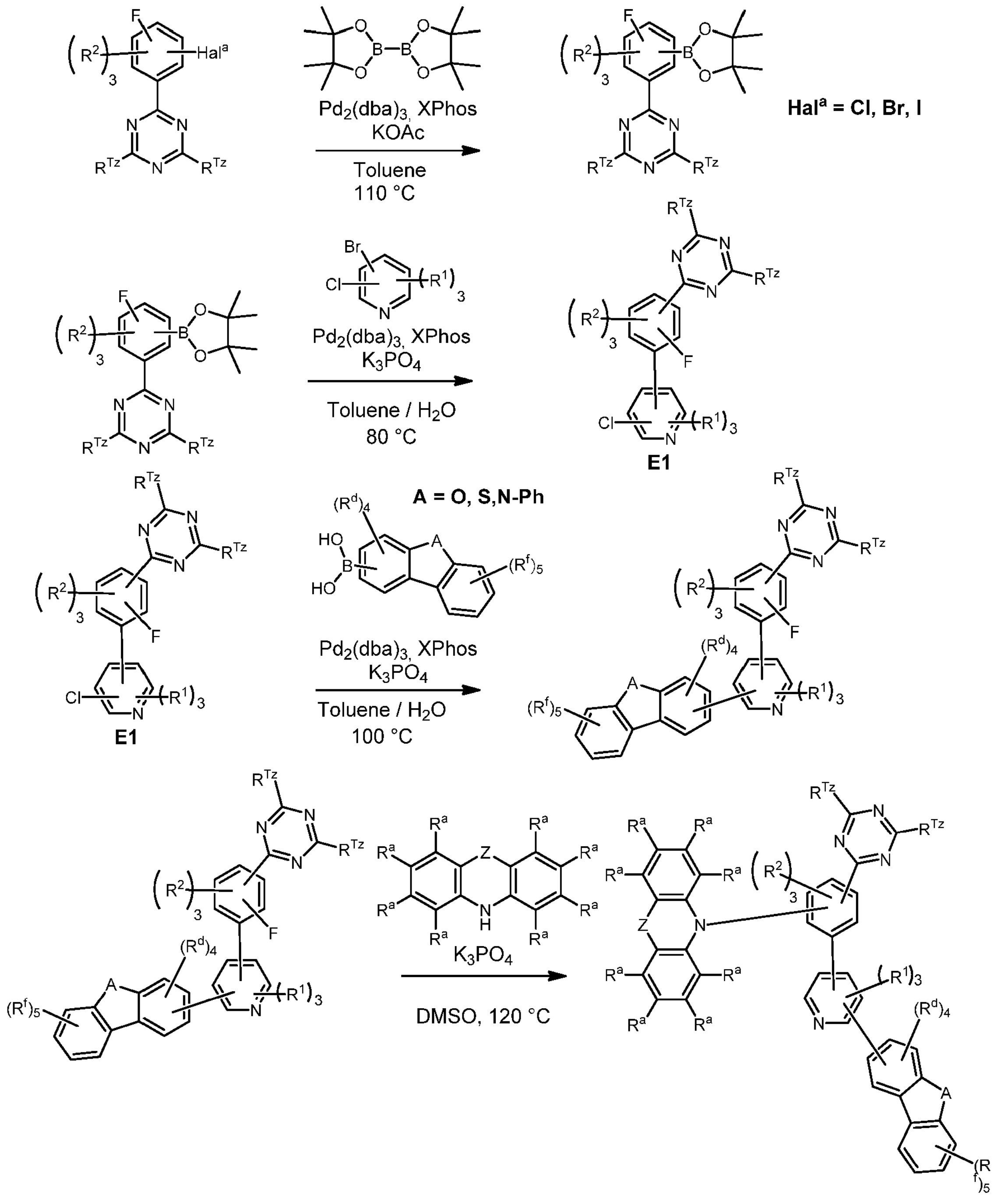


wherein Hal$^a$ is selected from the group consisting of Cl, Br and I;
and A is selected from O, S, N-Ph.

Preferably, Hal$^a$-fluorophenyl-1,3,5-triazine is selected from bromo-fluorophenyl-1,3,5-triazines and chloro-fluorophenyl-1,3,5-triazines. Exemplary Hal$^a$-fluorophenyl-1,3,5-triazines are 2-(3-Bromo-4-fluorophenyl)-4,6-diphenyl-1,3,5-triazine, 2-(3-Chloro-4-fluorophenyl)-4,6-diphenyl-1,3,5-triazine, 2-(3-Bromo-4-fluorophenyl)-4,6-methyl-1,3,5-triazine, 2-(3-Chloro-4-fluorophenyl)-4,6-methyl-1,3,5-triazine, 2-(4-Bromo-3-fluorophenyl)-4,6-diphenyl-1,3,5-triazine, 2-(4-Chloro-3-fluorophenyl)-4,6-diphenyl-1,3,5-triazine, 2-(4-Bromo-3-fluorophenyl)-4,6-methyl-1,3,5-triazine, 2-(4-Chloro-3-fluorophenyl)-4,6-methyl-1,3,5-triazine.

Typically, Pd$_2$(dba)$_3$ (tris(dibenzylideneacetone)dipalladium(0)) is used as a Pd catalyst, but alternatives are known in the art. For example, the ligand may be selected from the group consisting of S-Phos ([2-dicyclohexylphoshino-2',6'-dimethoxy-1,1'-biphenyl]; or SPhos), X-Phos (2-(dicyclohexylphosphino)-2",4",6"-triisopropylbiphenyl; or XPhos), and P(Cy)$_3$ (tricyclohexylphosphine). The salt is, for example, selected from tribasic potassium phosphate

and potassium acetate and the solvent can be a pure solvent, such as toluene or dioxane, or a mixture, such as toluene/dioxane/water or dioxane/toluene. A person of skill in the art can determine which Pd catalyst, ligand, salt and solvent combination will result in high reaction yields.

A person of skill in the pertinent art is aware that instead of the *in-situ* generation of a boronic acid ester by reacting a halo-species with bis-(pinacolato)diboron and then reacting it with another halo-species as described here, alternative synthesis routes can be chosen. For example, one of the two halo-species, i.e. the $Hal^a$-fluorophenyl-1,3,5-triazine and the halo-fluoro-pyridine, can be replaced by the corresponding boronic acid or boronic acid ester species and reacted with the respective other halo-species in a typical cross-coupling reaction.

For the reaction of **E1** with a nitrogen heterocycle in a nucleophilic aromatic substitution with an aryl halide, preferably an aryl fluoride, typical conditions include the use of a base, such as tribasic potassium phosphate or sodium hydride, for example, in an aprotic polar solvent, such as dimethyl sulfoxide (DMSO) or N,N-dimethylformamide (DMF), for example.

An alternative synthesis route comprises the introduction of a nitrogen heterocycle via copper- or palladium-catalyzed coupling to an aryl halide or aryl pseudohalide, preferably an aryl bromide, an aryl iodide, aryl triflate or an aryl tosylate.

A further aspect of the invention relates to the use of an organic molecule according to the invention as a luminescent emitter or as an absorber, and/or as host material and/or as electron transport material, and/or as hole injection material, and/or as hole blocking material in an optoelectronic device.

The optoelectronic device may be understood in the broadest sense as any device based on organic materials that is suitable for emitting light in the visible or nearest ultraviolet (UV) range, i.e., in the range of a wavelength of from 380 to 800 nm. More preferably, the optoelectronic device may be able to emit light in the visible range, i.e., of from 400 to 800 nm.

In the context of such use, the optoelectronic device is more particularly selected from the group consisting of:

- organic light-emitting diodes (OLEDs),
- light-emitting electrochemical cells,
- OLED sensors, especially in gas and vapour sensors not hermetically externally shielded,
- organic diodes,
- organic solar cells,
- organic transistors,
- organic field-effect transistors,
- organic lasers and
- down-conversion elements.

In a preferred embodiment in the context of such use, the optoelectronic device is a device selected from the group consisting of an organic light emitting diode (OLED), a light emitting electrochemical cell (LEC), and a light-emitting transistor.

In the case of the use, the fraction of the organic molecule according to the invention in the emission layer in an optoelectronic device, more particularly in OLEDs, is 1 % to 99 % by weight, more particularly 5 % to 80 % by weight. In an alternative embodiment, the proportion of the organic molecule in the emission layer is 100 % by weight.

In one embodiment, the light-emitting layer comprises not only the organic molecules according to the invention but also a host material whose triplet (T1) and singlet (S1) energy levels are energetically higher than the triplet (T1) and singlet (S1) energy levels of the organic molecule.

A further aspect of the invention relates to a composition comprising or consisting of:

(a) at least one organic molecule according to the invention, in particular in the form of an emitter and/or a host, and
(b) one or more emitter and/or host materials, which differ from the organic molecule according to the invention and
(c) optional one or more dyes and/or one or more solvents.

In one embodiment, the light-emitting layer comprises (or (essentially) consists of) a composition comprising or consisting of:

(a) at least one organic molecule according to the invention, in particular in the form of an emitter and/or a host, and
(b) one or more emitter and/or host materials, which differ from the organic molecule according to the invention and
(c) optional one or more dyes and/or one or more solvents.

Particularly preferably the light-emitting layer EML comprises (or (essentially) consists of) a composition comprising or consisting of:

(i) 1-50 % by weight, preferably 5-40 % by weight, in particular 10-30 % by weight, of one or more organic molecules according to the invention E;
(ii) 5-99 % by weight, preferably 30-94.9 % by weight, in particular 40-89% by weight, of at least one host compound H; and
(iii) optionally 0-94 % by weight, preferably 0.1-65 % by weight, in particular 1-50 % by weight, of at least one further host compound D with a structure differing from the structure of the molecules according to the invention; and
(iv) optionally 0-94 % by weight, preferably 0-65 % by weight, in particular 0-50 % by weight, of a solvent; and
(v) optionally 0-30 % by weight, in particular 0-20 % by weight, preferably 0-5 % by weight, of at least one further emitter molecule F with a structure differing from the structure of the molecules according to the invention.

Preferably, energy can be transferred from the host compound H to the one or more organic molecules according to the invention E, in particular transferred from the first excited triplet state T1(H) of the host compound H to the first excited triplet state T1(E) of the one or more organic molecules according to the invention E and/ or from the first excited singlet state S1(H) of the host compound H to the first excited singlet state S1(E) of the one or more organic molecules according to the invention E.

In a further embodiment, the light-emitting layer EML comprises (or (essentially) consists of) a composition comprising or consisting of:

(i) 1-50 % by weight, preferably 5-40 % by weight, in particular 10-30 % by weight, of one organic molecule according to the invention E;
(ii) 5-99 % by weight, preferably 30-94.9 % by weight, in particular 40-89% by weight, of one host compound H; and
(iii) optionally 0-94 % by weight, preferably 0.1-65 % by weight, in particular 1-50 % by weight, of at least one further host compound D with a structure differing from the structure of the molecules according to the invention; and
(iv) optionally 0-94 % by weight, preferably 0-65 % by weight, in particular 0-50 % by weight, of a solvent; and
(v) optionally 0-30 % by weight, in particular 0-20 % by weight, preferably 0-5 % by weight, of at least one further emitter molecule F with a structure differing from the structure of the molecules according to the invention.

In one embodiment, the host compound H has a highest occupied molecular orbital HOMO(H) having an energy $E^{HOMO}(H)$ in the range of from -5 to -6.5 eV and the at least one further host compound D has a highest occupied molecular orbital HOMO(D) having an energy $E^{HOMO}(D)$, wherein $E^{HOMO}(H) > E^{HOMO}(D)$.

In a further embodiment, the host compound H has a lowest unoccupied molecular orbital LUMO(H) having an energy $E^{LUMO}(H)$ and the at least one further host compound D has a lowest unoccupied molecular orbital LUMO(D) having an energy $E^{LUMO}(D)$, wherein $E^{LUMO}(H) > E^{LUMO}(D)$.

In one embodiment, the host compound H has a highest occupied molecular orbital HOMO(H) having an energy $E^{HOMO}(H)$ and a lowest unoccupied molecular orbital LUMO(H) having an energy $E^{LUMO}(H)$, and

the at least one further host compound D has a highest occupied molecular orbital HOMO(D) having an energy $E^{HOMO}(D)$ and a lowest unoccupied molecular orbital LUMO(D) having an energy $E^{LUMO}(D)$,
the organic molecule according to the invention E has a highest occupied molecular orbital HOMO(E) having an energy $E^{HOMO}(E)$ and a lowest unoccupied molecular orbital LUMO(E) having an energy $E^{LUMO}(E)$,

wherein
$E^{HOMO}(H) > E^{HOMO}(D)$ and the difference between the energy level of the highest occupied molecular orbital HOMO(E) of the organic molecule according to the invention E ($E^{HOMO}(E)$) and the energy level of the highest occupied molecular orbital HOMO(H) of the host compound H ($E^{HOMO}(H)$) is between -0.5 eV and 0.5 eV, more preferably between -0.3 eV and 0.3 eV, even more preferably between -0.2 eV and 0.2 eV or even between -0.1 eV and 0.1 eV; and $E^{LUMO}(H) > E^{LUMO}(D)$ and the difference between the energy level of the lowest unoccupied molecular orbital LUMO(E) of the organic molecule according to the invention E ($E^{LUMO}(E)$) and the lowest unoccupied molecular orbital LUMO(D) of the at least one further host compound D ($E^{LUMO}(D)$) is between -0.5 eV and 0.5 eV, more preferably between -0.3 eV and 0.3 eV, even more preferably between -0.2 eV and 0.2 eV or even between -0.1 eV and 0.1 eV.

In a further aspect, the invention relates to an optoelectronic device comprising an organic molecule or a composition of the type described here, more particularly in the form of a device selected from the group consisting of organic light-emitting diode (OLED), light-emitting electrochemical cell, OLED sensor, more particularly gas and vapour sensors not hermetically externally shielded, organic diode, organic solar cell, organic transistor, organic field-effect transistor, organic laser and down-conversion element.

In a preferred embodiment, the optoelectronic device is a device selected from the group consisting of an organic light emitting diode (OLED), a light emitting electrochemical cell (LEC), and a light-emitting transistor.

In one embodiment of the optoelectronic device of the invention, the organic molecule according to the invention

E is used as emission material in a light-emitting layer EML.

In one embodiment of the optoelectronic device of the invention the light-emitting layer EML consists of the composition according to the invention described here.

Exemplarily, when the optoelectronic device is an OLED, it may exhibit the following layer structure:

1. substrate
2. anode layer A
3. hole injection layer, HIL
4. hole transport layer, HTL
5. electron blocking layer, EBL
6. emitting layer, EML
7. hole blocking layer, HBL
8. electron transport layer, ETL
9. electron injection layer, EIL
10. cathode layer,

wherein the OLED comprises each layer only optionally, different layers may be merged and the OLED may comprise more than one layer of each layer type defined above.

Furthermore, the optoelectronic device may optionally comprise one or more protective layers protecting the device from damaging exposure to harmful species in the environment including, exemplarily moisture, vapor and/or gases.

In one embodiment of the invention, the optoelectronic device is an OLED, which exhibits the following inverted layer structure:

1. substrate
2. cathode layer
3. electron injection layer, EIL
4. electron transport layer, ETL
5. hole blocking layer, HBL
6. emitting layer, B
7. electron blocking layer, EBL
8. hole transport layer, HTL
9. hole injection layer, HIL
10. anode layer A

Wherein the OLED with an inverted layer structure comprises each layer only optionally, different layers may be merged and the OLED may comprise more than one layer of each layer types defined above.

In one embodiment of the invention, the optoelectronic device is an OLED, which may exhibit stacked architecture. In this architecture, contrary to the typical arrangement, where the OLEDs are placed side by side, the individual units are stacked on top of each other. Blended light may be generated with OLEDs exhibiting a stacked architecture, in particular white light may be generated by stacking blue, green and red OLEDs. Furthermore, the OLED exhibiting a stacked architecture may optionally comprise a charge generation layer (CGL), which is typically located between two OLED subunits and typically consists of a n-doped and p-doped layer with the n-doped layer of one CGL being typically located closer to the anode layer.

In one embodiment of the invention, the optoelectronic device is an OLED, which comprises two or more emission layers between anode and cathode. In particular, this so-called tandem OLED comprises three emission layers, wherein one emission layer emits red light, one emission layer emits green light and one emission layer emits blue light, and optionally may comprise further layers such as charge generation layers, blocking or transporting layers between the individual emission layers. In a further embodiment, the emission layers are adjacently stacked. In a further embodiment, the tandem OLED comprises a charge generation layer between each two emission layers. In addition, adjacent emission layers or emission layers separated by a charge generation layer may be merged.

The substrate may be formed by any material or composition of materials. Most frequently, glass slides are used as substrates. Alternatively, thin metal layers (e.g., copper, gold, silver or aluminum films) or plastic films or slides may be used. This may allow a higher degree of flexibility. The anode layer A is mostly composed of materials allowing to obtain an (essentially) transparent film. As at least one of both electrodes should be (essentially) transparent in order to allow light emission from the OLED, either the anode layer A or the cathode layer C is transparent. Preferably, the anode layer A comprises a large content or even consists of transparent conductive oxides (TCOs). Such anode layer A may exemplarily comprise indium tin oxide, aluminum zinc oxide, fluorine doped tin oxide, indium zinc oxide, PbO, SnO, zirconium oxide,

molybdenum oxide, vanadium oxide, wolfram oxide, graphite, doped Si, doped Ge, doped GaAs, doped polyaniline, doped polypyrrol and/or doped polythiophene.

Particularly preferably, the anode layer A (essentially) consists of indium tin oxide (ITO) (e.g., $(InO_3)0.9(SnO_2)0.1$). The roughness of the anode layer A caused by the transparent conductive oxides (TCOs) may be compensated by using a hole injection layer (HIL). Further, the HIL may facilitate the injection of quasi charge carriers (i.e., holes) in that the transport of the quasi charge carriers from the TCO to the hole transport layer (HTL) is facilitated. The hole injection layer (HIL) may comprise poly-3,4-ethylendioxy thiophene (PEDOT), polystyrene sulfonate (PSS), $MoO_2$, $V_2O_5$, CuPC or CuI, in particular a mixture of PEDOT and PSS. The hole injection layer (HIL) may also prevent the diffusion of metals from the anode layer A into the hole transport layer (HTL). The HIL may exemplarily comprise PEDOT:PSS (poly-3,4-ethylendioxy thiophene: polystyrene sulfonate), PEDOT (poly-3,4-ethylendioxy thiophene), mMTDATA (4,4',4"-tris[phenyl(m-tolyl) amino]triphenylamine), Spiro-TAD (2,2',7,7'-tetrakis(n,n-diphenylamino)-9,9'-spirobifluorene), DNTPD (N1,N1'-(biphenyl-4,4'-diyl)bis(N1-phenyl-N4,N4-di-m-tolylbenzene-1,4-diamine), NPB (N,N'-nis-(1-naphthalenyl)-N,N'-bisphenyl-(1,1'-biphenyl)-4,4'-diamine), NPNPB (N,N'-diphenyl-N,N'-di-[4-(N,N-diphenyl-amino)phenyl]benzidine), MeO-TPD (N,N,N',N'-tetrakis(4-methoxyphenyl)benzidine), HAT-CN (1,4,5,8,9,11-hexaazatriphenylen-hexacarbonitrile) and/or Spiro-NPD (N,N'-diphenyl-N,N'-bis-(1-naphthyl)-9,9'-spirobifluorene-2,7-diamine).

Adjacent to the anode layer A or hole injection layer (HIL) typically a hole transport layer (HTL) is located. Herein, any hole transport compound may be used. Exemplarily, electron-rich heteroaromatic compounds such as triarylamines and/or carbazoles may be used as hole transport compound. The HTL may decrease the energy barrier between the anode layer A and the light-emitting layer EML. The hole transport layer (HTL) may also be an electron blocking layer (EBL). Preferably, hole transport compounds bear comparably high energy levels of their triplet states T1. Exemplarily the hole transport layer (HTL) may comprise a star-shaped heterocycle such as tris(4-carbazoyl-9-ylphenyl)amine (TCTA), poly-TPD (poly(4-butylphenyl-diphenyl-amine)), [alpha]-NPD (poly(4-butylphenyl-diphenyl-amine)), TAPC (4,4'-cyclohexyliden-bis[N,N-bis(4-methylphenyl)benzenamine]), 2-TNATA (4,4',4"-tris[2-naphthyl(phenyl)amino]triphenylamine), Spiro-TAD, DNTPD, NPB, NPNPB, MeO-TPD, HAT-CN and/or TrisPcz (9,9'-diphenyl-6-(9-phenyl-9H-carbazol-3-yl)-9H,9'H-3,3'-bicarbazole). In addition, the HTL may comprise a p-doped layer, which may be composed of an inorganic or organic dopant in an organic hole-transporting matrix. Transition metal oxides such as vanadium oxide, molybdenum oxide or tungsten oxide may exemplarily be used as inorganic dopant. Tetrafluorotetracyanoquinodimethane ($F_4$-TCNQ), copper-pentafluorobenzoate (Cu(I)pFBz) or transition metal complexes may exemplarily be used as organic dopant.

The EBL may exemplarily comprise mCP (1,3-bis(carbazol-9-yl)benzene), TCTA, 2-TNATA, mCBP (3,3-di(9H-carbazol-9-yl)biphenyl), tris-Pcz, CzSi (9-(4-tert-Butylphenyl)-3,6-bis(triphenylsilyl)-9H-carbazole), and/or DCB (N,N'-dicarbazolyl-1,4-dimethylbenzene).

Adjacent to the hole transport layer (HTL), typically, the light-emitting layer EML is located. The light-emitting layer EML comprises at least one light emitting molecule. Particularly, the EML comprises at least one light emitting molecule according to the invention E. In one embodiment, the light-emitting layer comprises only the organic molecules according to the invention E. Typically, the EML additionally comprises one or more host materials H. Exemplarily, the host material H is selected from CBP (4,4'-Bis-(N-carbazolyl)-biphenyl), mCP, mCBP Sif87 (dibenzo[b,d]thiophen-2-yltriphenylsilane), CzSi, Sif88 (dibenzo[b,d]thiophen-2-yl)diphenylsilane), DPEPO (bis[2-(diphenylphosphino)phenyl] ether oxide), 9-[3-(dibenzofuran-2-yl)phenyl]-9H-carbazole, 9-[3-(dibenzofuran-2-yl)phenyl]-9H-carbazole, 9-[3-(dibenzothiophen-2-yl)phenyl]-9H-carbazole, 9-[3,5-bis(2-dibenzofuranyl)phenyl]-9H-carbazole, 9-[3,5-bis(2-dibenzothiophenyl) phenyl]-9H-carbazole, T2T (2,4,6-tris(biphenyl-3-yl)-1,3,5-triazine), T3T (2,4,6-tris(triphenyl-3-yl)-1,3,5-triazine) and/or TST (2,4,6-tris(9,9'-spirobifluorene-2-yl)-1,3,5-triazine). The host material H typically should be selected to exhibit first triplet (T1) and first singlet (S1) energy levels, which are energetically higher than the first triplet (T1) and first singlet (S1) energy levels of the organic molecule.

In one embodiment of the invention, the EML comprises a so-called mixed-host system with at least one hole-dominant host and one electron-dominant host. In a particular embodiment, the EML comprises exactly one light emitting molecule according to the invention E and a mixed-host system comprising T2T as electron-dominant host and a host selected from CBP, mCP, mCBP, 9-[3-(dibenzofuran-2-yl)phenyl]-9H-carbazole, 9-[3-(dibenzofuran-2-yl)phenyl]-9H-carbazole, 9-[3-(dibenzothiophen-2-yl)phenyl]-9H-carbazole, 9-[3,5-bis(2-dibenzofuranyl)phenyl]-9H-carbazole and 9-[3,5-bis(2-dibenzothiophenyl)phenyl]-9H-carbazole as hole-dominant host. In a further embodiment the EML comprises 50-80 % by weight, preferably 60-75 % by weight of a host selected from CBP, mCP, mCBP, 9-[3-(dibenzofuran-2-yl) phenyl]-9H-carbazole, 9-[3-(dibenzofuran-2-yl)phenyl]-9H-carbazole, 9-[3-(dibenzothiophen-2-yl)phenyl]-9H-carbazole, 9-[3,5-bis(2-dibenzofuranyl)phenyl]-9H-carbazole and 9-[3,5-bis(2-dibenzothiophenyl)phenyl]-9H-carbazole; 10-45 % by weight, preferably 15-30 % by weight of T2T and 5-40 % by weight, preferably 10-30 % by weight of light emitting molecule according to the invention.

Adjacent to the light-emitting layer EML an electron transport layer (ETL) may be located. Herein, any electron transporter may be used. Exemplarily, electron-poor compounds such as, e.g., benzimidazoles, pyridines, triazoles, oxadiazoles (e.g., 1,3,4-oxadiazole), phosphinoxides and sulfone, may be used. An electron transporter may also be a star-shaped heterocycle such as 1,3,5-tri(1-phenyl-1H-benzo[d]imidazol-2-yl)phenyl (TPBi). The ETL may comprise

NBphen (2,9-bis(naphthalen-2-yl)-4,7-diphenyl-1,10-phenanthroline), $Alq_3$ (Aluminum-tris(8-hydroxyquinoline)), TSPO1 (diphenyl-4-triphenylsilylphenyl-phosphinoxide), BPyTP2 (2,7-di(2,2'-bipyridin-5-yl)triphenyle), Sif87 (dibenzo[b,d]thiophen-2-yltriphenylsilane), Sif88 (dibenzo[b,d]thiophen-2-yl)diphenylsilane), BmPyPhB (1,3-bis[3,5-di(pyridin-3-yl)phenyl]benzene) and/or BTB (4,4'-bis-[2-(4,6-diphenyl-1,3,5-triazinyl)]-1,1'-biphenyl). Optionally, the ETL may be doped with materials such as Liq. The electron transport layer (ETL) may also block holes or a holeblocking layer (HBL) is introduced.

The HBL may exemplarily comprise BCP (2,9-dimethyl-4,7-diphenyl-1,10-phenanthroline = Bathocuproine), BAlq (bis(8-hydroxy-2-methylquinoline)-(4-phenylphenoxy)aluminum), NBphen (2,9-bis(naphthalen-2-yl)-4,7-diphenyl-1,10-phenanthroline), $Alq_3$ (Aluminum-tris(8-hydroxyquinoline)), TSPO1 (diphenyl-4-triphenylsilylphenyl-phosphinoxide), T2T (2,4,6-tris(biphenyl-3-yl)-1,3,5-triazine), T3T (2,4,6-tris(triphenyl-3-yl)-1,3,5-triazine), TST (2,4,6-tris(9,9'-spirobifluorene-2-yl)-1,3,5-triazine), and/or TCB/TCP (1,3,5-tris(N-carbazolyl)benzol/ 1,3,5-tris(carbazol)-9-yl) benzene).

Adjacent to the electron transport layer (ETL), a cathode layer C may be located. Exemplarily, the cathode layer C may comprise or may consist of a metal (e.g., Al, Au, Ag, Pt, Cu, Zn, Ni, Fe, Pb, LiF, Ca, Ba, Mg, In, W, or Pd) or a metal alloy. For practical reasons, the cathode layer may also consist of (essentially) non-transparent metals such as Mg, Ca or Al. Alternatively or additionally, the cathode layer C may also comprise graphite and or carbon nanotubes (CNTs). Alternatively, the cathode layer C may also consist of nanoscalic silver wires.

An OLED may further, optionally, comprise a protection layer between the electron transport layer (ETL) and the cathode layer C (which may be designated as electron injection layer (EIL)). This layer may comprise lithium fluoride, cesium fluoride, silver, Liq (8-hydroxyquinolinolatolithium), $Li_2O$, $BaF_2$, MgO and/or NaF.

Optionally, also the electron transport layer (ETL) and/or a hole blocking layer (HBL) may comprise one or more host compounds H.

In order to modify the emission spectrum and/or the absorption spectrum of the light-emitting layer EML further, the light-emitting layer EML may further comprise one or more further emitter molecules F. Such an emitter molecule F may be any emitter molecule known in the art. Preferably such an emitter molecule F is a molecule with a structure differing from the structure of the molecules according to the invention E. The emitter molecule F may optionally be a TADF emitter. Alternatively, the emitter molecule F may optionally be a fluorescent and/or phosphorescent emitter molecule which is able to shift the emission spectrum and/or the absorption spectrum of the light-emitting layer EML. Exemplarily, the triplet and/or singlet excitons may be transferred from the emitter molecule according to the invention E to the emitter molecule F before relaxing to the ground state S0 by emitting light typically red-shifted in comparison to the light emitted by emitter molecule E. Optionally, the emitter molecule F may also provoke two-photon effects (i.e., the absorption of two photons of half the energy of the absorption maximum).

Optionally, an optoelectronic device (e.g., an OLED) may, for example, be an essentially white optoelectronic device. For example, such white optoelectronic device may comprise at least one (deep) blue emitter molecule and one or more emitter molecules emitting green and/or red light. Then, there may also be energy transmittance between two or more molecules as described above.

As used herein, if not defined more specifically in the particular context, the designation of the colors of emitted and/or absorbed light is as follows:

| | |
|---|---|
| violet: | wavelength range of >380-420 nm; |
| deep blue: | wavelength range of >420-480 nm; |
| sky blue: | wavelength range of >480-500 nm; |
| green: | wavelength range of >500-560 nm; |
| yellow: | wavelength range of >560-580 nm; |
| orange: | wavelength range of >580-620 nm; |
| red: | wavelength range of >620-800 nm. |

With respect to emitter molecules, such colors refer to the emission maximum. Therefore, exemplarily, a deep blue emitter has an emission maximum in the range of from >420 to 480 nm, a sky blue emitter has an emission maximum in the range of from >480 to 500 nm, a green emitter has an emission maximum in a range of from >500 to 560 nm, a red emitter has an emission maximum in a range of from >620 to 800 nm.

A deep blue emitter may preferably have an emission maximum of below 480 nm, more preferably below 470 nm, even more preferably below 465 nm or even below 460 nm. It will typically be above 420 nm, preferably above 430 nm, more preferably above 440 nm or even above 450 nm.

Accordingly, a further aspect of the present invention relates to an OLED, which exhibits an external quantum efficiency at 1000 $cd/m^2$ of more than 8 %, more preferably of more than 10 %, more preferably of more than 13 %, even more preferably of more than 15 % or even more than 20 % and/or exhibits an emission maximum between 420 nm and 500

nm, preferably between 430 nm and 490 nm, more preferably between 440 nm and 480 nm, even more preferably between 450 nm and 470 nm and/or exhibits a LT80 value at 500 cd/$m^2$ of more than 100 h, preferably more than 200 h, more preferably more than 400 h, even more preferably more than 750 h or even more than 1000 h. Accordingly, a further aspect of the present invention relates to an OLED, whose emission exhibits a CIEy color coordinate of less than 0.45, preferably less than 0.30, more preferably less than 0.20 or even more preferably less than 0.15 or even less than 0.10.

A further aspect of the present invention relates to an OLED, which emits light at a distinct color point. According to the present invention, the OLED emits light with a narrow emission band (small full width at half maximum (FWHM)). In one aspect, the OLED according to the invention emits light with a FWHM of the main emission peak of less than 0.50 eV, preferably less than 0.48 eV, more preferably less than 0.45 eV, even more preferably less than 0.43 eV or even less than 0.40 eV.

A further aspect of the present invention relates to an OLED, which emits light with CIEx and CIEy color coordinates close to the CIEx (= 0.131) and CIEy (= 0.046) color coordinates of the primary color blue (CIEx = 0.131 and CIEy = 0.046) as defined by ITU-R Recommendation BT.2020 (Rec. 2020) and thus is suited for the use in Ultra High Definition (UHD) displays, e.g. UHD-TVs. Accordingly, a further aspect of the present invention relates to an OLED, whose emission exhibits a CIEx color coordinate of between 0.02 and 0.30, preferably between 0.03 and 0.25, more preferably between 0.05 and 0.20 or even more preferably between 0.08 and 0.18 or even between 0.10 and 0.15 and/ or a a CIEy color coordinate of between 0.00 and 0.45, preferably between 0.01 and 0.30, more preferably between 0.02 and 0.20 or even more preferably between 0.03 and 0.15 or even between 0.04 and 0.10.

In a further aspect, the invention relates to a method for producing an optoelectronic component. In this case an organic molecule of the invention is used.

The optoelectronic device, in particular the OLED according to the present invention can be produced by any means of vapor deposition and/ or liquid processing. Accordingly, at least one layer is

- prepared by means of a sublimation process,
- prepared by means of an organic vapor phase deposition process,
- prepared by means of a carrier gas sublimation process,
- solution processed or printed.

The methods used to produce the optoelectronic device, in particular the OLED according to the present invention are known in the art. The different layers are individually and successively deposited on a suitable substrate by means of subsequent deposition processes. The individual layers may be deposited using the same or differing deposition methods.

Vapor deposition processes exemplarily comprise thermal (co)evaporation, chemical vapor deposition and physical vapor deposition. For active matrix OLED display, an AMOLED backplane is used as substrate. The individual layer may be processed from solutions or dispersions employing adequate solvents. Solution deposition process exemplarily comprise spin coating, dip coating and jet printing. Liquid processing may optionally be carried out in an inert atmosphere (e.g., in a nitrogen atmosphere) and the solvent may optionally be completely or partially removed by means known in the state of the art.

## Examples

General synthesis scheme I

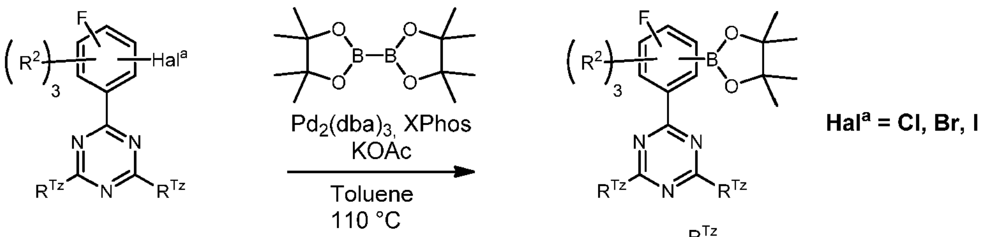


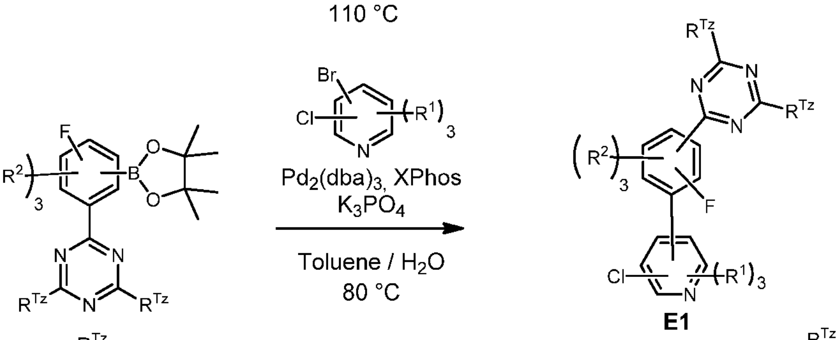


E1

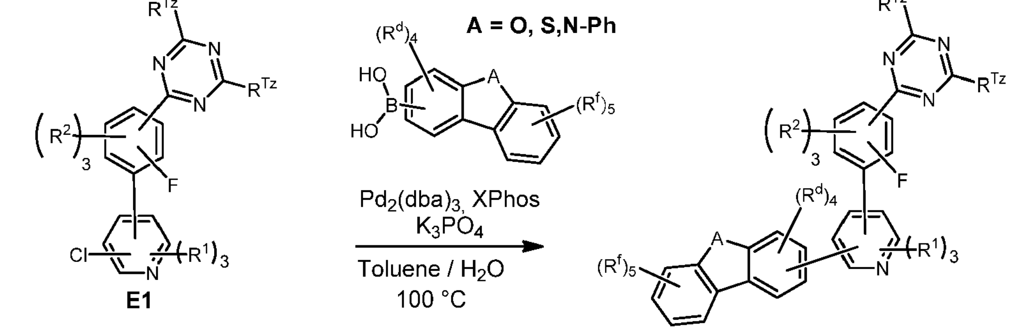


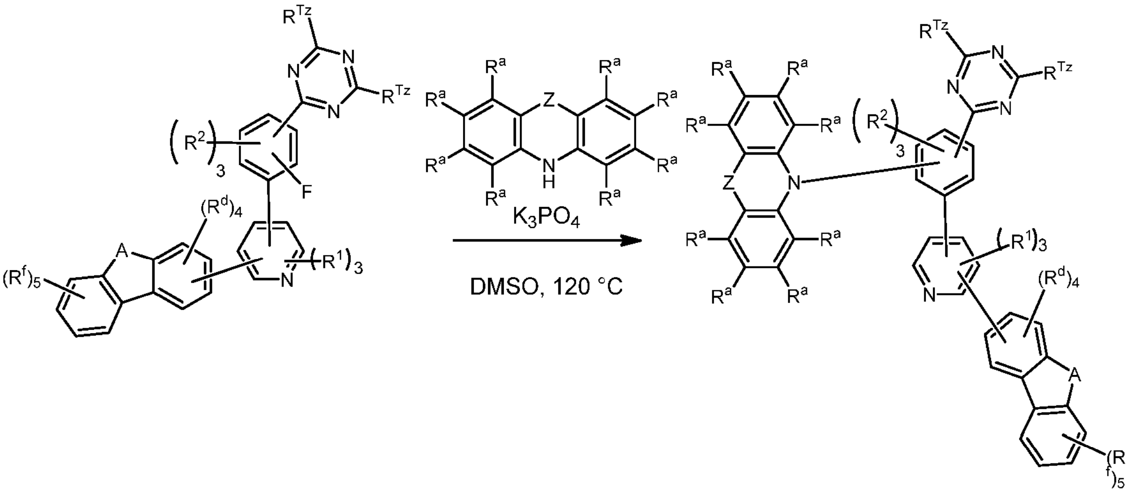


*General procedure for synthesis* ***AAV1***

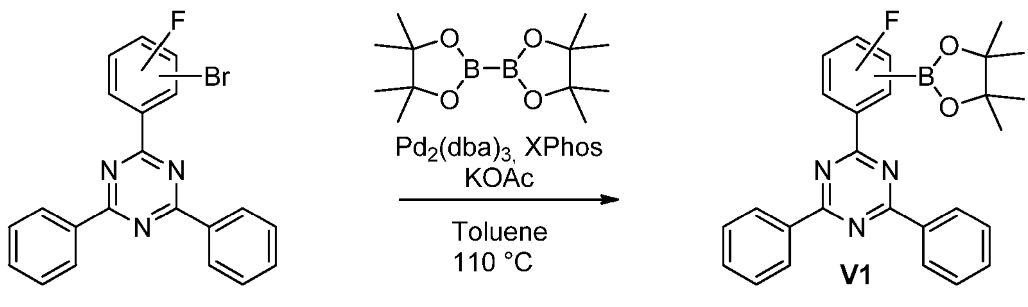


V1

2-(Bromo-fluorophenyl)-4,6-diphenyl-1,3,5-triazine (1.00 equivalent), bis-(pinacolato)diboron (1.5 equivalents, CAS: 73183-34-3), tris(dibenzylideneacetone)dipalladium(0) $Pd_2(dba)_3$ (0.04 equivalents, CAS: 51364-51-3), XPhos (0.08 equivalents, CAS 564483-18-7) and potassium acetate (KOAc, 3.0 equivalents) are stirred under nitrogen atmosphere in dry toluene at 110 °C for 16 h. After cooling down to room temperature (RT) the reaction mixture is extracted with ethyl acetate/brine. The organic phases are collected, washed with brine and dried over $MgSO_4$. The organic solvent is removed, the crude product was washed with cyclohexane and recrystallized from EtOH.

*General procedure for synthesis* ***AAV2:***

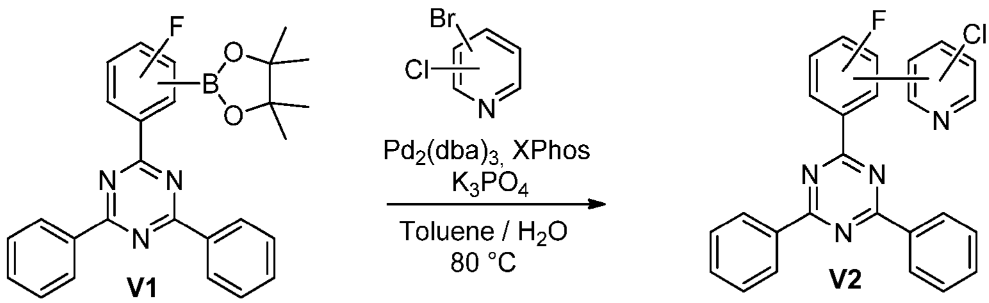


**V1** (1.00 equivalent), bromo-chloro-pyridine (1.1 equivalents), tris(dibenzylideneacetone)dipalladium(0) $Pd_2(dba)_3$ (0.04 equivalents, CAS: 51364-51-3), X-Phos (0.08 equivalents, CAS 564483-18-7) and potassium phosphate ($K_3PO_4$, 3.0 equivalents) are stirred under nitrogen atmosphere in toluene/$H_2O$ (6:1) at 80°C for 16 h. After cooling down to room temperature (RT) the reaction mixture is extracted with ethyl acetate/brine. Organic phases are collected, solvent removed and the crude product is purified by flash chromatography.

*General procedure for synthesis* ***AAV3:***

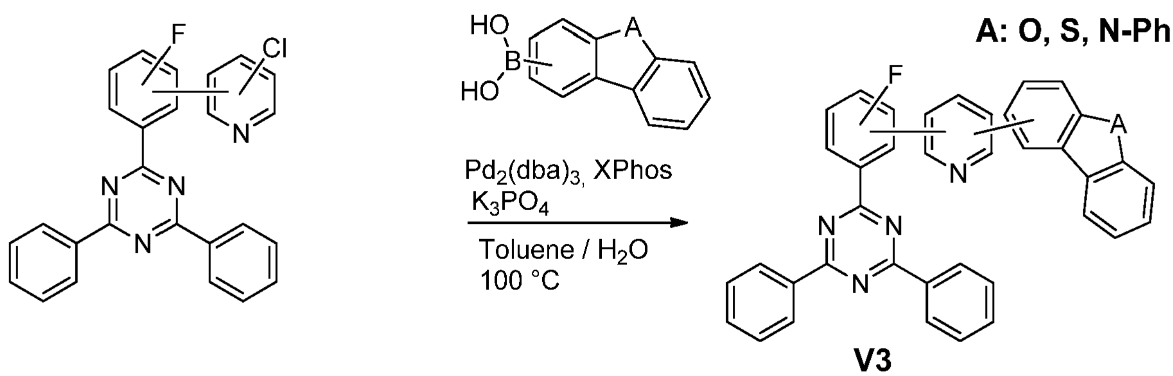


**V3** (1.00 equivalent), (1.00 equivalent), tris(dibenzylideneacetone)dipalladium(0) $Pd_2(dba)_3$ (0.04 equivalents, CAS: 51364-51-3), X-Phos (0.08 equivalents, CAS 564483-18-7) and potassium phosphate ($K_3PO_4$, 3.0 equivalents) are stirred under nitrogen atmosphere in toluene/$H_2O$ (10:1) at 100 °C for 16 h. After cooling down to room temperature (RT) the reaction mixture is extracted with ethyl acetate/brine. The organic phases are collected, washed with brine and dried over $MgSO_4$. The organic solvent is removed and the crude product is purified by flash chromatography and additionally recrystallized form EtOH.

*General procedure for synthesis* ***AAV4:***

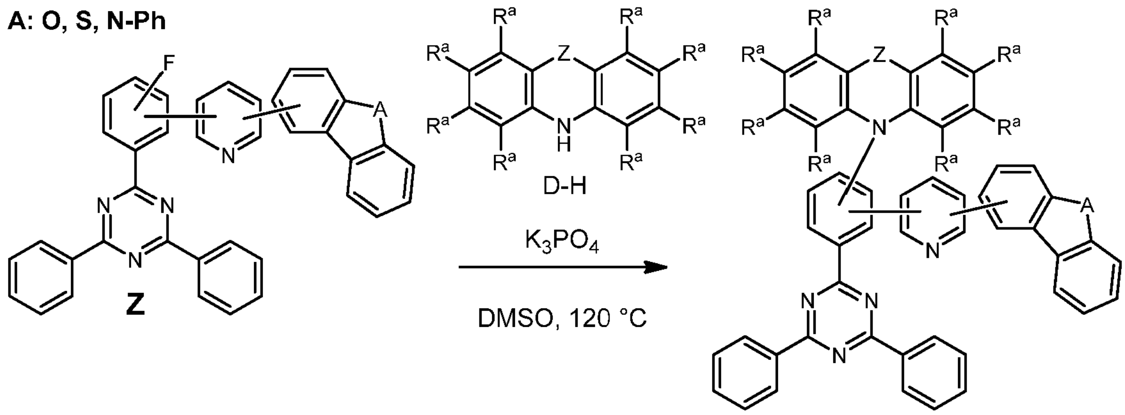


**V3** (1 equivalent), the corresponding donor molecule D-H (1.00 equivalents) and tribasic potassium phosphate (3.00 equivalents) are suspended under nitrogen atmosphere in dry DMSO and stirred at 120 °C (16 h). After cooling down to room temperature (RT) the reaction mixture is extracted with ethyl acetate/brine. Organic phases are collected, washed with brine and dried over $MgSO_4$. The solvent is evaporated under reduced pressure. The crude product is purified by recrystallization or by flash chromatography.

In particular, the donor molecule D-H is a 3,6-substituted carbazole (e.g., 3,6-dimethylcarbazole, 3,6-diphenylcarbazole, 3,6-di-tert-butylcarbazole), a 2,7-substituted carbazole (e.g., 2,7-dimethylcarbazole, 2,7-diphenylcarbazole, 2,7-di-tert-butylcarbazole), a 1,8-substituted carbazole (e.g., 1,8-dimethylcarbazole, 1,8-diphenylcarbazole, 1,8-di-tert-butylcarbazole), a 1-substituted carbazole (e.g., 1-methylcarbazole, 1-phenylcarbazole, 1-tert-butylcarbazole), a 2-substituted carbazole (e.g., 2-methylcarbazole, 2-phenylcarbazole, 2-tert-butylcarbazole), or a 3-substituted carbazole (e.g., 3-methylcarbazole, 3-phenylcarbazole, 3-tert-butylcarbazole).

For example, a halogen-substituted carbazole, particularly 3-bromocarbazole, can be used as D-H.

In a subsequent reaction, a boronic acid ester functional group or boronic acid functional group may, for example, be introduced at the position of the one or more halogen substituents, which was introduced via D-H, to yield the corresponding carbazol-3-ylboronic acid ester or carbazol-3-ylboronic acid, e.g., via the reaction with bis(pinacolato) diboron (CAS No. 73183-34-3). Subsequently, one or more substituents $R^a$ may be introduced in place of the boronic acid ester group or the boronic acid group via a coupling reaction with the corresponding halogenated reactant $R^a$-Hal, preferably $R^a$-Cl and $R^a$-Br.

Alternatively, one or more substituents $R^a$ may be introduced at the position of the one or more halogen substituents, which was introduced via D-H, via the reaction with a boronic acid of the substituent $R^a$ [$R^a$-$B(OH)_2$] or a corresponding boronic acid ester.

*Cyclic voltammetry*

Cyclic voltammograms are measured from solutions having concentration of $10^{-3}$ mol/L of the organic molecules in dichloromethane or a suitable solvent and a suitable supporting electrolyte (e.g. 0.1 mol/L of tetrabutylammonium hexafluorophosphate). The measurements are conducted at room temperature under nitrogen atmosphere with a three-electrode assembly (Working and counter electrodes: Pt wire, reference electrode: Pt wire) and calibrated using $FeCp_2/FeCp_2^+$ as internal standard. The HOMO data was corrected using ferrocene as internal standard against a saturated calomel electrode (SCE).

*Density functional theory calculation*

Molecular structures are optimized employing the BP86 functional and the resolution of identity approach (RI). Excitation energies are calculated using the (BP86) optimized structures employing Time-Dependent DFT (TD-DFT) methods. Orbital and excited state energies are calculated with the B3LYP functional. Def2-SVP basis sets (and a m4-grid for numerical integration are used. The Turbomole program package is used for all calculations.

*Photophysical measurements*

Sample pretreatment: Spin-coating
Apparatus: Spin150, SPS euro.

The sample concentration is 10 mg/ml, dissolved in a suitable solvent.

Program: 1) 3 s at 400 U/min; 20 s at 1000 U/min at 1000 Upm/s. 3) 10 s at 4000 U/min at 1000 Upm/s. After coating, the films are dried at 70 °C for 1 min.

Photoluminescence spectroscopy and TCSPC (*Time-correlated single-photon counting*) Steady-state emission spectroscopy is measured by a Horiba Scientific, Modell FluoroMax-4 equipped with a 150 W Xenon-Arc lamp, excitation- and emissions monochromators and a Hamamatsu R928 photomultiplier and a time-correlated single-photon counting option. Emissions and excitation spectra are corrected using standard correction fits.

Excited state lifetimes are determined employing the same system using the TCSPC method with FM-2013 equipment and a Horiba Yvon TCSPC hub.

Excitation sources:

NanoLED 370 (wavelength: 371 nm, puls duration: 1,1 ns)
NanoLED 290 (wavelength: 294 nm, puls duration: <1 ns)
SpectraLED 310 (wavelength: 314 nm)
SpectraLED 355 (wavelength: 355 nm).

Data analysis (exponential fit) is done using the software suite DataStation and DAS6 analysis software. The fit is specified using the chi-squared-test.

Photoluminescence quantum yield measurements

For photoluminescence quantum yield (PLQY) measurements an *Absolute PL Quantum Yield Measurement* C9920-03G system (*Hamamatsu Photonics*) is used. Quantum yields and CIE coordinates are determined using the software U6039-05 version 3.6.0.

Emission maxima are given in nm, quantum yields Φ in % and CIE coordinates as x,y values. PLQY is determined using the following protocol:

1) Quality assurance: Anthracene in ethanol (known concentration) is used as reference
2) Excitation wavelength: the absorption maximum of the organic molecule is determined and the molecule is excited using this wavelength
3) Measurement
Quantum yields are measured for sample of solutions or films under nitrogen atmosphere. The yield is calculated using the equation:

$$\Phi_{PL} = \frac{n_{photon}, emited}{n_{photon}, absorbed} = \frac{\int \frac{\lambda}{hc}\left[Int_{emitted}^{sample}(\lambda) - Int_{absorbed}^{sample}(\lambda)\right]d\lambda}{\int \frac{\lambda}{hc}\left[Int_{emitted}^{reference}(\lambda) - Int_{absorbed}^{reference}(\lambda)\right]d\lambda}$$

wherein $n_{photon}$ denotes the photon count and Int. the intensity.

**Production and characterization of optoelectronic devices**

OLED devices comprising organic molecules according to the invention can be produced via vacuum-deposition methods. If a layer contains more than one compound, the weight-percentage of one or more compounds is given in %. The total weight-percentage values amount to 100 %, thus if a value is not given, the fraction of this compound equals to the difference between the given values and 100 %.

The not fully optimized OLEDs are characterized using standard methods and measuring electroluminescence spectra, the external quantum efficiency (in %) in dependency on the intensity, calculated using the light detected by the photodiode, and the current. The OLED device lifetime is extracted from the change of the luminance during operation at constant current density. The LT50 value corresponds to the time, where the measured luminance decreased to 50 % of the initial luminance, analogously LT80 corresponds to the time point, at which the measured luminance decreased to 80 % of the initial luminance, LT 95 to the time point, at which the measured luminance decreased to 95 % of the initial luminance etc.

Accelerated lifetime measurements are performed (e.g. applying increased current densities). Exemplarily LT80

values at 500 cd/m2 are determined using the following equation:

$$\mathrm{LT80}\left(500\,\frac{cd^2}{m^2}\right) = \mathrm{LT80}(L_0)\left(\frac{L_0}{500\,\frac{cd^2}{m^2}}\right)^{1.6}$$

wherein $L_0$ denotes the initial luminance at the applied current density.

The values correspond to the average of several pixels (typically two to eight), the standard deviation between these pixels is given.

**Example 1**

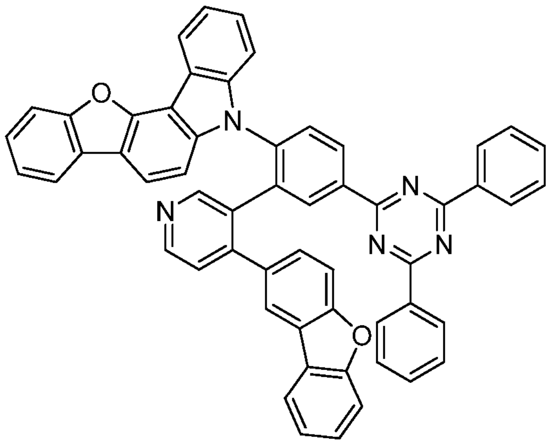

Example 1 was synthesized according to ***AAV1,*** wherein 2-(4-fluoro-3-bromo-phenyl)-4,6-diphenyl-1,3,5-triazine was used as reactant (yield: 62%),
***AAV2,*** wherein 3-bromo-4-chloropyridine (CAS 36953-42-1) was used as reactant (yield: 81%),
***AAV3,*** wherein Dibenzo[b,d]furan-2-ylboronic acid (CAS 402936-15-6) was used as reactant (yield: 66%),
and ***AAV4*** (77 % yield).
HPLC-MS: 15.87 min (807.52 m/z (100%))

Figure 1 depicts the emission spectrum of example **1** (10 % by weight in PMMA). The emission maximum ($\lambda_{max}$) is at 457 nm. The photoluminescence quantum yield (PLQY) is 83 %, the full width at half maximum (FWHM) is 0.43 eV. The resulting $CIE_x$ coordinate is determined at 0.16 and the CIEy coordinate at 0.15.

**Example 2**

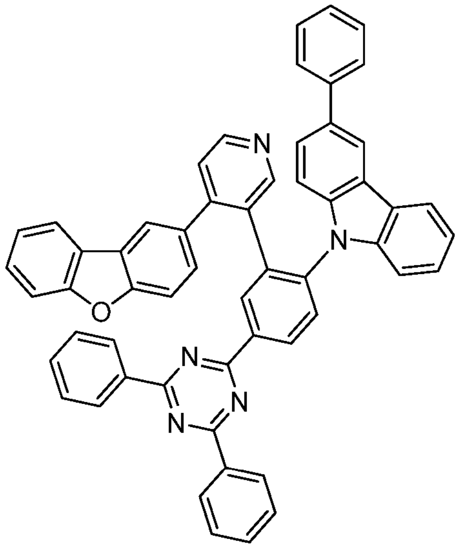

Example **2** was synthesized according to ***AAV1,*** wherein 2-(4-fluoro-3-bromo-phenyl)-4,6-diphenyl-1,3,5-triazine was used as reactant (yield: 62%),
***AAV2,*** wherein 3-bromo-4-chloropyridine (CAS 36953-42-1) was used as reactant (yield: 81%),
***AAV3,*** wherein Dibenzo[b,d]furan-2-ylboronic acid (CAS 402936-15-6) was used as reactant (yield: 66%),
and ***AAV4*** (90 % yield).
HPLC-MS: 11.77 min (793.35 m/z (100%))

Figure 2 shows the emission spectrum of example **2** (10 % by weight in PMMA). The emission maximum ($\lambda_{max}$) is at 454 nm. The photoluminescence quantum yield (PLQY) is 86 %, the full width at half maximum (FWHM) is 0.43 eV. The resulting $CIE_x$ coordinate is determined at 0.16 and the CIEy coordinate at 0.15.

**Additional Examples of Organic Molecules of the Invention**

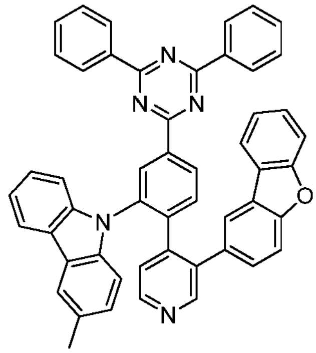

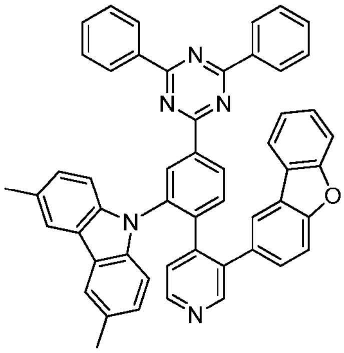

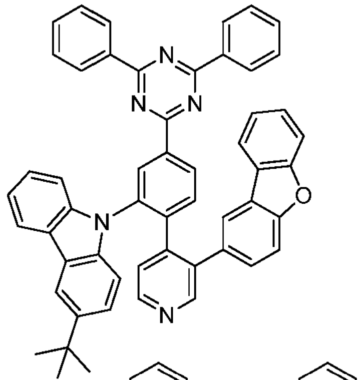

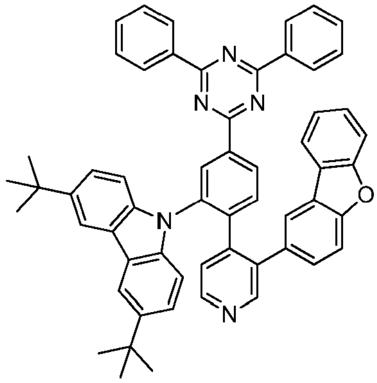

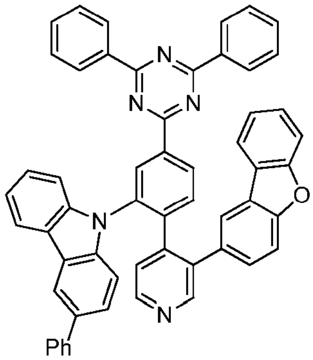

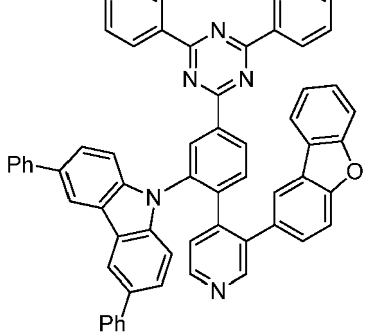

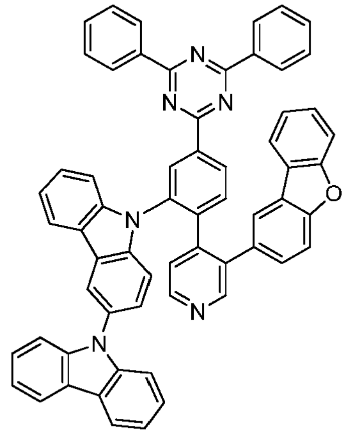

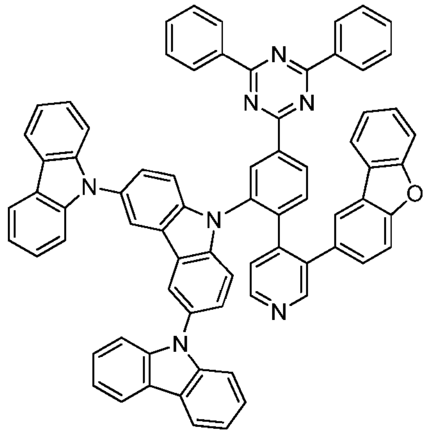

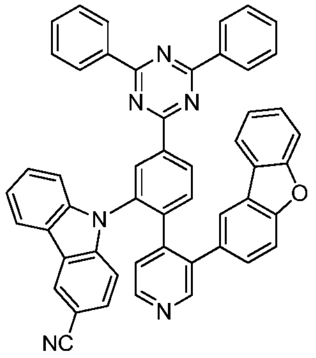

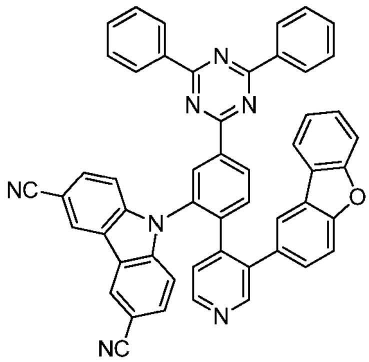

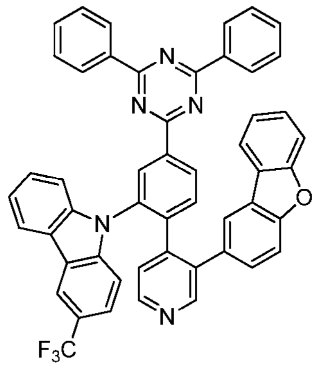

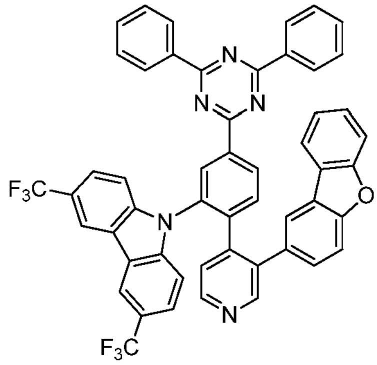

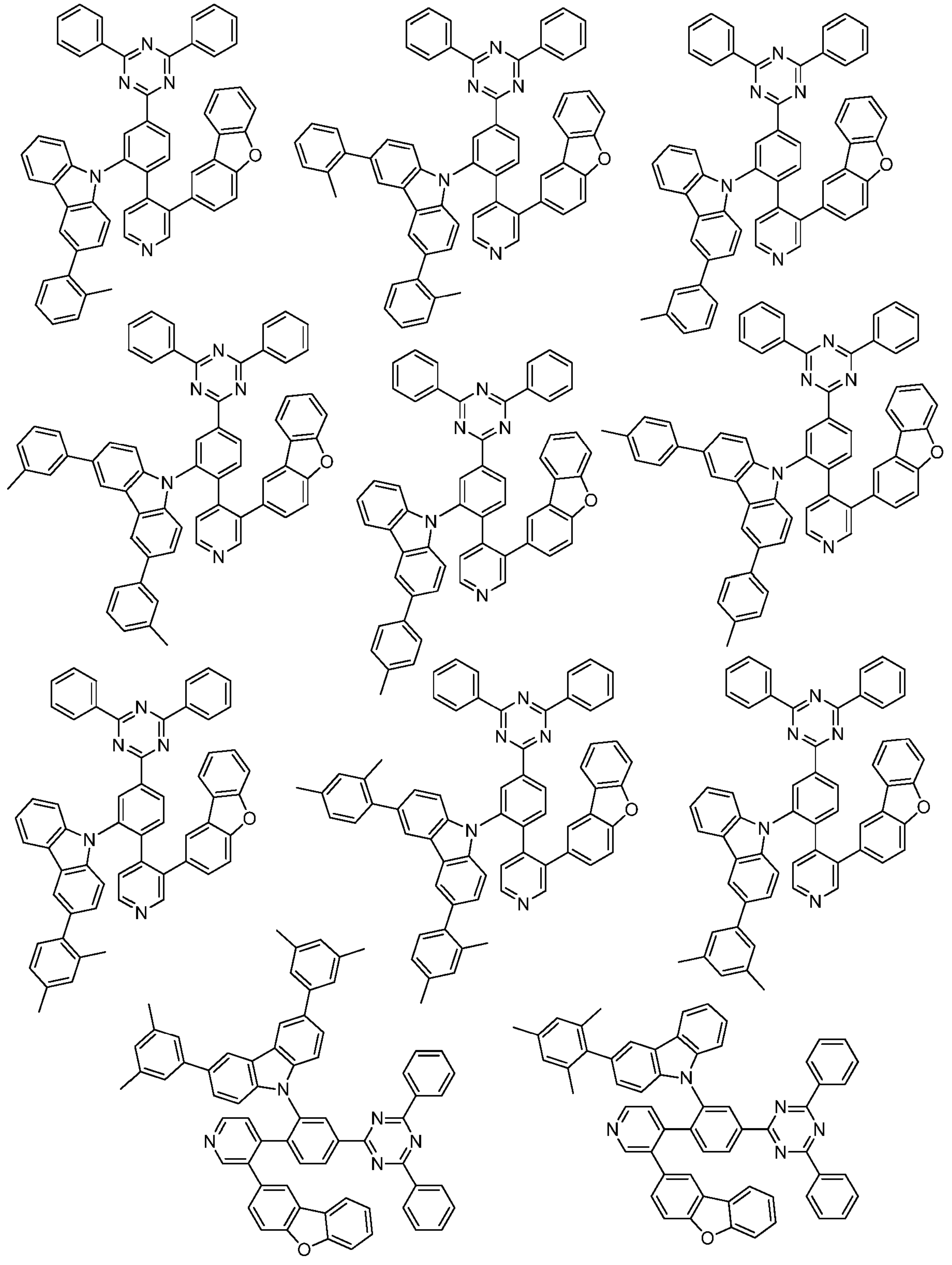

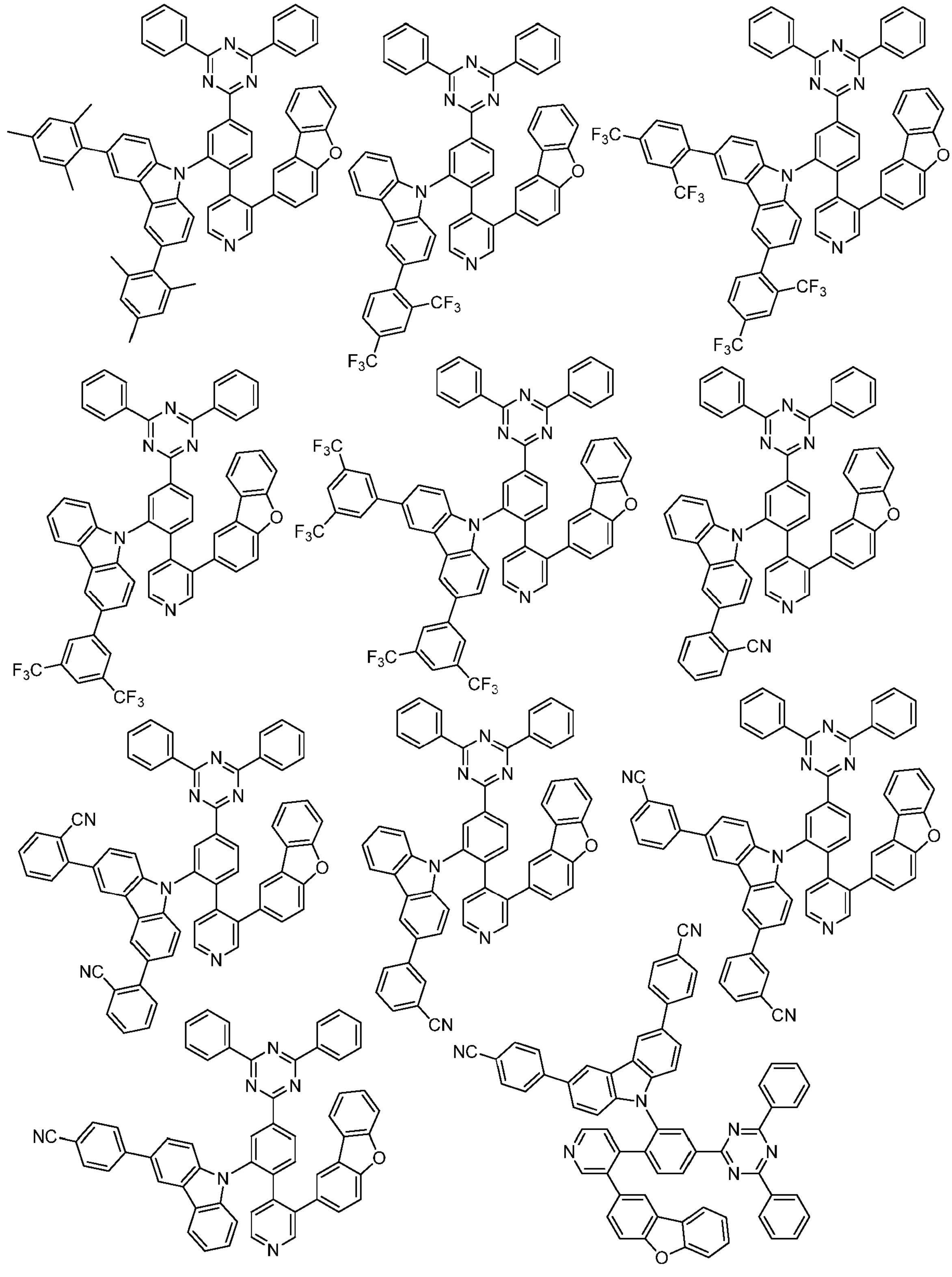

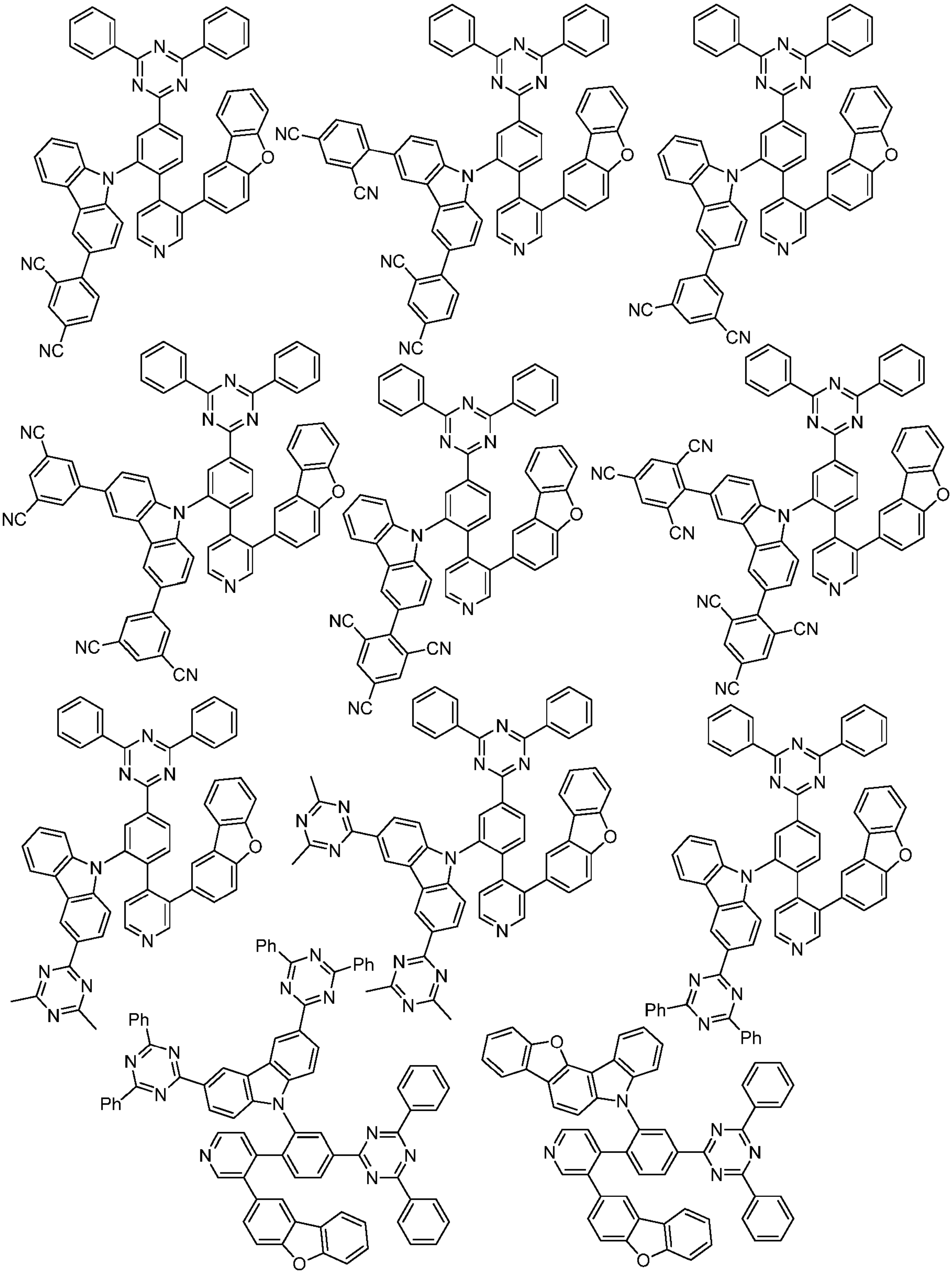

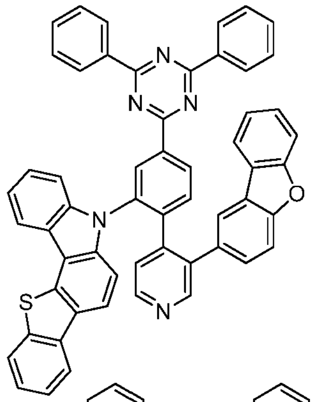
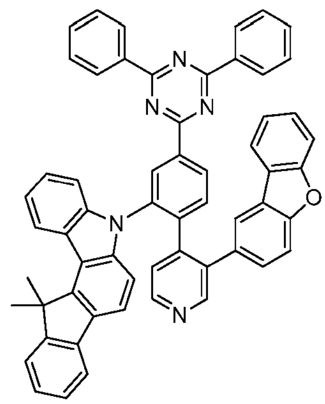
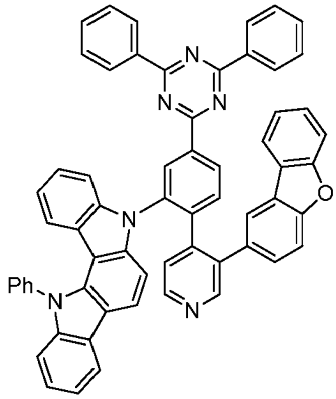
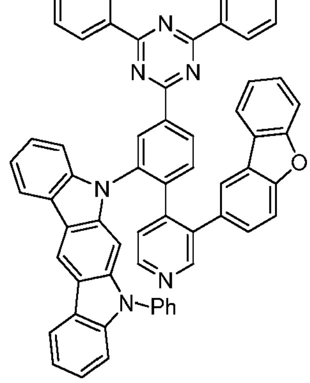
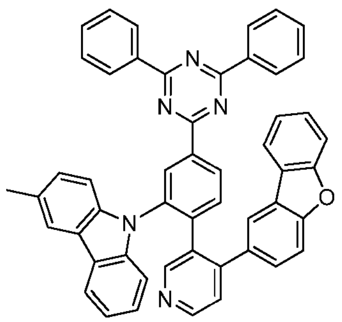
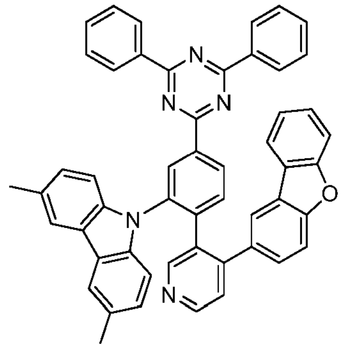
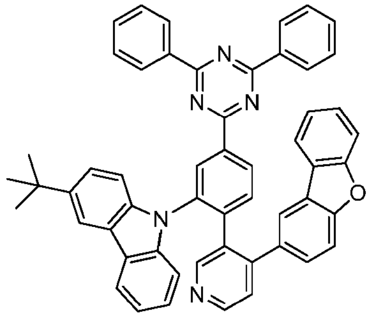
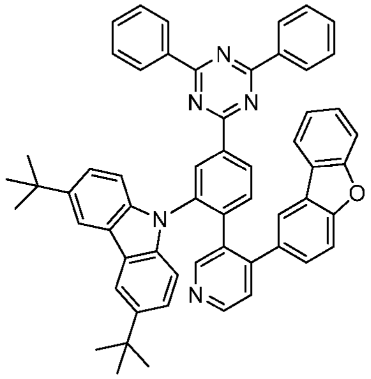
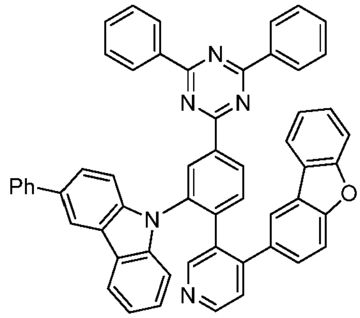
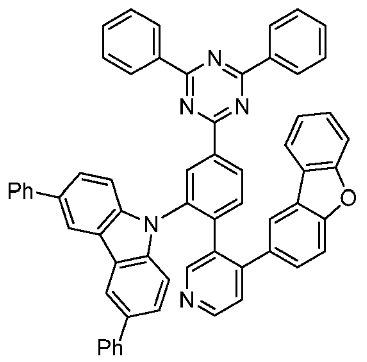
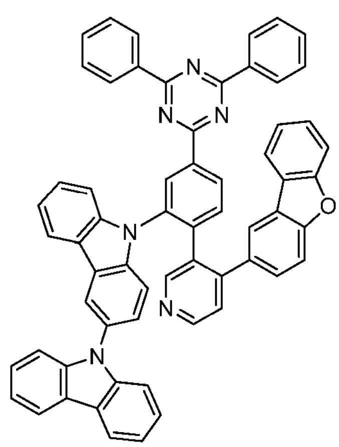
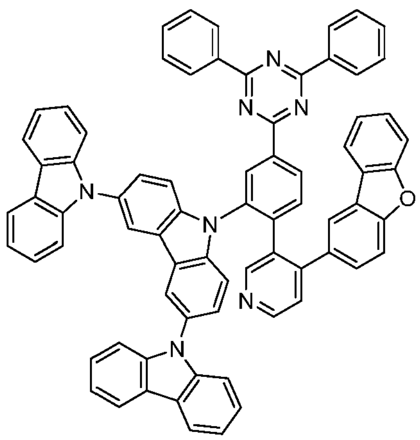

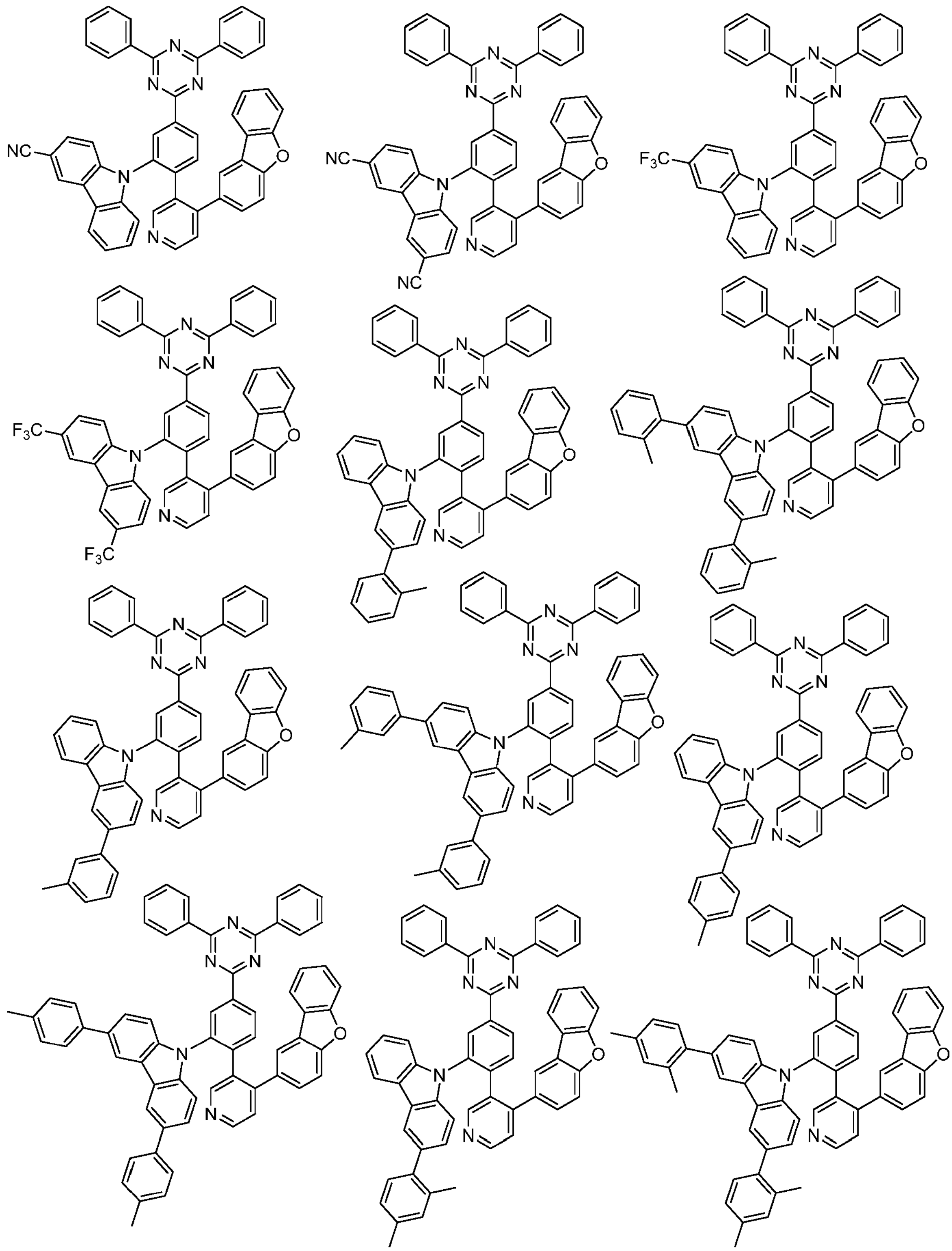

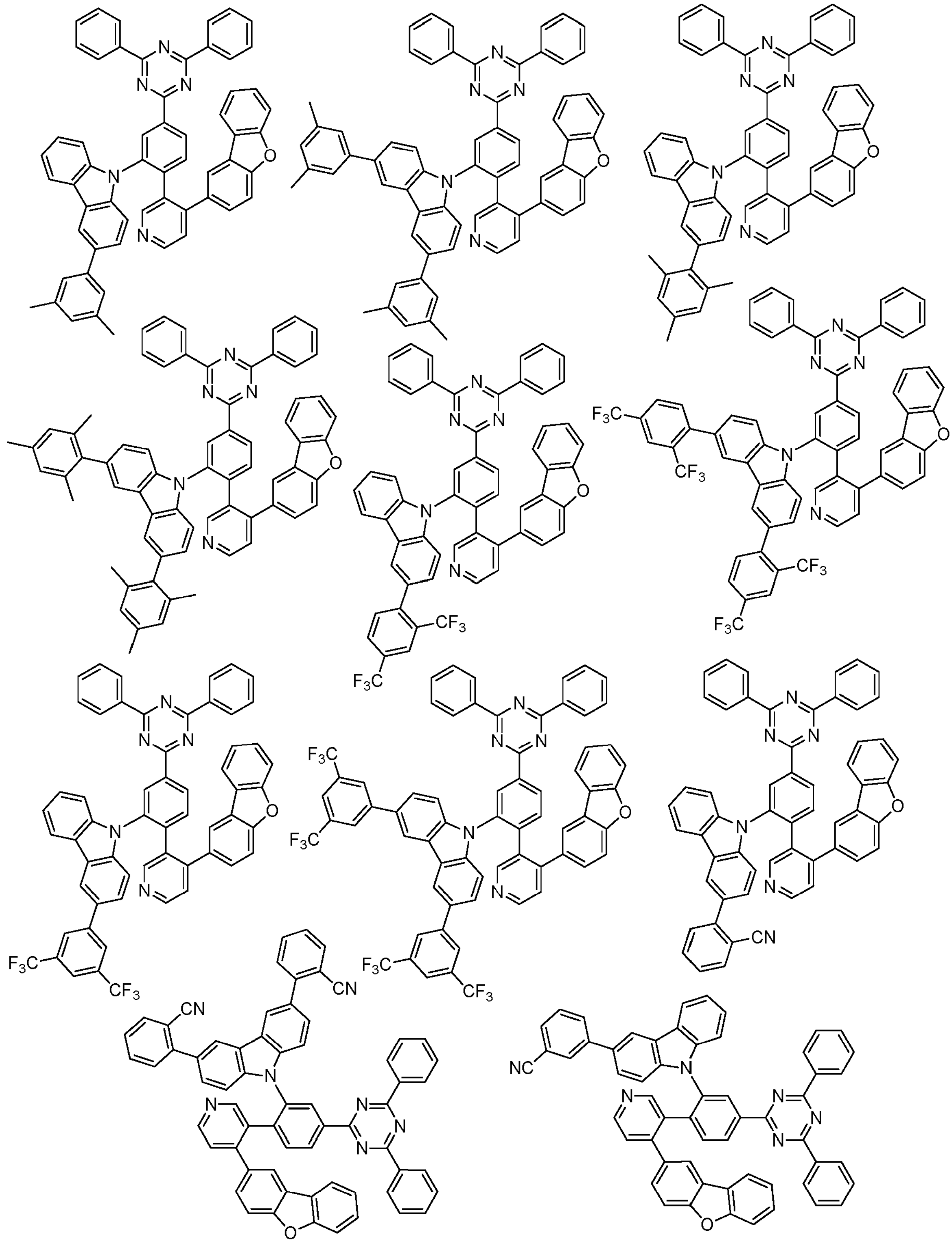

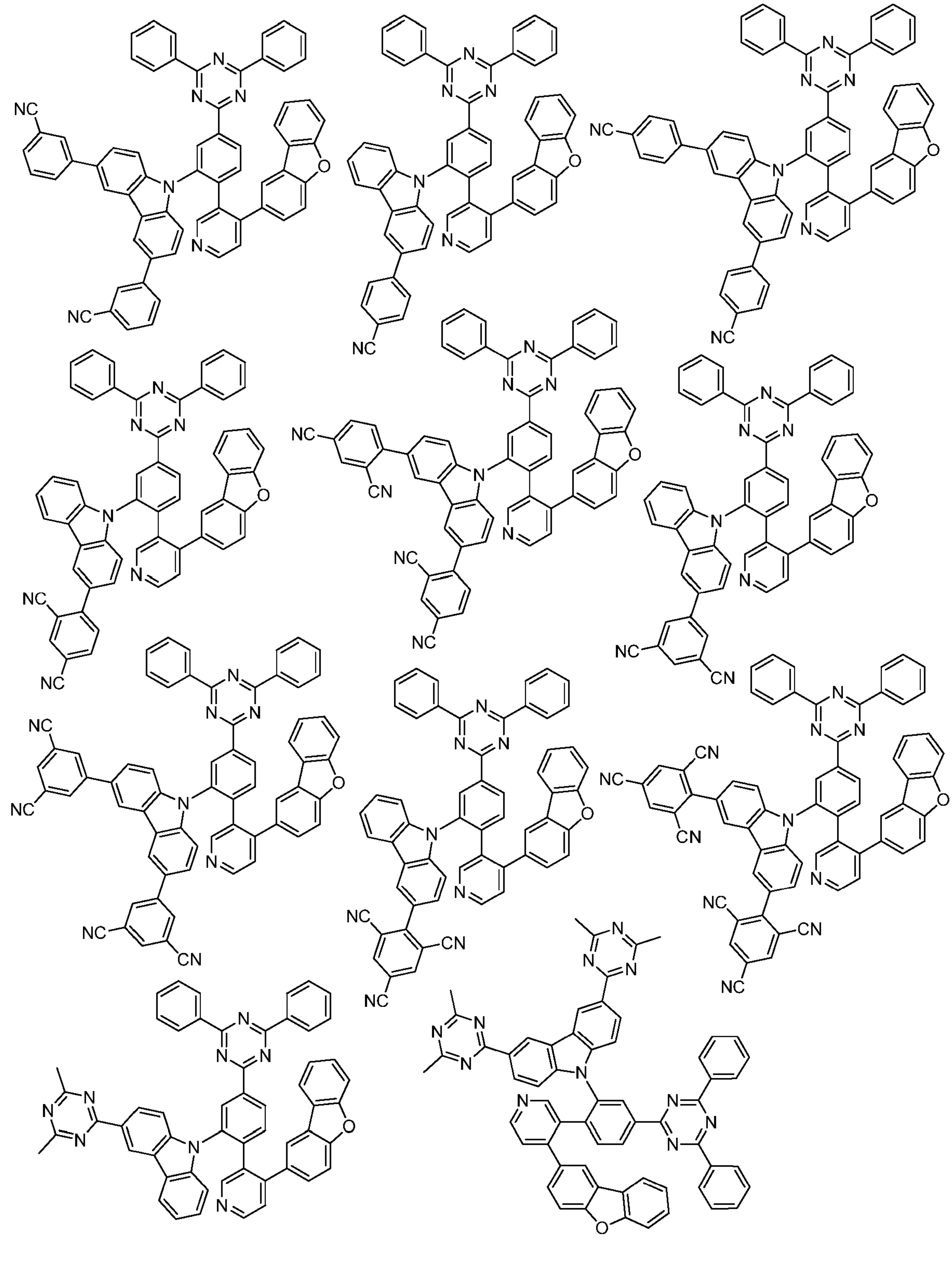

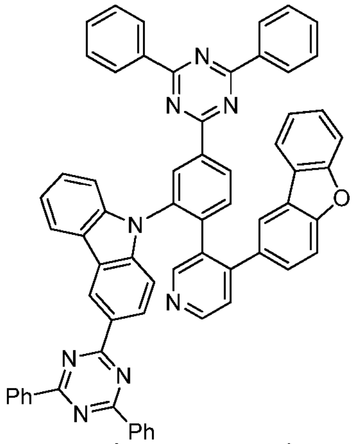
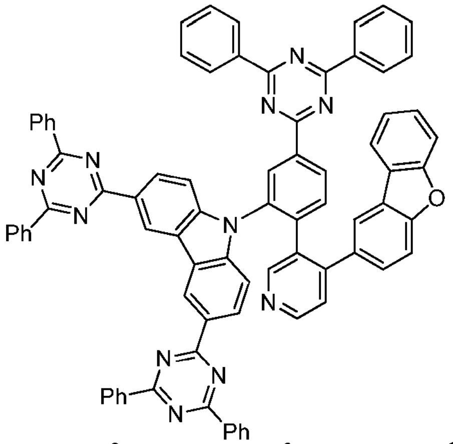
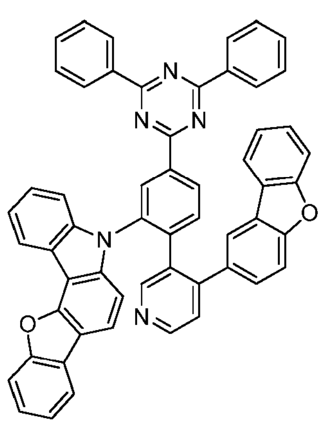

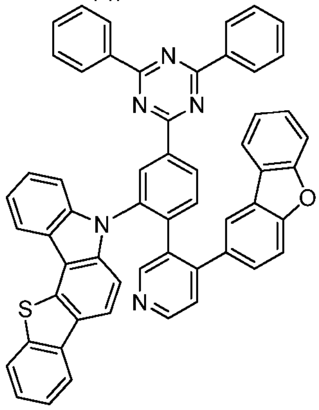
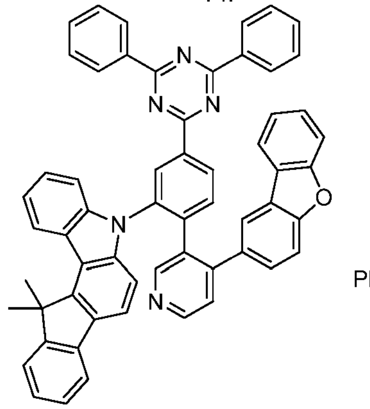
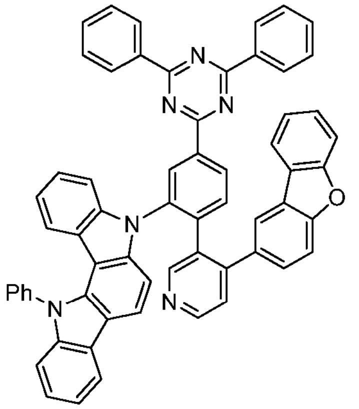

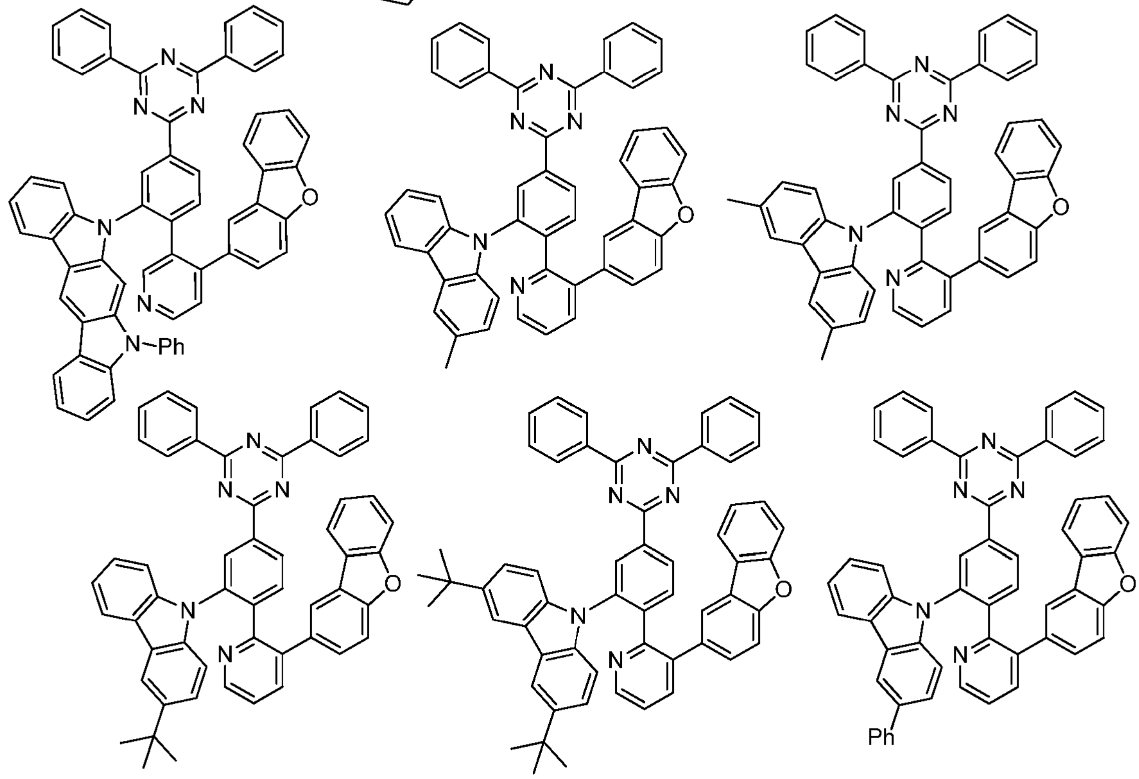

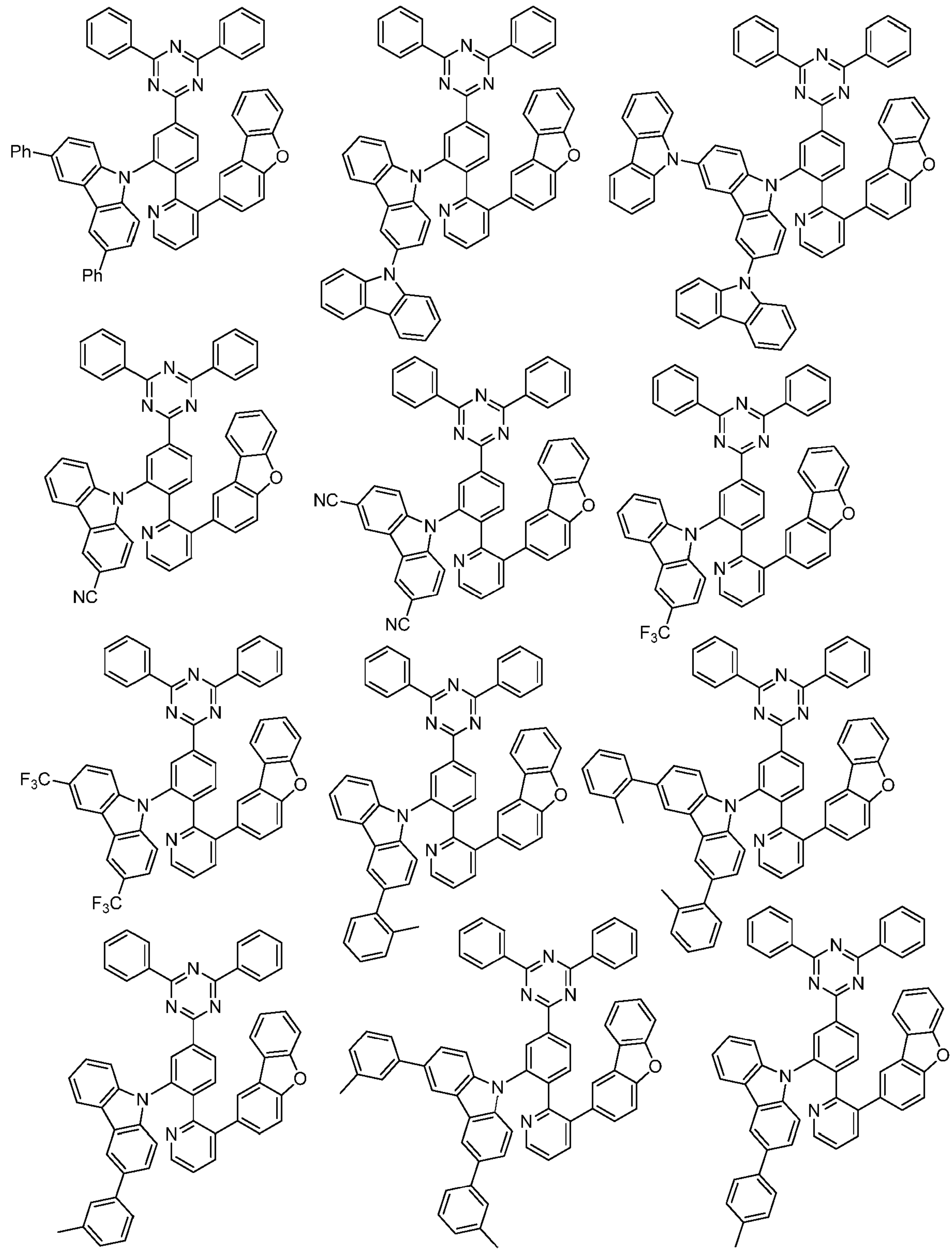

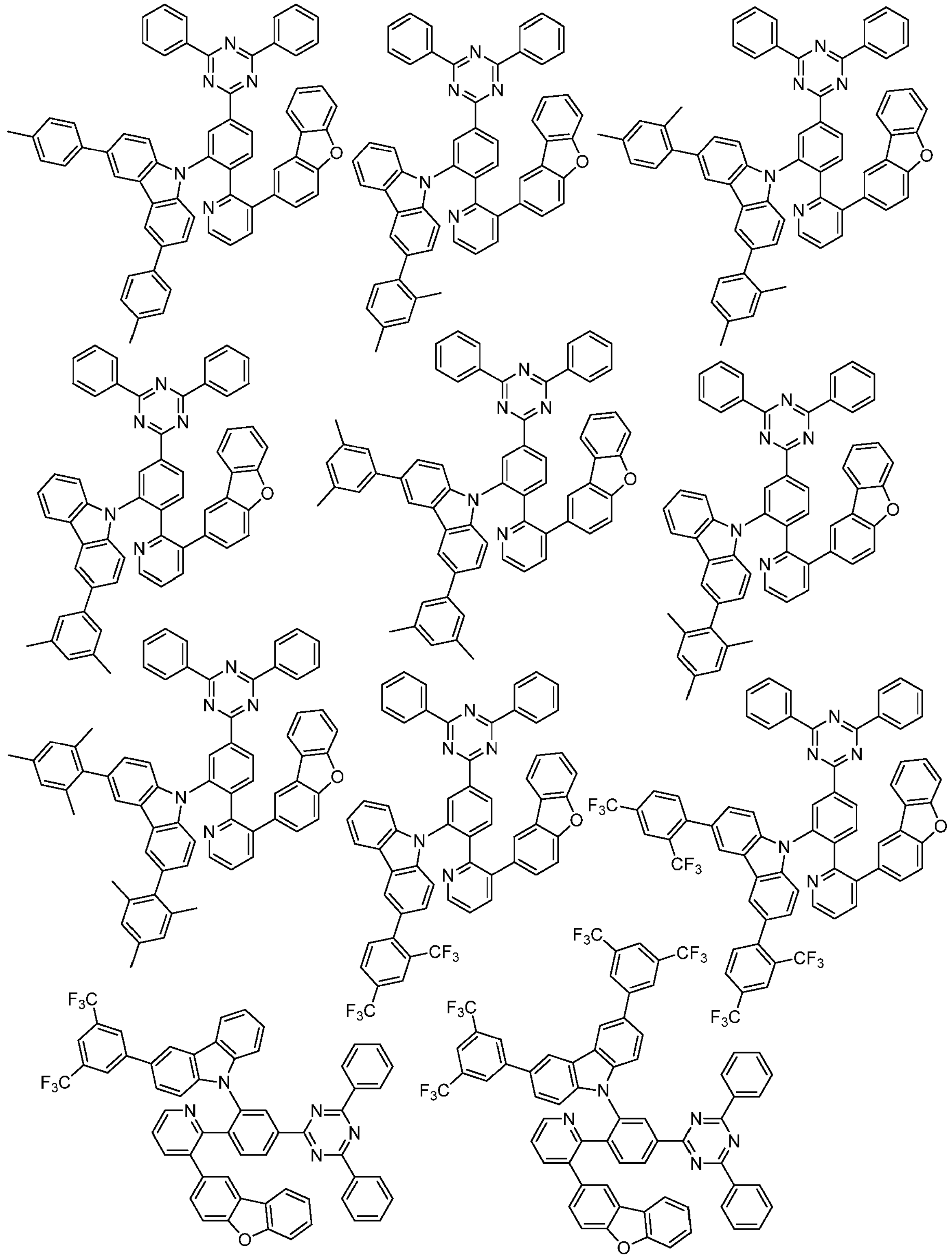

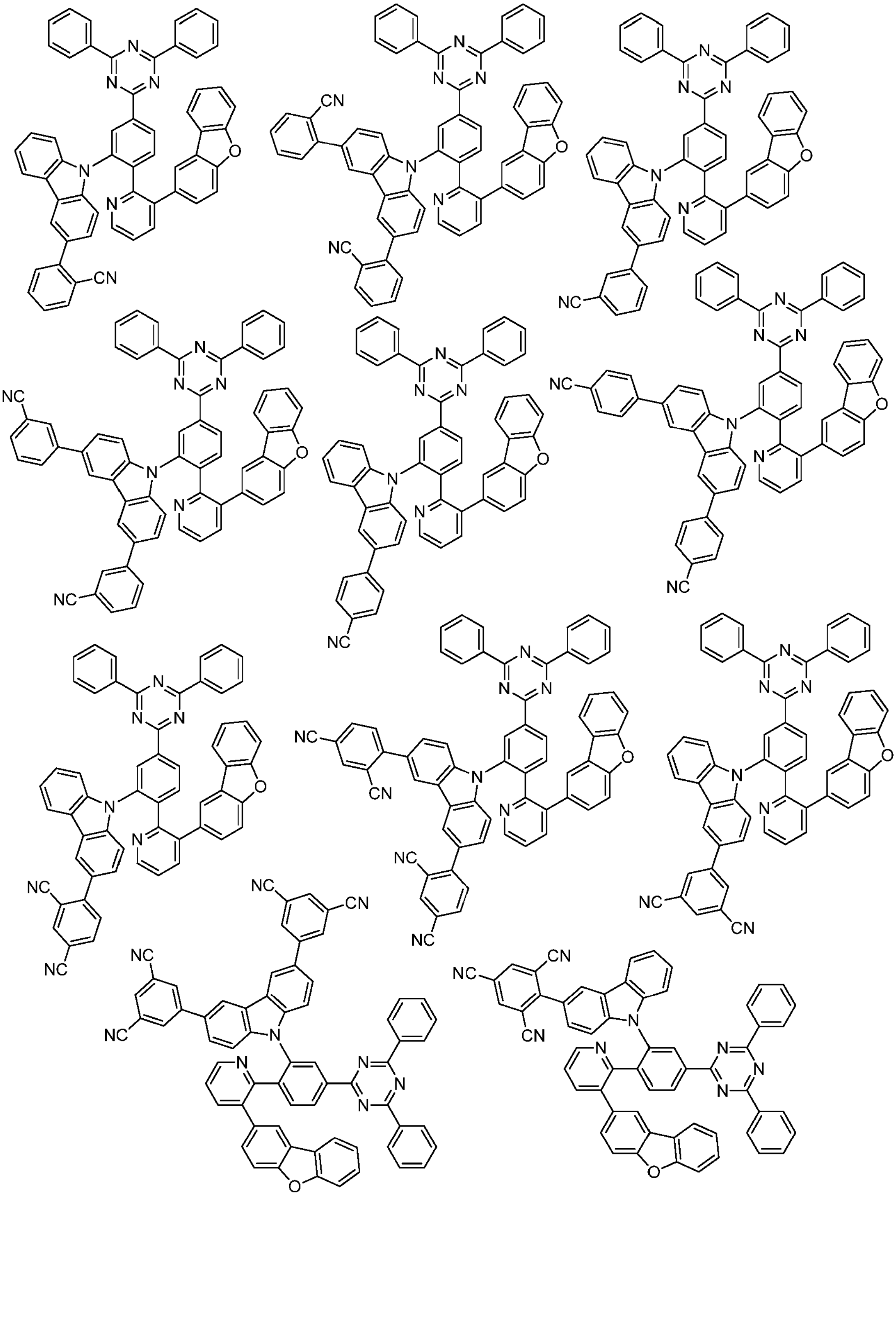

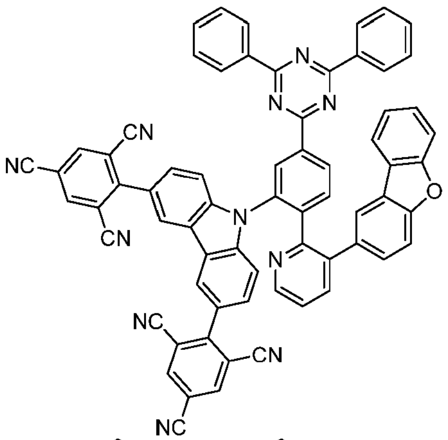

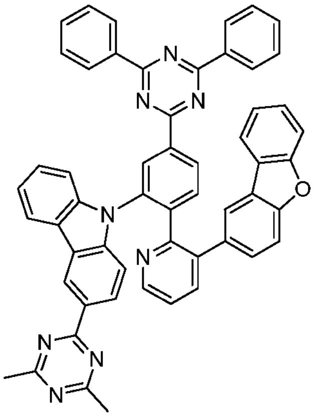

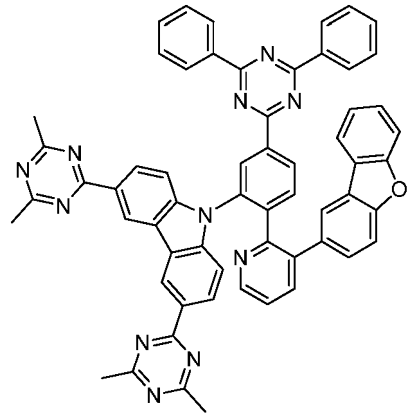

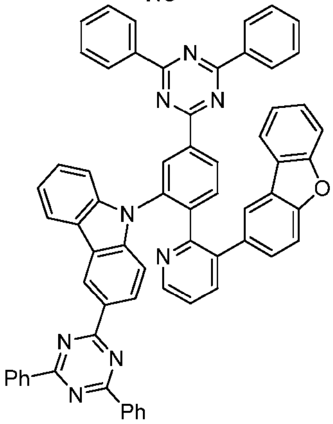

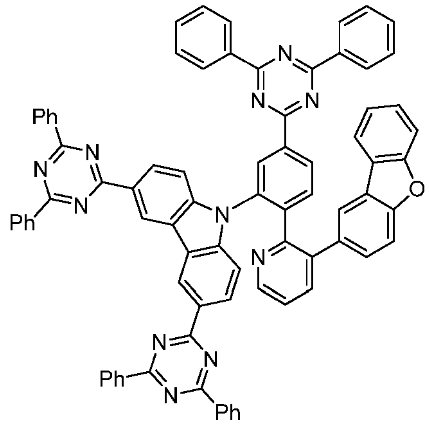

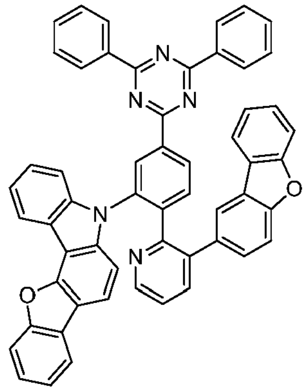

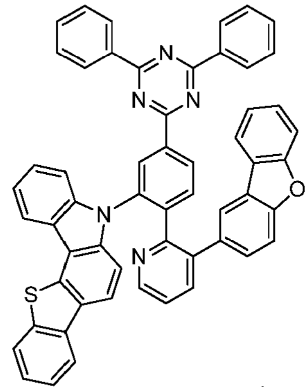

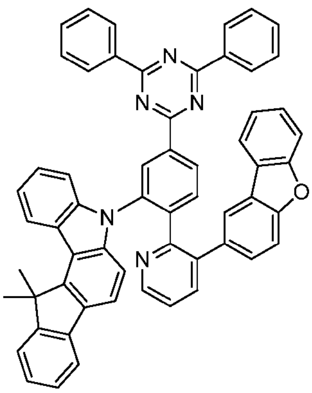

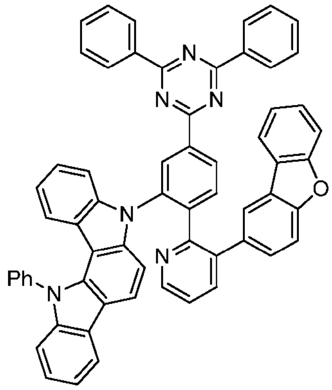

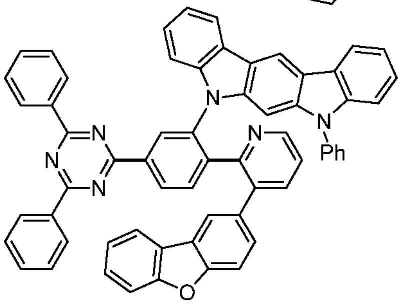

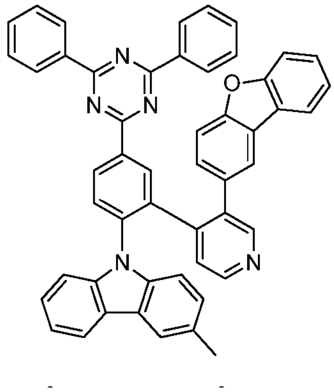

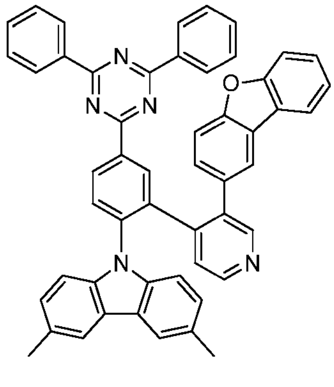

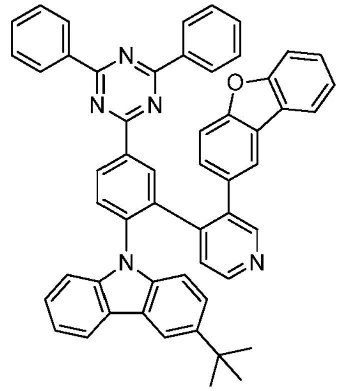

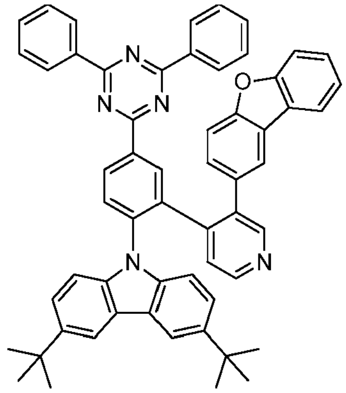

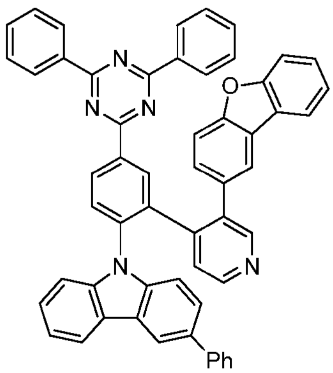

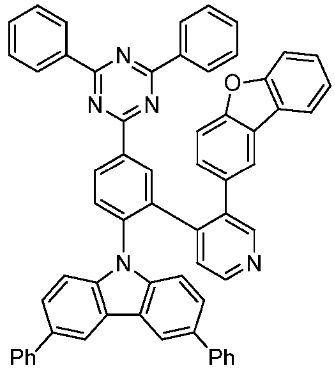

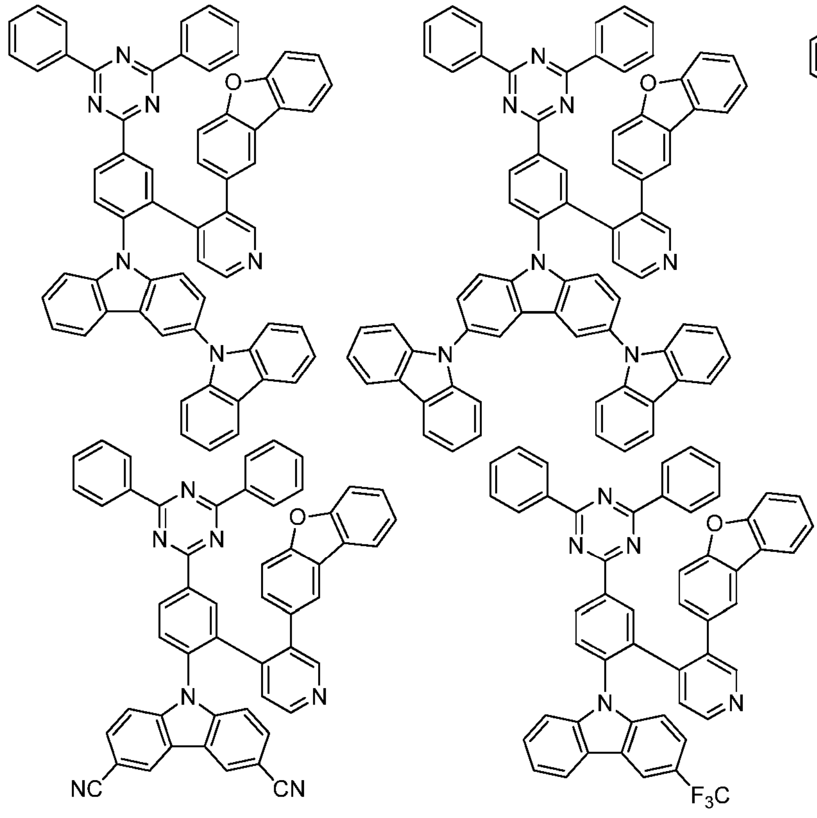

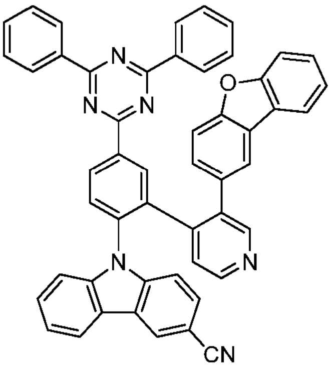

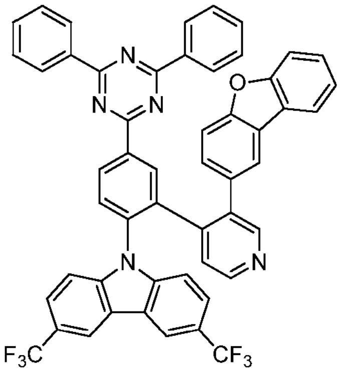

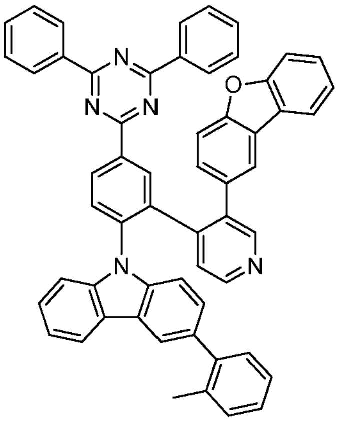

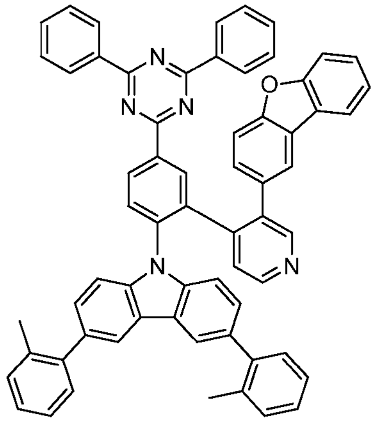

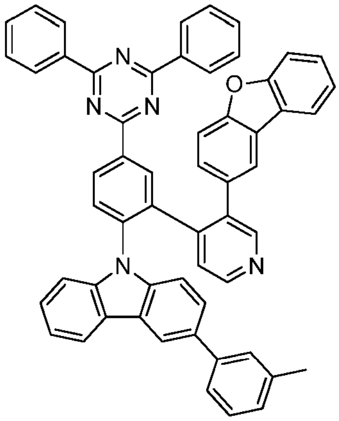

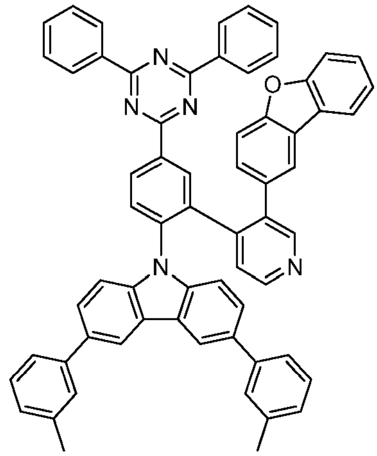

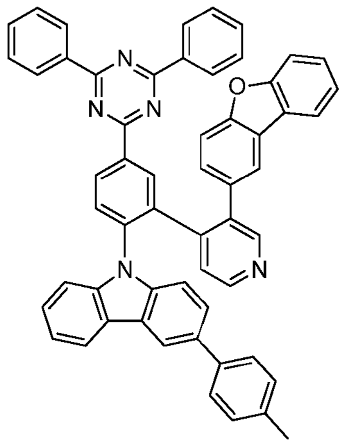

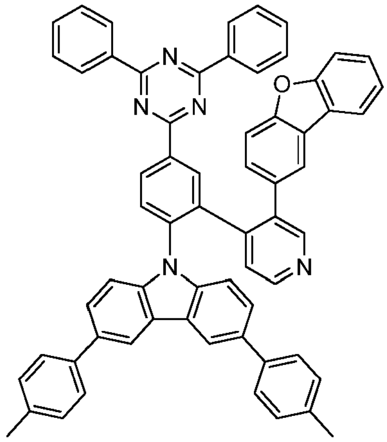

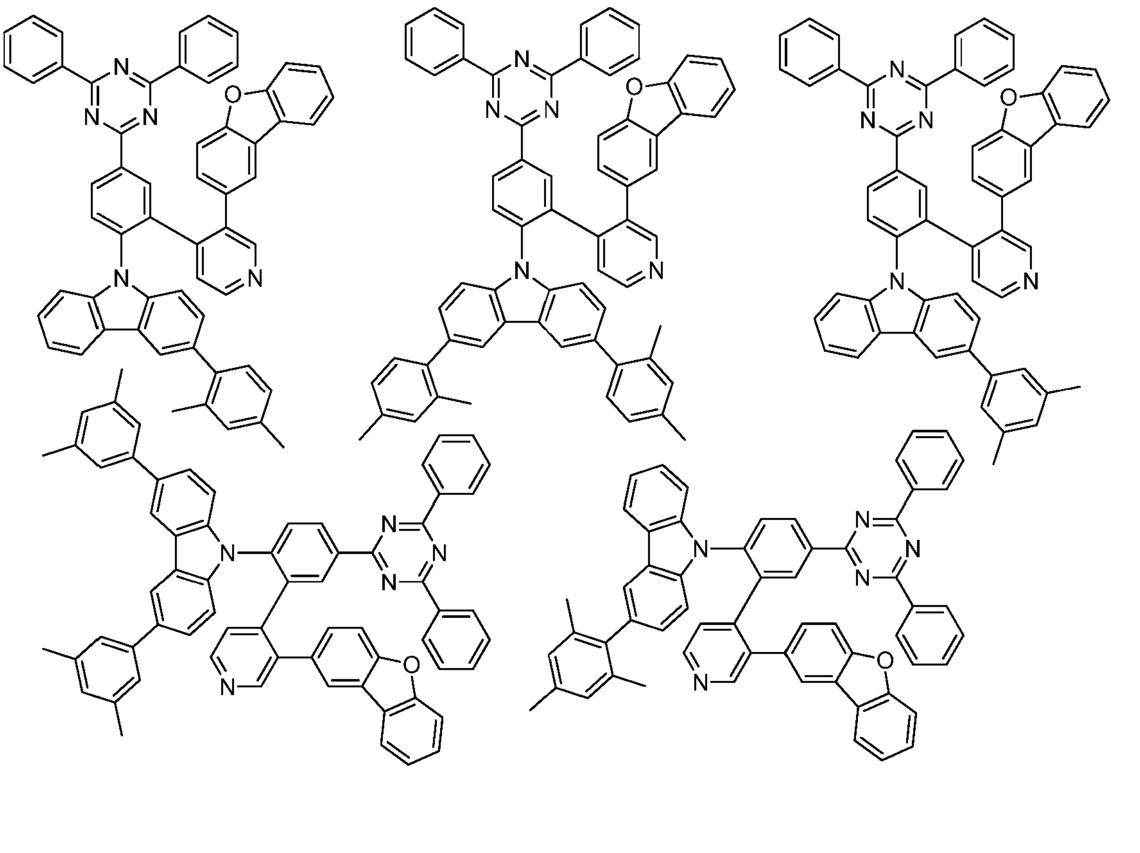

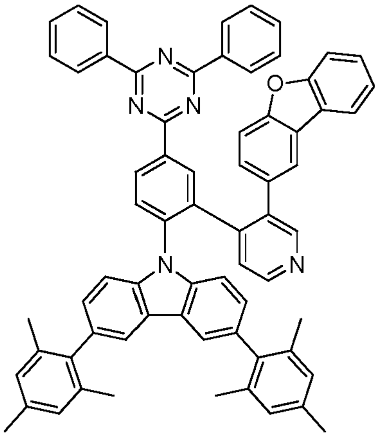

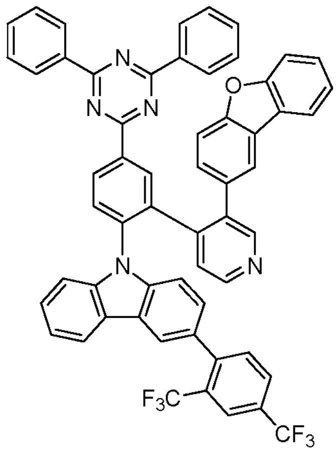

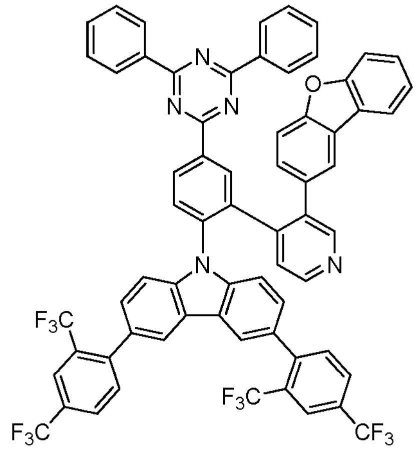

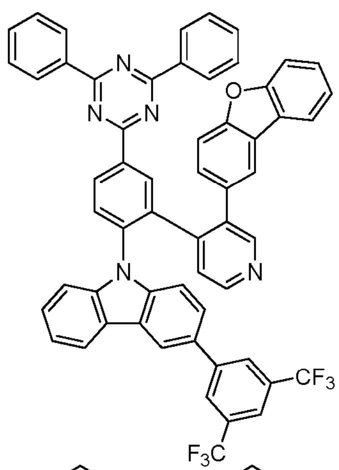

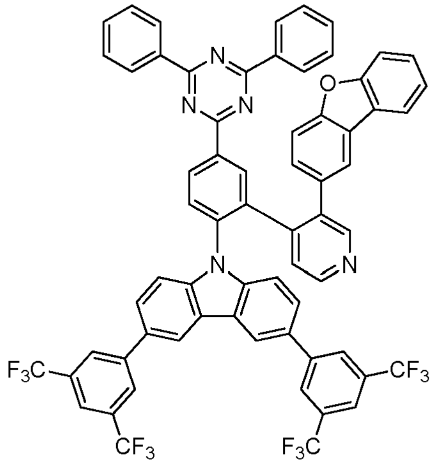

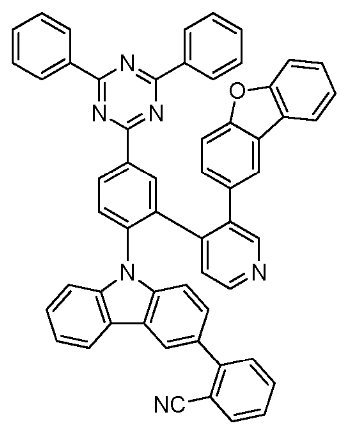

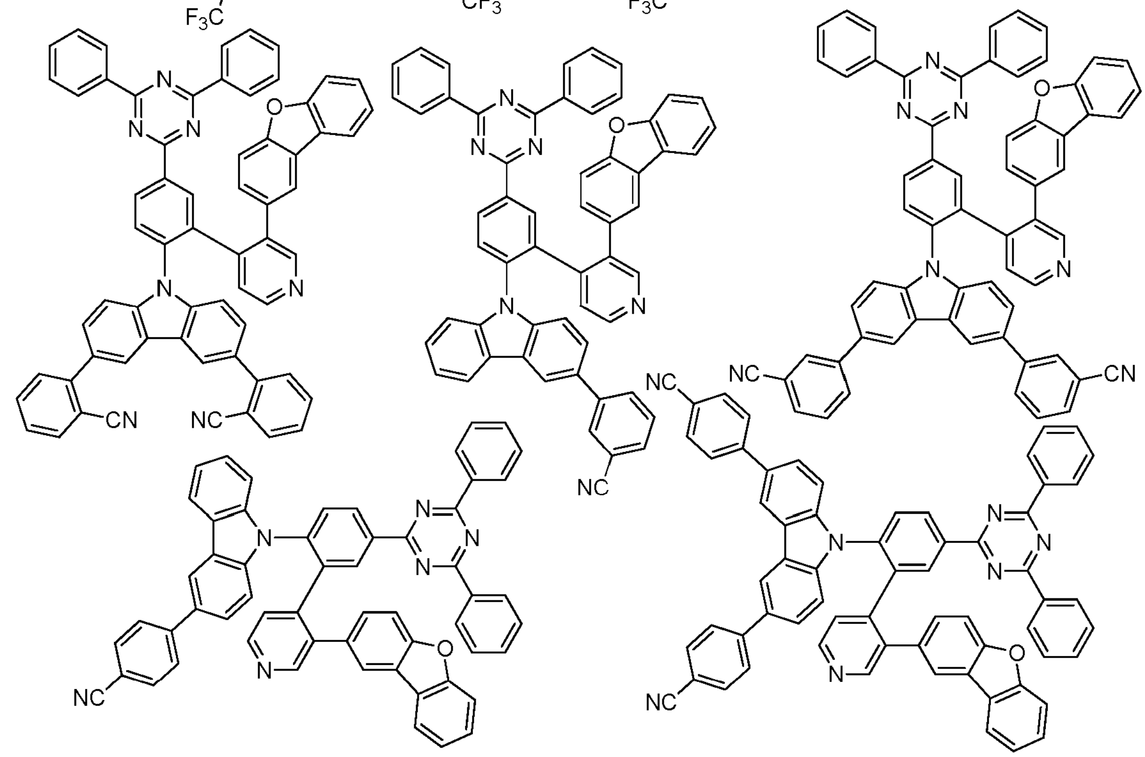

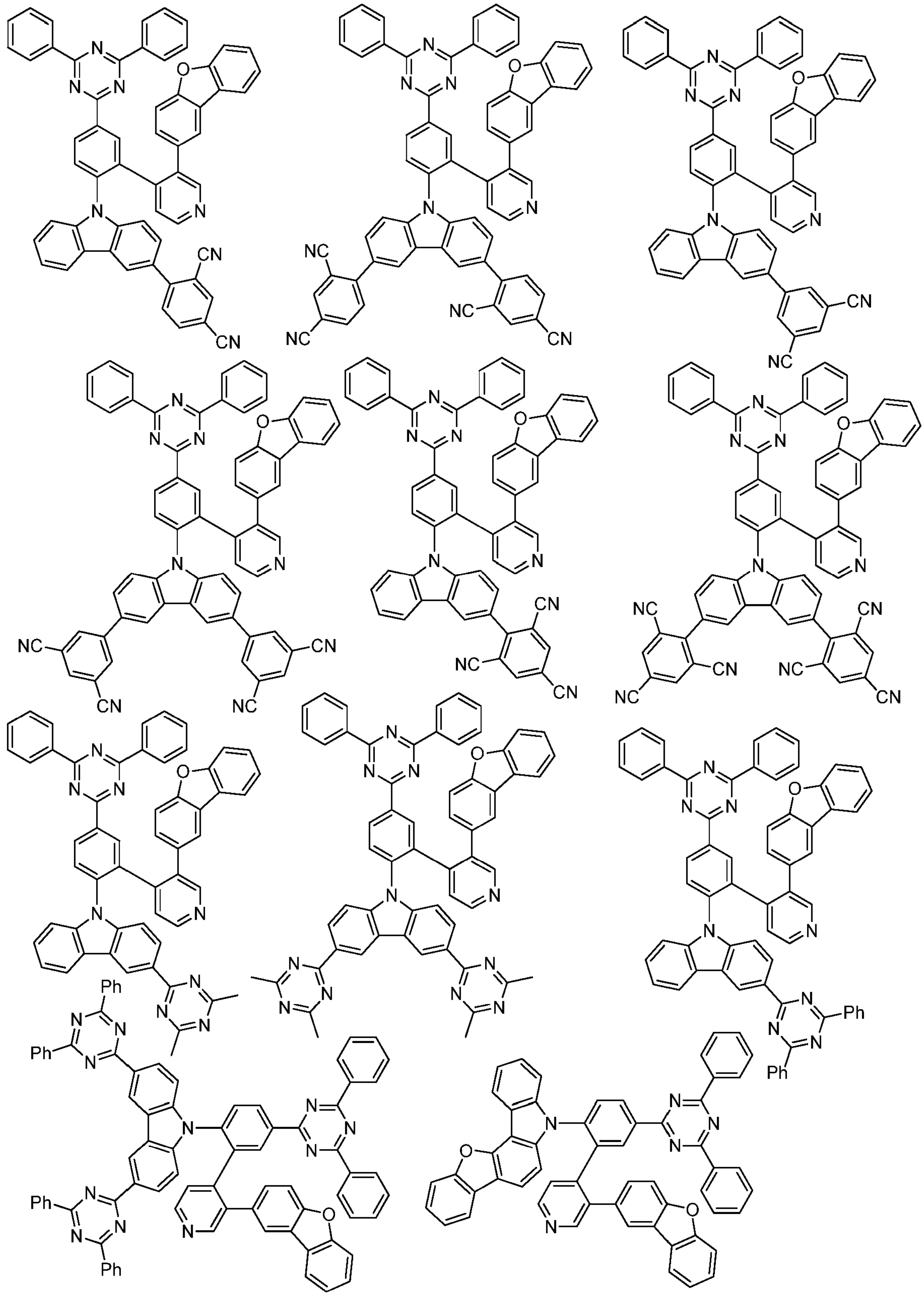

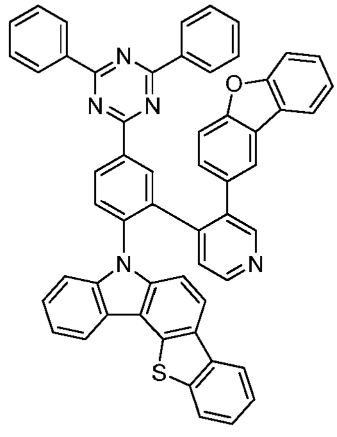
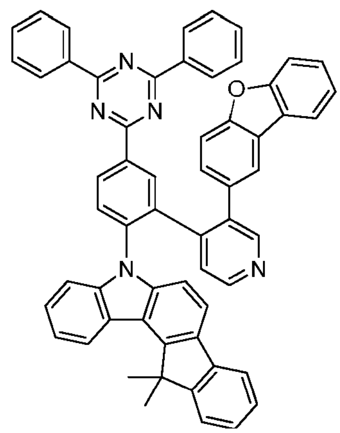
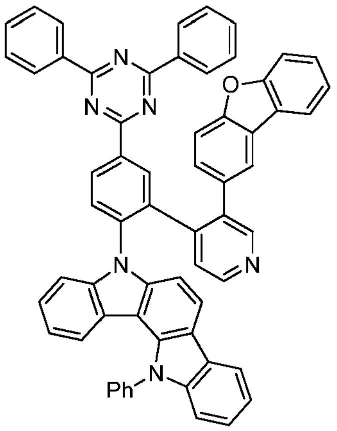
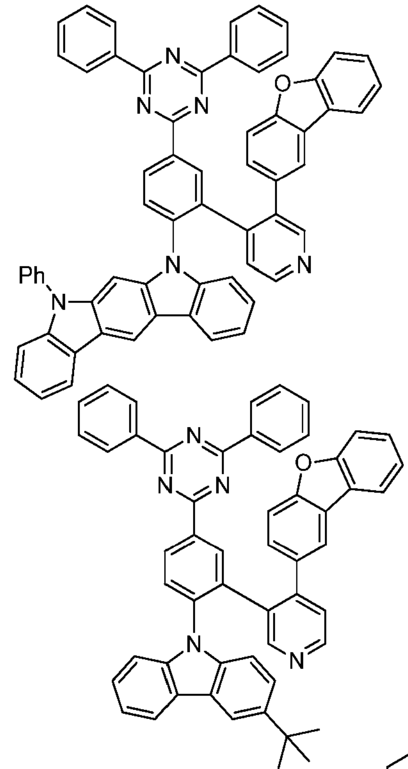
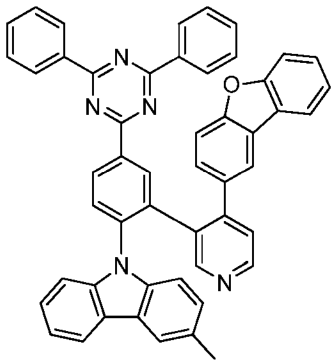
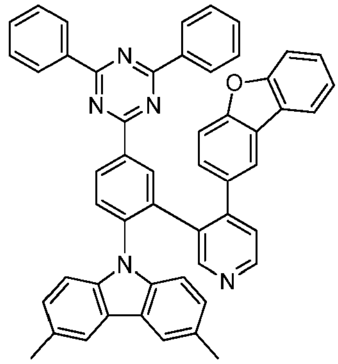
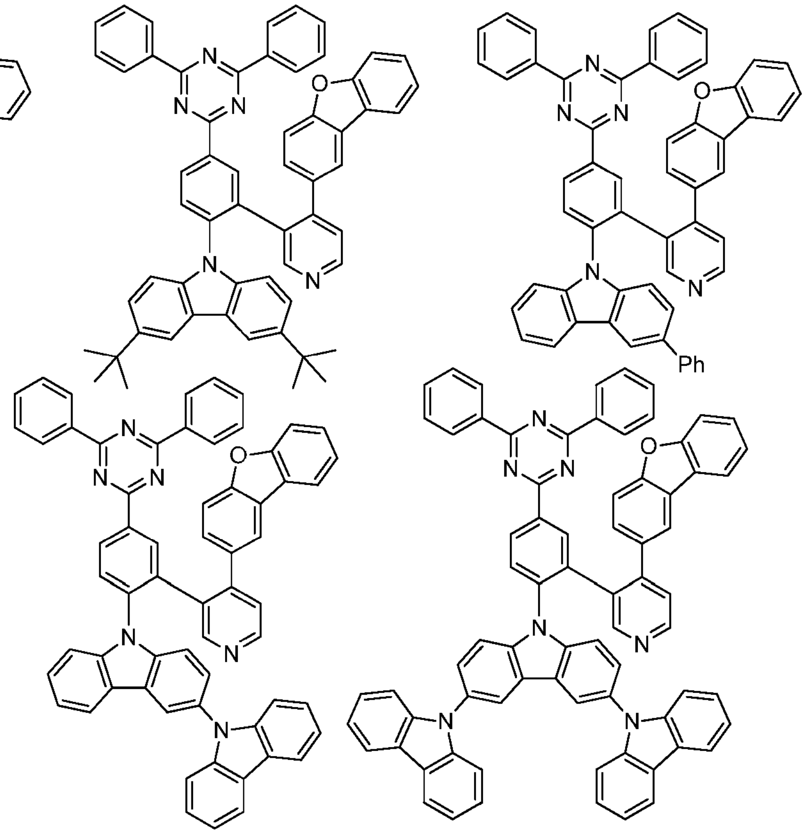
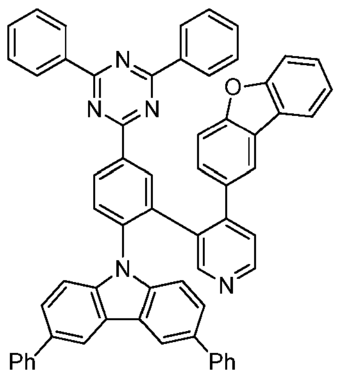

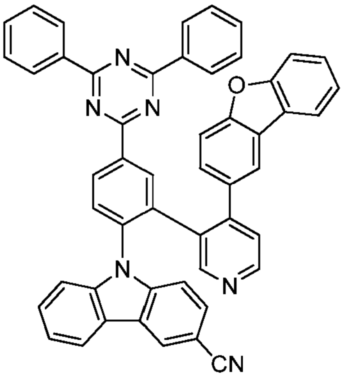

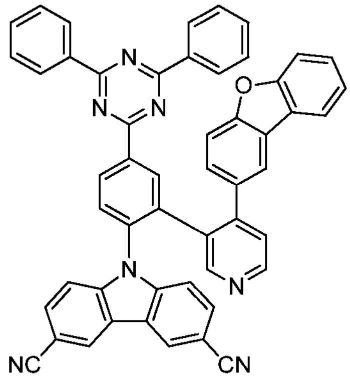

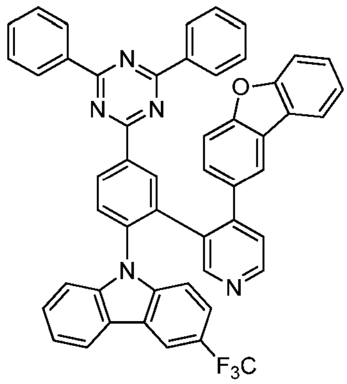

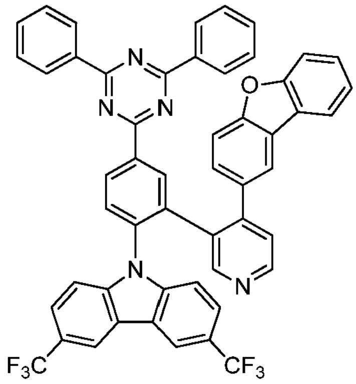

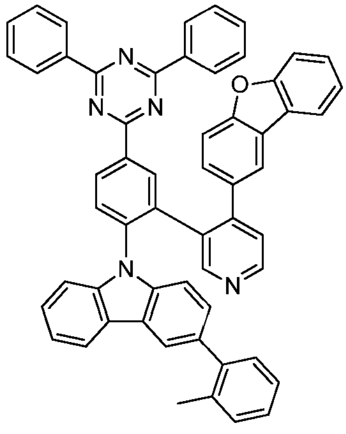

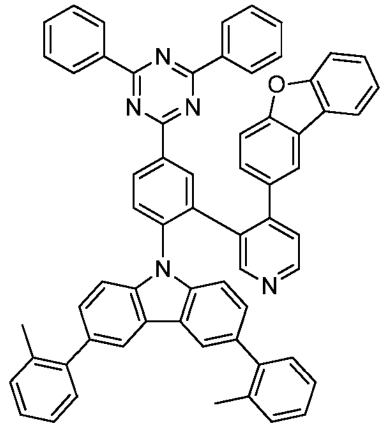

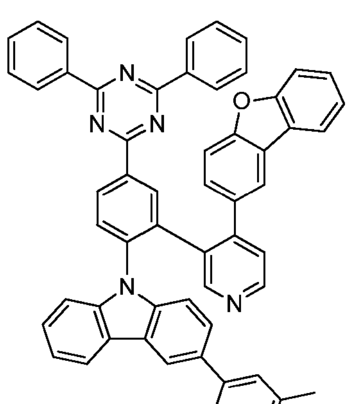

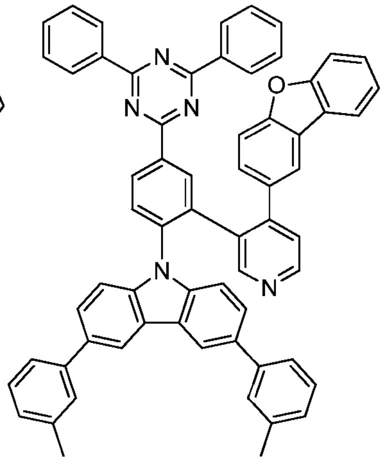

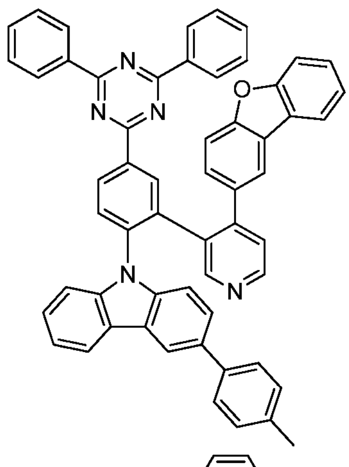

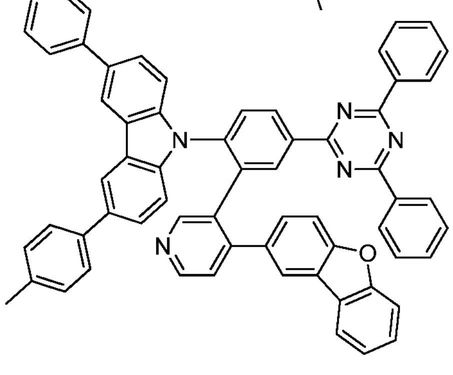

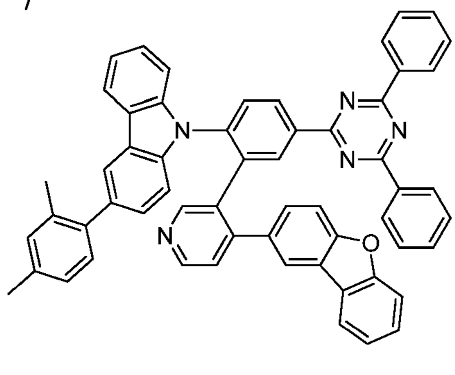

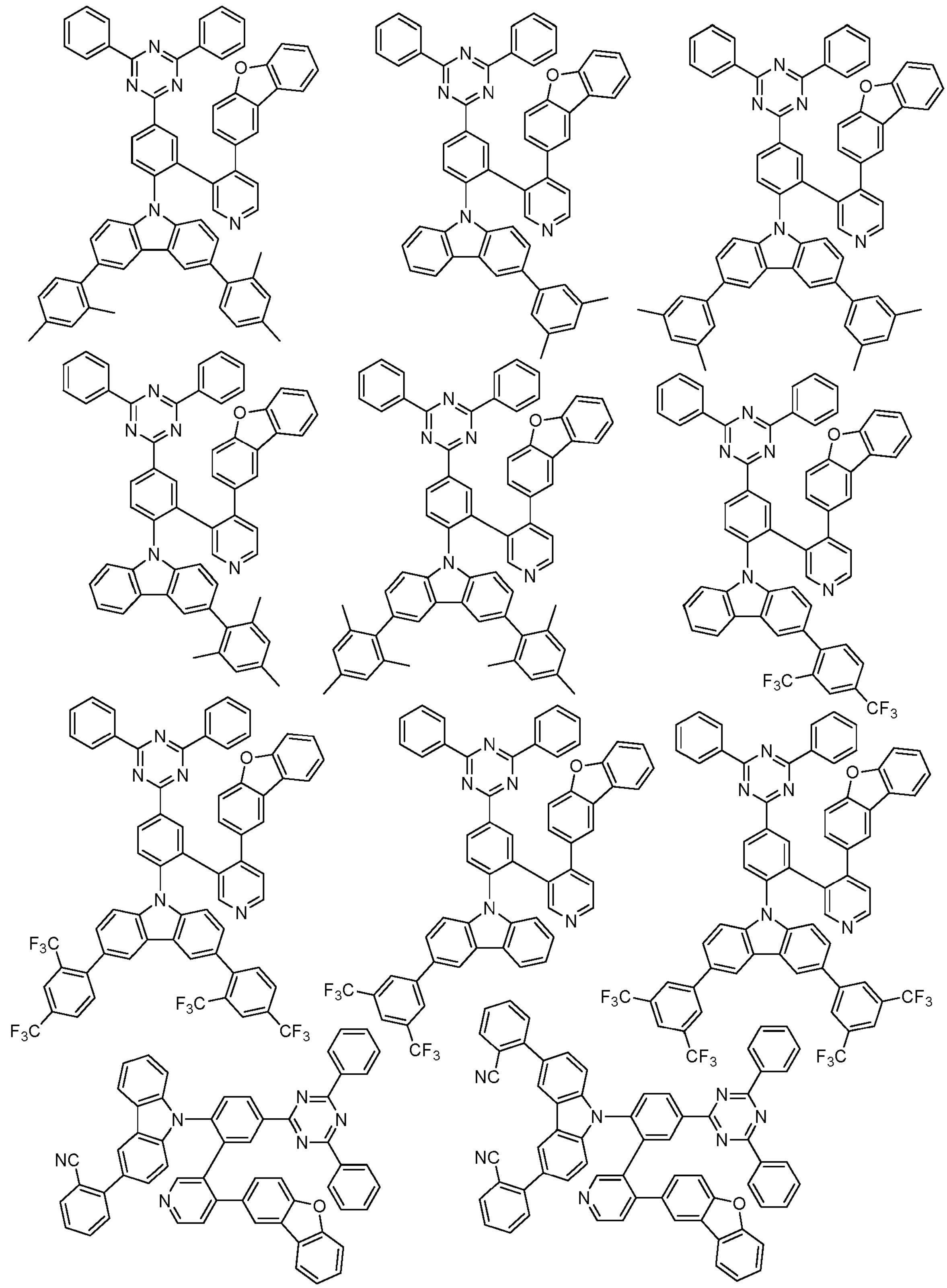

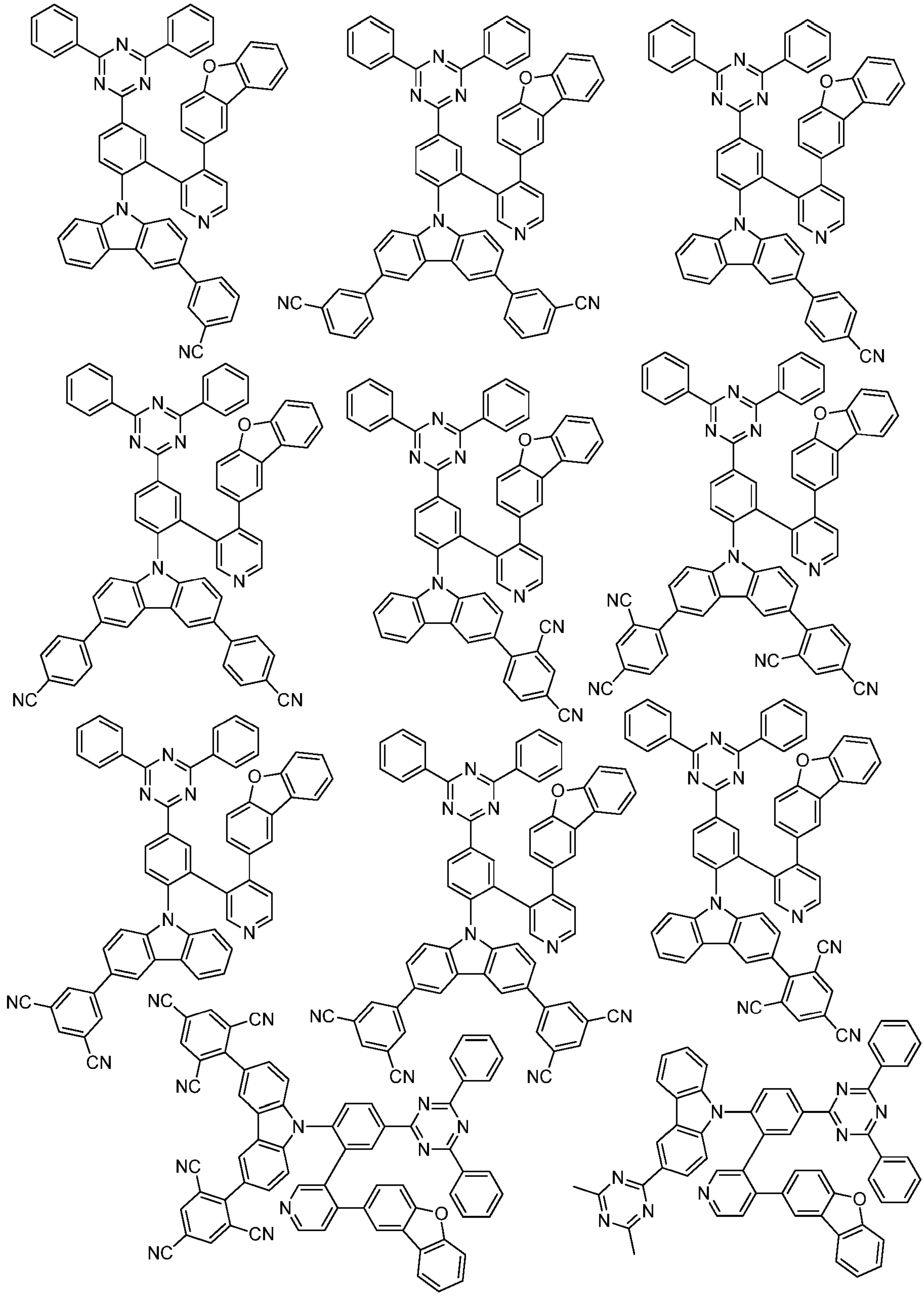

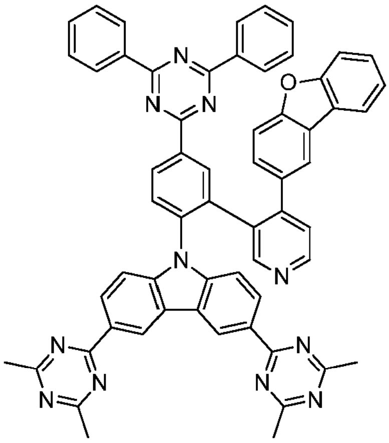
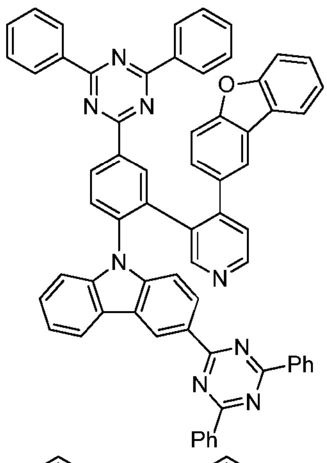
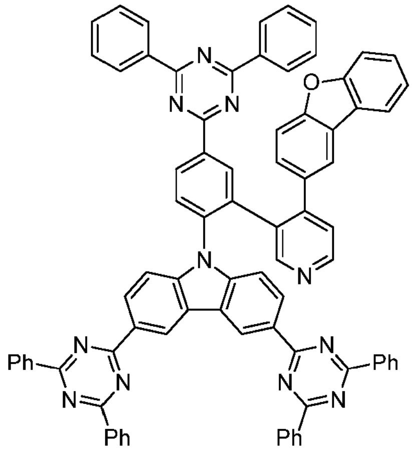
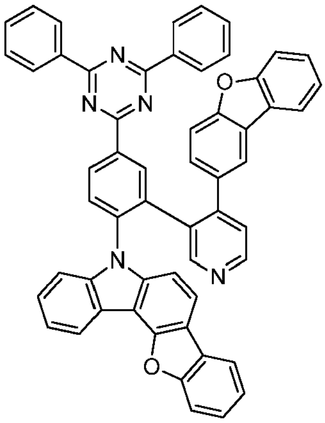
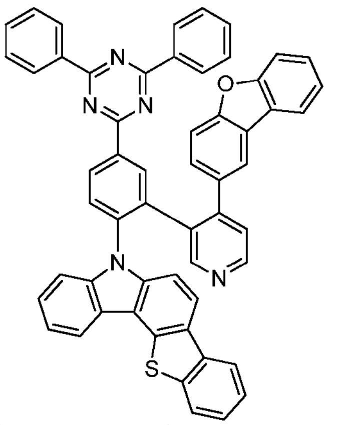
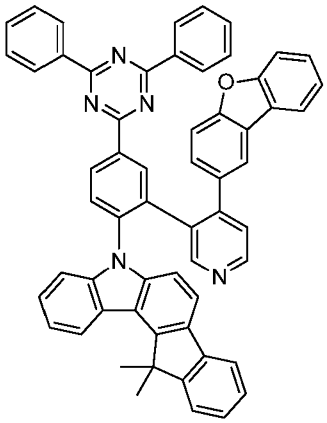
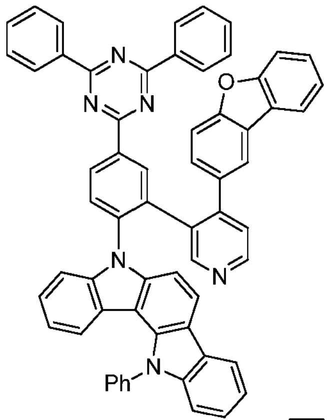
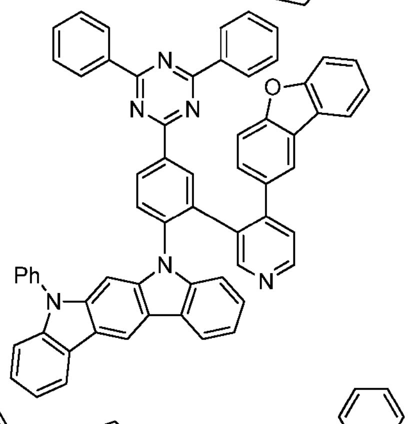
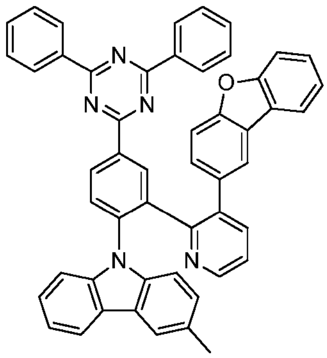
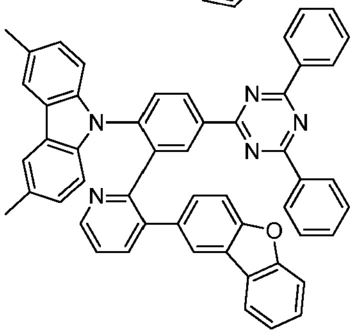
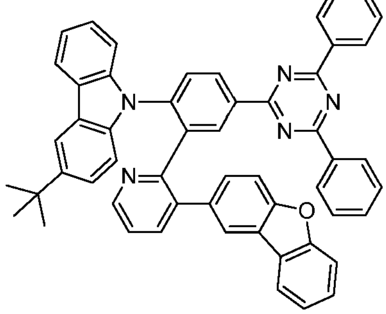
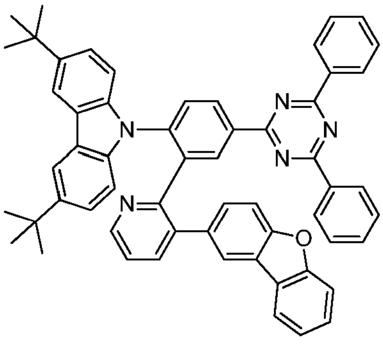

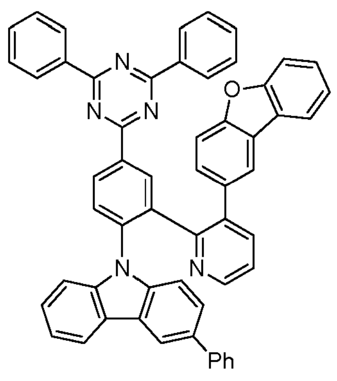

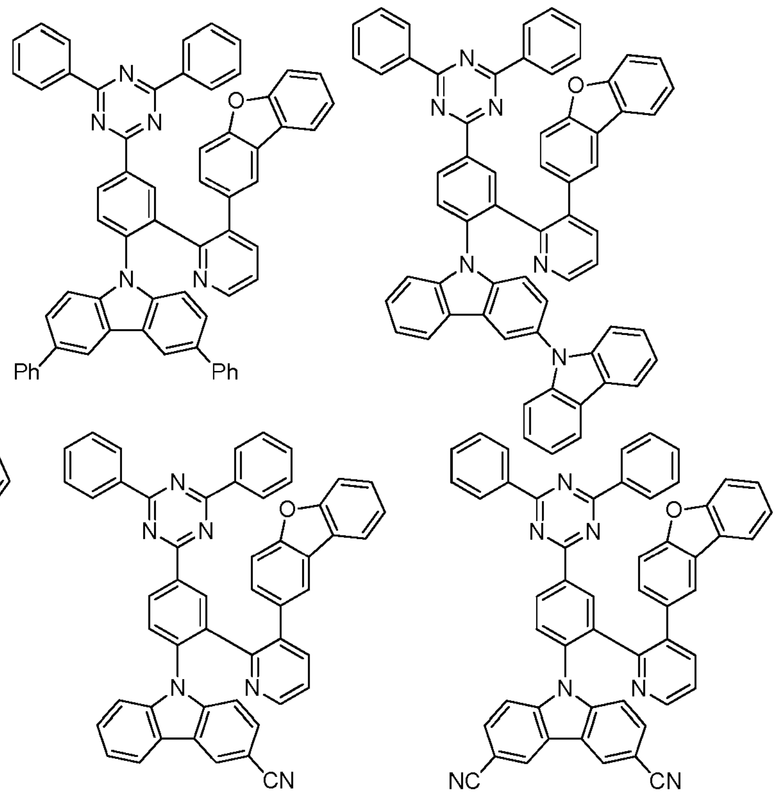

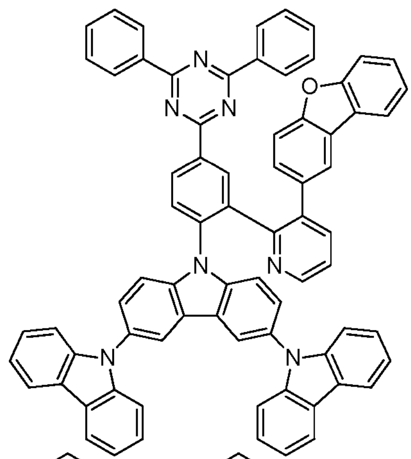

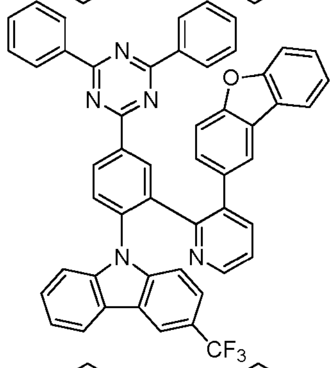

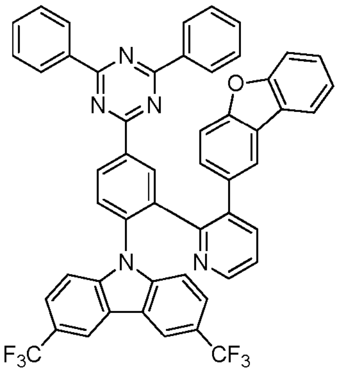

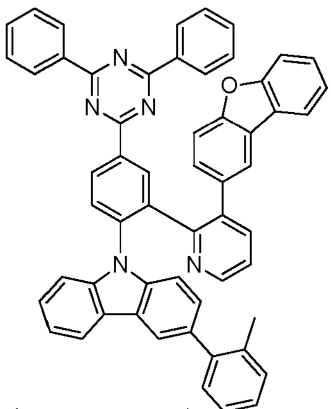

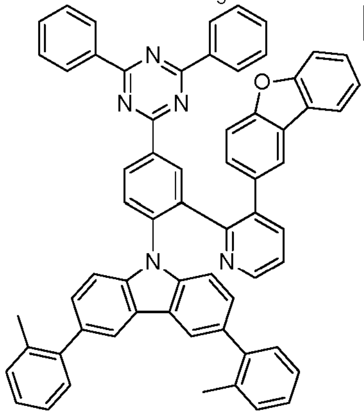

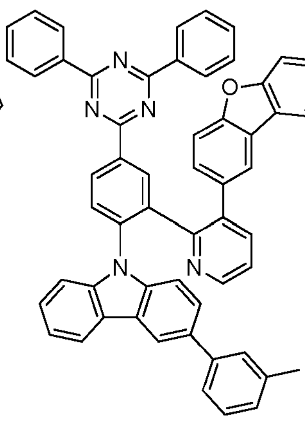

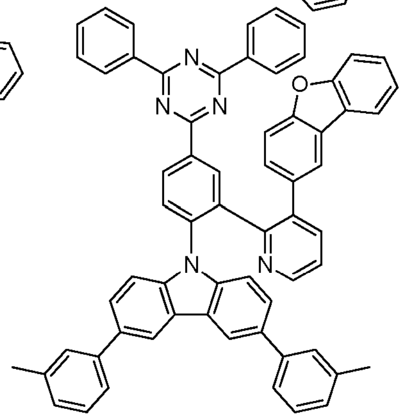

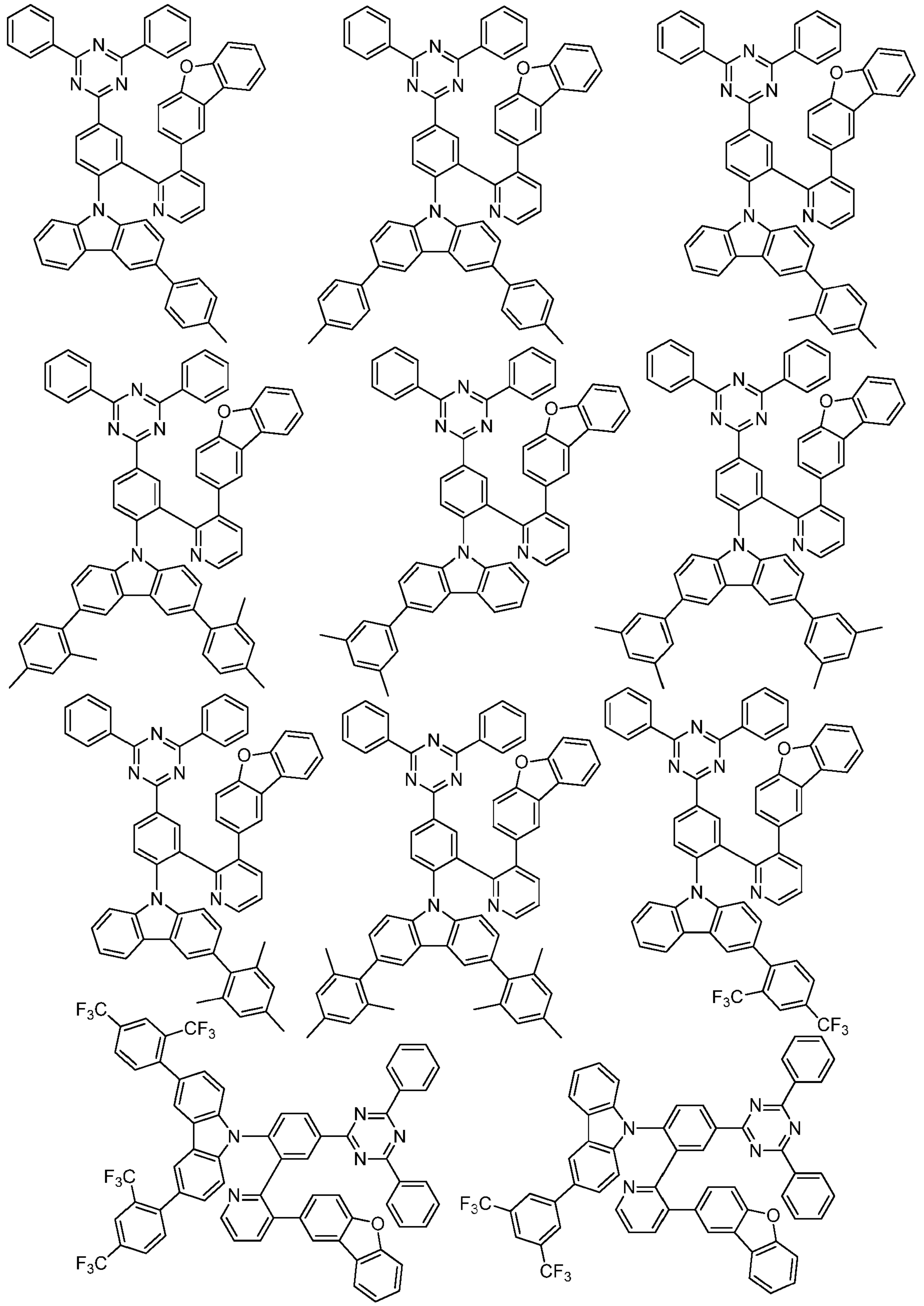

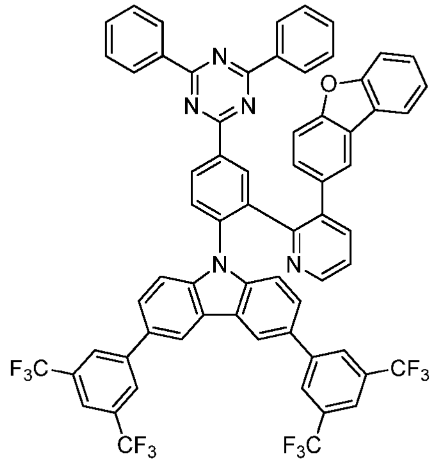

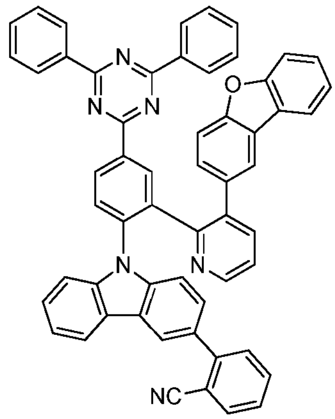

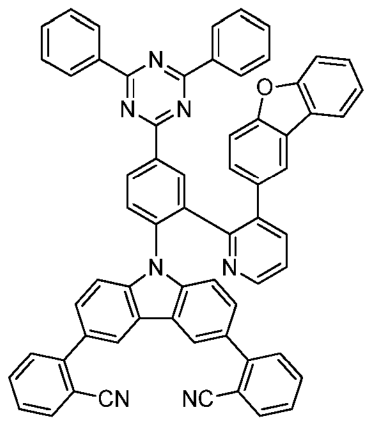

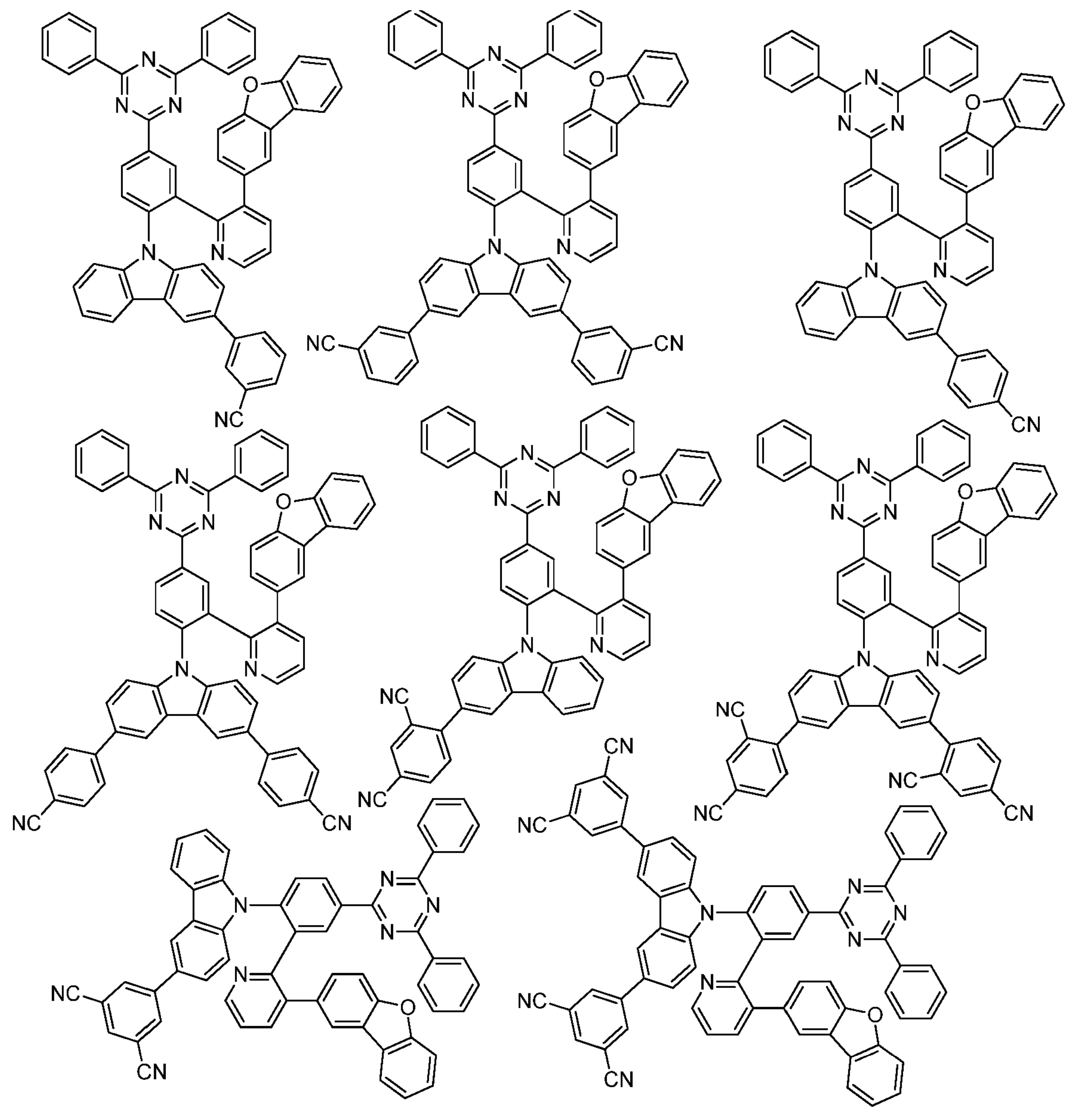

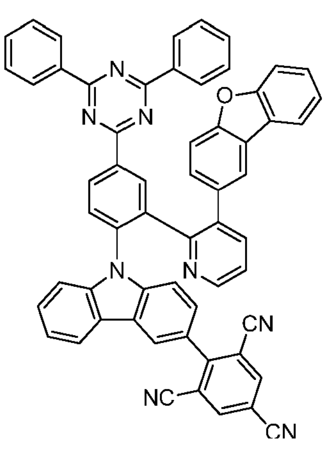
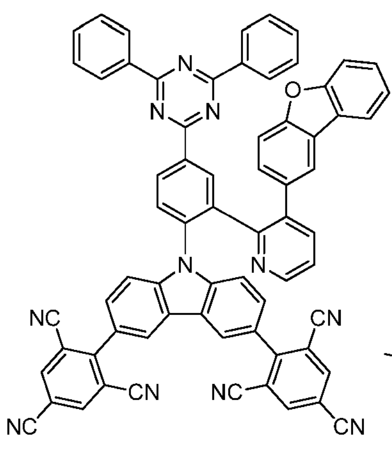
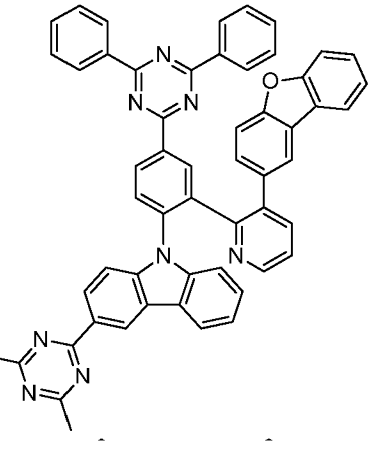
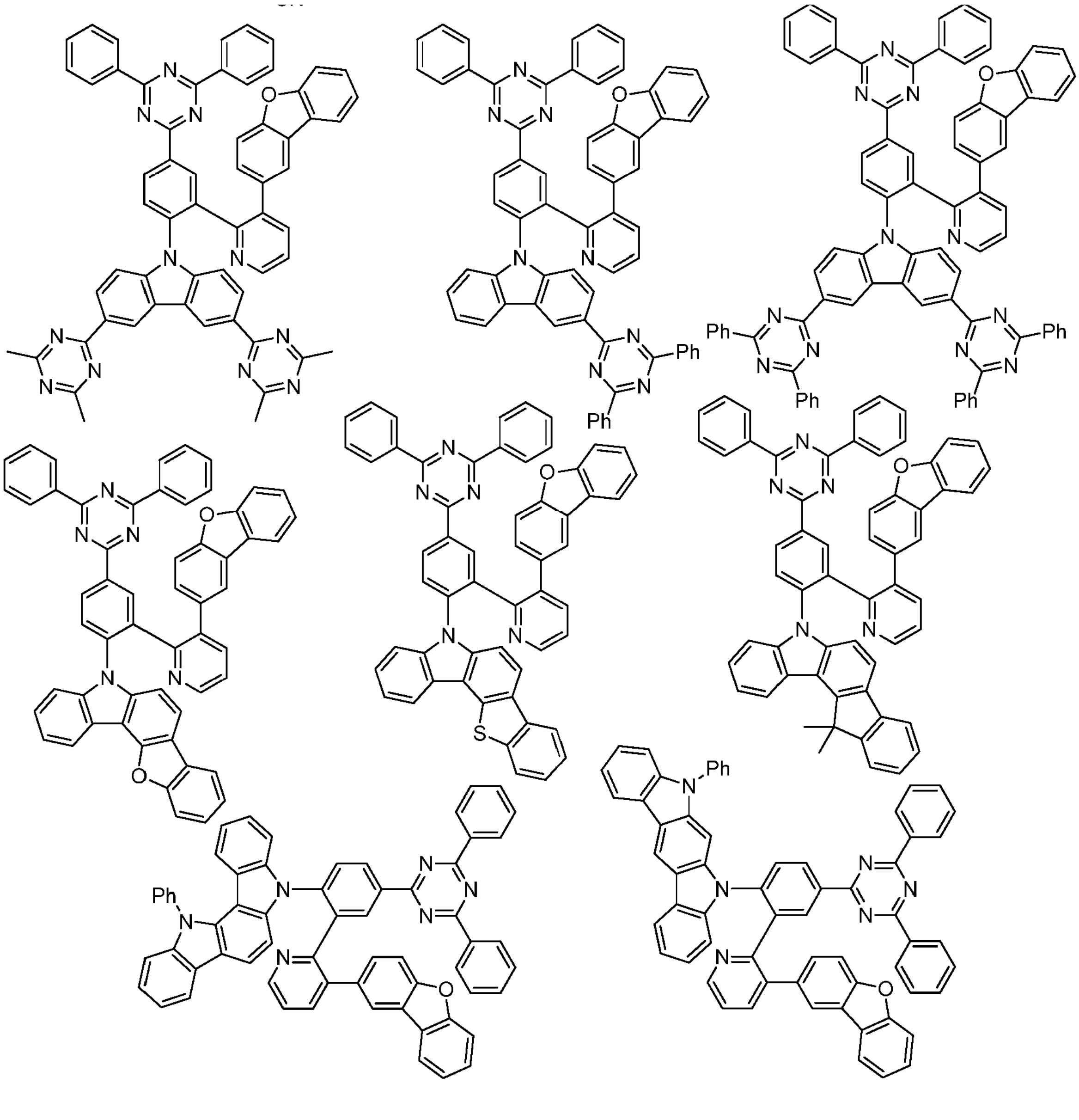

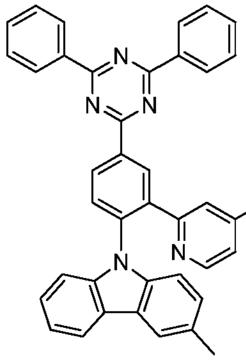
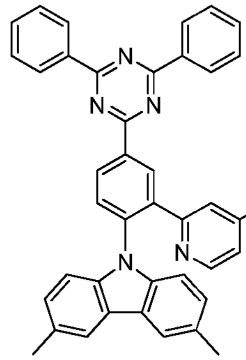
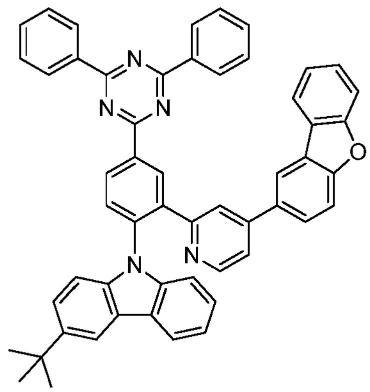

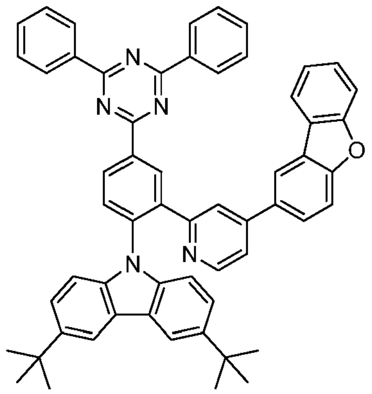
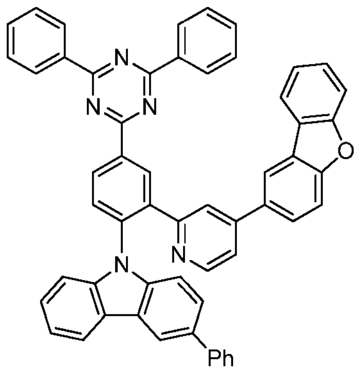
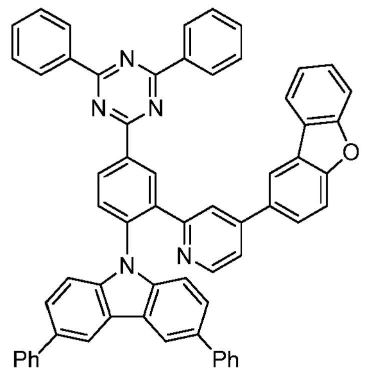

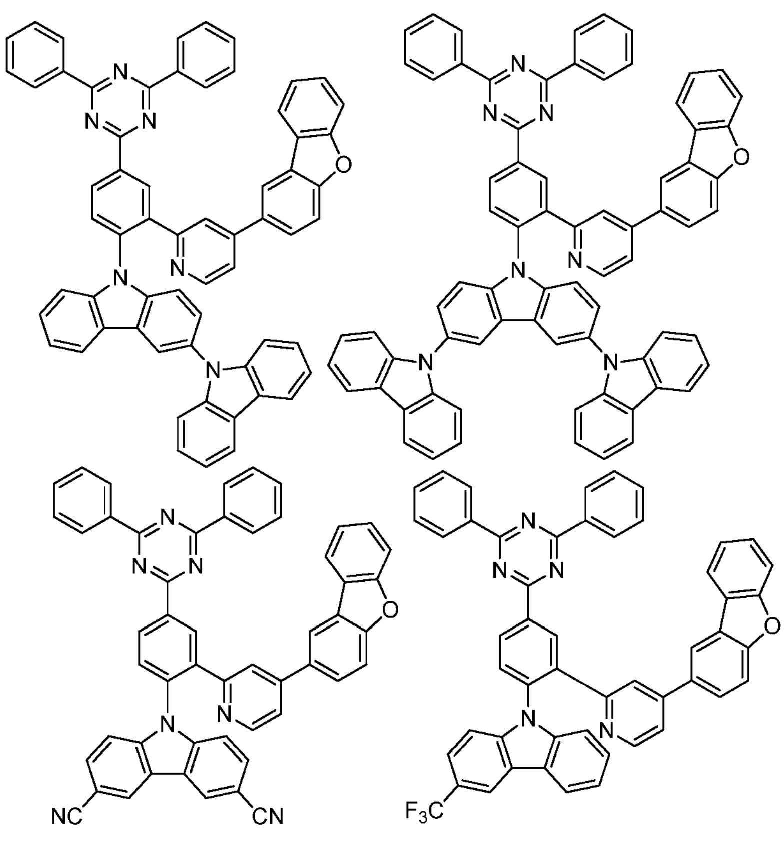
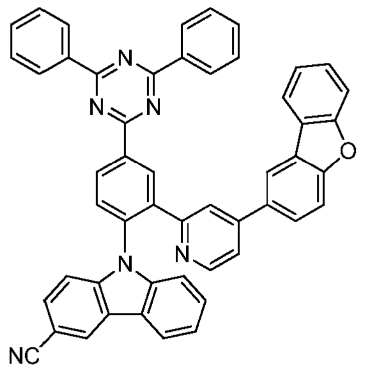

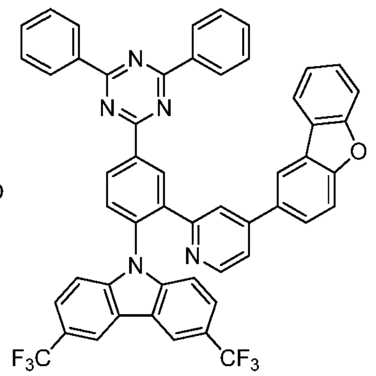

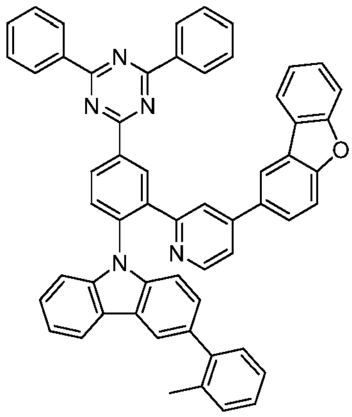

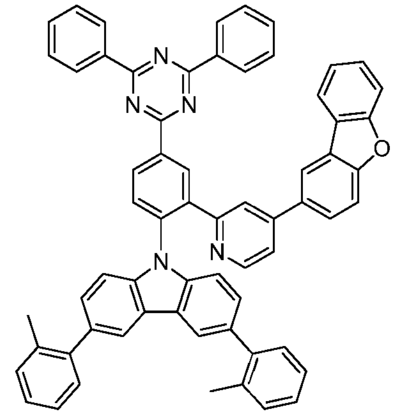

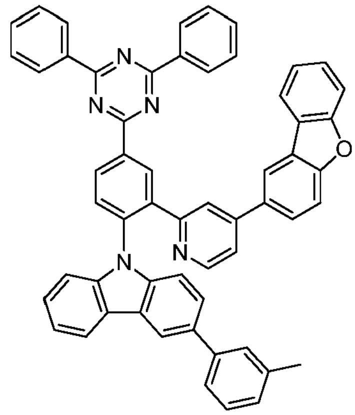

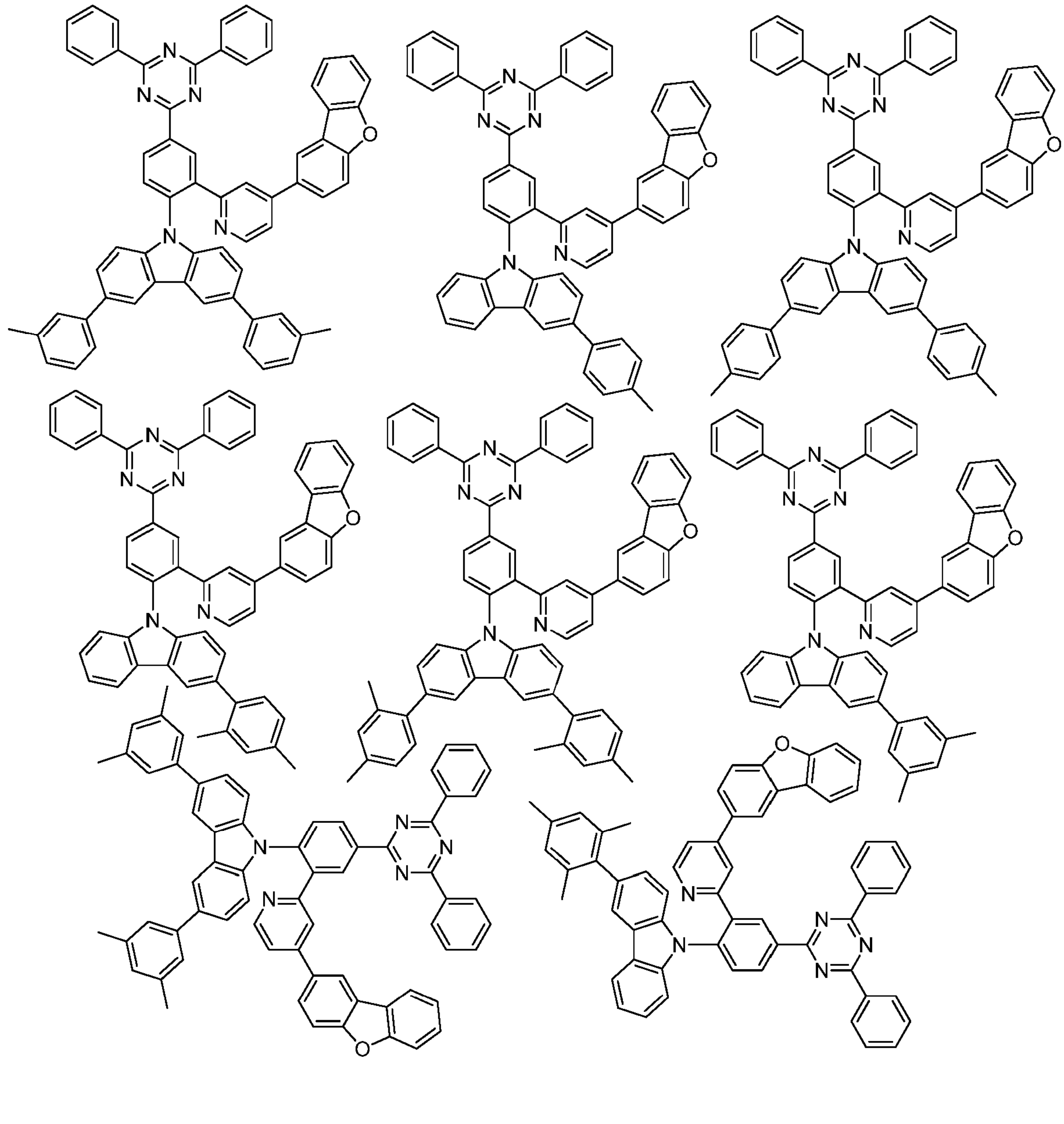

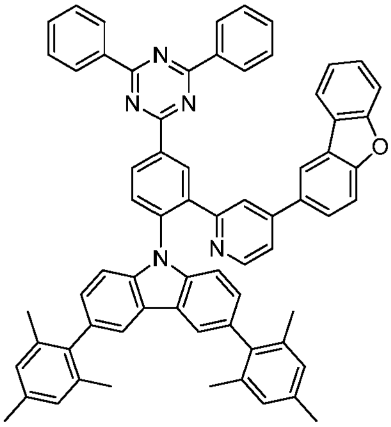

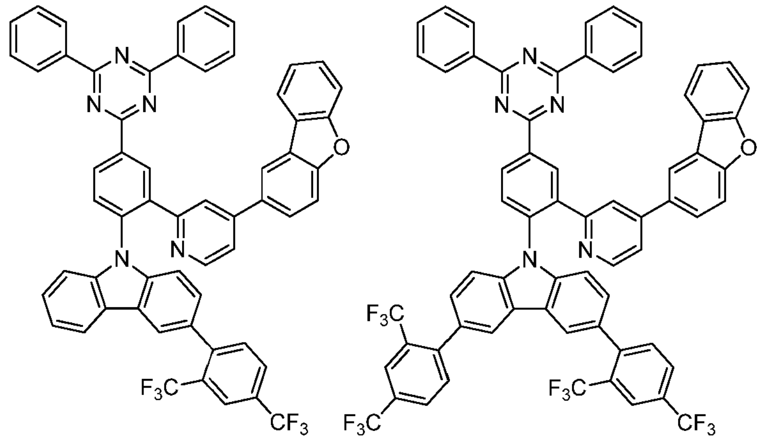

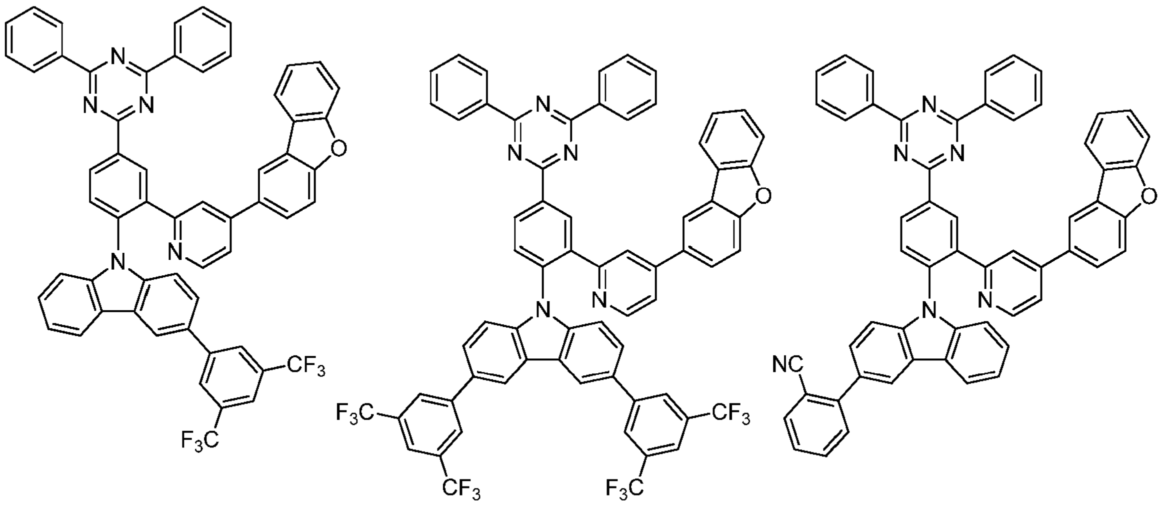

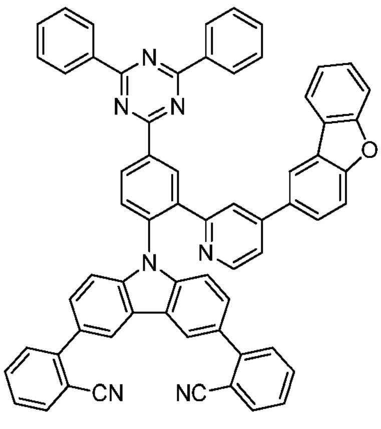

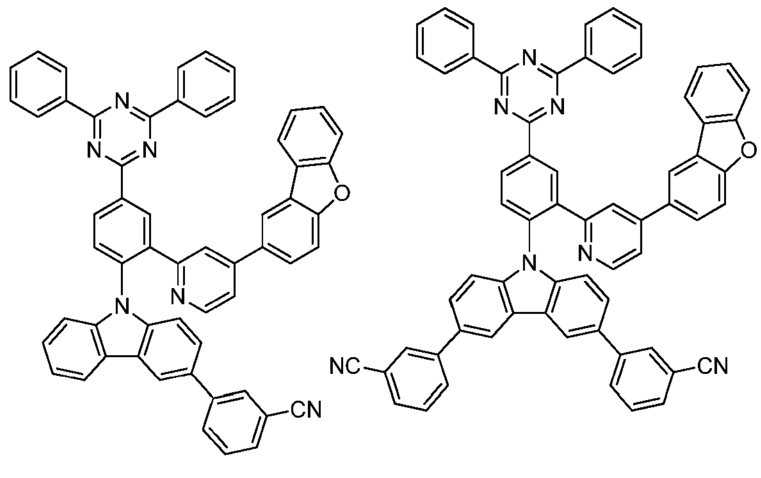

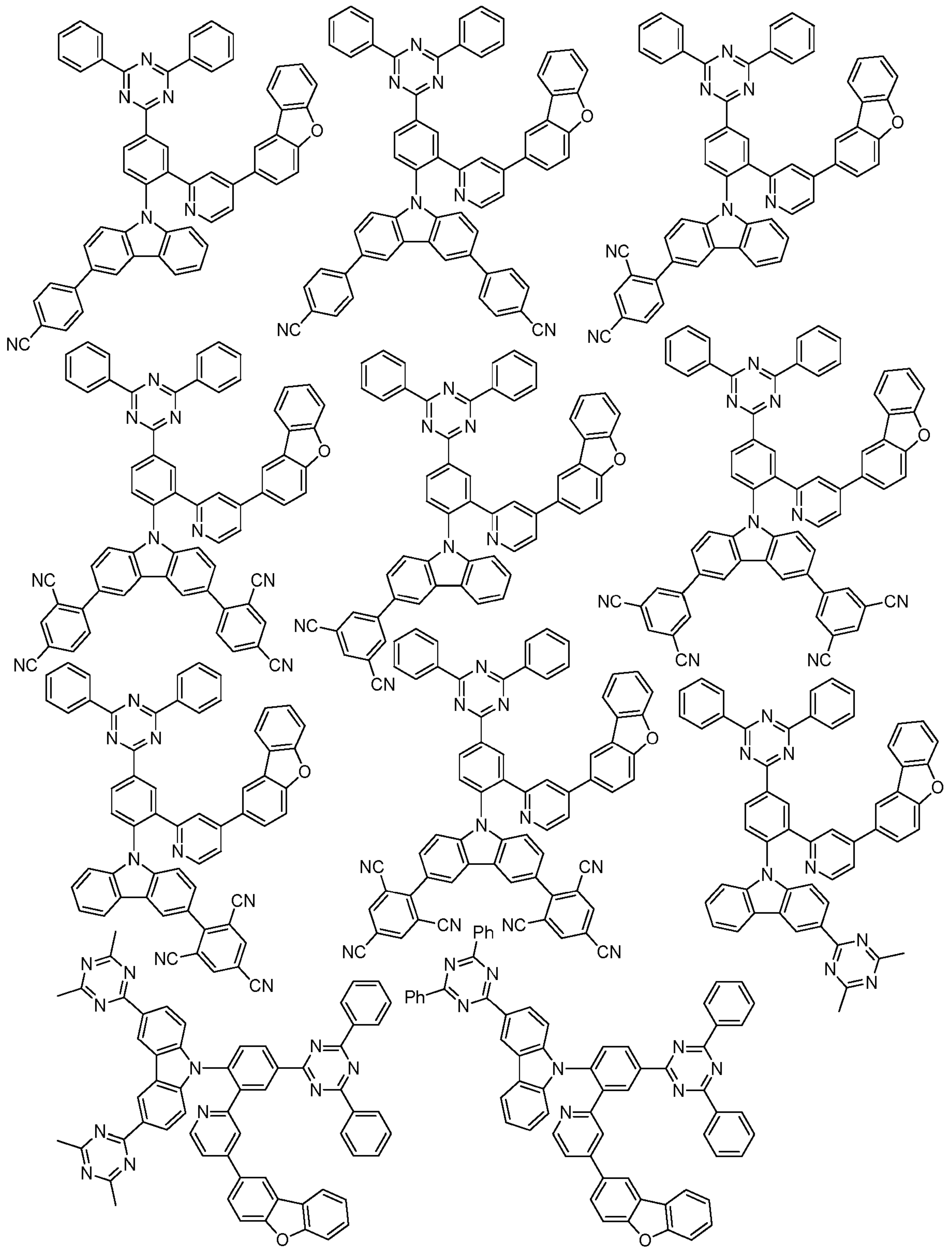

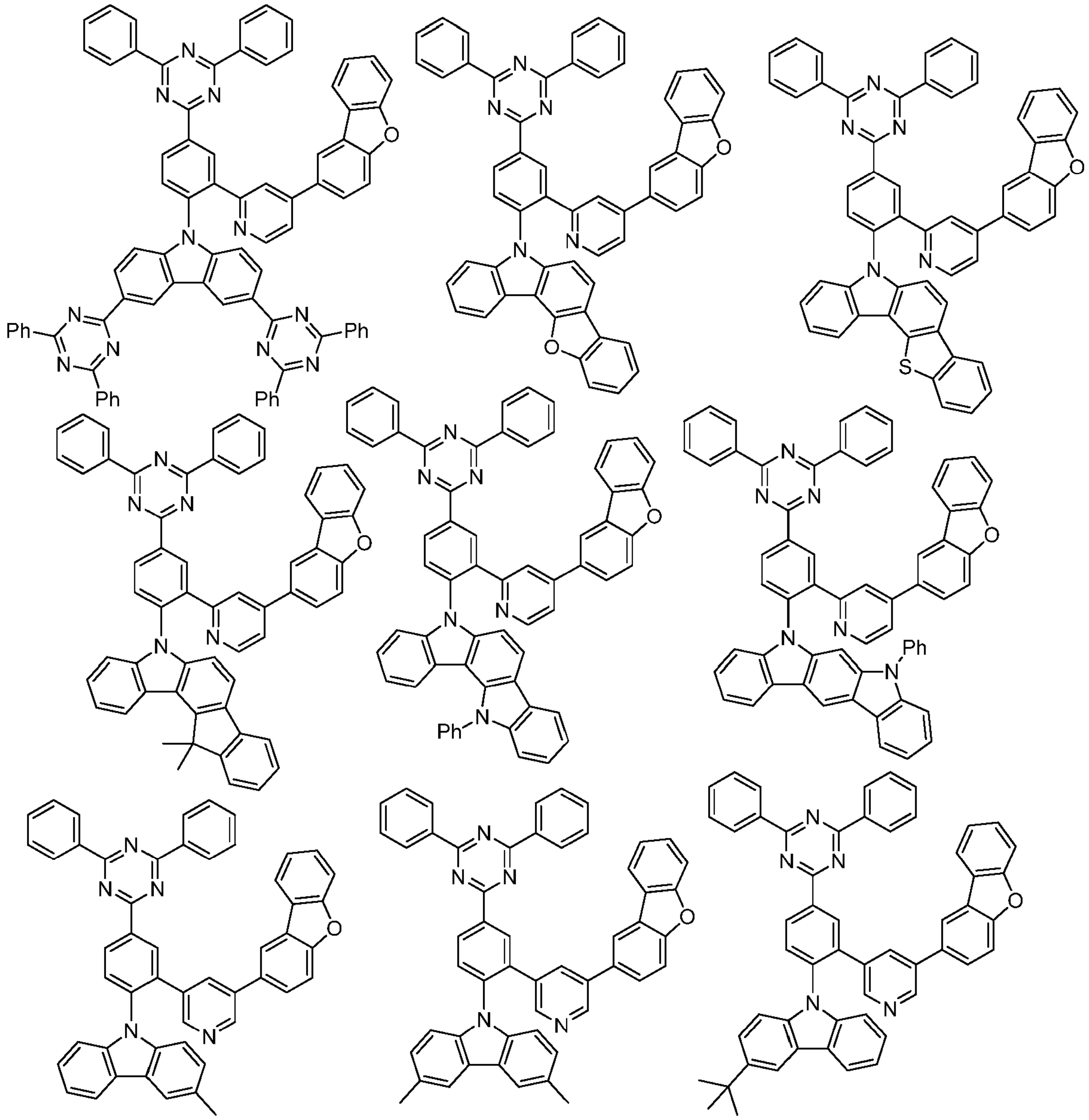

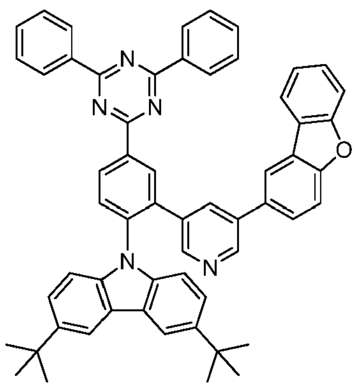

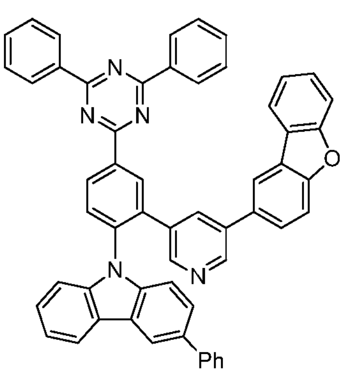

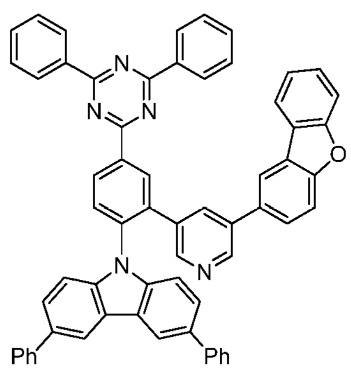

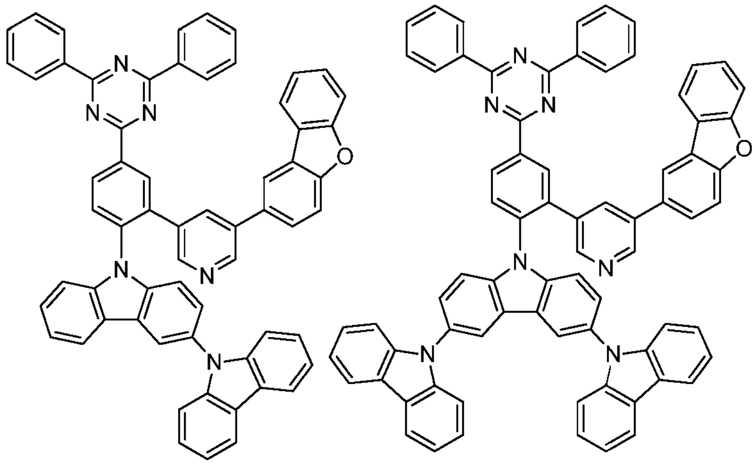

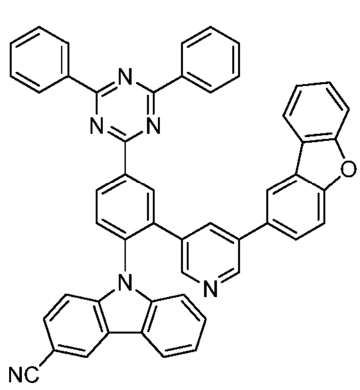

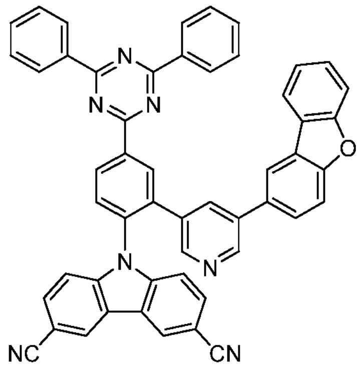

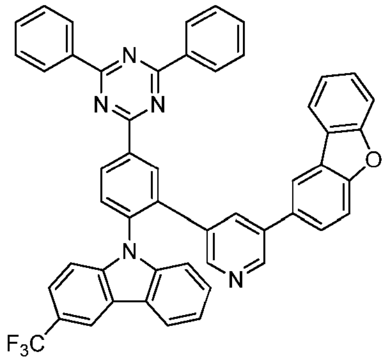

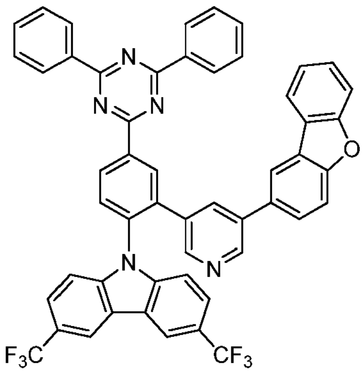

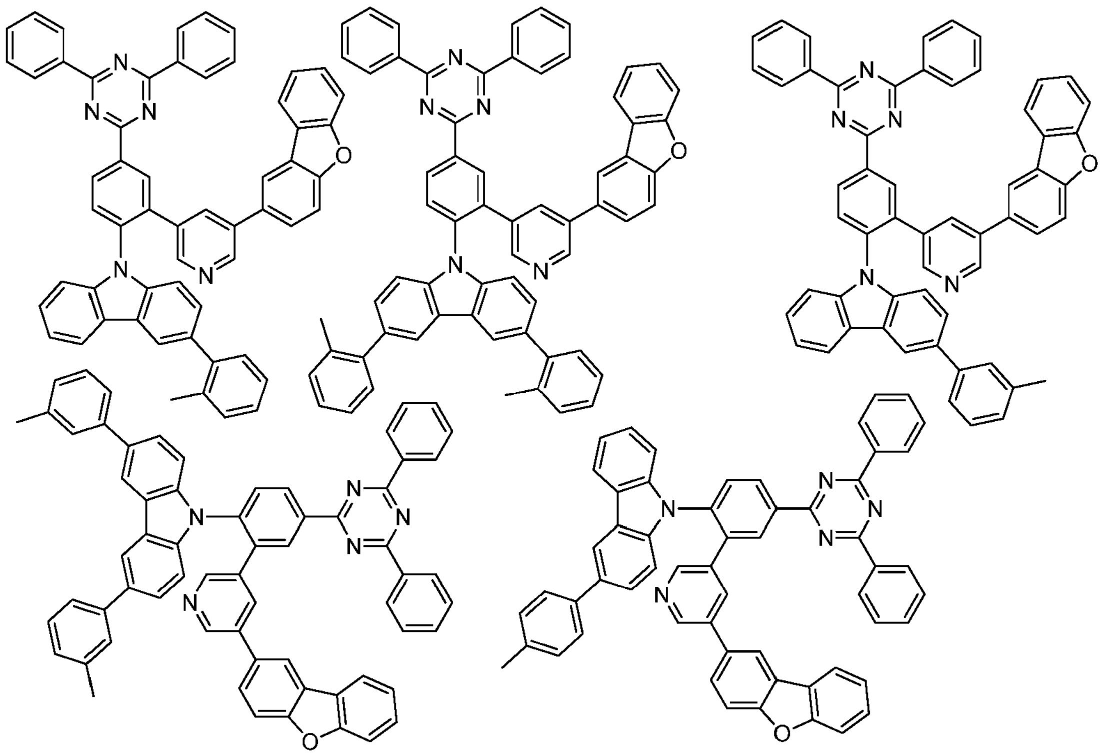

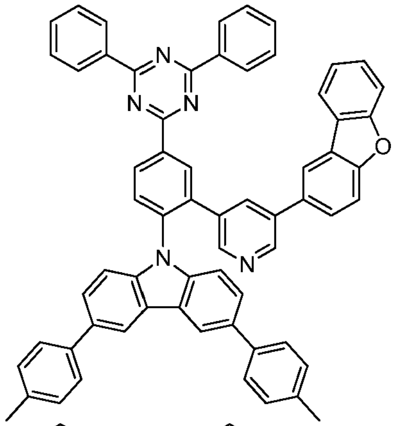
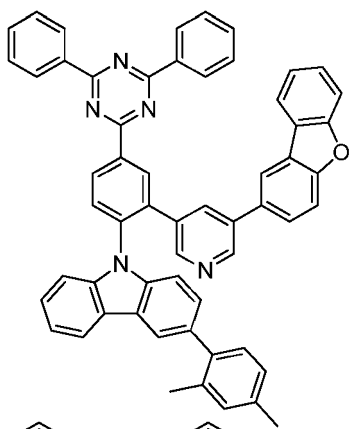
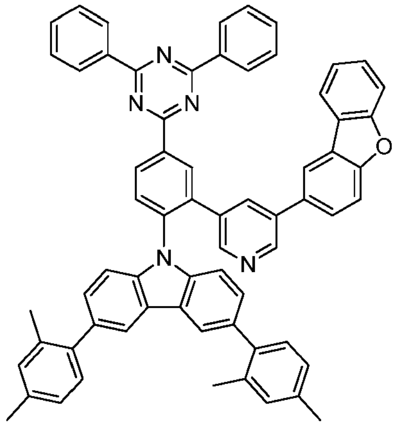
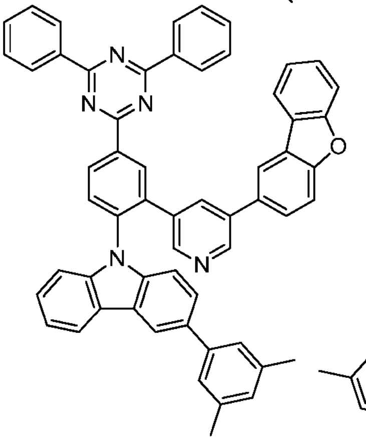
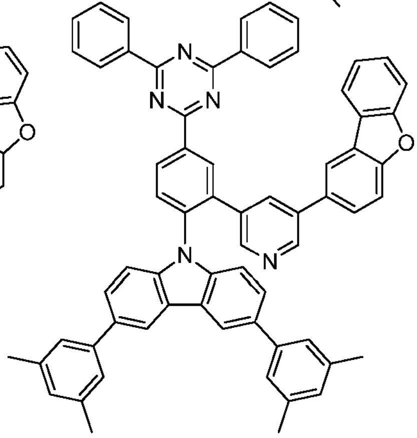
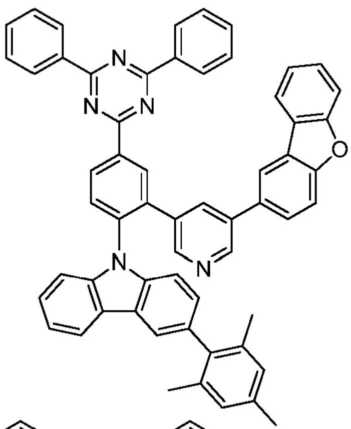
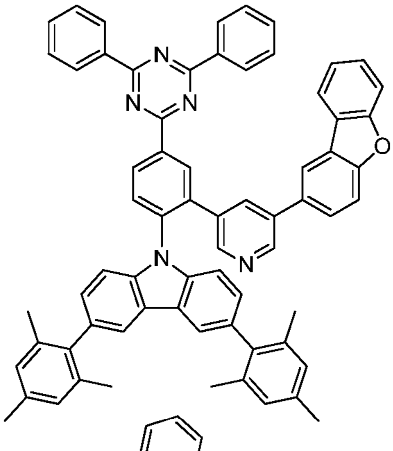
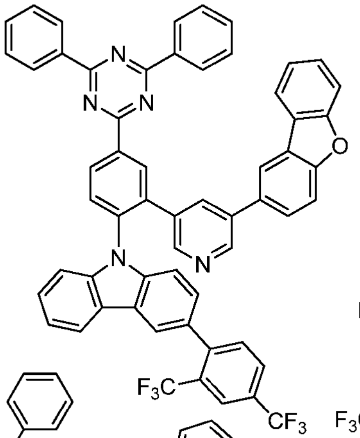
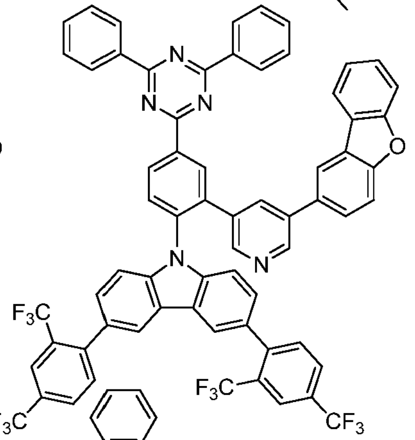
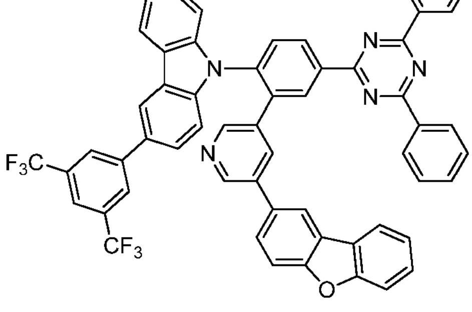
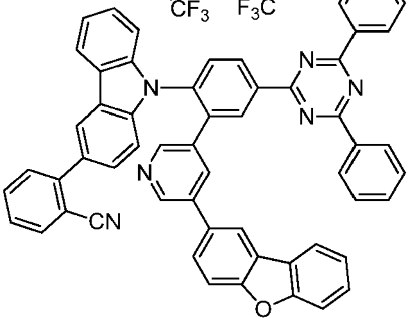

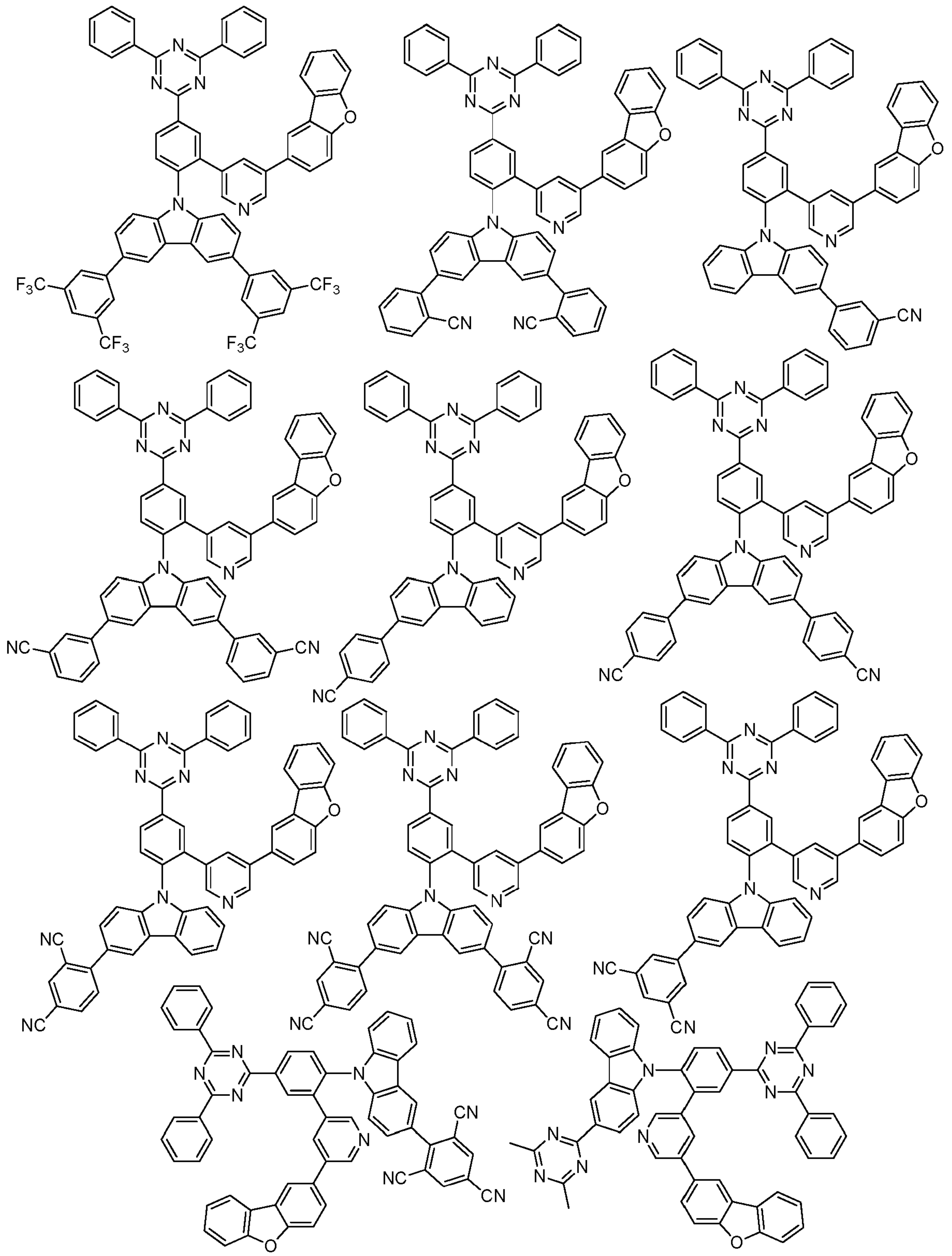

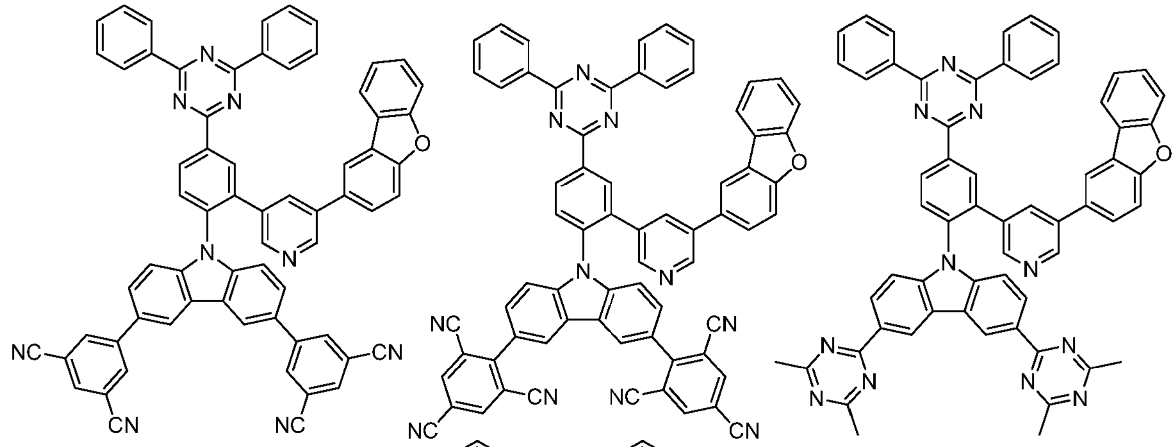

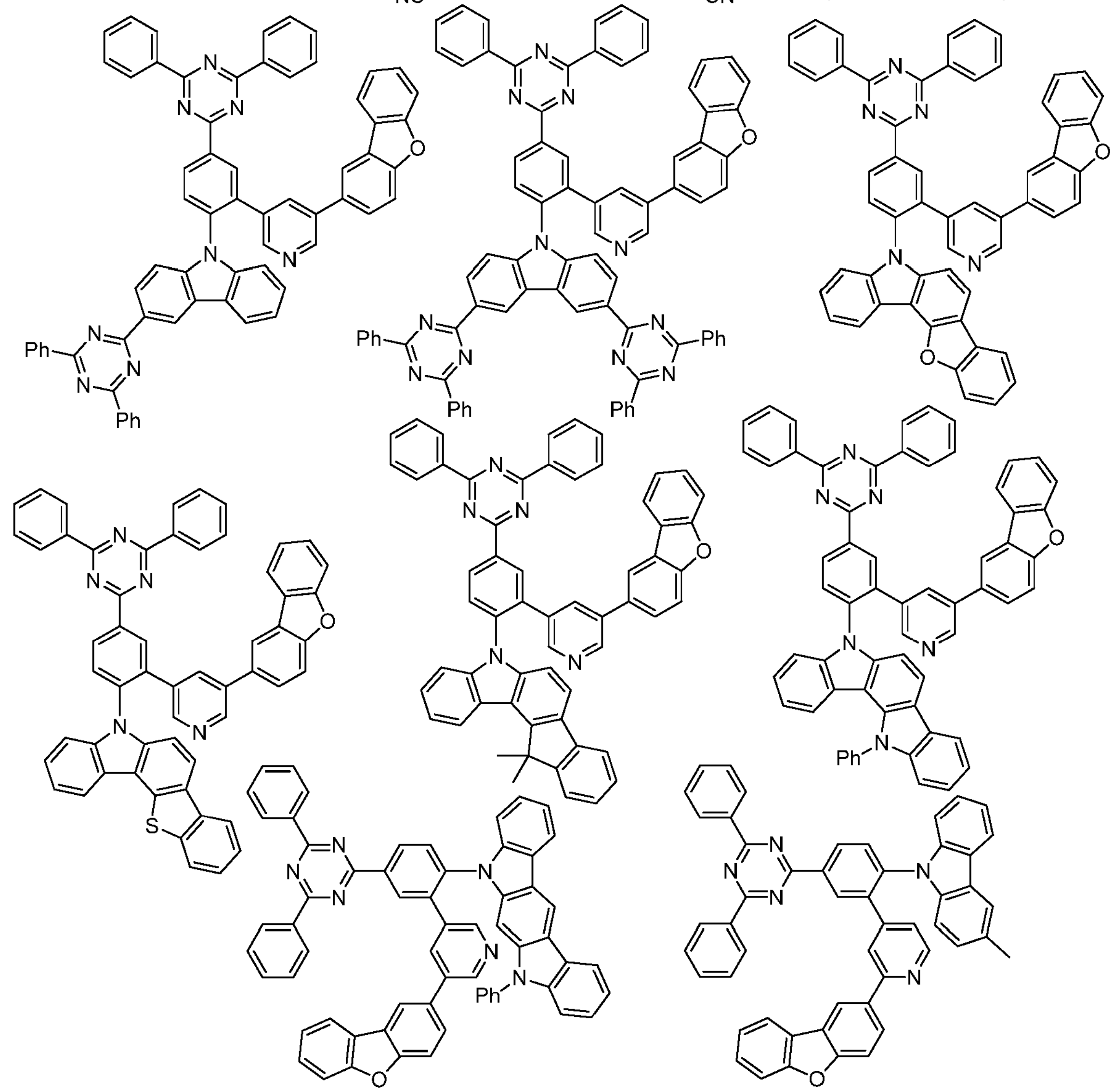

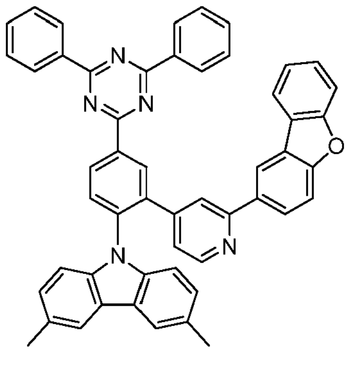
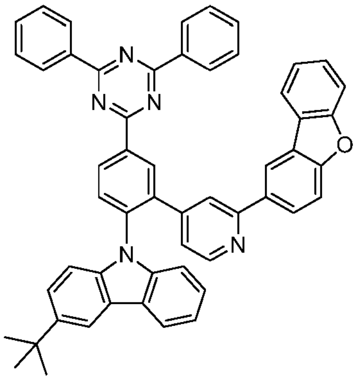
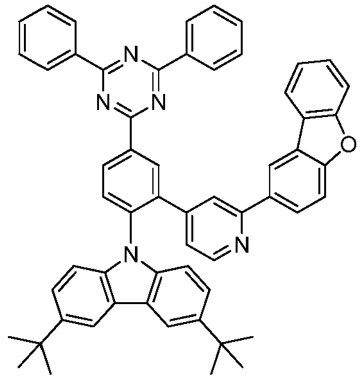

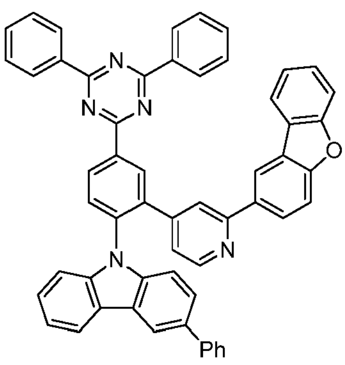
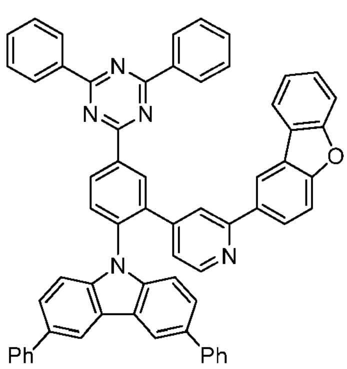
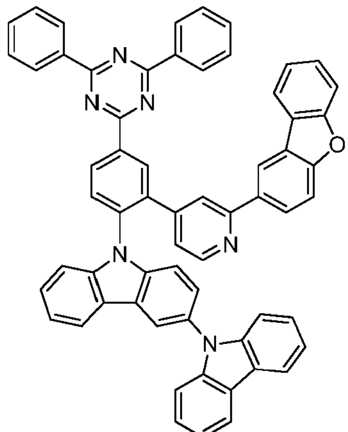

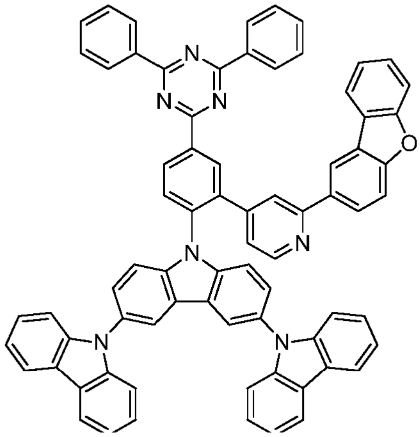
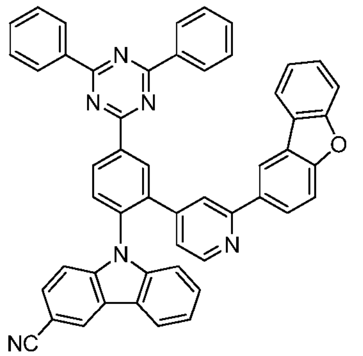
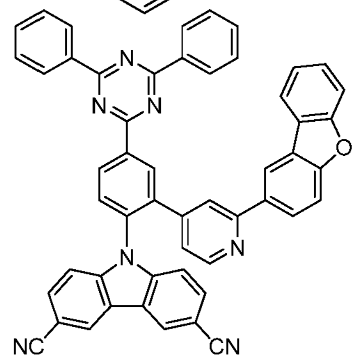

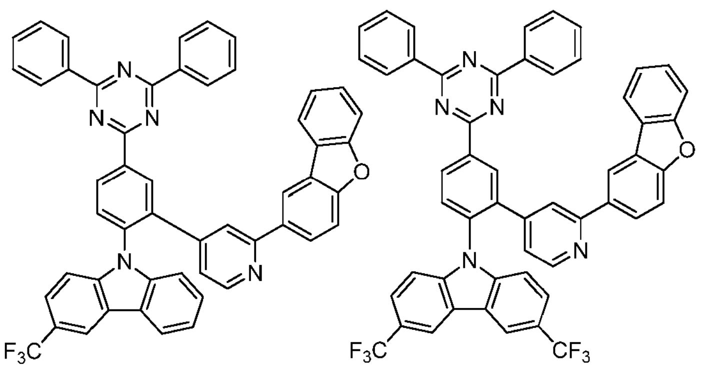
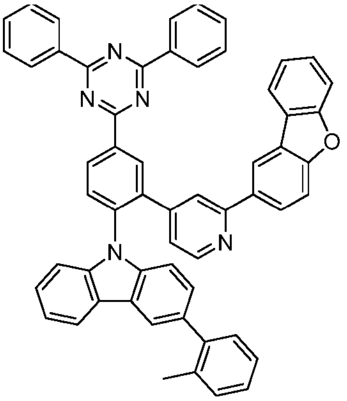

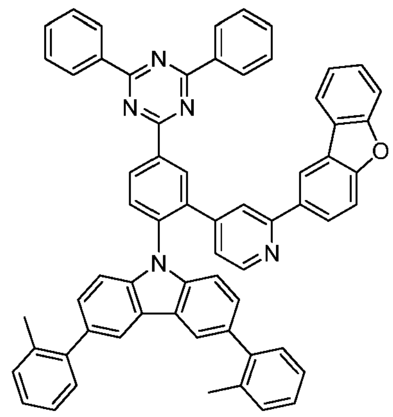

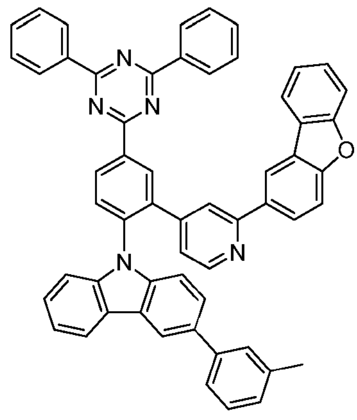

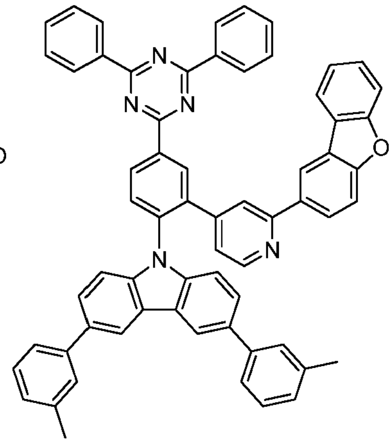

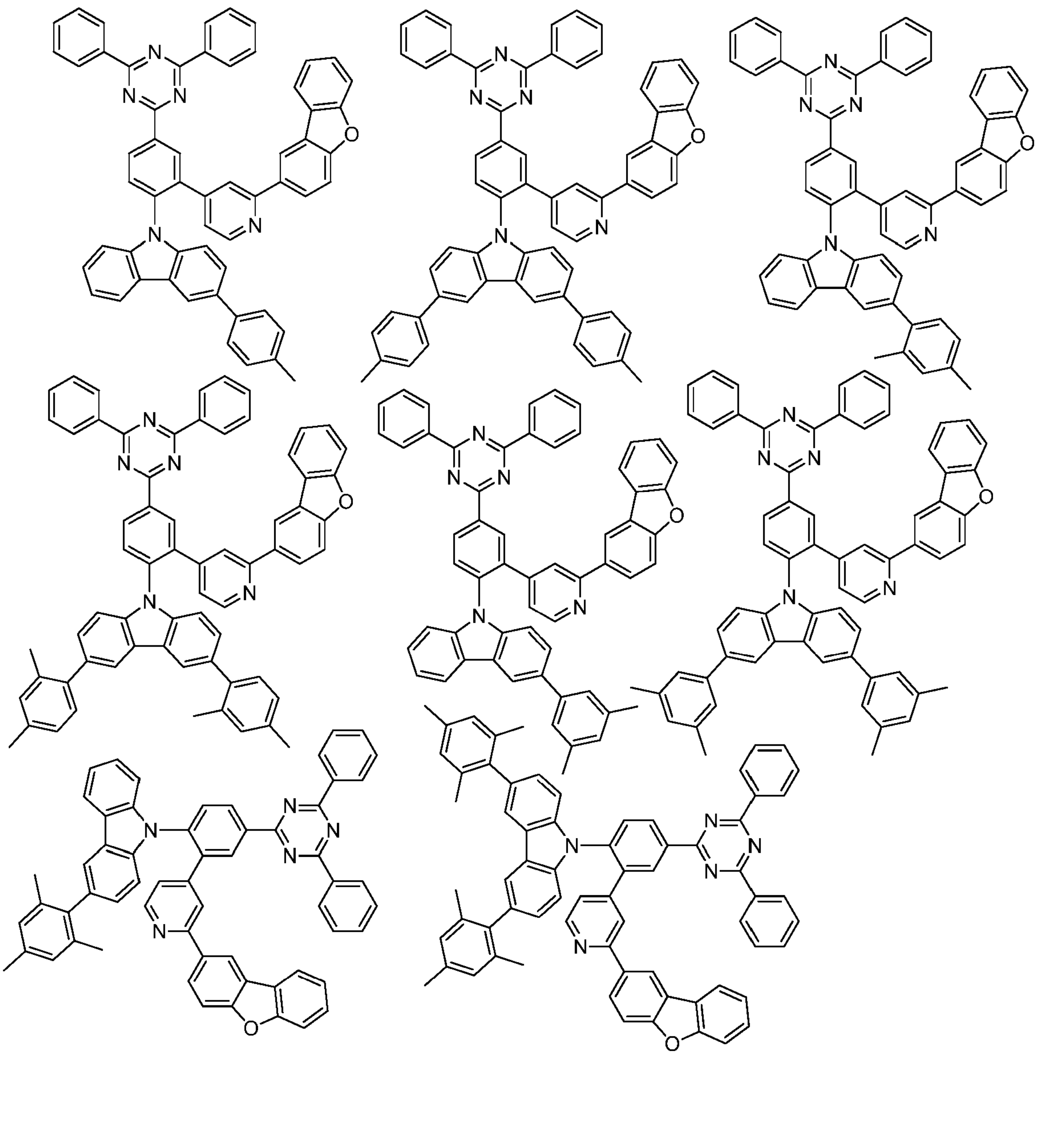

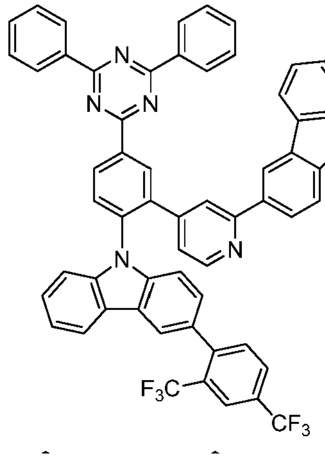

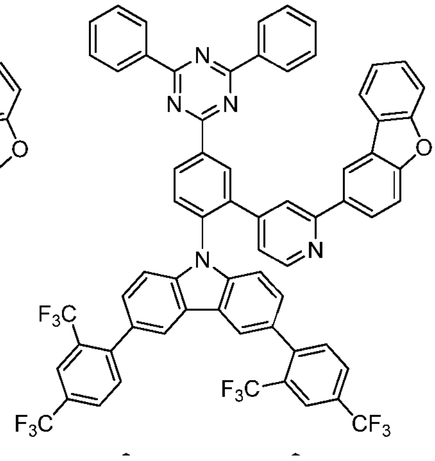

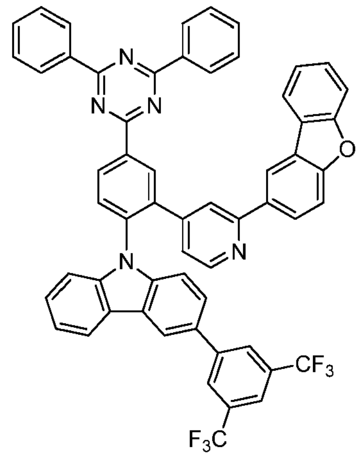

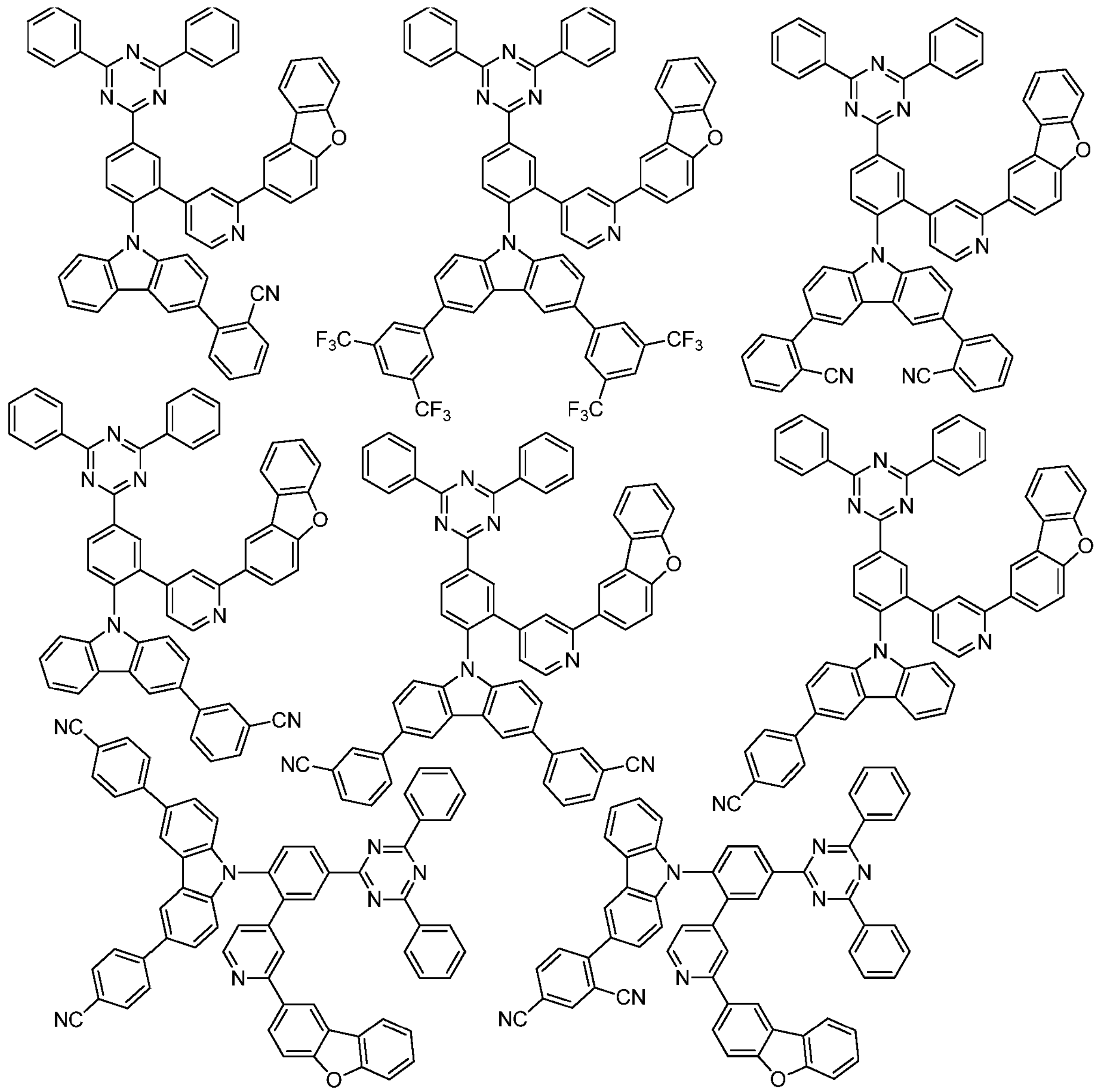

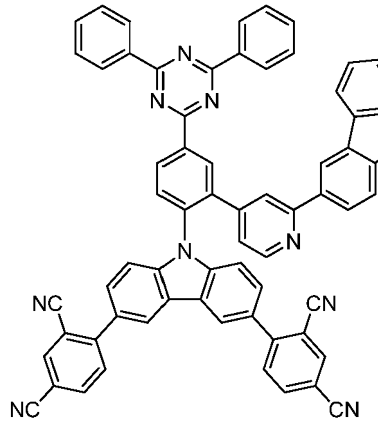
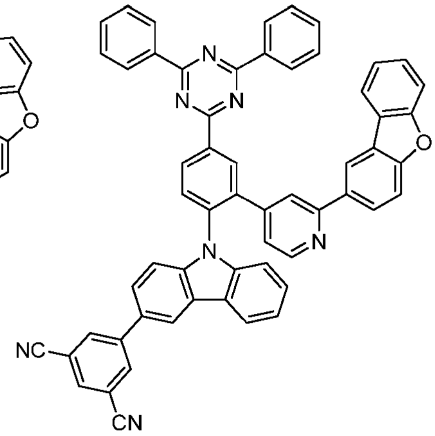
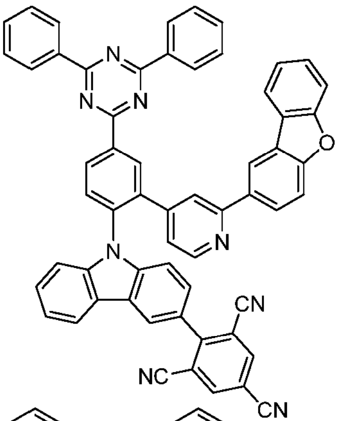
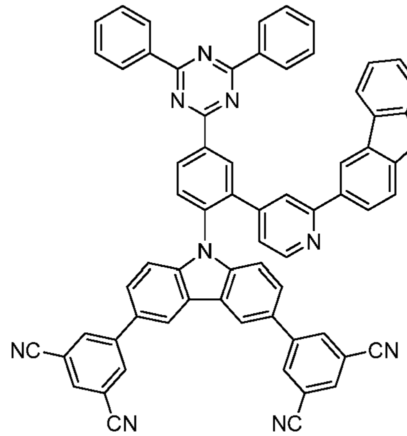
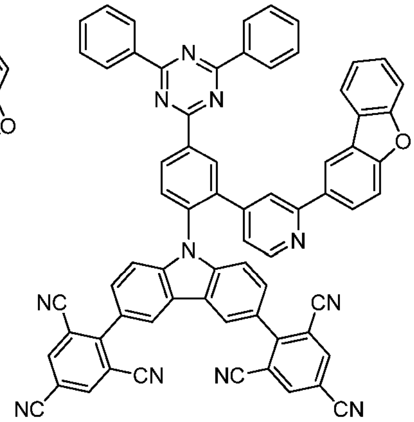
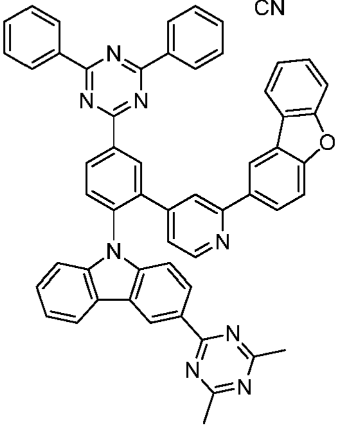
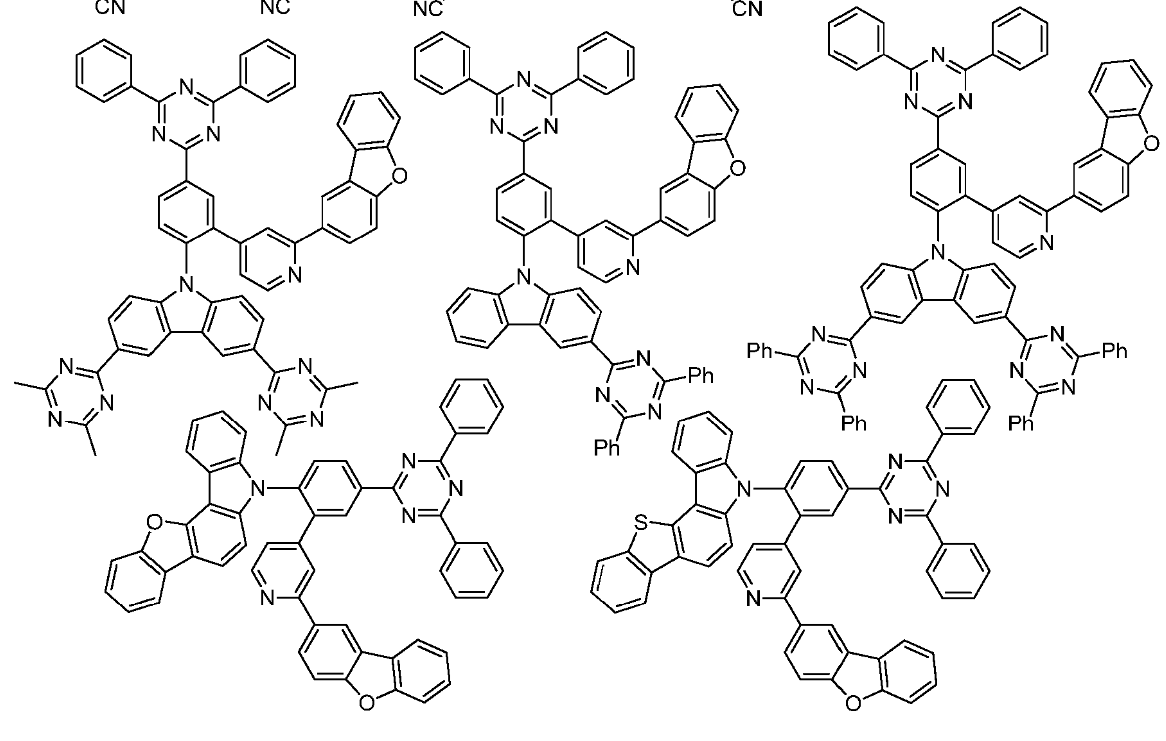

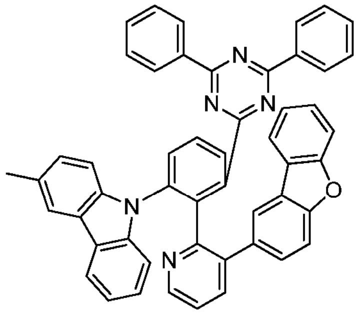 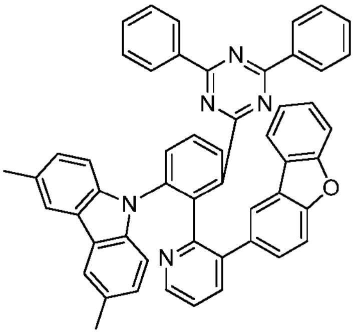 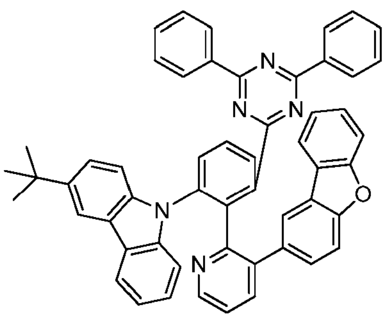

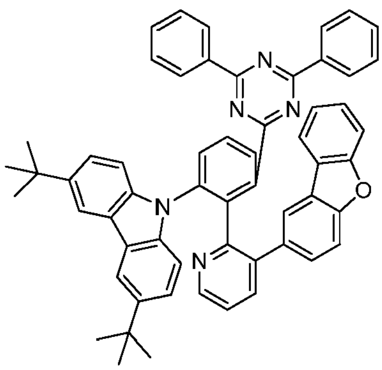 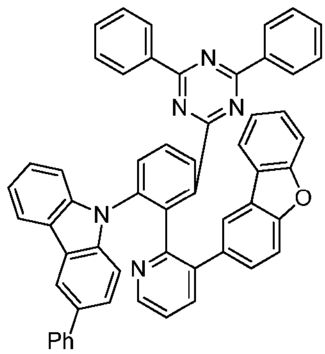 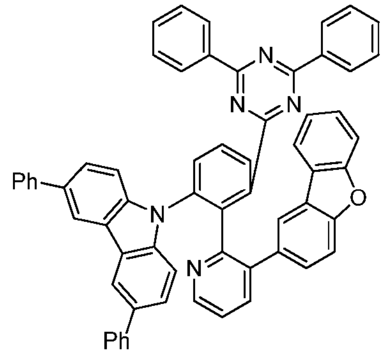

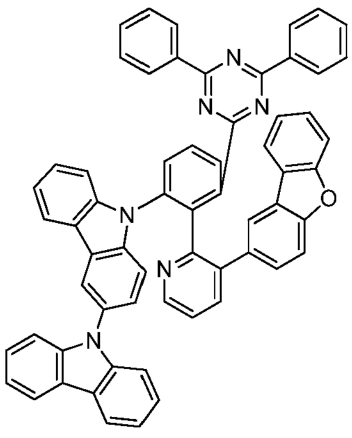 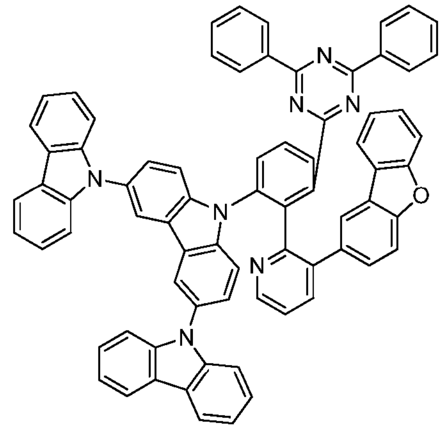 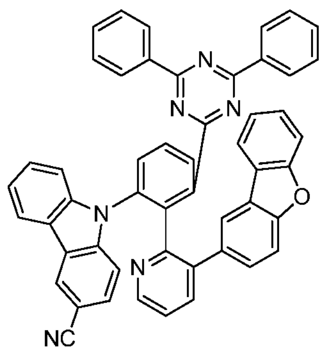

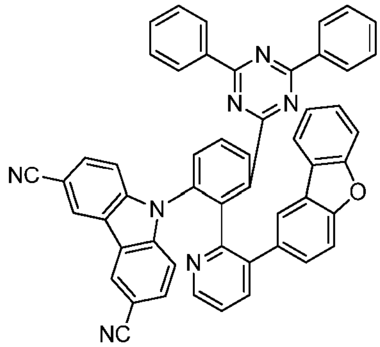 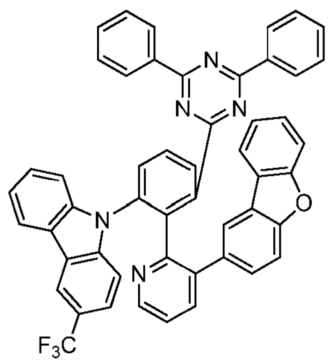 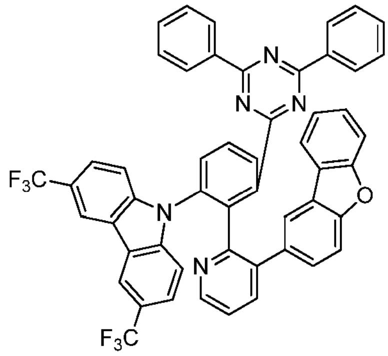

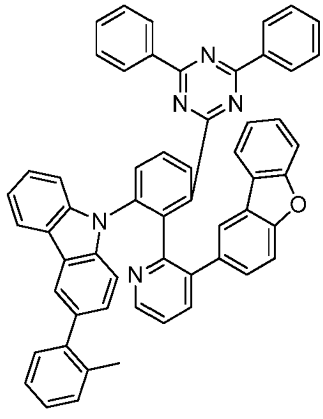

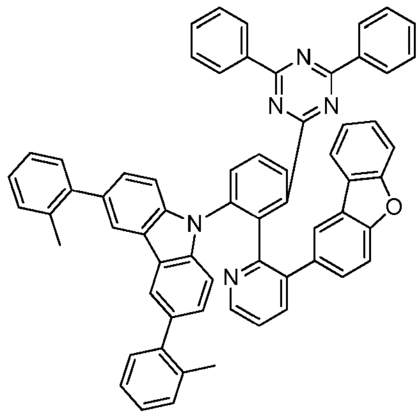

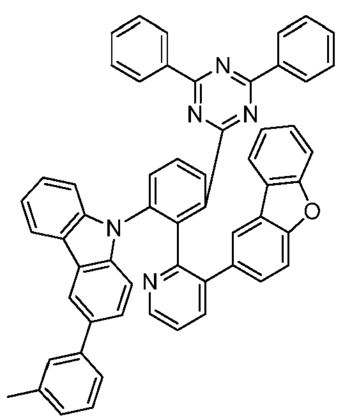

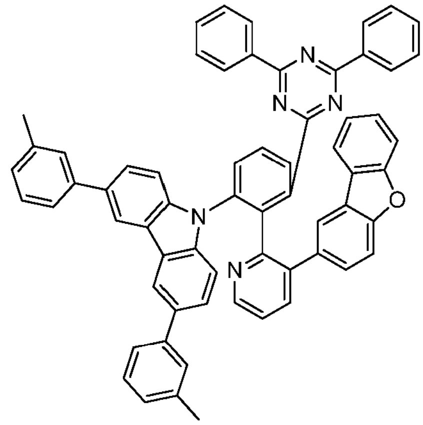

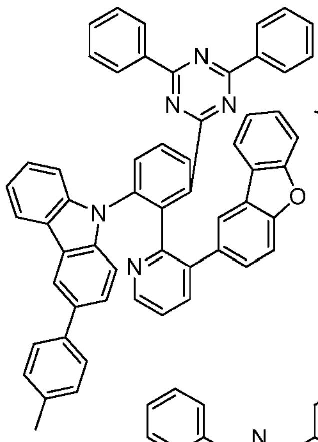

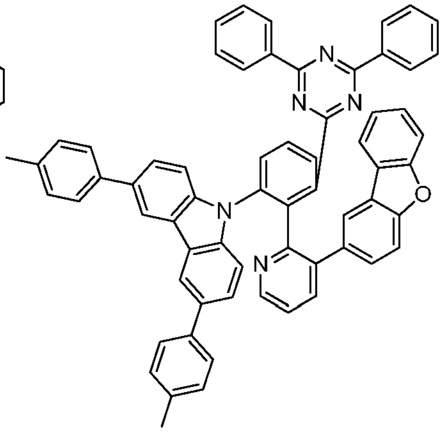

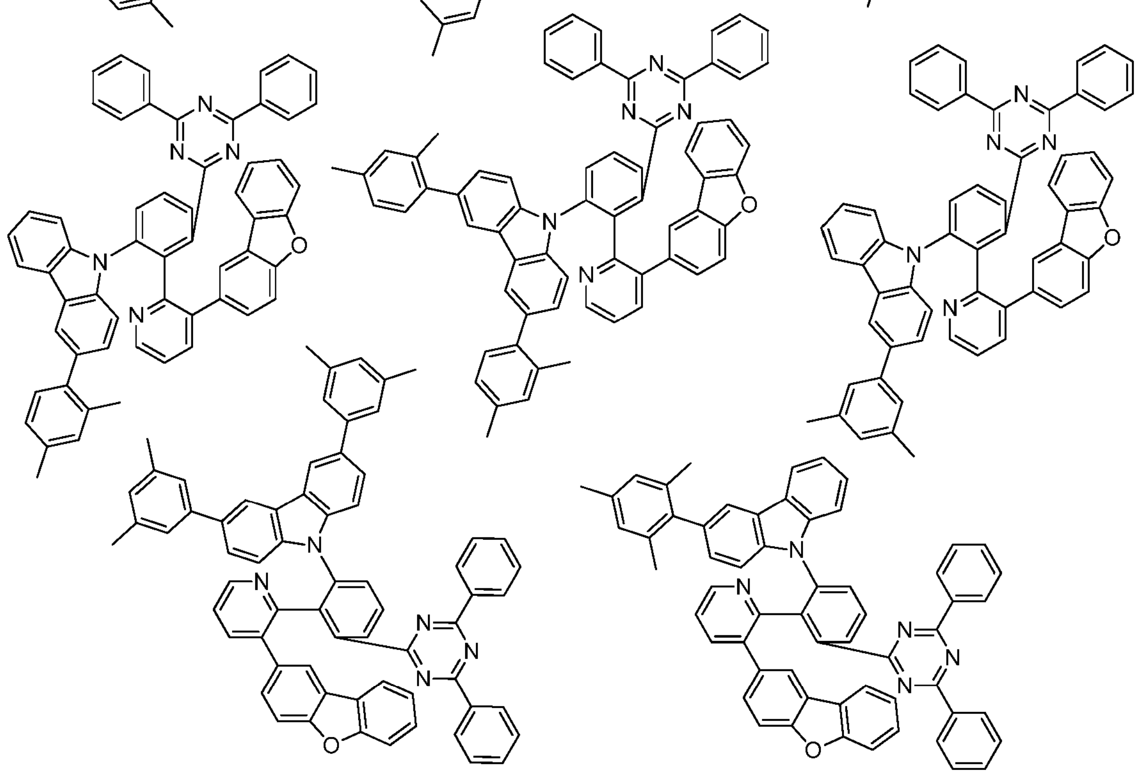

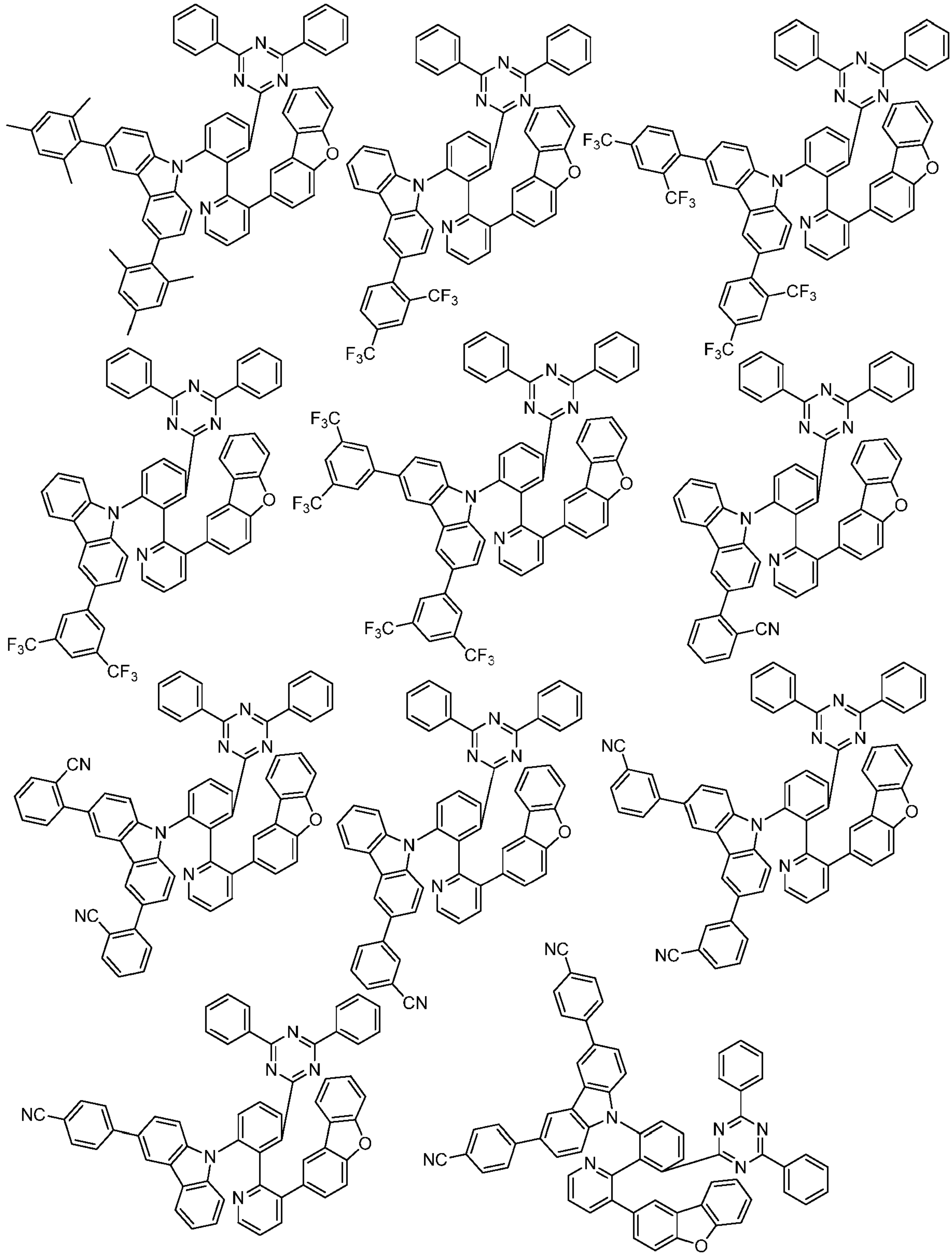

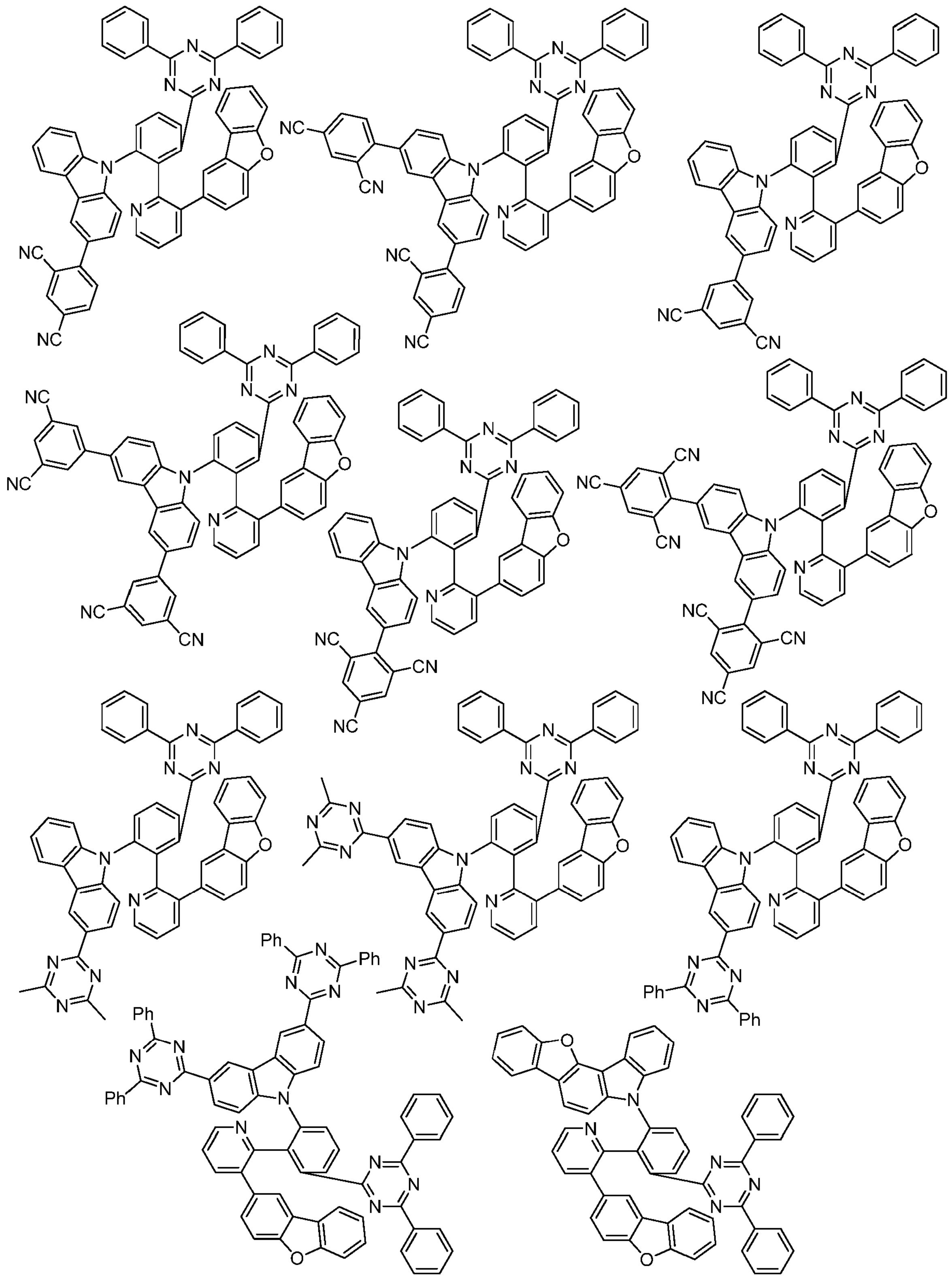

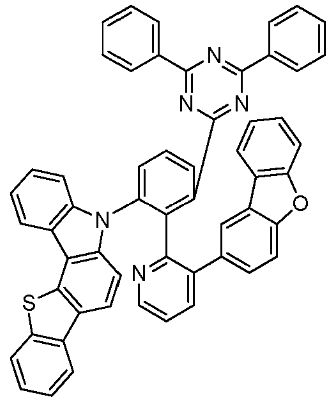
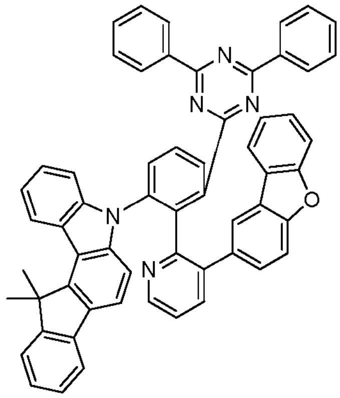
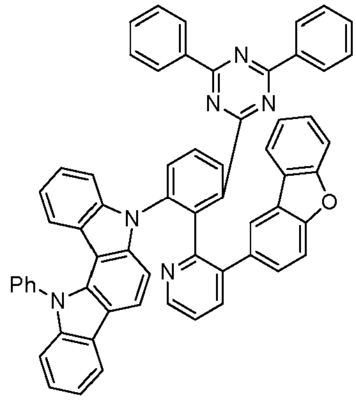

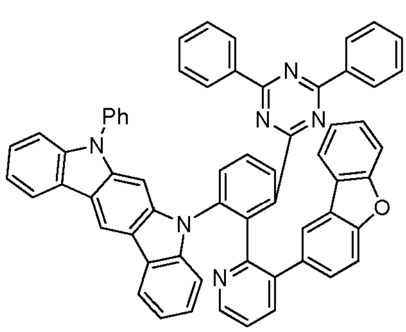
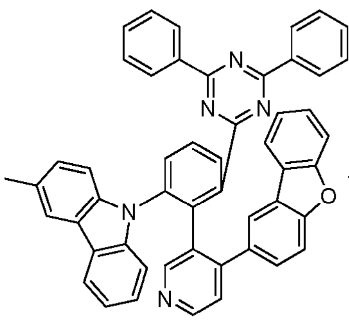
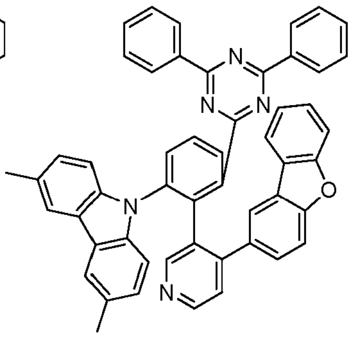

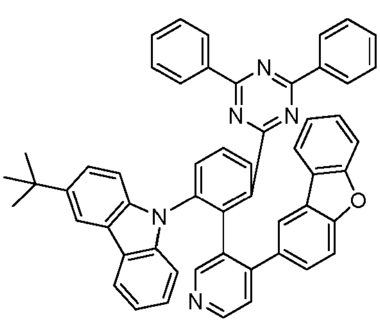
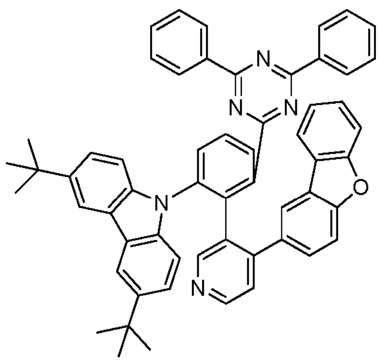
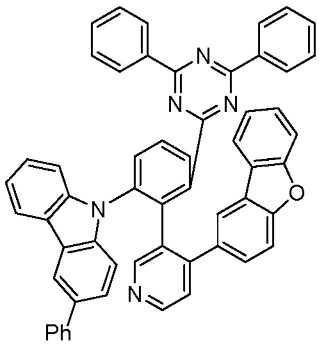

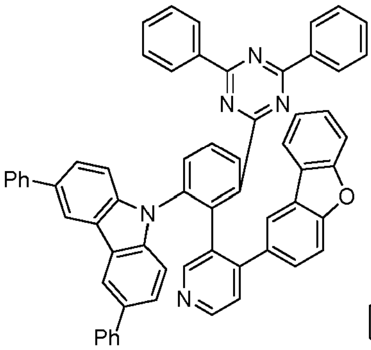
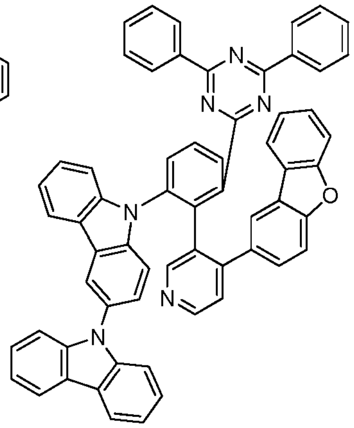
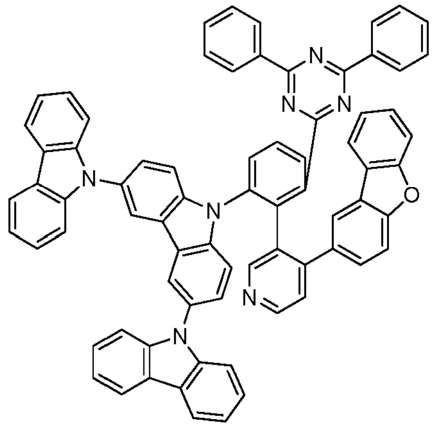

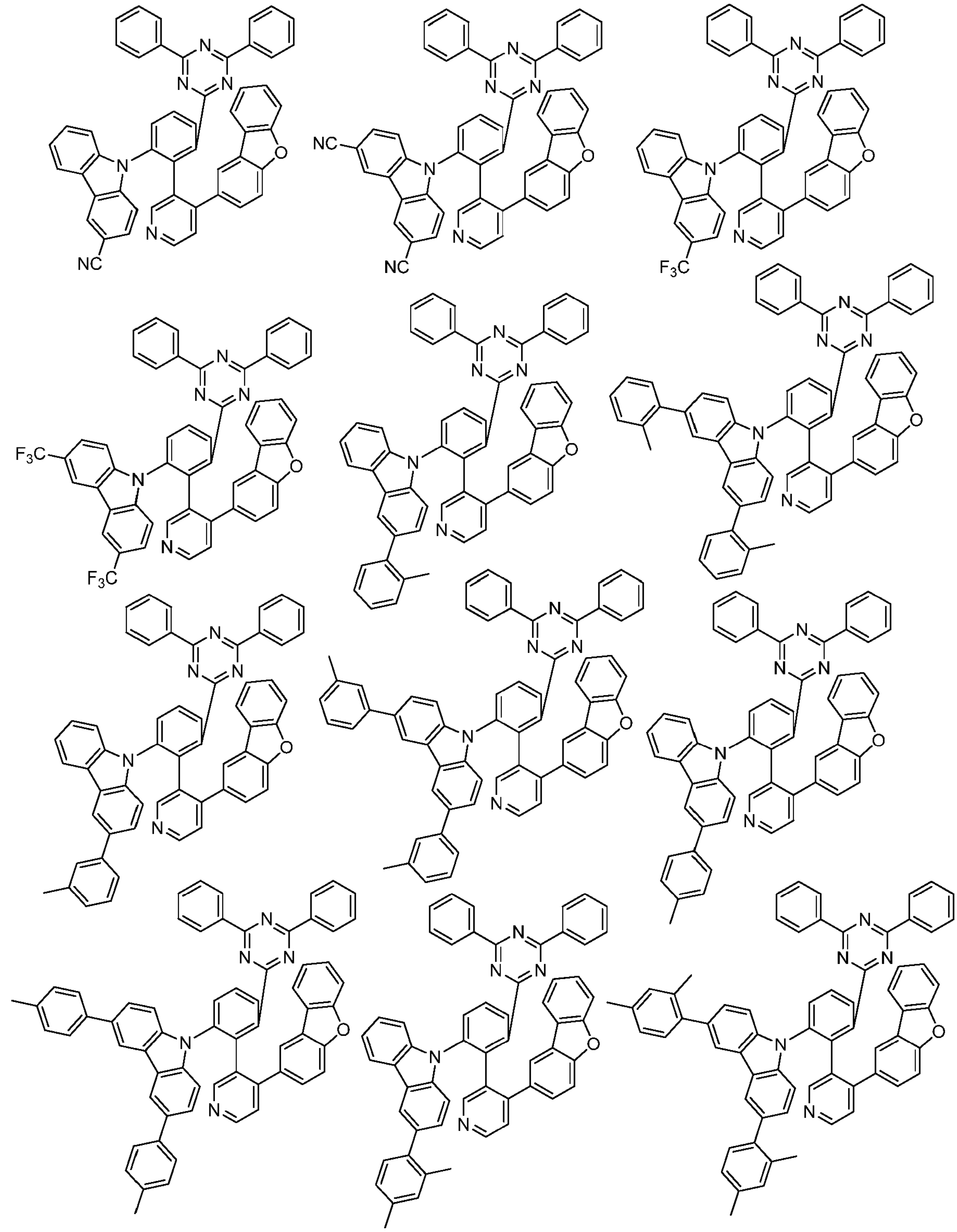

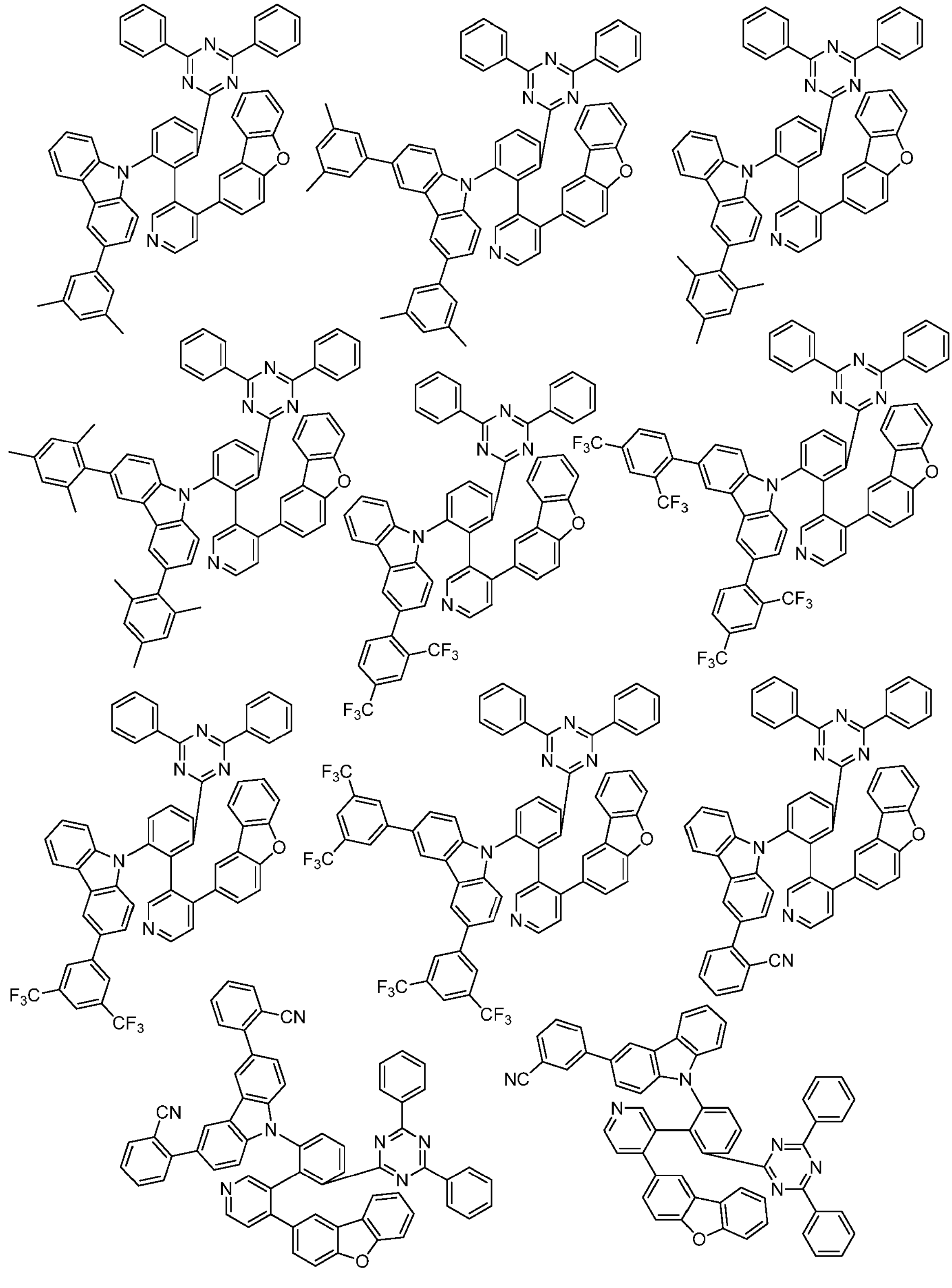

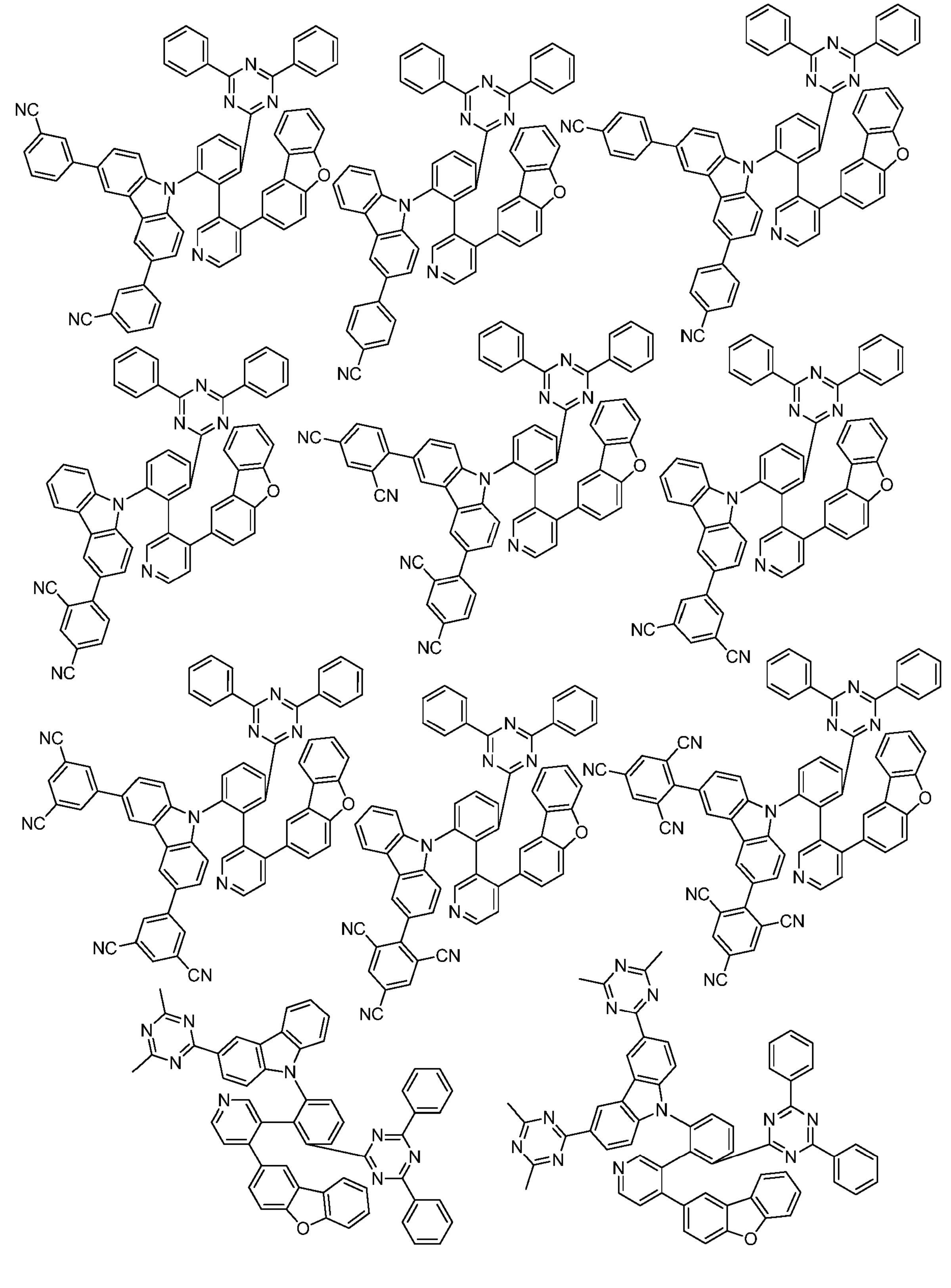

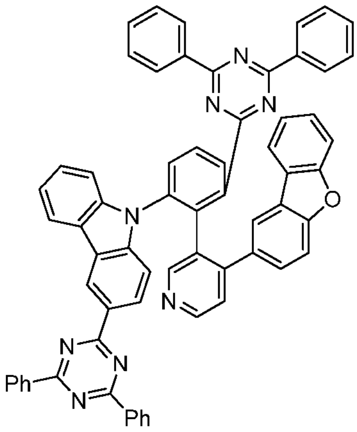
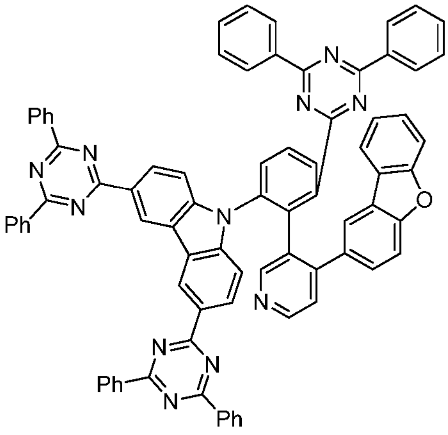
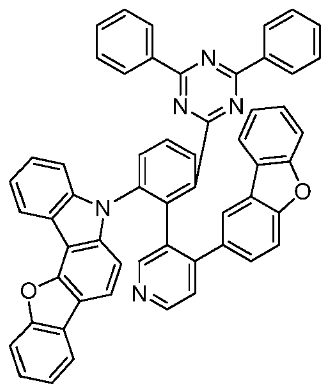

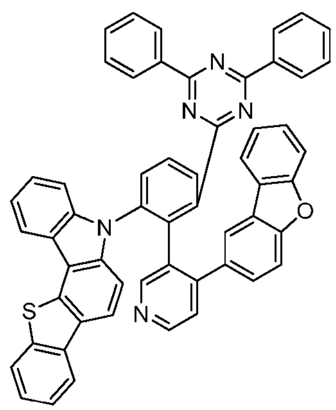
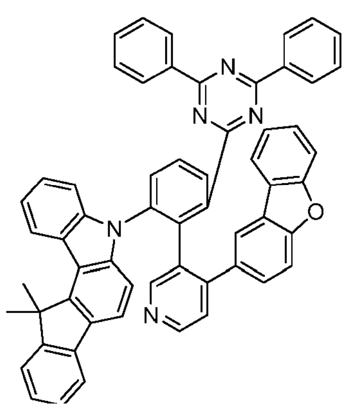
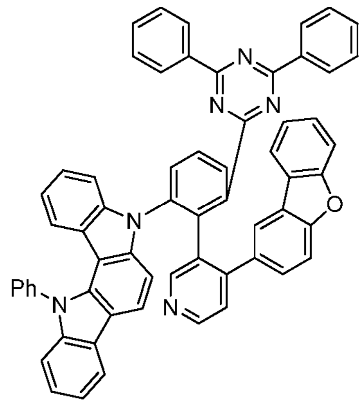

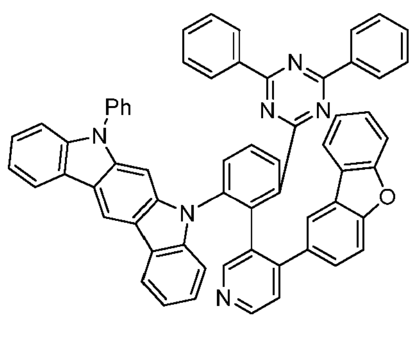
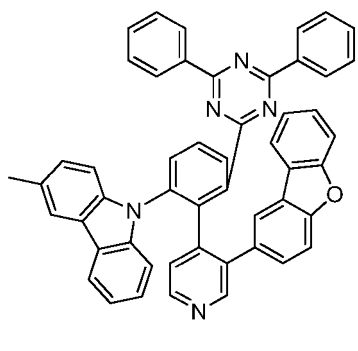
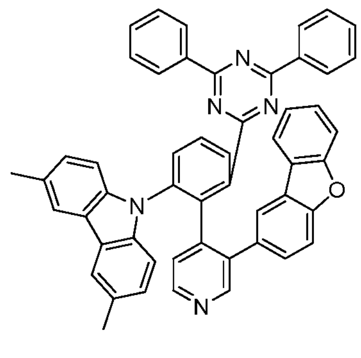

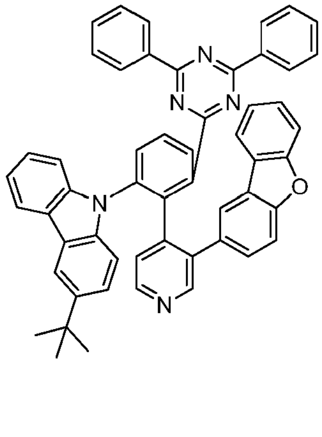
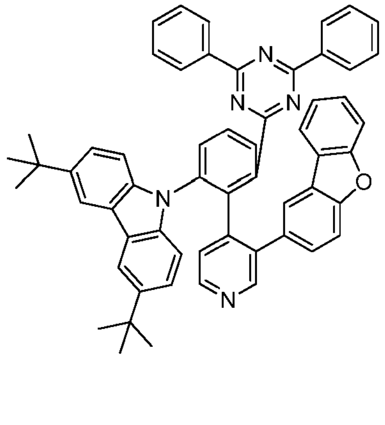
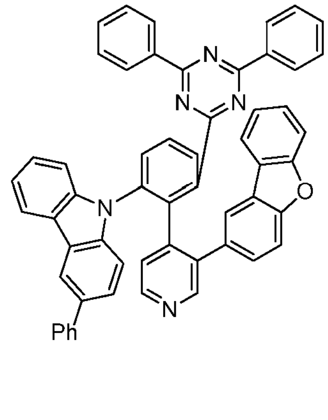

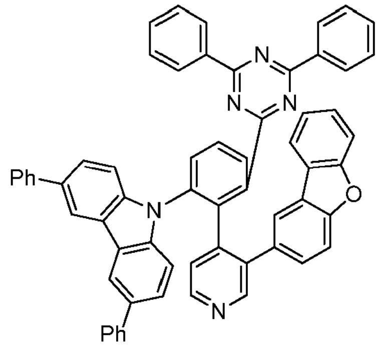
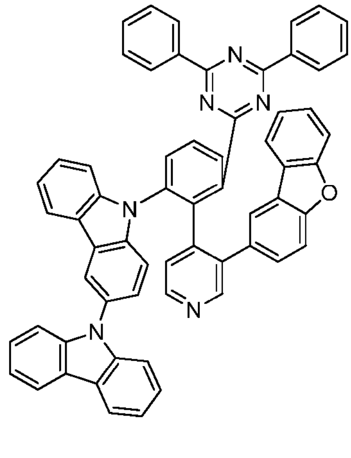
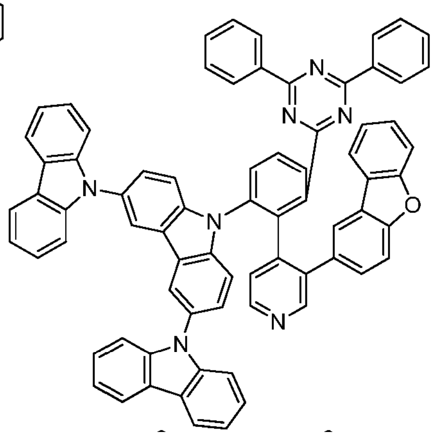

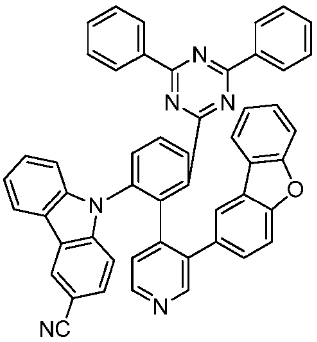
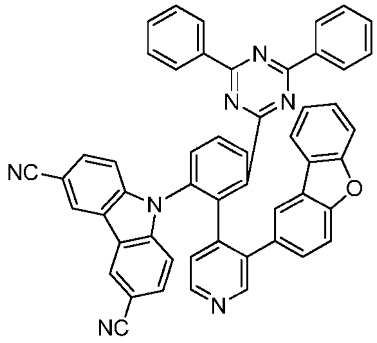
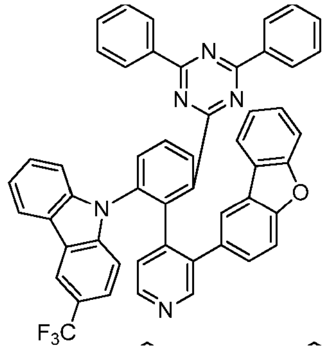

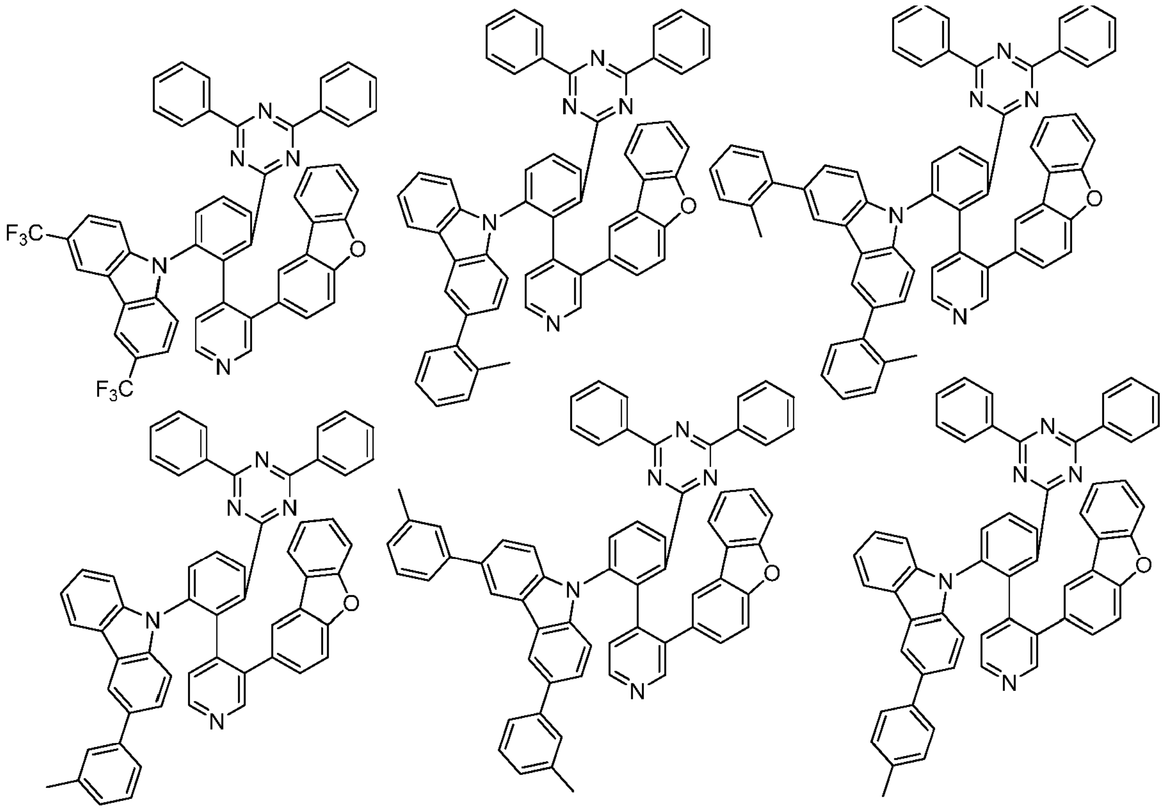

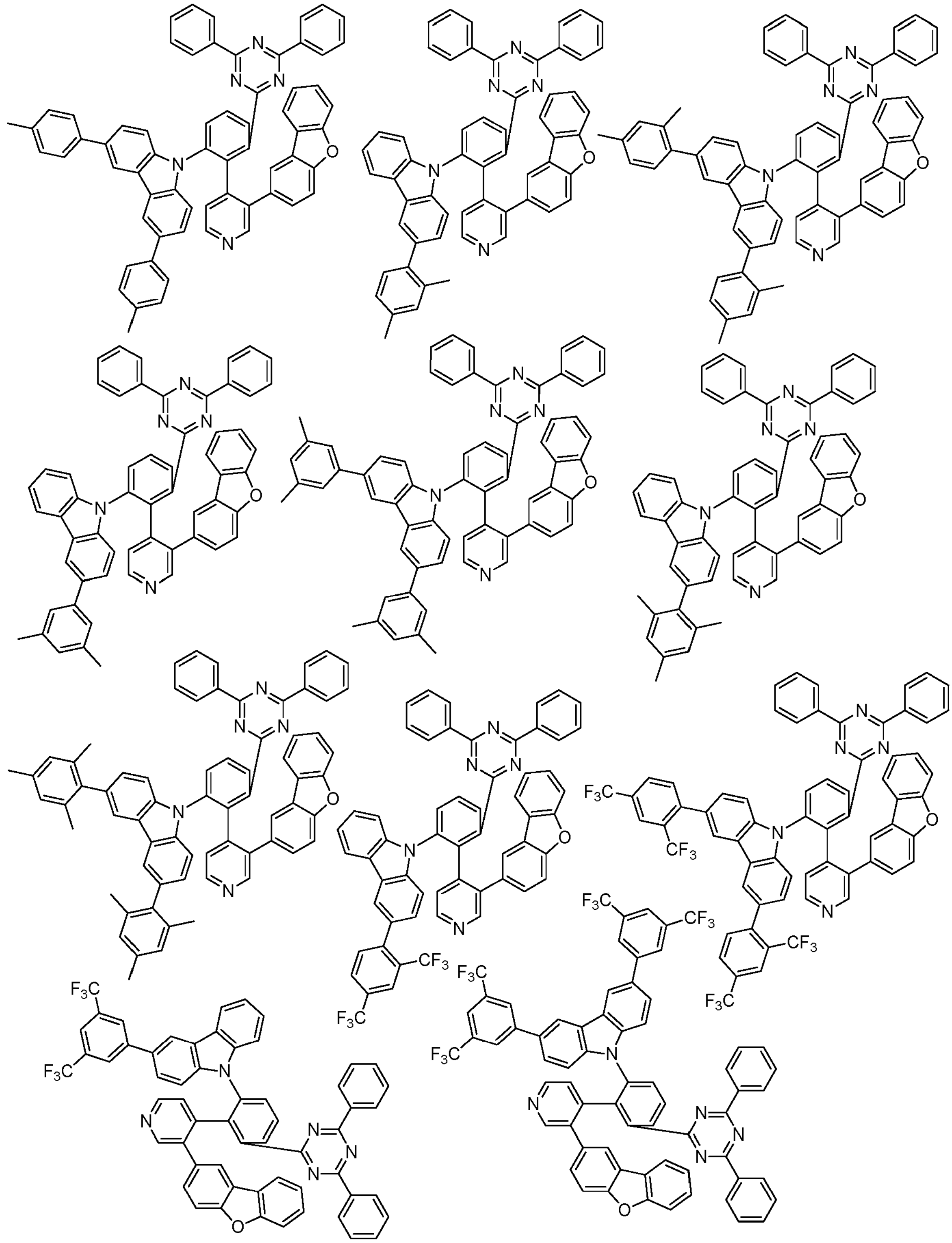

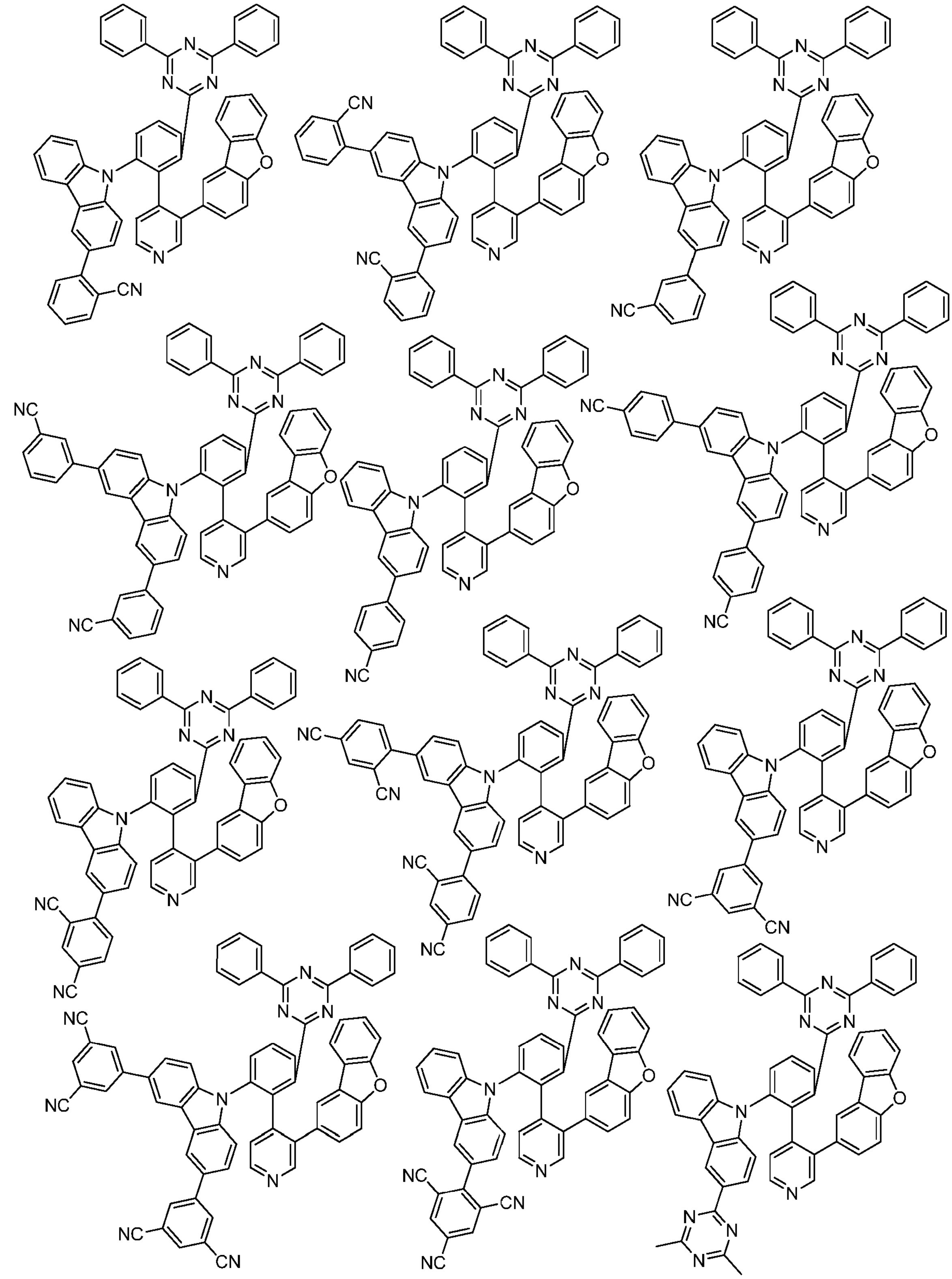

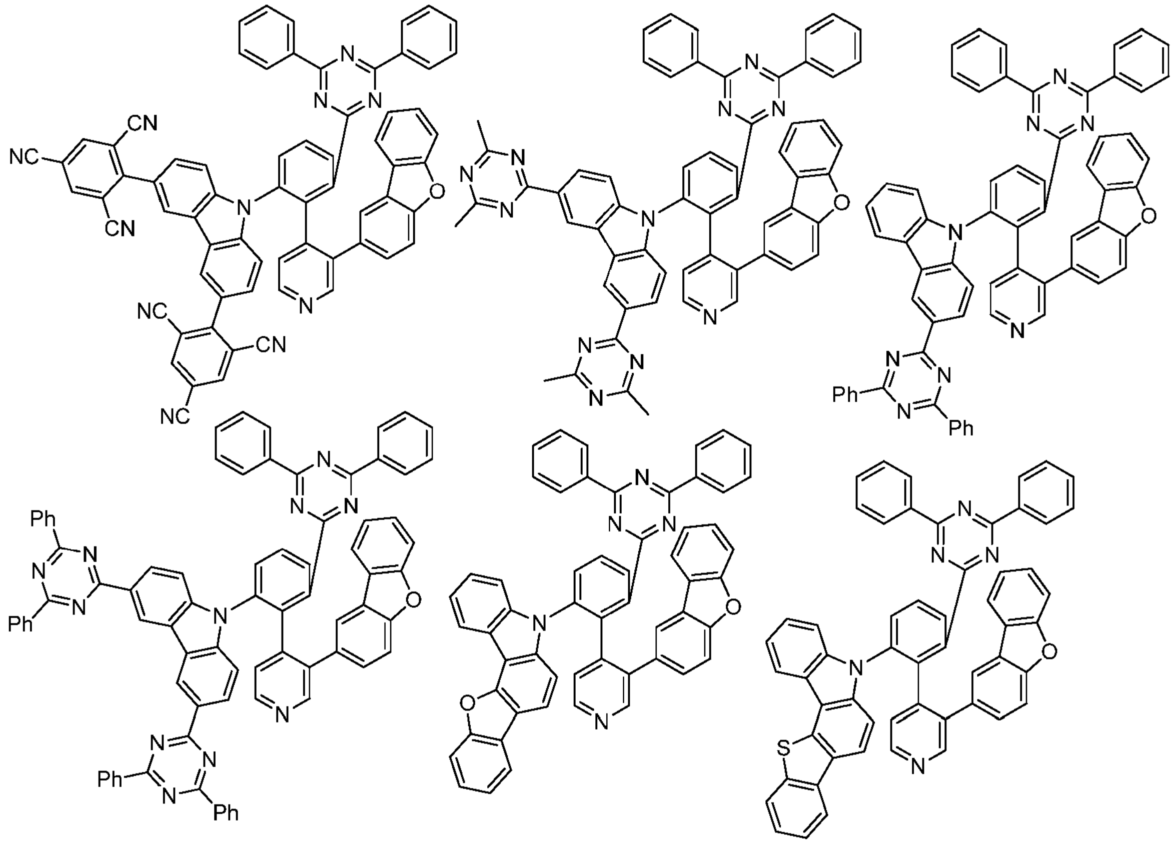

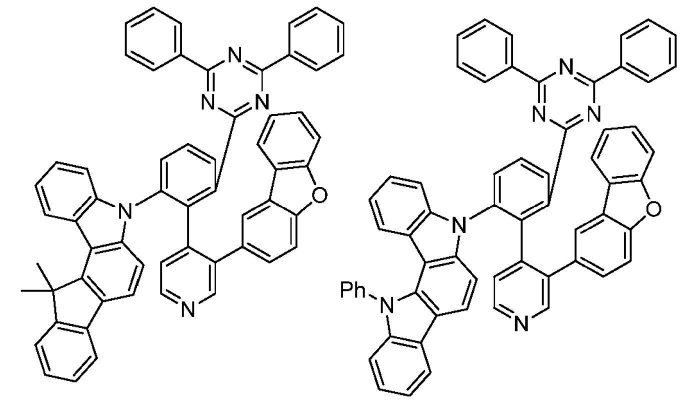

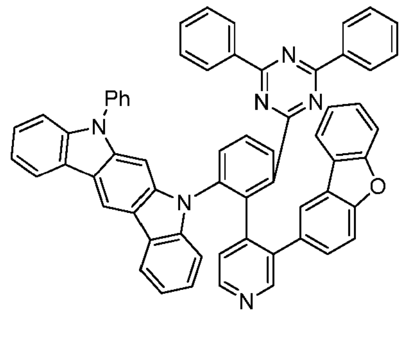

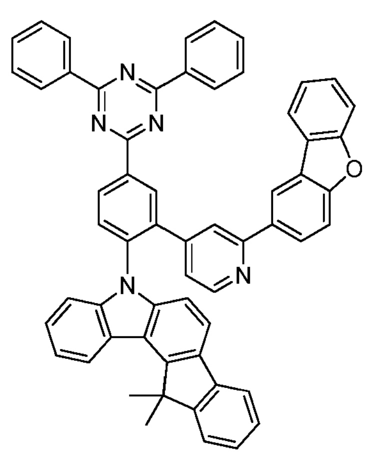

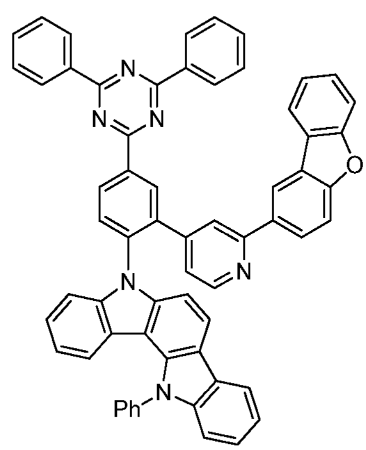

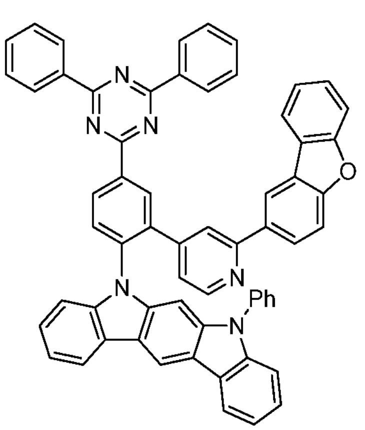

## Figures

The figures show:

Figure 1 Emission spectrum of example **1** (10% by weight) in PMMA.
Figure 2 Emission spectrum of example **2** (10% by weight) in PMMA.

## Claims

**1.** An organic molecule, comprising

- one first chemical moiety comprising a structure of Formula I,

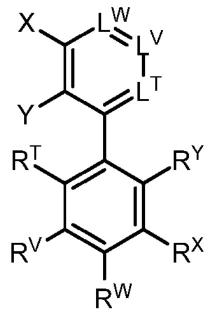

Formula I

- one second chemical moiety comprising a structure of Formula II,

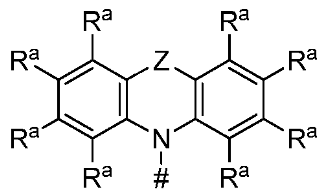

Formula II

wherein the first chemical moiety is linked to the second chemical moiety via a single bond; and
- one third chemical moiety comprising a structure of Formula III,

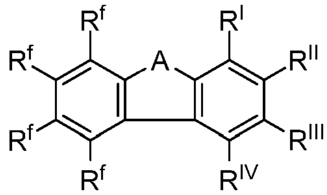

Formula III

wherein the first chemical moiety is linked to the third chemical moiety via a single bond;
wherein
$L^T$ is N or C-T;
$L^V$ is N or C-V;
$L^W$ is N or C-W;
T is the binding site of a single bond linking the first chemical moiety to the third chemical moiety or is $R^1$;
V is the binding site of a single bond linking the first chemical moiety to the third chemical moiety or is $R^1$;
W is the binding site of a single bond linking the first chemical moiety to the third chemical moiety or is $R^1$;
X is the binding site of a single bond linking the first chemical moiety to the third chemical moiety or is $R^1$;

Y is the binding site of a single bond linking the first chemical moiety to the third chemical moiety or is $R^1$;

$R^T$ is selected from the group consisting of $R^A$ and $R^2$;
$R^V$ is selected from the group consisting of $R^A$ and $R^2$;
$R^W$ is the binding site of a single bond linking the first chemical moiety to the second chemical moiety or is selected from the group consisting of $R^A$ and $R^2$;
$R^X$ is selected from the group consisting of $R^A$ and $R^2$;
$R^Y$ is the binding site of a single bond linking the first chemical moiety to the second chemical moiety or is selected from the group consisting of $R^A$ and $R^2$;

# represents the binding site of a single bond linking the second chemical moiety to the first chemical moiety;

Z is at each occurrence independently from another selected from the group consisting of a direct bond, $CR^3R^4$, $C=CR^3R^4$, C=O, $C=NR^3$, $NR^3$, O, $SiR^3R^4$, S, S(O) and $S(O)_2$;

A is selected from the group consisting of O, S and N-Ph;
$R^I$, $R^{II}$, $R^{III}$, and $R^{IV}$ is at each occurrence independently from another selected from the group consisting of $ and $R^d$;
$ represents the binding site of a single bond linking the first chemical moiety to the third chemical moiety;
$R^d$ is at each occurrence independently from another selected from the group consisting of hydrogen, deuterium,
$C_6$-$C_{18}$-aryl,
which is optionally substituted with one or more substituents independently from another selected from the group consisting of

$C_1$-$C_5$-alkyl,
wherein optionally one or more hydrogen atoms are independently from each other substituted by deuterium, CN, $CF_3$, or F;
and
$C_6$-$C_{18}$-aryl,

which is optionally substituted with one or more $C_1$-$C_5$-alkyl substituents;
$R^A$ comprises a structure of Formula Tz:

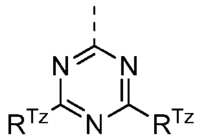

Formula Tz

wherein the dotted bond represents the binding site of $R^A$ to the single bond linking the first chemical moiety and $R^A$;
$R^1$ is independently from each other at each occurrence independently from another selected from the group consisting of:

hydrogen, deuterium,
$C_1$-$C_5$-alkyl,
wherein one or more hydrogen atoms are optionally substituted by deuterium;
$C_2$-$C_8$-alkenyl,
wherein one or more hydrogen atoms are optionally substituted by deuterium;
$C_2$-$C_8$-alkynyl,
wherein one or more hydrogen atoms are optionally substituted by deuterium; and
$C_6$-$C_{18}$-aryl,
which is optionally substituted with one or more substituents $R^6$;
$R^2$ is independently from each other at each occurrence independently from another selected from the group consisting of:

hydrogen, deuterium,
$C_1$-$C_5$-alkyl,
wherein one or more hydrogen atoms are optionally substituted by deuterium;
$C_2$-$C_8$-alkenyl,
wherein one or more hydrogen atoms are optionally substituted by deuterium;
$C_2$-$C_8$-alkynyl,
wherein one or more hydrogen atoms are optionally substituted by deuterium; and
$C_6$-$C_{18}$-aryl,
which is optionally substituted with one or more substituents $R^6$;
$R^{Tz}$ is at each occurrence independently from another selected from the group consisting of:
hydrogen, deuterium,
$C_1$-$C_5$-alkyl,
wherein one or more hydrogen atoms are optionally substituted by deuterium;
$C_6$-$C_{18}$-aryl,
which is optionally substituted with one or more substituents $R^6$, and
$C_3$-$C_{17}$-heteroaryl,
which is optionally substituted with one or more substituents $R^6$;
$R^a$, $R^3$ and $R^4$ is at each occurrence independently from another selected from the group consisting of: hydrogen, deuterium, $N(R^5)_2$, $OR^5$, $Si(R^5)_3$, $B(OR^5)_2$, $OSO_2R^5$, $CF_3$, CN, F, Br, I,
$C_1$-$C_{40}$-alkyl,

which is optionally substituted with one or more substituents $R^5$ and
wherein one or more non-adjacent $CH_2$-groups are optionally substituted by $R^5C=CR^5$, C≡C, $Si(R^5)_2$, $Ge(R^5)_2$, $Sn(R^5)_2$, C=O, C=S, C=Se, $C=NR^5$, $P(=O)(R^5)$, SO, $SO_2$, $NR^5$, O, S or $CONR^5$;

$C_1$-$C_{40}$-alkoxy,

which is optionally substituted with one or more substituents $R^5$ and
wherein one or more non-adjacent $CH_2$-groups are optionally substituted by $R^5C=CR^5$, C≡C, $Si(R^5)_2$, $Ge(R^5)_2$, $Sn(R^5)_2$, C=O, C=S, C=Se, $C=NR^5$, $P(=O)(R^5)$, SO, $SO_2$, $NR^5$, O, S or $CONR^5$;

$C_1$-$C_{40}$-thioalkoxy,

which is optionally substituted with one or more substituents $R^5$ and
wherein one or more non-adjacent $CH_2$-groups are optionally substituted by $R^5C=CR^5$, C≡C, $Si(R^5)_2$, $Ge(R^5)_2$, $Sn(R^5)_2$, C=O, C=S, C=Se, $C=NR^5$, $P(=O)(R^5)$, SO, $SO_2$, $NR^5$, O, S or $CONR^5$;

$C_2$-$C_{40}$-alkenyl,

which is optionally substituted with one or more substituents $R^5$ and
wherein one or more non-adjacent $CH_2$-groups are optionally substituted by $R^5C=CR^5$, C≡C, $Si(R^5)_2$, $Ge(R^5)_2$, $Sn(R^5)_2$, C=O, C=S, C=Se, $C=NR^5$, $P(=O)(R^5)$, SO, $SO_2$, $NR^5$, O, S or $CONR^5$;

$C_2$-$C_{40}$-alkynyl,

which is optionally substituted with one or more substituents $R^5$ and
wherein one or more non-adjacent $CH_2$-groups are optionally substituted by $R^5C=CR^5$, C≡C, $Si(R^5)_2$, $Ge(R^5)_2$, $Sn(R^5)_2$, C=O, C=S, C=Se, $C=NR^5$, $P(=O)(R^5)$, SO, $SO_2$, $NR^5$, O, S or $CONR^5$;

$C_6$-$C_{60}$-aryl,
which is optionally substituted with one or more substituents $R^5$; and
$C_3$-$C_{57}$-heteroaryl,
which is optionally substituted with one or more substituents $R^5$;

$R^5$ is at each occurrence independently from another selected from the group consisting of hydrogen, deuterium, $N(R^6)_2$, $OR^6$, $Si(R^6)_3$, $B(OR^6)_2$, $OSO_2R^6$, $CF_3$, CN, **F,** Br, I,
$C_1$-$C_{40}$-alkyl,

which is optionally substituted with one or more substituents $R^6$ and
wherein one or more non-adjacent $CH_2$-groups are optionally substituted by $R^6C=CR^6$, C≡C, $Si(R^6)_2$, $Ge(R^6)_2$, $Sn(R^6)_2$, C=O, C=S, C=Se, $C=NR^6$, $P(=O)(R^6)$, SO, $SO_2$, $NR^6$, O, S or $CONR^6$;

$C_1$-$C_{40}$-alkoxy,

which is optionally substituted with one or more substituents $R^6$ and
wherein one or more non-adjacent $CH_2$-groups are optionally substituted by $R^6C=CR^6$, C≡C, $Si(R^6)_2$, $Ge(R^6)_2$, $Sn(R^6)_2$, C=O, C=S, C=Se, $C=NR^6$, $P(=O)(R^6)$, SO, $SO_2$, $NR^6$, O, S or $CONR^6$;

$C_1$-$C_{40}$-thioalkoxy,

which is optionally substituted with one or more substituents $R^6$ and
wherein one or more non-adjacent $CH_2$-groups are optionally substituted by $R^6C=CR^6$, C≡C, $Si(R^6)_2$, $Ge(R^6)_2$, $Sn(R^6)_2$, C=O, C=S, C=Se, $C=NR^6$, $P(=O)(R^6)$, SO, $SO_2$, $NR^6$, O, S or $CONR^6$;

$C_2$-$C_{40}$-alkenyl,

which is optionally substituted with one or more substituents $R^6$ and
wherein one or more non-adjacent $CH_2$-groups are optionally substituted by $R^6C=CR^6$, C≡C, $Si(R^6)_2$, $Ge(R^6)_2$, $Sn(R^6)_2$, C=O, C=S, C=Se, $C=NR^6$, $P(=O)(R^6)$, SO, $SO_2$, $NR^6$, O, S or $CONR^6$;

$C_2$-$C_{40}$-alkynyl,

which is optionally substituted with one or more substituents $R^6$ and
wherein one or more non-adjacent $CH_2$-groups are optionally substituted by $R^6C=CR^6$, C≡C, $Si(R^6)_2$, $Ge(R^6)_2$, $Sn(R^6)_2$, C=O, C=S, C=Se, $C=NR^6$, $P(=O)(R^6)$, SO, $SO_2$, $NR^6$, O, S or $CONR^6$;

$C_6$-$C_{60}$-aryl,
which is optionally substituted with one or more substituents $R^6$; and
$C_3$-$C_{57}$-heteroaryl,
which is optionally substituted with one or more substituents $R^6$;
$R^f$ is at each occurrence independently from another selected from the group consisting of hydrogen,
deuterium,

$N(R^{5f})_2$,

$OR^{5f}$,

$Si(R^{5f})_3$,

$B(OR^{5f})_2$,

$OSO_2R^{5f}$,

$CF_3$,

CN,

F,

Br,

I,

$C_1$-$C_{40}$-alkyl,

which is optionally substituted with one or more substituents $R^{5f}$ and wherein one or more non-adjacent $CH_2$-groups are optionally substituted by $R^{5f}C=CR^{5f}$, C≡C, $Si(R^{5f})_2$, $Ge(R^{5f})_2$, $Sn(R^{5f})_2$, C=O, C=S, C=Se, $C=NR^{5f}$, $P(=O)(R^{5f})$, SO, $SO_2$, $NR^{5f}$, O, S or $CONR^{5f}$;

$C_1$-$C_{40}$-alkoxy,

which is optionally substituted with one or more substituents $R^{5f}$ and wherein one or more non-adjacent $CH_2$-groups are optionally substituted by $R^{5f}C=CR^{5f}$, C≡C, $Si(R^{5f})_2$, $Ge(R^{5f})_2$, $Sn(R^{5f})_2$, C=O, C=S, C=Se, $C=NR^{5f}$, $P(=O)(R^{5f})$, SO, $SO_2$, $NR^{5f}$, O, S or $CONR^{5f}$;

$C_1$-$C_{40}$-thioalkoxy,

which is optionally substituted with one or more substituents $R^{5f}$ and wherein one or more non-adjacent $CH_2$-groups are optionally substituted by $R^{5f}C=CR^{5f}$, C≡C, $Si(R^{5f})_2$, $Ge(R^{5f})_2$, $Sn(R^{5f})_2$, C=O, C=S, C=Se, $C=NR^{5f}$, $P(=O)(R^{5f})$, SO, $SO_2$, $NR^{5f}$, O, S or $CONR^{5f}$;

$C_2$-$C_{40}$-alkenyl,

which is optionally substituted with one or more substituents $R^{5f}$ and wherein one or more non-adjacent $CH_2$-groups are optionally substituted by $R^{5f}C=CR^{5f}$, C≡C, $Si(R^{5f})_2$, $Ge(R^{5f})_2$, $Sn(R^{5f})_2$, C=O, C=S, C=Se, $C=NR^{5f}$, $P(=O)(R^{5f})$, SO, $SO_2$, $NR^{5f}$, O, S or $CONR^{5f}$;

$C_2$-$C_{40}$-alkynyl,

which is optionally substituted with one or more substituents $R^{5f}$ and wherein one or more non-adjacent $CH_2$-groups are optionally substituted by $R^{5f}C=CR^{5f}$, C≡C, $Si(R^{5f})_2$, $Ge(R^{5f})_2$, $Sn(R^{5f})_2$, C=O, C=S, C=Se, $C=NR^{5f}$, $P(=O)(R^{5f})$, SO, $SO_2$, $NR^{5f}$, O, S or $CONR^{5f}$;

$C_6$-$C_{60}$-aryl,
which is optionally substituted with one or more substituents $R^{5f}$; and
$C_3$-$C_{57}$-heteroaryl,
which is optionally substituted with one or more substituents $R^{5f}$.
$R^{5f}$ is at each occurrence independently from another selected from the group consisting of hydrogen,
deuterium,

$N(R^6)_2$,

$OR^6$,

$Si(R^6)_3$,

$B(OR^6)_2$,

$OSO_2R^6$,

$CF_3$,

CN,

F,

Br,

I,

$C_1$-$C_{40}$-alkyl,

which is optionally substituted with one or more substituents $R^6$ and wherein one or more non-adjacent $CH_2$-groups are optionally substituted by $R^6C=CR^6$, C≡C, $Si(R^6)_2$, $Ge(R^6)_2$, $Sn(R^6)_2$, C=O, C=S, C=Se, $C=NR^6$, $P(=O)(R^6)$, SO, $SO_2$, $NR^6$, O, S or $CONR^6$;

$C_1$-$C_{40}$-alkoxy,

which is optionally substituted with one or more substituents $R^6$ and wherein one or more non-adjacent $CH_2$-groups are optionally substituted by $R^6C=CR^6$, C≡C, $Si(R^6)_2$, $Ge(R^6)_2$, $Sn(R^6)_2$, C=O, C=S, C=Se, $C=NR^6$, $P(=O)(R^6)$, SO, $SO_2$, $NR^6$, O, S or $CONR^6$;

$C_1$-$C_{40}$-thioalkoxy,

which is optionally substituted with one or more substituents $R^6$ and wherein one or more non-adjacent $CH_2$-groups are optionally substituted by $R^6C=CR^6$, C≡C, $Si(R^6)_2$, $Ge(R^6)_2$, $Sn(R^6)_2$, C=O, C=S, C=Se, $C=NR^6$, $P(=O)(R^6)$, SO, $SO_2$, $NR^6$, O, S or $CONR^6$;

$C_2$-$C_{40}$-alkenyl,

which is optionally substituted with one or more substituents $R^6$ and wherein one or more non-adjacent $CH_2$-groups are optionally substituted by $R^6C=CR^6$, C≡C, $Si(R^6)_2$, $Ge(R^6)_2$, $Sn(R^6)_2$, C=O, C=S, C=Se, $C=NR^6$, $P(=O)(R^6)$, SO, $SO_2$, $NR^6$, O, S or $CONR^6$;

$C_2$-$C_{40}$-alkynyl,

which is optionally substituted with one or more substituents $R^6$ and wherein one or more non-adjacent $CH_2$-groups are optionally substituted by $R^6C=CR^6$, C≡C, $Si(R^6)_2$, $Ge(R^6)_2$, $Sn(R^6)_2$, C=O, C=S, C=Se, $C=NR^6$, $P(=O)(R^6)$, SO, $SO_2$, $NR^6$, O, S or $CONR^6$;

$C_6$-$C_{60}$-aryl,
which is optionally substituted with one or more substituents $R^6$; and
$C_3$-$C_{57}$-heteroaryl,
which is optionally substituted with one or more substituents $R^6$.
$R^6$ is at each occurrence independently from another selected from the group consisting of:

hydrogen, deuterium, OPh, $CF_3$, CN, F,
$C_1$-$C_5$-alkyl,
wherein one or more hydrogen atoms are optionally, independently from each other substituted by deuterium, CN, $CF_3$, or F;
$C_1$-$C_5$-alkoxy,

wherein one or more hydrogen atoms are optionally, independently from each other substituted by deuterium, CN, $CF_3$, or F;
$C_1$-$C_5$-thioalkoxy,
wherein one or more hydrogen atoms are optionally, independently from each other substituted by deuterium, CN, $CF_3$, or F;
$C_2$-$C_5$-alkenyl,
wherein one or more hydrogen atoms are optionally, independently from each other substituted by deuterium, CN, $CF_3$, or F;
$C_2$-$C_5$-alkynyl,
wherein one or more hydrogen atoms are optionally, independently from each other substituted by deuterium, CN, $CF_3$, or F;
$C_6$-$C_{18}$-aryl,
which is optionally substituted with one or more $C_1$-$C_5$-alkyl substituents;
$C_3$-$C_{17}$-heteroaryl,
which is optionally substituted with one or more $C_1$-$C_5$-alkyl substituents;
$N(C_6$-$C_{18}$-aryl$)_2$;
$N(C_3$-$C_{17}$-heteroaryl$)_2$,
and $N(C_3$-$C_{17}$-heteroaryl$)(C_6$-$C_{18}$-aryl$)$;
wherein the substituents $R^a$, $R^3$, $R^4$ or $R^5$ independently from each other optionally form a mono- or polycyclic, aliphatic, aromatic and/or benzo-fused ring system with one or more substituents $R^a$, $R^3$, $R^4$ or $R^5$;
optionally, one or more of the substituents $R^f$ or $R^{5f}$ independently from each other form a mono- or polycyclic, aliphatic, aromatic and/or benzo-fused ring system with one or more other substituents $R^f$ or $R^{5f}$;
wherein

- exactly one ring member selected from the group consisting of $L^T$, $L^V$ and $L^W$ is N,
- exactly one substituent selected from the group consisting of $R^T$, $R^V$, $R^W$, $R^X$ and $R^Y$ is $R^A$,
- exactly one substituent selected from the group consisting of T, V, W, X and Y is the binding
- site of a single bond linking the first chemical moiety to the third chemical moiety, and
- exactly one substituent selected from the group consisting of $R^W$ and $R^Y$ represents the binding site of a single bond linking the first chemical moiety and the second chemical moiety.

**2.** The organic molecule according to claim 1, wherein the first chemical moiety comprises a structure of Formula Ia:

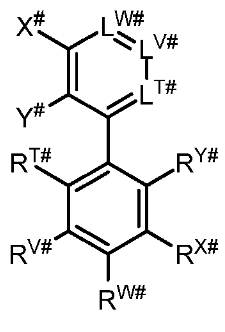

Formula Ia

wherein $R^1$, $R^2$, $R^I$ and $R^A$ are defined as above;
$L^{T\#}$ is N or C-$R^1$;
$L^{V\#}$ is N or C- $R^1$,
$L^{W\#}$ is N or C-$W^\#$;
$W^\#$ is the binding site of a single bond linking the first chemical moiety to the third chemical moiety or is $R^1$;
$X^\#$ is the binding site of a single bond linking the first chemical moiety to the third chemical moiety or is $R^1$;
$Y^\#$ is the binding site of a single bond linking the first chemical moiety to the third chemical moiety or is $R^1$;
$R^{T\#}$ is selected from the group consisting of $R^A$ and $R^2$;

$R^{V\#}$ is selected from the group consisting of $R^A$ and $R^2$;
$R^{W\#}$ is the binding site of a single bond linking the first chemical moiety to the second chemical moiety or is selected from the group consisting of $R^A$ and $R^2$;
$R^{X\#}$ is $R^2$;
$R^{Y\#}$ is the binding site of a single bond linking the first chemical moiety to the second chemical moiety or is $R^2$;
wherein
exactly one ring member (atom or group) selected from the group consisting of $L^{T\#}$, $L^{V\#}$ and $L^{W\#}$ is N;
exactly one substituent selected from the group consisting of $R^{T\#}$, $R^{V\#}$ and $R^{W\#}$ is $R^A$, exactly one substituent selected from the group consisting of $W^{\#}$, $X^{\#}$ and $Y^{\#}$ is the binding site of a single bond linking the first chemical moiety to the third chemical moiety
and exactly one substituent selected from the group consisting of $R^{W\#}$ and $R^{Y\#}$ represents the binding site of a single bond linking the first chemical moiety and the second chemical moiety;
and wherein apart from that the definitions in claim 1 apply.

3. The organic molecule according to claim 1 or 2, wherein $R^1$ and $R^2$ is independently from each other at each occurrence independently from another selected from the group consisting of H, methyl, mesityl, tolyl and phenyl.

4. The organic molecule according to one or more of claims 1 to 3, wherein $R^{Tz}$ is independently from each other selected from the group consisting of H, methyl and phenyl.

5. The organic molecule according to one or more of claims 1 to 4, wherein the second chemical moiety comprises a structure of Formula IIa:

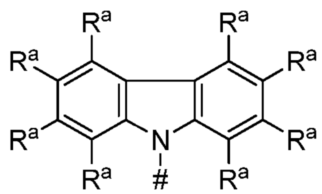

Formula IIa

wherein # and $R^a$ are defined as in claim 1.

6. The organic molecule according to one or more of claims 1 to 5, wherein the second chemical moiety comprises a structure of Formula IIb:

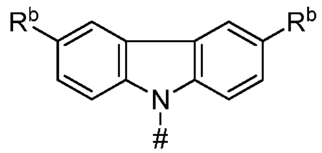

Formula IIb

wherein

$R^b$ is at each occurrence independently from another selected from the group consisting of: deuterium, $N(R^5)_2$, $OR^5$, $Si(R^5)_3$, $B(OR^5)_2$, $OSO_2R^5$, $CF_3$, CN, F, Br, I,
$C_1$-$C_{40}$-alkyl,

which is optionally substituted with one or more substituents $R^5$ and
wherein one or more non-adjacent $CH_2$-groups are optionally substituted by $R^5C=CR^5$, C≡C, $Si(R^5)_2$, $Ge(R^5)_2$, $Sn(R^5)_2$, C=O, C=S, C=Se, $C=NR^5$, $P(=O)(R^5)$, SO, $SO_2$, $NR^5$, O, S or $CONR^5$;

$C_1$-$C_{40}$-alkoxy,

which is optionally substituted with one or more substituents $R^5$ and

wherein one or more non-adjacent $CH_2$-groups are optionally substituted by $R^5C=CR^5$, C≡C, $Si(R^5)_2$, $Ge(R^5)_2$, $Sn(R^5)_2$, C=O, C=S, C=Se, $C=NR^5$, $P(=O)(R^5)$, SO, $SO_2$, $NR^5$, O, S or $CONR^5$;

$C_1$-$C_{40}$-thioalkoxy,

which is optionally substituted with one or more substituents $R^5$ and
wherein one or more non-adjacent $CH_2$-groups are optionally substituted by $R^5C=CR^5$, C≡C, $Si(R^5)_2$, $Ge(R^5)_2$, $Sn(R^5)_2$, C=O, C=S, C=Se, $C=NR^5$, $P(=O)(R^5)$, SO, $SO_2$, $NR^5$, O, S or $CONR^5$;

$C_2$-$C_{40}$-alkenyl,

which is optionally substituted with one or more substituents $R^5$ and
wherein one or more non-adjacent $CH_2$-groups are optionally substituted by $R^5C=CR^5$, C≡C, $Si(R^5)_2$, $Ge(R^5)_2$, $Sn(R^5)_2$, C=O, C=S, C=Se, $C=NR^5$, $P(=O)(R^5)$, SO, $SO_2$, $NR^5$, O, S or $CONR^5$;

$C_2$-$C_{40}$-alkynyl,

which is optionally substituted with one or more substituents $R^5$ and
wherein one or more non-adjacent $CH_2$-groups are optionally substituted by $R^5C=CR^5$, C≡C, $Si(R^5)_2$, $Ge(R^5)_2$, $Sn(R^5)_2$, C=O, C=S, C=Se, $C=NR^5$, $P(=O)(R^5)$, SO, $SO_2$, $NR^5$, O, S or $CONR^5$;

$C_6$-$C_{60}$-aryl,
which is optionally substituted with one or more substituents $R^5$; and
$C_3$-$C_{57}$-heteroaryl,
which is optionally substituted with one or more substituents $R^5$;
and wherein apart from that the definitions in claim 1 apply.

**7.** The organic molecule according to one or more of claims 1 to 5, wherein the second chemical moiety comprises a structure of Formula IIc:

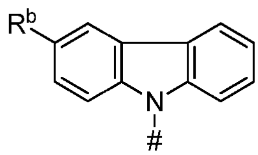

Formula IIc

wherein

$R^b$ is at each occurrence independently from another selected from the group consisting of deuterium, $N(R^5)_2$, $OR^5$, $Si(R^5)_3$, $B(OR^5)_2$, $OSO_2R^5$, $CF_3$, CN, F, Br, I,
$C_1$-$C_{40}$-alkyl,

which is optionally substituted with one or more substituents $R^5$ and
wherein one or more non-adjacent $CH_2$-groups are optionally substituted by $R^5C=CR^5$, C≡C, $Si(R^5)_2$, $Ge(R^5)_2$, $Sn(R^5)_2$, C=O, C=S, C=Se, $C=NR^5$, $P(=O)(R^5)$, SO, $SO_2$, $NR^5$, O, S or $CONR^5$;

$C_1$-$C_{40}$-alkoxy,

which is optionally substituted with one or more substituents $R^5$ and
wherein one or more non-adjacent $CH_2$-groups are optionally substituted by $R^5C=CR^5$, C≡C, $Si(R^5)_2$, $Ge(R^5)_2$, $Sn(R^5)_2$, C=O, C=S, C=Se, $C=NR^5$, $P(=O)(R^5)$, SO, $SO_2$, $NR^5$, O, S or $CONR^5$;

$C_1$-$C_{40}$-thioalkoxy,

which is optionally substituted with one or more substituents $R^5$ and
wherein one or more non-adjacent $CH_2$-groups are optionally substituted by $R^5C=CR^5$, C≡C, $Si(R^5)_2$,

$Ge(R^5)_2$, $Sn(R^5)_2$, C=O, C=S, C=Se, $C=NR^5$, $P(=O)(R^5)$, SO, $SO_2$, $NR^5$, O, S or $CONR^5$;

$C_2$-$C_{40}$-alkenyl,

which is optionally substituted with one or more substituents $R^5$ and
wherein one or more non-adjacent $CH_2$-groups are optionally substituted by $R^5C=CR^5$, C≡C, $Si(R^5)_2$, $Ge(R^5)_2$, $Sn(R^5)_2$, C=O, C=S, C=Se, $C=NR^5$, $P(=O)(R^5)$, SO, $SO_2$, $NR^5$, O, S or $CONR^5$;

$C_2$-$C_{40}$-alkynyl,

which is optionally substituted with one or more substituents $R^5$ and
wherein one or more non-adjacent $CH_2$-groups are optionally substituted by $R^5C=CR^5$, C≡C, $Si(R^5)_2$, $Ge(R^5)_2$, $Sn(R^5)_2$, C=O, C=S, C=Se, $C=NR^5$, $P(=O)(R^5)$, SO, $SO_2$, $NR^5$, O, S or $CONR^5$;

$C_6$-$C_{60}$-aryl,
which is optionally substituted with one or more substituents $R^5$; and
$C_3$-$C_{57}$-heteroaryl,
which is optionally substituted with one or more substituents $R^5$;
and wherein apart from that the definitions in claim 1 apply.

**8.** The organic molecule according to claim 6 or 7, wherein $R^b$ is at each occurrence independently from another selected from the group consisting of

- Me, $^i$Pr, $^t$Bu, CN, $CF_3$,
- Ph, which is optionally substituted with one or more substituents independently from each other selected from the group consisting of Me, $^i$Pr, $^t$Bu, CN, $CF_3$ and Ph;
- pyridinyl, which is optionally substituted with one or more substituents independently from each other selected from the group consisting of Me, $^i$Pr, $^t$Bu, CN, $CF_3$ and Ph;
- pyrimidinyl, which is optionally substituted with one or more substituents independently from each other selected from the group consisting of Me, $^i$Pr, $^t$Bu, CN, $CF_3$ and Ph;
- carbazolyl, which is optionally substituted with one or more substituents independently from each other selected from the group consisting of Me, $^i$Pr, $^t$Bu, CN, $CF_3$ and Ph;
- triazinyl, which is optionally substituted with one or more substituents independently from each other selected from the group consisting of Me, $^i$Pr, $^t$Bu, CN, $CF_3$, and Ph;

and
- $N(Ph)_2$.

**9.** Use of an organic molecule according to one or more of claims 1 to 8 as a luminescent emitter and/or a host material and/or an electron transport material and/or a hole injection material and/or a hole blocking material in an optoelectronic device.

**10.** The use according to claim 9, wherein the optoelectronic device is selected from the group consisting of:

• organic light-emitting diodes (OLEDS),
• light-emitting electrochemical cells,
• OLED-sensors,
• organic diodes,
• organic solar cells,
• organic transistors,
• organic field-effect transistors,
• organic lasers and
• down-conversion elements.

**11.** A composition, comprising

(a) at least one organic molecule according to one or more of claims 1 to 8, in particular in the form of an emitter and/or a host, and
(b) one or more emitter and/or host materials, which differ from the organic molecule of one or more of claims 1 to

8, and
(c) optionally, one or more dyes and/or one or more solvents.

**12.** An optoelectronic device, comprising an organic molecule according to one or more of claims 1 to 8 or a composition according to claim 11, in particular in form of a device selected from the group consisting of organic light-emitting diode (OLED), light-emitting electrochemical cell, OLED-sensor, organic diode, organic solar cell, organic transistor, organic field-effect transistor, organic laser, and down-conversion element.

**13.** The optoelectronic device according to claim 12, comprising

- a substrate,
- an anode and
- a cathode, wherein the anode or the cathode are disposed on the substrate, and
- at least a light-emitting layer, which is arranged between the anode and the cathode and which comprises an organic molecule according to one or more of claims 1 to 8 or a composition according to claim 11.

**14.** A method for producing an optoelectronic device, wherein an organic molecule according to any one of claims 1 to 8 or a composition according to claim 11 is used.

**15.** The method according to claim 14, comprising the processing of the organic molecule by a vacuum evaporation method or from a solution.

**Patentansprüche**

**1.** Organisches Molekül, umfassend

- eine erste chemische Einheit, umfassend eine Struktur der Formel I,

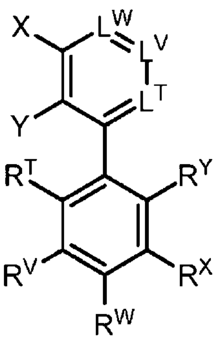

Formel I

- eine zweite chemische Einheit, umfassend eine Struktur der Formel II,

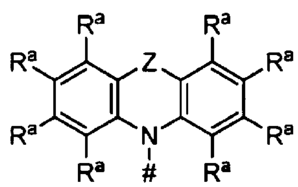

Formel II

wobei die erste chemische Einheit mit der zweiten chemischen Einheit über eine Einfachbindung verbunden ist; und
- eine dritte chemische Einheit, umfassend eine Struktur der Formel III,

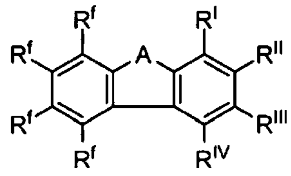

Formel III

wobei die erste chemische Einheit mit der dritten chemischen Einheit über eine Einfachbindung verbunden ist;
wobei
$L^T$ für N oder C-T steht;
$L^V$ für N oder C-V steht;
$L^W$ für N oder C-W steht;
T die Bindungsstelle einer Einfachbindung ist, die die erste chemische Einheit mit der dritten chemischen Einheit verbindet, oder $R^1$ ist;
V die Bindungsstelle einer Einfachbindung ist, die die erste chemische Einheit mit der dritten chemischen Einheit verbindet, oder $R^1$ ist;
W die Bindungsstelle einer Einfachbindung ist, die die erste chemische Einheit mit der dritten chemischen Einheit verbindet, oder $R^1$ ist;
X die Bindungsstelle einer Einfachbindung ist, die die erste chemische Einheit mit der dritten chemischen Einheit verbindet, oder $R^1$ ist;
Y die Bindungsstelle einer Einfachbindung ist, die die erste chemische Einheit mit der dritten chemischen Einheit verbindet, oder $R^1$ ist;
$R^T$ aus der Gruppe ausgewählt ist, die aus $R^A$ und $R^2$ besteht;
$R^V$ aus der Gruppe ausgewählt ist, die aus $R^A$ und $R^2$ besteht;
$R^W$ die Bindungsstelle einer Einfachbindung ist, die die erste chemische Einheit mit der zweiten chemischen Einheit verbindet, oder aus der Gruppe ausgewählt ist, die aus $R^A$ und $R^2$ besteht;
$R^X$ aus der Gruppe ausgewählt ist, die aus $R^A$ und $R^2$ besteht;
$R^Y$ die Bindungsstelle einer Einfachbindung ist, die die erste chemische Einheit mit der zweiten chemischen Einheit verbindet, oder aus der Gruppe ausgewählt ist, die aus $R^A$ und $R^2$ besteht;
# die Bindungsstelle einer Einfachbindung darstellt, die die zweite chemische Einheit mit der ersten chemischen Einheit verbindet;
Z bei jedem Vorkommen unabhängig voneinander aus der Gruppe ausgewählt ist, die aus einer Direktbindung, $CR^3R^4$, $C=CR^3R^4$, C=O, $C=NR^3$, $NR^3$, O, $SiR^3R^4$, S, S(O) und $S(O)^2$ besteht;
A aus der Gruppe ausgewählt ist, die aus O, S und N-Ph besteht;
$R^I$, $R^{II}$, $R^{III}$ und $R^{IV}$ bei jedem Vorkommen unabhängig voneinander aus der Gruppe ausgewählt sind, die aus $ und $R^d$ besteht;
$ die Bindungsstelle einer Einfachbindung darstellt, die die erste chemische Einheit mit der dritten chemischen Einheit verbindet;
$R^d$ bei jedem Vorkommen unabhängig voneinander aus der Gruppe ausgewählt ist, die aus Folgendem besteht:

Wasserstoff, Deuterium,
$C_6$-$C_{18}$-Aryl,
das optional mit einem oder mehreren Substituenten substituiert ist, die unabhängig voneinander aus der Gruppe ausgewählt sind, die aus Folgendem besteht:

$C_1$-$C_5$-Alkyl,
wobei optional ein oder mehrere Wasserstoffatome unabhängig voneinander durch Deuterium, CN, $CF_3$ oder F substituiert sind;
und
$C_6$-$C_{18}$-Aryl,
das optional mit einem oder mehreren $C_1$-$C_5$-Alkyl-Substituenten substituiert ist; $R^A$ eine Struktur der Formel Tz umfasst:

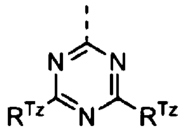

Formel Tz

wobei die gestrichelte Bindung die Bindungsstelle von $R^A$ an die Einfachbindung darstellt, die die erste chemische Einheit und $R^A$ verbindet;
$R^1$ unabhängig voneinander bei jedem Vorkommen unabhängig voneinander aus der Gruppe ausgewählt ist, die aus Folgendem besteht:

Wasserstoff, Deuterium,
$C_1$-$C_5$-Alkyl,
wobei ein oder mehrere Wasserstoffatome optional durch Deuterium substituiert sind;
$C_2$-$C_8$-Alkenyl,
wobei ein oder mehrere Wasserstoffatome optional durch Deuterium substituiert sind;
$C_2$-$C_8$-Alkynyl,
wobei ein oder mehrere Wasserstoffatome optional durch Deuterium substituiert sind; und
$C_6$-$C_{18}$-Aryl,
das optional mit einem oder mehreren Substituenten $R^6$ substituiert ist;
$R^2$ unabhängig voneinander bei jedem Vorkommen unabhängig voneinander aus der Gruppe ausgewählt ist, die aus Folgendem besteht:

Wasserstoff, Deuterium,
$C_1$-$C_5$-Alkyl,
wobei ein oder mehrere Wasserstoffatome optional durch Deuterium substituiert sind;
$C_2$-$C_8$-Alkenyl,
wobei ein oder mehrere Wasserstoffatome optional durch Deuterium substituiert sind;
$C_2$-$C_8$-Alkynyl,
wobei ein oder mehrere Wasserstoffatome optional durch Deuterium substituiert sind; und
$C_6$-$C_{18}$-Aryl,
das optional mit einem oder mehreren Substituenten $R^6$ substituiert ist;
$R^{Tz}$ bei jedem Vorkommen unabhängig voneinander aus der Gruppe ausgewählt ist, die aus Folgendem besteht:

Wasserstoff, Deuterium,
$C_1$-$C_5$-Alkyl,
wobei ein oder mehrere Wasserstoffatome optional durch Deuterium substituiert sind;
$C_6$-$C_{18}$-Aryl,
das optional mit einem oder mehreren Substituenten $R^6$ substituiert ist, und
$C_3$-$C_{17}$-Heteroaryl,
das optional mit einem oder mehreren Substituenten $R^6$ substituiert ist;
$R^a$, $R^3$ und $R^4$ bei jedem Vorkommen unabhängig voneinander aus der Gruppe ausgewählt sind, die aus Folgendem besteht: Wasserstoff, Deuterium, $N(R^5)_2$, $OR^5$, $Si(R^5)_3$, $B(OR^5)_2$, $OSO_2R^5$, $CF_3$, CN, F, Br, I,
$C_1$-$C_{40}$-Alkyl,
das optional mit einem oder mehreren Substituenten $R^5$ substituiert ist und
wobei eine oder mehrere nicht benachbarte $CH_2$-Gruppen optional durch $R^5C=CR^5$, $C\equiv C$, $Si(R^5)_2$, $Ge(R^5)_2$, $Sn(R^5)_2$, C=O, C=S, C=Se, $C=NR^5$, $P(=O)(R^5)$, SO, $SO_2$, $NR^5$, O, S oder $CONR^5$ substituiert sind;
$C_1$-$C_{40}$-Alkoxy,
das optional mit einem oder mehreren Substituenten $R^5$ substituiert ist und
wobei eine oder mehrere nicht benachbarte $CH_2$-Gruppen optional durch $R^5C=CR^5$, $C\equiv C$, $Si(R^5)_2$, $Ge(R^5)_2$, $Sn(R^5)_2$, C=O, C=S, C=Se, $C=NR^5$, $P(=O)(R^5)$, SO, $SO_2$, $NR^5$, O, S oder $CONR^5$ substituiert sind;
$C_1$-$C_{40}$-Thioalkoxy,
das optional mit einem oder mehreren Substituenten $R^5$ substituiert ist und

wobei eine oder mehrere nicht benachbarte $CH_2$-Gruppen optional durch $R^5C=CR^5$, C≡C, $Si(R^5)_2$, $Ge(R^5)_2$, $Sn(R^5)_2$, C=O, C=S, C=Se, $C=NR^5$, $P(=O)(R^5)$, SO, $SO_2$, $NR^5$, O, S oder $CONR^5$ substituiert sind;
$C_2$-$C_{40}$-Alkenyl,
das optional mit einem oder mehreren Substituenten $R^5$ substituiert ist und wobei eine oder mehrere nicht benachbarte $CH_2$-Gruppen optional durch $R^5C=CR^5$, C≡C, $Si(R^5)_2$, $Ge(R^5)_2$, $Sn(R^5)_2$, C=O, C=S, C=Se, $C=NR^5$, $P(=O)(R^5)$, SO, $SO_2$, $NR^5$, O, S oder $CONR^5$ substituiert sind;
$C_2$-$C_{40}$-Alkynyl,
das optional mit einem oder mehreren Substituenten $R^5$ substituiert ist und wobei eine oder mehrere nicht benachbarte $CH_2$-Gruppen optional durch $R^5C=CR^5$, C≡C, $Si(R^5)_2$, $Ge(R^5)_2$, $Sn(R^5)_2$, C=O, C=S, C=Se, $C=NR^5$, $P(=O)(R^5)$, SO, $SO_2$, $NR^5$, O, S oder $CONR^5$ substituiert sind;
$C_6$-$C_{60}$-Aryl,
das optional mit einem oder mehreren Substituenten $R^5$ substituiert ist, und
$C_3$-$C_{57}$-Heteroaryl,
das optional mit einem oder mehreren Substituenten $R^5$ substituiert ist;
$R^5$ bei jedem Vorkommen unabhängig voneinander aus der Gruppe ausgewählt ist, die aus Folgendem besteht:

Wasserstoff, Deuterium, $N(R^6)_2$, $OR^6$, $Si(R^6)_3$, $B(OR^6)_2$, $OSO_2R^6$, $CF_3$, CN, F, Br, I, $C_1$-$C_{40}$-Alkyl,
das optional mit einem oder mehreren Substituenten $R^6$ substituiert ist und wobei eine oder mehrere nicht benachbarte $CH_2$-Gruppen optional durch $R^6C=CR^6$, C≡C, $Si(R^6)_2$, $Ge(R^6)_2$, $Sn(R^6)_2$, C=O, C=S, C=Se, $C=NR^6$, $P(=O)(R^6)$, SO, $SO_2$, $NR^6$, O, S oder $CONR^6$ substituiert sind;
$C_1$-$C_{40}$-Alkoxy,
das optional mit einem oder mehreren Substituenten $R^6$ substituiert ist und wobei eine oder mehrere nicht benachbarte $CH_2$-Gruppen optional durch $R^6C=CR^6$, C≡C, $Si(R^6)_2$, $Ge(R^6)_2$, $Sn(R^6)_2$, C=O, C=S, C=Se, $C=NR^6$, $P(=O)(R^6)$, SO, $SO_2$, $NR^6$, O, S oder $CONR^6$ substituiert sind;
$C_1$-$C_{40}$-Thioalkoxy,
das optional mit einem oder mehreren Substituenten $R^6$ substituiert ist und wobei eine oder mehrere nicht benachbarte $CH_2$-Gruppen optional durch $R^6C=CR^6$, C≡C, $Si(R^6)_2$, $Ge(R^6)_2$, $Sn(R^6)_2$, C=O, C=S, C=Se, $C=NR^6$, $P(=O)(R^6)$, SO, $SO_2$, $NR^6$, O, S oder $CONR^6$ substituiert sind;
$C_2$-$C_{40}$-Alkenyl,
das optional mit einem oder mehreren Substituenten $R^6$ substituiert ist und wobei eine oder mehrere nicht benachbarte $CH_2$-Gruppen optional durch $R^6C=CR^6$, C≡C, $Si(R^6)_2$, $Ge(R^6)_2$, $Sn(R^6)_2$, C=O, C=S, C=Se, $C=NR^6$, $P(=O)(R^6)$, SO, $SO_2$, $NR^6$, O, S oder $CONR^6$ substituiert sind;
$C_2$-$C_{40}$-Alkynyl,
das optional mit einem oder mehreren Substituenten $R^6$ substituiert ist und wobei eine oder mehrere nicht benachbarte $CH_2$-Gruppen optional durch $R^6C=CR^6$, C≡C, $Si(R^6)_2$, $Ge(R^6)_2$, $Sn(R^6)_2$, C=O, C=S, C=Se, $C=NR^6$, $P(=O)(R^6)$, SO, $SO_2$, $NR^6$, O, S oder $CONR^6$ substituiert sind;
$C_6$-$C_{60}$-Aryl,
das optional mit einem oder mehreren Substituenten $R^6$ substituiert ist; und
$C_3$-$C_{57}$-Heteroaryl,
das optional mit einem oder mehreren Substituenten $R^6$ substituiert ist;
$R^f$ bei jedem Vorkommen unabhängig voneinander aus der Gruppe ausgewählt ist,
die aus Folgendem besteht:

Wasserstoff,
Deuterium,

$N(R^{5f})_2$,

$OR^{5f}$,

$Si(R^{5f})_3$,

$B(OR^{5f})_2$,

$OSO_2R^{5f}$,

$CF_3$,

CN,

F,

Br,

I,

$C_1$-$C_{40}$-Alkyl,
das optional mit einem oder mehreren Substituenten $R^{5f}$ substituiert ist und wobei eine oder mehrere nicht benachbarte $CH_2$-Gruppen optional durch $R^{5f}C=CR^{5f}$, C≡C, $Si(R^{5f})_2$, $Ge(R^{5f})_2$, $Sn(R^{5f})_2$, **C=O, C=S,** C=Se, $C=NR^{5f}$, $P(=O)(R^{5f})$, SO, $SO_2$, $NR^{5f}$, O, S oder $CONR^{5f}$ substituiert sind;
$C_1$-$C_{40}$-Alkoxy,
das optional mit einem oder mehreren Substituenten $R^{5f}$ substituiert ist und wobei eine oder mehrere nicht benachbarte $CH_2$-Gruppen optional durch $R^{5f}C=CR^{5f}$, C≡C, $Si(R^{5f})_2$, $Ge(R^{5f})_2$, $Sn(R^{5f})_2$, C=O, C=S, C=Se, $C=NR^{5f}$, $P(=O)(R^{5f})$, SO, $SO_2$, $NR^{5f}$, O, S oder $CONR^{5f}$ substituiert sind;
$C_1$-$C_{40}$-Thioalkoxy,
das optional mit einem oder mehreren Substituenten $R^{5f}$ substituiert ist und wobei eine oder mehrere nicht benachbarte $CH_2$-Gruppen optional durch $R^{5f}C=CR^{5f}$, C≡C, $Si(R^{5f})_2$, $Ge(R^{5f})_2$, $Sn(R^{5f})_2$, C=O, C=S, C=Se, $C=NR^{5f}$, $P(=O)(R^{5f})$, SO, $SO_2$, $NR^{5f}$, O, S oder $CONR^{5f}$ substituiert sind;
$C_2$-$C_{40}$-Alkenyl,
das optional mit einem oder mehreren Substituenten $R^{5f}$ substituiert ist und wobei eine oder mehrere nicht benachbarte $CH_2$-Gruppen optional durch $R^{5f}C=CR^{5f}$, C≡C, $Si(R^{5f})_2$, $Ge(R^{5f})_2$, $Sn(R^{5f})_2$, C=O, C=S, C=Se, $C=NR^{5f}$, $P(=O)(R^{5f})$, SO, $SO_2$, $NR^{5f}$, O, S oder $CONR^{5f}$ substituiert sind;
$C_2$-$C_{40}$-Alkynyl,
das optional mit einem oder mehreren Substituenten $R^{5f}$ substituiert ist und wobei eine oder mehrere nicht benachbarte $CH_2$-Gruppen optional durch $R^{5f}C=CR^{5f}$, C≡C, $Si(R^{5f})_2$, $Ge(R^{5f})_2$, $Sn(R^{5f})_2$, C=O, C=S, C=Se, $C=NR^{5f}$, $P(=O)(R^{5f})$, SO, $SO_2$, $NR^{5f}$, O, S oder $CONR^{5f}$ substituiert sind;
$C_6$-$C_{60}$-Aryl,
das optional mit einem oder mehreren Substituenten $R^{5f}$ substituiert ist; und
$C_3$-$C_{57}$-Heteroaryl,
das optional mit einem oder mehreren Substituenten $R^{5f}$ substituiert ist,
$R^{5f}$ bei jedem Vorkommen unabhängig voneinander aus der Gruppe ausgewählt ist, die aus Folgendem besteht:

Wasserstoff,
Deuterium,

$N(R^6)_2$,

$OR^6$,

$Si(R^6)_3$,

$B(OR^6)_2$,

$OSO_2R^6$,

$CF_3$,

CN,

F,

Br,

I,

$C_1$-$C_{40}$-Alkyl,
das optional mit einem oder mehreren Substituenten $R^6$ substituiert ist und
wobei eine oder mehrere nicht benachbarte $CH_2$-Gruppen optional durch $R^6C=CR^6$, C≡C, $Si(R^6)_2$, $Ge(R^6)_2$, $Sn(R^6)_2$, C=O, C=S, C=Se, $C=NR^6$, $P(=O)(R^6)$, SO, $SO_2$, $NR^6$, O, S oder $CONR^6$ substituiert sind;
$C_1$-$C_{40}$-Alkoxy,
das optional mit einem oder mehreren Substituenten $R^6$ substituiert ist und
wobei eine oder mehrere nicht benachbarte $CH_2$-Gruppen optional durch $R^6C=CR^6$, C≡C, $Si(R^6)_2$, $Ge(R^6)_2$, $Sn(R^6)_2$, C=O, C=S, C=Se, $C=NR^6$, $P(=O)(R^6)$, SO, $SO_2$, $NR^6$, O, S oder $CONR^6$ substituiert sind;
$C_1$-$C_{40}$-Thioalkoxy,
das optional mit einem oder mehreren Substituenten $R^6$ substituiert ist und
wobei eine oder mehrere nicht benachbarte $CH_2$-Gruppen optional durch $R^6C=CR^6$, C≡C, $Si(R^6)_2$, $Ge(R^6)_2$, $Sn(R^6)_2$, C=O, C=S, C=Se, $C=NR^6$, $P(=O)(R^6)$, SO, $SO_2$, $NR^6$, O, S oder $CONR^6$ substituiert sind;
$C_2$-$C_{40}$-Alkenyl,
das optional mit einem oder mehreren Substituenten $R^6$ substituiert ist und
wobei eine oder mehrere nicht benachbarte $CH_2$-Gruppen optional durch $R^6C=CR^6$, C≡C, $Si(R^6)_2$, $Ge(R^6)_2$, $Sn(R^6)_2$, C=O, C=S, C=Se, $C=NR^6$, $P(=O)(R^6)$, SO, $SO_2$, $NR^6$, O, S oder $CONR^6$ substituiert sind;
$C_2$-$C_{40}$-Alkynyl,
das optional mit einem oder mehreren Substituenten $R^6$ substituiert ist und
wobei eine oder mehrere nicht benachbarte $CH_2$-Gruppen optional durch $R^6C=CR^6$, C≡C, $Si(R^6)_2$, $Ge(R^6)_2$, $Sn(R^6)_2$, C=O, C=S, C=Se, $C=NR^6$, $P(=O)(R^6)$, SO, $SO_2$, $NR^6$, O, S oder $CONR^6$ substituiert sind;
$C_6$-$C_{60}$-Aryl,
das optional mit einem oder mehreren Substituenten $R^6$ substituiert ist; und
$C_3$-$C_{57}$-Heteroaryl,
das optional mit einem oder mehreren Substituenten $R^6$ substituiert ist,
$R^6$ bei jedem Vorkommen unabhängig voneinander aus der Gruppe ausgewählt ist, die aus Folgendem besteht:

Wasserstoff, Deuterium, OPh, $CF_3$, CN, F,
$C_1$-$C_5$-Alkyl,
wobei ein oder mehrere Wasserstoffatome optional unabhängig voneinander durch Deuterium, CN, $CF_3$ oder F substituiert sind;

$C_1$-$C_5$-Alkoxy,
wobei ein oder mehrere Wasserstoffatome optional unabhängig voneinander durch Deuterium, CN, $CF_3$ oder F substituiert sind;
$C_1$-$C_5$-Thioalkoxy,
wobei ein oder mehrere Wasserstoffatome optional unabhängig voneinander durch Deuterium, CN, $CF_3$ oder F substituiert sind;
$C_2$-$C_5$-Alkenyl,
wobei ein oder mehrere Wasserstoffatome optional unabhängig voneinander durch Deuterium, CN, $CF_3$ oder F substituiert sind;
$C_2$-$C_5$-Alkynyl,
wobei ein oder mehrere Wasserstoffatome optional unabhängig voneinander durch Deuterium, CN, $CF_3$ oder F substituiert sind;
$C_6$-$C_{18}$-Aryl,
das optional mit einem oder mehreren $C_1$-$C_5$-Alkyl-Substituenten substituiert ist; $C_3$-$C_{17}$-Heteroaryl,
das optional mit einem oder mehreren $C_1$-$C_5$-Alkyl-Substituenten substituiert ist; $N(C_6$-$C_{18}$-$Aryl)_2$;
$N(C_3$-$C_{17}$-$Heteroaryl)_2$,
und $N(C_3$-$C_{17}$-Heteroaryl)($C_6$-$C_{18}$-Aryl);
wobei die Substituenten $R^a$, $R^3$, $R^4$ oder $R^5$ unabhängig voneinander optional ein mono- oder polycyclisches, aliphatisches, aromatisches und/oder benzo-kondensiertes Ringsystem mit einem oder mehreren Substituenten $R^a$, $R^3$, $R^4$ oder $R^5$ bilden;
wobei optional einer oder mehrere der Substituenten $R^f$ oder $R^{5f}$ unabhängig voneinander ein mono- oder polycyclisches, aliphatisches, aromatisches und/oder benzo-kondensiertes Ringsystem mit einem oder mehreren anderen Substituenten $R^f$ oder $R^{5f}$ bilden;
wobei

- genau ein Ringglied, das aus der Gruppe ausgewählt ist, die aus $L^T$, $L^V$ und $L^W$ besteht, N ist,
- genau ein Substituent, der aus der Gruppe ausgewählt ist, die aus $R^T$, $R^V$, $R^W$, $R^X$ und $R^Y$ besteht, $R^A$ ist,
- genau ein Substituent, der aus der Gruppe ausgewählt ist, die aus T, V, W, X und Y besteht, die Bindungsstelle einer Einfachbindung ist, die die erste chemische Einheit mit der dritten chemischen Einheit verbindet, und
- genau ein Substituent, der aus der Gruppe ausgewählt ist, die aus $R^W$ und $R^Y$ besteht, die Bindungsstelle einer Einfachbindung darstellt, die die erste chemische Einheit und die zweite chemische Einheit verbindet.

**2.** Organisches Molekül nach Anspruch 1, wobei die erste chemische Einheit eine Struktur der Formel Ia umfasst:

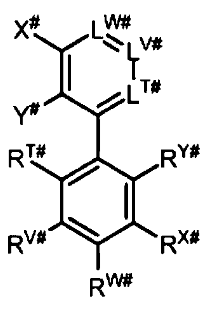

Formel Ia

wobei $R^1$, $R^2$, $R^I$ und $R^A$ wie oben definiert sind;
$L^{T\#}$ für N oder C-$R^1$ steht;

$L^{V\#}$ für N oder C-$R^1$ steht;
$L^{W\#}$ für N oder C-$W^\#$ steht;
$W^\#$ die Bindungsstelle einer Einfachbindung ist, die die erste chemische Einheit mit der dritten chemischen Einheit verbindet, oder $R^1$ ist;
$X^\#$ die Bindungsstelle einer Einfachbindung ist, die die erste chemische Einheit mit der dritten chemischen Einheit verbindet, oder $R^1$ ist;
$Y^\#$ die Bindungsstelle einer Einfachbindung ist, die die erste chemische Einheit mit der dritten chemischen Einheit verbindet, oder $R^1$ ist;
$R^{T\#}$ aus der Gruppe ausgewählt ist, die aus $R^A$ und $R^2$ besteht;
$R^{V\#}$ aus der Gruppe ausgewählt ist, die aus $R^A$ und $R^2$ besteht;
$R^{W\#}$ die Bindungsstelle einer Einfachbindung ist, die die erste chemische Einheit mit der zweiten chemischen Einheit verbindet, oder aus der Gruppe ausgewählt ist, die aus $R^A$ und $R^2$ besteht;
$R^{X\#}$ für $R^2$ steht;
$R^{Y\#}$ die Bindungsstelle einer Einfachbindung ist, die die erste chemische Einheit mit der zweiten chemischen Einheit verbindet, oder $R^2$ ist;
wobei
genau ein Ringglied (Atom oder Gruppe), das aus der Gruppe ausgewählt ist, die aus $L^{T\#}$, $L^{V\#}$ und $L^{W\#}$ besteht, N ist;
genau ein Substituent, der aus der Gruppe ausgewählt ist, die aus $R^{T\#}$, $R^{V\#}$ und $R^{W\#}$ besteht, $R^A$ ist,
genau ein Substituent, der aus der Gruppe ausgewählt ist, die aus $W^\#$, $X^\#$ und $Y^\#$ besteht, die Bindungsstelle einer Einfachbindung ist, die die erste chemische Einheit mit der dritten chemischen Einheit verbindet,
und genau ein Substituent, der aus der Gruppe ausgewählt ist, die aus $R^{W\#}$ und $R^{Y\#}$ besteht, die Bindungsstelle einer Einfachbindung darstellt, die die erste chemische Einheit und die zweite chemische Einheit verbindet;
und wobei ansonsten die Definitionen in Anspruch 1 gelten.

**3.** Organisches Molekül nach Anspruch 1 oder 2, wobei $R^1$ und $R^2$ unabhängig voneinander bei jedem Vorkommen unabhängig voneinander aus der Gruppe ausgewählt sind, die aus H, Methyl, Mesityl, Tolyl und Phenyl besteht.

**4.** Organisches Molekül nach einem oder mehreren der Ansprüche 1 bis 3, wobei $R^{Tz}$ unabhängig voneinander aus der Gruppe ausgewählt ist, die aus H, Methyl und Phenyl besteht.

**5.** Organisches Molekül nach einem oder mehreren der Ansprüche 1 bis 4, wobei die zweite chemische Einheit eine Struktur der Formel IIa umfasst:

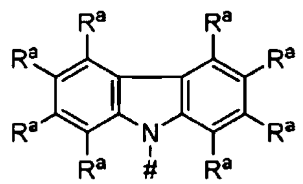

Formel IIa

wobei # und $R^a$ wie in Anspruch 1 definiert sind.

**6.** Organisches Molekül nach einem oder mehreren der Ansprüche 1 bis 5, wobei die zweite chemische Einheit eine Struktur der Formel IIb umfasst:

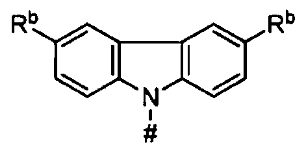

Formel IIb

wobei
$R^b$ bei jedem Vorkommen unabhängig voneinander aus der Gruppe ausgewählt ist, die aus Folgendem besteht:

Deuterium, $N(R^5)_2$, $OR^5$, $Si(R^5)_3$, $B(OR^5)_2$, $OSO_2R^5$, $CF_3$, CN, F, Br, I,
$C_1$-$C_{40}$-Alkyl,
das optional mit einem oder mehreren Substituenten $R^5$ substituiert ist und
wobei eine oder mehrere nicht benachbarte $CH_2$-Gruppen optional durch $R^5C=CR^5$, C≡C, $Si(R^5)_2$, $Ge(R^5)_2$, $Sn(R^5)_2$, C=O, C=S, C=Se, $C=NR^5$, $P(=O)(R^5)$, SO, $SO_2$, $NR^5$, O, S oder $CONR^5$ substituiert sind;
$C_1$-$C_{40}$-Alkoxy,
das optional mit einem oder mehreren Substituenten $R^5$ substituiert ist und
wobei eine oder mehrere nicht benachbarte $CH_2$-Gruppen optional durch $R^5C=CR^5$, C≡C, $Si(R^5)_2$, $Ge(R^5)_2$, $Sn(R^5)_2$, C=O, C=S, C=Se, $C=NR^5$, $P(=O)(R^5)$, SO, $SO_2$, $NR^5$, O, S oder $CONR^5$ substituiert sind;
$C_1$-$C_{40}$-Thioalkoxy,
das optional mit einem oder mehreren Substituenten $R^5$ substituiert ist und
wobei eine oder mehrere nicht benachbarte $CH_2$-Gruppen optional durch $R^5C=CR^5$, C≡C, $Si(R^5)_2$, $Ge(R^5)_2$, $Sn(R^5)_2$, C=O, C=S, C=Se, $C=NR^5$, $P(=O)(R^5)$, SO, $SO_2$, $NR^5$, O, S oder $CONR^5$ substituiert sind;
$C_2$-$C_{40}$-Alkenyl,
das optional mit einem oder mehreren Substituenten $R^5$ substituiert ist und
wobei eine oder mehrere nicht benachbarte $CH_2$-Gruppen optional durch $R^5C=CR^5$, C≡C, $Si(R^5)_2$, $Ge(R^5)_2$, $Sn(R^5)_2$, C=O, C=S, C=Se, $C=NR^5$, $P(=O)(R^5)$, SO, $SO_2$, $NR^5$, O, S oder $CONR^5$ substituiert sind;
$C_2$-$C_{40}$-Alkynyl,
das optional mit einem oder mehreren Substituenten $R^5$ substituiert ist und
wobei eine oder mehrere nicht benachbarte $CH_2$-Gruppen optional durch $R^5C=CR^5$, C≡C, $Si(R^5)_2$, $Ge(R^5)_2$, $Sn(R^5)_2$, C=O, C=S, C=Se, $C=NR^5$, $P(=O)(R^5)$, SO, $SO_2$, $NR^5$, O, S oder $CONR^5$ substituiert sind;
$C_6$-$C_{60}$-Aryl,
das optional mit einem oder mehreren Substituenten $R^5$ substituiert ist; und
$C_3$-$C_{57}$-Heteroaryl,
das optional mit einem oder mehreren Substituenten $R^5$ substituiert ist;
und wobei ansonsten die Definitionen in Anspruch 1 gelten.

7. Organisches Molekül nach einem oder mehreren der Ansprüche 1 bis 5, wobei die zweite chemische Einheit eine Struktur der Formel IIc umfasst:

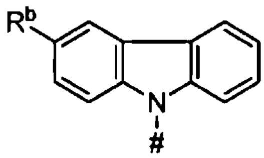

Formel IIc

wobei
$R^b$ bei jedem Vorkommen unabhängig voneinander aus der Gruppe ausgewählt ist, die aus Folgendem besteht:

Deuterium, $N(R^5)_2$, $OR^5$, $Si(R^5)_3$, $B(OR^5)_2$, $OSO_2R^5$, $CF_3$, CN, F, Br, I,
$C_1$-$C_{40}$-Alkyl,
das optional mit einem oder mehreren Substituenten $R^5$ substituiert ist und
wobei eine oder mehrere nicht benachbarte $CH_2$-Gruppen optional durch $R^5C=CR^5$, C≡C, $Si(R^5)_2$, $Ge(R^5)_2$, $Sn(R^5)_2$, C=O, C=S, C=Se, $C=NR^5$, $P(=O)(R^5)$, SO, $SO_2$, $NR^5$, O, S oder $CONR^5$ substituiert sind;
$C_1$-$C_{40}$-Alkoxy,
das optional mit einem oder mehreren Substituenten $R^5$ substituiert ist und
wobei eine oder mehrere nicht benachbarte $CH_2$-Gruppen optional durch $R^5C=CR^5$, C≡C, $Si(R^5)_2$, $Ge(R^5)_2$, $Sn(R^5)_2$, C=O, C=S, C=Se, $C=NR^5$, $P(=O)(R^5)$, SO, $SO_2$, $NR^5$, O, S oder $CONR^5$ substituiert sind;
$C_1$-$C_{40}$-Thioalkoxy,
das optional mit einem oder mehreren Substituenten $R^5$ substituiert ist und
wobei eine oder mehrere nicht benachbarte $CH_2$-Gruppen optional durch $R^5C=CR^5$, C≡C, $Si(R^5)_2$, $Ge(R^5)_2$, $Sn(R^5)_2$, C=O, C=S, C=Se, $C=NR^5$, $P(=O)(R^5)$, SO, $SO_2$, $NR^5$, O, S oder $CONR^5$ substituiert sind;
$C_2$-$C_{40}$-Alkenyl,
das optional mit einem oder mehreren Substituenten $R^5$ substituiert ist und
wobei eine oder mehrere nicht benachbarte $CH_2$-Gruppen optional durch $R^5C=CR^5$, C≡C, $Si(R^5)_2$, $Ge(R^5)_2$, $Sn(R^5)_2$, C=O, C=S, C=Se, $C=NR^5$, $P(=O)(R^5)$, SO, $SO_2$, $NR^5$, O, S oder $CONR^5$ substituiert sind;

$C_2$-$C_{40}$-Alkynyl,
das optional mit einem oder mehreren Substituenten $R^5$ substituiert ist und
wobei eine oder mehrere nicht benachbarte $CH_2$-Gruppen optional durch $R^5C$=$CR^5$, C≡C, $Si(R^5)_2$, $Ge(R^5)_2$, $Sn(R^5)_2$, C=O, C=S, C=Se, C=$NR^5$, P(=O)($R^5$), SO, $SO_2$, $NR^5$, O, S oder $CONR^5$ substituiert sind;
$C_6$-$C_{60}$-Aryl,
das optional mit einem oder mehreren Substituenten $R^5$ substituiert ist; und
$C_3$-$C_{57}$-Heteroaryl,
das optional mit einem oder mehreren Substituenten $R^5$ substituiert ist;
und wobei ansonsten die Definitionen in Anspruch 1 gelten.

**8.** Organisches Molekül nach Anspruch 6 oder 7, wobei $R^b$ bei jedem Vorkommen unabhängig voneinander aus der Gruppe ausgewählt ist, die aus Folgendem besteht:

- Me, $^i$Pr, $^t$Bu, CN, $CF_3$,
- Ph, das optional mit einem oder mehreren Substituenten substituiert ist, die unabhängig voneinander aus der Gruppe ausgewählt sind, die aus Me, $^i$Pr, $^t$Bu, CN, $CF_3$ und Ph besteht;
- Pyridinyl, das optional mit einem oder mehreren Substituenten substituiert ist, die unabhängig voneinander aus der Gruppe ausgewählt sind, die aus Me, $^i$Pr, $^t$Bu, CN, $CF_3$ und Ph besteht;
- Pyrimidinyl, das optional mit einem oder mehreren Substituenten substituiert ist, die unabhängig voneinander aus der Gruppe ausgewählt sind, die aus Me, $^i$Pr, $^t$Bu, CN, $CF_3$ und Ph besteht;
- Carbazolyl, das optional mit einem oder mehreren Substituenten substituiert ist, die unabhängig voneinander aus der Gruppe ausgewählt sind, die aus Me, $^i$Pr, $^t$Bu, CN, $CF_3$ und Ph besteht;
- Triazinyl, das optional mit einem oder mehreren Substituenten substituiert ist, die unabhängig voneinander aus der Gruppe ausgewählt sind, die aus Me, $^i$Pr, $^t$Bu, CN, $CF_3$ und Ph besteht; und
- $N(Ph)_2$.

**9.** Verwendung eines organischen Moleküls nach einem oder mehreren der Ansprüche 1 bis 8 als lumineszierender Emitter und/oder als Wirtsmaterial und/oder als Elektronentransportmaterial und/oder als Lochinjektionsmaterial und/oder als Lochblockierungsmaterial in einer optoelektronischen Vorrichtung.

**10.** Verwendung nach Anspruch 9, wobei die optoelektronische Vorrichtung aus der Gruppe ausgewählt ist, die aus Folgendem besteht:

- organischen Leuchtdioden (OLEDs),
- lichtemittierenden elektrochemischen Zellen,
- OLED-Sensoren,
- organischen Dioden,
- organischen Solarzellen,
- organischen Transistoren,
- organischen Feldeffekttransistoren,
- organischen Lasern und
- Abwärtskonversionselementen.

**11.** Zusammensetzung, umfassend

(a) mindestens ein organisches Molekül nach einem oder mehreren der Ansprüche 1 bis 8, insbesondere in Form eines Emitters und/oder eines Wirts, und
(b) ein oder mehrere Emitter- und/oder Wirtsmaterialien, die sich von dem organischen Molekül nach einem oder mehreren der Ansprüche 1 bis 8 unterscheiden, und
(c) optional einen oder mehrere Farbstoffe und/oder ein oder mehrere Lösungsmittel.

**12.** Optoelektronische Vorrichtung, umfassend ein organisches Molekül nach einem oder mehreren der Ansprüche 1 bis 8 oder eine Zusammensetzung nach Anspruch 11, insbesondere in Form einer Vorrichtung, ausgewählt aus der Gruppe bestehend aus einer organischen Leuchtdiode (OLED), einer lichtemittierenden elektrochemischen Zelle, einem OLED-Sensor, einer organischen Diode, einer organischen Solarzelle, einem organischen Transistor, einem organischen Feldeffekttransistor, einem organischen Laser und einem Abwärtskonversionselement.

**13.** Optoelektronische Vorrichtung nach Anspruch 12, umfassend

- ein Substrat,
- eine Anode und
- eine Kathode, wobei die Anode oder die Kathode auf dem Substrat angeordnet sind, und
- mindestens eine lichtemittierende Schicht, die zwischen der Anode und der Kathode angeordnet ist und die ein organisches Molekül nach einem oder mehreren der Ansprüche 1 bis 8 oder eine Zusammensetzung nach Anspruch 11 umfasst.

**14.** Verfahren zur Herstellung einer optoelektronischen Vorrichtung, wobei ein organisches Molekül nach einem der Ansprüche 1 bis 8 oder eine Zusammensetzung nach Anspruch 11 verwendet wird.

**15.** Verfahren nach Anspruch 14, umfassend die Verarbeitung des organischen Moleküls durch ein Vakuumverdampfungsverfahren oder aus einer Lösung.

## Revendications

**1.** Molécule organique, comprenant

- une première fraction chimique comprenant une structure de formule I,

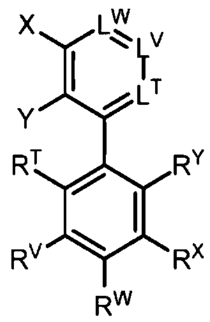

Formule I

- une deuxième fraction chimique comprenant une structure de formule II,

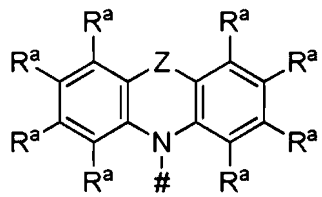

Formule II

dans laquelle la première fraction chimique est reliée à la deuxième fraction chimique via une simple liaison ; et
- une troisième fraction chimique comprenant une structure de formule III,

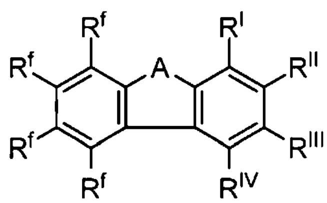

Formule III

dans laquelle la première fraction chimique est reliée à la troisième fraction chimique via une simple liaison ;
dans laquelle
$L^T$ est N ou C-T ;
$L^V$ est N ou C-V ;
$L^W$ est N ou C-W ;
T est le site de liaison d'une simple liaison reliant la première fraction chimique à la troisième fraction chimique ou est $R^1$ ;
V est le site de liaison d'une simple liaison reliant la première fraction chimique à la troisième fraction chimique ou est $R^1$ ;
W est le site de liaison d'une simple liaison reliant la première fraction chimique à la troisième fraction chimique ou est $R^1$ ;
X est le site de liaison d'une simple liaison reliant la première fraction chimique à la troisième fraction chimique ou est $R^1$ ;
Y est le site de liaison d'une simple liaison reliant la première fraction chimique à la troisième fraction chimique ou est $R^1$ ;
$R^T$ est choisi dans le groupe consistant en $R^A$ et $R^2$ ;
$R^V$ est choisi dans le groupe consistant en $R^A$ et $R^2$ ;
$R^W$ est le site de liaison d'une simple liaison reliant la première fraction chimique à la deuxième fraction chimique ou est choisi dans le groupe consistant en $R^A$ et $R^2$ ;
$R^X$ est choisi dans le groupe consistant en $R^A$ et $R^2$ ;
$R^Y$ est le site de liaison d'une simple liaison reliant la première fraction chimique à la deuxième fraction chimique ou est choisi dans le groupe consistant en $R^A$ et $R^2$ ;
# représente le site de liaison d'une simple liaison reliant la deuxième fraction chimique à la première fraction chimique ;
Z est, à chaque occurrence indépendamment d'une autre, choisi dans le groupe consistant en une liaison directe, $CR^3R^4$, $C=CR^3R^4$, C=O, $C=NR^3$, $NR^3$, O, $SiR^3R^4$, S, S(O) et $S(O)_2$ ;
A est choisi dans le groupe consistant en O, S et N-Ph ;
$R^I$, $R^{II}$, $R^{III}$ et $R^{IV}$ sont, à chaque occurrence indépendamment d'une autre, choisis dans le groupe consistant en $ et $R^d$ ;
$ représente le site de liaison d'une simple liaison reliant la première fraction chimique à la troisième fraction chimique ;
$R^d$ est, à chaque occurrence indépendamment d'une autre, choisi dans le groupe consistant en
hydrogène, deutérium,
aryle en $C_6$ à $C_{18}$,

qui est facultativement substitué par un ou plusieurs substituants choisis indépendamment les uns des autres dans le groupe consistant en
alkyle en $C_1$ à $C_5$,

dans lequel facultativement un ou plusieurs atomes d'hydrogène sont indépendamment les uns des autres substitués par du deutérium, CN, $CF_3$ ou F ;
et
aryle en $C_6$ à $C_{18}$,
qui est facultativement substitué par un ou plusieurs substituants alkyle en $C_1$ à $C_5$ ; $R^A$ comprend une structure de formule Tz :

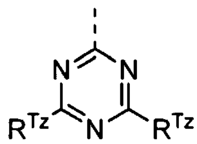

Formule Tz

dans laquelle la liaison pointillée représente le site de liaison de $R^A$ à la simple liaison reliant la première fraction chimique et $R^A$ ;
$R^1$ est indépendamment d'un autre, à chaque occurrence indépendamment d'une autre, choisi dans le groupe consistant en :

hydrogène, deutérium,
alkyle en $C_1$ à $C_5$,
dans lequel un ou plusieurs atomes d'hydrogène sont facultativement substitués par du deutérium ;
alcényle en $C_2$ à $C_8$,
dans lequel un ou plusieurs atomes d'hydrogène sont facultativement substitués par du deutérium ;
alcynyle en $C_2$ à $C_8$,
dans lequel un ou plusieurs atomes d'hydrogène sont facultativement substitués par du deutérium ; et
aryle en $C_6$ à $C_{18}$,
qui est facultativement substitué par un ou plusieurs substituants $R^6$ ;
$R^2$ est indépendamment d'un autre, à chaque occurrence indépendamment d'une autre, choisi dans le groupe consistant en :

hydrogène, deutérium,
alkyle en $C_1$ à $C_5$,
dans lequel un ou plusieurs atomes d'hydrogène sont facultativement substitués par du deutérium ;
alcényle en $C_2$ à $C_8$,
dans lequel un ou plusieurs atomes d'hydrogène sont facultativement substitués par du deutérium ;
alcynyle en $C_2$ à $C_8$,
dans lequel un ou plusieurs atomes d'hydrogène sont facultativement substitués par du deutérium ; et
aryle en $C_6$ à $C_{18}$,
qui est facultativement substitué par un ou plusieurs substituants $R^6$ ;
$R^{TZ}$ est, à chaque occurrence indépendamment d'une autre, choisi dans le groupe consistant en :

hydrogène, deutérium,
alkyle en $C_1$ à $C_5$,
dans lequel un ou plusieurs atomes d'hydrogène sont facultativement substitués par du deutérium ;
aryle en $C_6$ à $C_{18}$,
qui est facultativement substitué par un ou plusieurs substituants $R^6$, et
hétéroaryle en $C_3$ à $C_{17}$,
qui est facultativement substitué par un ou plusieurs substituants $R^6$ ;
$R^a$, $R^3$ et $R^4$ sont, à chaque occurrence indépendamment d'une autre, choisis dans le groupe consistant en : hydrogène, deutérium, $N(R^5)_2$, $OR^5$, $Si(R^5)_3$, $B(OR^5)_2$, $OSO_2R^5$, $CF_3$, CN, F, Br, I,
alkyle en $C_1$ à $C_{40}$,
qui est facultativement substitué par un ou plusieurs substituants $R^5$ et
dans lequel un ou plusieurs groupes $CH_2$ non adjacents sont facultativement substitués par $R^5C=CR^5$, C≡C, $Si(R^5)_2$, $Ge(R^5)_2$, $Sn(R^5)_2$, C=O, C=S, C=Se, $C=NR^5$, $P(=O)(R^5)$, SO, $SO_2$, $NR^5$, O, S ou $CONR^5$ ;
alcoxy en $C_1$ à $C_{40}$,
qui est facultativement substitué par un ou plusieurs substituants $R^5$ et
dans lequel un ou plusieurs groupes $CH_2$ non adjacents sont facultativement substitués par $R^5C=CR^5$, C≡C, $Si(R^5)_2$, $Ge(R^5)_2$, $Sn(R^5)_2$, C=O, C=S, C=Se, $C=NR^5$, $P(=O)(R^5)$, SO, $SO_2$, $NR^5$, O, S ou $CONR^5$ ;
thioalcoxy en $C_1$ à $C_{40}$,
qui est facultativement substitué par un ou plusieurs substituants $R^5$ et
dans lequel un ou plusieurs groupes $CH_2$ non adjacents sont facultativement substitués par $R^5C=CR^5$, C≡C, $Si(R^5)_2$, $Ge(R^5)_2$, $Sn(R^5)_2$, C=O, C=S, C=Se, $C=NR^5$, $P(=O)(R^5)$, SO, $SO_2$, $NR^5$, O, S ou $CONR^5$ ;
alcényle en $C_2$ à $C_{40}$,
qui est facultativement substitué par un ou plusieurs substituants $R^5$ et
dans lequel un ou plusieurs groupes $CH_2$ non adjacents sont facultativement substitués par $R^5C=CR^5$, C≡C, $Si(R^5)_2$, $Ge(R^5)_2$, $Sn(R^5)_2$, C=O, C=S, C=Se, $C=NR^5$, $P(=O)(R^5)$, SO, $SO_2$, $NR^5$, O, S ou $CONR^5$ ;
alcynyle en $C_2$ à $C_{40}$,
qui est facultativement substitué par un ou plusieurs substituants $R^5$ et
dans lequel un ou plusieurs groupes $CH_2$ non adjacents sont facultativement substitués par

$R^5C=CR^5$, C≡C, $Si(R^5)_2$, $Ge(R^5)_2$, $Sn(R^5)_2$, C=O, C=S, C=Se, $C=NR^5$, $P(=O)(R^5)$, SO, $SO_2$, $NR^5$, O, S ou $CONR^5$ ;
aryle en $C_6$ à $C_{60}$,
qui est facultativement substitué par un ou plusieurs substituants $R^5$ ; et hétéroaryle en $C_3$ à $C_{57}$,
qui est facultativement substitué par un ou plusieurs substituants $R^5$ ;
$R^5$ est, à chaque occurrence indépendamment d'une autre, choisi dans le groupe consistant en hydrogène, deutérium, $N(R^6)_2$, $OR^6$, $Si(R^6)_3$, $B(OR^6)_2$, $OSO_2R^6$, $CF_3$, CN, F, Br, I, alkyle en $C_1$ à $C_{40}$,
qui est facultativement substitué par un ou plusieurs substituants $R^6$ et
dans lequel un ou plusieurs groupes $CH_2$ non adjacents sont facultativement substitués par $R^6C=CR^6$, C≡C, $Si(R^6)_2$, $Ge(R^6)_2$, $Sn(R^6)_2$, C=O, C=S, C=Se, $C=NR^6$, $P(=O)(R^6)$, SO, $SO_2$, $NR^6$, O, S ou $CONR^6$ ;
alcoxy en $C_1$ à $C_{40}$,
qui est facultativement substitué par un ou plusieurs substituants $R^6$ et
dans lequel un ou plusieurs groupes $CH_2$ non adjacents sont facultativement substitués par $R^6C=CR^6$, C≡C, $Si(R^6)_2$, $Ge(R^6)_2$, $Sn(R^6)_2$, C=O, C=S, C=Se, $C=NR^6$, $P(=O)(R^6)$, SO, $SO_2$, $NR^6$, O, S ou $CONR^6$ ;
thioalcoxy en $C_1$ à $C_{40}$,
qui est facultativement substitué par un ou plusieurs substituants $R^6$ et
dans lequel un ou plusieurs groupes $CH_2$ non adjacents sont facultativement substitués par $R^6C=CR^6$, C≡C, $Si(R^6)_2$, $Ge(R^6)_2$, $Sn(R^6)_2$, C=O, C=S, C=Se, $C=NR^6$, $P(=O)(R^6)$, SO, $SO_2$, $NR^6$, O, S ou $CONR^6$ ;
alcényle en $C_2$ à $C_{40}$,
qui est facultativement substitué par un ou plusieurs substituants $R^6$ et dans lequel un ou plusieurs groupes $CH_2$ non adjacents sont facultativement substitués par $R^6C=CR^6$, C≡C, $Si(R^6)_2$, $Ge(R^6)_2$, $Sn(R^6)_2$, C=O, C=S, C=Se, $C=NR^6$, $P(=O)(R^6)$, SO, $SO_2$, $NR^6$, O, S ou $CONR^6$ ;
alcynyle en $C_2$ à $C_{40}$,
qui est facultativement substitué par un ou plusieurs substituants $R^6$ et dans lequel un ou plusieurs groupes $CH_2$ non adjacents sont facultativement substitués par $R^6C=CR^6$, C≡C, $Si(R^6)_2$, $Ge(R^6)_2$, $Sn(R^6)_2$, C=O, C=S, C=Se, $C=NR^6$, $P(=O)(R^6)$, SO, $SO_2$, $NR^6$, O, S ou $CONR^6$ ;
aryle en $C_6$ à $C_{60}$,
qui est facultativement substitué par un ou plusieurs substituants $R^6$ ; et hétéroaryle en $C_3$ à $C_{57}$,
qui est facultativement substitué par un ou plusieurs substituants $R^6$ ;
$R^f$ est, à chaque occurrence indépendamment d'une autre, choisi dans le groupe consistant en hydrogène,
deutérium,

$N(R^{5f})_2$,

$OR^{5f}$,

$Si(R^{5f})_3$,

$B(OR^{5f})_2$,

$OSO_2R^{5f}$,

$CF_3$,

CN,

F,

Br,

I,

alkyle en $C_1$ à $C_{40}$,
qui est facultativement substitué par un ou plusieurs substituants $R^{5f}$ et
dans lequel un ou plusieurs groupes $CH_2$ non adjacents sont facultativement substitués par $R^{5f}C=CR^{5f}$, C≡C, $Si(R^{5f})_2$, $Ge(R^{5f})_2$, $Sn(R^{5f})_2$, C=O, C=S, C=Se, $C=NR^{5f}$, $P(=O)(R^{5f})$, SO, $SO_2$, $NR^{5f}$, O, S ou $CONR^{5f}$ ;
alcoxy en $C_1$ à $C_{40}$,
qui est facultativement substitué par un ou plusieurs substituants $R^{5f}$ et
dans lequel un ou plusieurs groupes $CH_2$ non adjacents sont facultativement substitués par $R^{5f}C=CR^{5f}$, C≡C, $Si(R^{5f})_2$, $Ge(R^{5f})_2$, $Sn(R^{5f})_2$, C=O, C=S, C=Se, $C=NR^{5f}$, $P(=O)(R^{5f})$, SO, $SO_2$, $NR^{5f}$, O, S ou $CONR^{5f}$ ;
thioalcoxy en $C_1$ à $C_{40}$,
qui est facultativement substitué par un ou plusieurs substituants $R^{5f}$ et
dans lequel un ou plusieurs groupes $CH_2$ non adjacents sont facultativement substitués par $R^{5f}C=CR^{5f}$, C≡C, $Si(R^{5f})_2$, $Ge(R^{5f})_2$, $Sn(R^{5f})_2$, C=O, C=S, C=Se, $C=NR^{5f}$, $P(=O)(R^{5f})$, SO, $SO_2$, $NR^{5f}$, O, S ou $CONR^{5f}$ ;
alcényle en $C_2$ à $C_{40}$,
qui est facultativement substitué par un ou plusieurs substituants $R^{5f}$ et
dans lequel un ou plusieurs groupes $CH_2$ non adjacents sont facultativement substitués par $R^{5f}C=CR^{5f}$, C≡C, $Si(R^{5f})_2$, $Ge(R^{5f})_2$, $Sn(R^{5f})_2$, C=O, C=S, C=Se, $C=NR^{5f}$, $P(=O)(R^{5f})$, SO, $SO_2$, $NR^{5f}$, O, S ou $CONR^{5f}$ ;
alcynyle en $C_2$ à $C_{40}$,
qui est facultativement substitué par un ou plusieurs substituants $R^{5f}$ et
dans lequel un ou plusieurs groupes $CH_2$ non adjacents sont facultativement substitués par $R^{5f}C=CR^{5f}$, C≡C, $Si(R^{5f})_2$, $Ge(R^{5f})_2$, $Sn(R^{5f})_2$, C=O, C=S, C=Se, $C=NR^{5f}$, $P(=O)(R^{5f})$, SO, $SO_2$, $NR^{5f}$, O, S ou $CONR^{5f}$ ;
aryle en $C_6$ à $C_{60}$,
qui est facultativement substitué par un ou plusieurs substituants $R^{5f}$ ; et
hétéroaryle en $C_3$ à $C_{57}$,
qui est facultativement substitué par un ou plusieurs substituants $R^{5f}$ ;
$R^{5f}$ est, à chaque occurrence indépendamment d'une autre, choisi dans le groupe consistant en
hydrogène,
deutérium,

$N(R^6)_2$,

$OR^6$,

$Si(R^6)_3$,

$B(OR^6)_2$,

$OSO_2R^6$,

$CF_3$,

CN,

F,

Br,

I,

alkyle en $C_1$ à $C_{40}$,
qui est facultativement substitué par un ou plusieurs substituants $R^6$ et
dans lequel un ou plusieurs groupes $CH_2$ non adjacents sont facultativement substitués par $R^6C=CR^6$, C≡C, $Si(R^6)_2$, $Ge(R^6)_2$, $Sn(R^6)_2$, C=O, C=S, C=Se, $C=NR^6$, $P(=O)(R^6)$, SO, $SO_2$,

$NR^6$, O, S ou $CONR^6$ ;
alcoxy en $C_1$ à $C_{40}$,
qui est facultativement substitué par un ou plusieurs substituants $R^6$ et
dans lequel un ou plusieurs groupes $CH_2$ non adjacents sont facultativement substitués par $R^6C=CR^6$, $C\equiv C$, $Si(R^6)_2$, $Ge(R^6)_2$, $Sn(R^6)_2$, C=O, C=S, C=Se, $C=NR^6$, $P(=O)(R^6)$, SO, $SO_2$, $NR^6$, O, S ou $CONR^6$ ;
thioalcoxy en $C_1$ à $C_{40}$,
qui est facultativement substitué par un ou plusieurs substituants $R^6$ et
dans lequel un ou plusieurs groupes $CH_2$ non adjacents sont facultativement substitués par $R^6C=CR^6$, $C\equiv C$, $Si(R^6)_2$, $Ge(R^6)_2$, $Sn(R^6)_2$, C=O, C=S, C=Se, $C=NR^6$, $P(=O)(R^6)$, SO, $SO_2$, $NR^6$, O, S ou $CONR^6$ ;
alcényle en $C_2$ à $C_{40}$,
qui est facultativement substitué par un ou plusieurs substituants $R^6$ et
dans lequel un ou plusieurs groupes $CH_2$ non adjacents sont facultativement substitués par $R^6C=CR^6$, $C\equiv C$, $Si(R^6)_2$, $Ge(R^6)_2$, $Sn(R^6)_2$, C=O, C=S, C=Se, $C=NR^6$, $P(=O)(R^6)$, SO, $SO_2$, $NR^6$, O, S ou $CONR^6$ ;
alcynyle en $C_2$ à $C_{40}$,
qui est facultativement substitué par un ou plusieurs substituants $R^6$ et
dans lequel un ou plusieurs groupes $CH_2$ non adjacents sont facultativement substitués par $R^6C=CR^6$, $C\equiv C$, $Si(R^6)_2$, $Ge(R^6)_2$, $Sn(R^6)_2$, C=O, C=S, C=Se, $C=NR^6$, $P(=O)(R^6)$, SO, $SO_2$, $NR^6$, O, S ou $CONR^6$ ;
aryle en $C_6$ à $C_{60}$,
qui est facultativement substitué par un ou plusieurs substituants $R^6$ ; et
hétéroaryle en $C_3$ à $C_{57}$,
qui est facultativement substitué par un ou plusieurs substituants $R^6$ ;
$R^6$ est, à chaque occurrence indépendamment d'une autre, choisi dans le groupe consistant en :

hydrogène, deutérium, OPh, $CF_3$, CN, F,
alkyle en $C_1$ à $C_5$,
dans lequel un ou plusieurs atomes d'hydrogène sont facultativement, indépendamment les uns des autres, substitués par du deutérium, CN, $CF_3$ ou F ;
alcoxy en $C_1$ à $C_5$,
dans lequel un ou plusieurs atomes d'hydrogène sont facultativement, indépendamment les uns des autres, substitués par du deutérium, CN, $CF_3$ ou F ;
thioalcoxy en $C_1$ à $C_5$,
dans lequel un ou plusieurs atomes d'hydrogène sont facultativement, indépendamment les uns des autres, substitués par du deutérium, CN, $CF_3$ ou F ;
alcényle en $C_2$ à $C_5$,
dans lequel un ou plusieurs atomes d'hydrogène sont facultativement, indépendamment les uns des autres, substitués par du deutérium, CN, $CF_3$ ou F ;
alcynyle en $C_2$ à $C_5$,
dans lequel un ou plusieurs atomes d'hydrogène sont facultativement, indépendamment les uns des autres, substitués par du deutérium, CN, $CF_3$ ou F ;
aryle en $C_6$ à $C_{18}$,
qui est facultativement substitué par un ou plusieurs substituants alkyle en $C_1$ à $C_5$ ;
hétéroaryle en $C_3$ à $C_{17}$,
qui est facultativement substitué par un ou plusieurs substituants alkyle en $C_1$ à $C_5$ ;
N(aryle en $C_6$ à $C_{18})_2$ ;
N(hétéroaryle en $C_3$ à $C_{17})_2$,
et N(hétéroaryl en $C_3$ à $C_{17}$)(aryle en $C_6$ à $C_{18}$) ;
dans laquelle les substituants $R^a$, $R^3$, $R^4$ ou $R^5$ indépendamment les uns des autres forment facultativement un système de cycle mono- ou polycyclique, aliphatique, aromatique et/ou benzo-fusionné avec un ou plusieurs substituants $R^a$, $R^3$, $R^4$ ou $R^5$ ;
facultativement, un ou plusieurs des substituants $R^f$ ou $R^{5f}$ indépendamment les uns des autres forment un système de cycle mono- ou polycyclique, aliphatique, aromatique et/ou benzo-fusionné avec un ou plusieurs autres substituants $R^f$ ou $R^{5f}$ ;
dans laquelle

- exactement un chaînon de cycle choisi dans le groupe consistant en $L^T$, $L^V$ et $L^W$ est N,
- exactement un substituant choisi dans le groupe consistant en $R^T$, $R^V$, $R^W$, $R^X$ et $R^Y$ est $R^A$,
- exactement un substituant choisi dans le groupe consistant en T, V, W, X et Y est le site de liaison d'une simple liaison reliant la première fraction chimique à la troisième fraction chimique, et
- exactement un substituant choisi dans le groupe consistant en $R^W$ et $R^Y$ représente le site de liaison d'une simple lorsque reliant la première fraction chimique et la deuxième fraction chimique.

**2.** Molécule organique selon la revendication 1, dans laquelle la première fraction chimique comprend une structure de formule Ia :

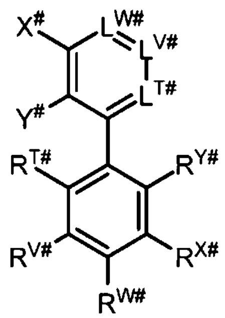

Formule Ia

dans laquelle $R^1$, $R^2$ ; $R^I$ et $R^A$ sont définis comme ci-dessus ;
$L^{T\#}$ est N ou C-$R^1$ ;
$L^{V\#}$ est N ou C-$R^1$ ;
$L^{W\#}$ est N ou C-$W^\#$ ;
$W^\#$ est le site de liaison d'une simple liaison reliant la première fraction chimique à la troisième fraction chimique ou est $R^1$ ;
$X^\#$ est le site de liaison d'une simple liaison reliant la première fraction chimique à la troisième fraction chimique ou est $R^1$ ;
$Y^\#$ est le site de liaison d'une simple liaison reliant la première fraction chimique à la troisième fraction chimique ou est $R^1$ ;
$R^{T\#}$ est choisi dans le groupe consistant en $R^A$ et $R^2$ ;
$R^{Y\#}$ est choisi dans le groupe consistant en $R^A$ et $R^2$ ;
$R^{W\#}$ est le site de liaison d'une simple liaison reliant la première fraction chimique à la deuxième fraction chimique ou est choisi dans le groupe consistant en $R^A$ et $R^2$ ;
$R^{X\#}$ est $R^2$ ;
$R^{Y\#}$ est le site de liaison d'une simple liaison reliant la première fraction chimique à la deuxième fraction chimique ou est $R^2$ ;
dans laquelle

- exactement un chaînon de cycle (atome ou groupe) choisi dans le groupe consistant en $L^{T\#}$, $L^{V\#}$ et $L^{W\#}$ est N ;
- exactement un substituant choisi dans le groupe consistant en $R^{T\#}$, $R^{V\#}$ et $R^{W\#}$ est $R^A$, exactement un substituant choisi dans le groupe consistant en $W^\#$, $X^\#$ et $Y^\#$ est le site de liaison d'une simple liaison reliant la première fraction chimique à la troisième fraction chimique

et exactement un substituant choisi dans le groupe consistant en $R^{W\#}$ et $R^{Y\#}$ représente le site de liaison d'une simple liaison reliant la première fraction chimique et la deuxième fraction chimique ;
et dans laquelle à part cela, les définitions de la revendication 1 s'appliquent.

**3.** Molécule organique selon la revendication 1 ou 2, dans laquelle $R^1$ et $R^2$ sont indépendamment l'un de l'autre, à chaque occurrence indépendamment d'une autre, choisis dans le groupe consistant en H, méthyle, mésityle, tolyle et

phényle.

4. Molécule organique selon une ou plusieurs des revendications 1 à 3, dans laquelle $R^{Tz}$ est, indépendamment d'un autre, choisi dans le groupe consistant en H, méthyle et phényle.

5. Molécule organique selon une ou plusieurs des revendications 1 à 4, dans laquelle la deuxième fraction chimique comprend une structure de formule IIa :

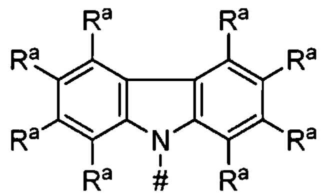

Formule IIa

dans laquelle # et $R^a$ sont définis comme en revendication 1.

6. Molécule organique selon une ou plusieurs des revendications 1 à 5, dans laquelle la deuxième fraction chimique comprend une structure de formule IIb :

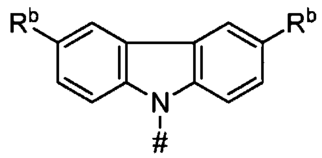

Formule IIb

dans laquelle
$R^b$ est, à chaque occurrence indépendamment d'une autre, choisi dans le groupe consistant en :

deutérium, $N(R^5)_2$, $OR^5$, $Si(R^5)_3$, $B(OR^5)_2$, $OSO_2R^5$, $CF_3$, CN, F, Br, I,
alkyle en $C_1$ à $C_{40}$,
qui est facultativement substitué par un ou plusieurs substituants $R^5$ et
dans lequel un ou plusieurs groupes $CH_2$ non adjacents sont facultativement substitués par $R^5C=CR^5$, $C\equiv C$, $Si(R^5)_2$, $Ge(R^5)_2$, $Sn(R^5)_2$, C=O, C=S, C=Se, $C=NR^5$, $P(=O)(R^5)$, SO, $SO_2$, $NR^5$, O, S ou $CONR^5$ ;
alcoxy en $C_1$ à $C_{40}$,
qui est facultativement substitué par un ou plusieurs substituants $R^5$ et
dans lequel un ou plusieurs groupes $CH_2$ non adjacents sont facultativement substitués par $R^5C=CR^5$, $C\equiv C$, $Si(R^5)_2$, $Ge(R^5)_2$, $Sn(R^5)_2$, C=O, C=S, C=Se, $C=NR^5$, $P(=O)(R^5)$, SO, $SO_2$, $NR^5$, O, S ou $CONR^5$ ;
thioalcoxy en $C_1$ à $C_{40}$,
qui est facultativement substitué par un ou plusieurs substituants $R^5$ et
dans lequel un ou plusieurs groupes $CH_2$ non adjacents sont facultativement substitués par $R^5C=CR^5$, $C\equiv C$, $Si(R^5)_2$, $Ge(R^5)_2$, $Sn(R^5)_2$, C=O, C=S, C=Se, $C=NR^5$, $P(=O)(R^5)$, SO, $SO_2$, $NR^5$, O, S ou $CONR^5$ ;
alcényle en $C_2$ à $C_{40}$,
qui est facultativement substitué par un ou plusieurs substituants $R^5$ et
dans lequel un ou plusieurs groupes $CH_2$ non adjacents sont facultativement substitués par $R^5C=CR^5$, $C\equiv C$, $Si(R^5)_2$, $Ge(R^5)_2$, $Sn(R^5)_2$, C=O, C=S, C=Se, $C=NR^5$, $P(=O)(R^5)$, SO, $SO_2$, $NR^5$, O, S ou $CONR^5$ ;
alcynyle en $C_2$ à $C_{40}$,
qui est facultativement substitué par un ou plusieurs substituants $R^5$ et
dans lequel un ou plusieurs groupes $CH_2$ non adjacents sont facultativement substitués par $R^5C=CR^5$, $C\equiv C$, $Si(R^5)_2$, $Ge(R^5)_2$, $Sn(R^5)_2$, C=O, C=S, C=Se, $C=NR^5$, $P(=O)(R^5)$, SO, $SO_2$, $NR^5$, O, S ou $CONR^5$ ;
aryle en $C_6$ à $C_{60}$,
qui est facultativement substitué par un ou plusieurs substituants $R^5$ ; et
hétéroaryle en $C_3$ à $C_{57}$,

qui est facultativement substitué par un ou plusieurs substituants $R^5$ ;
et dans laquelle à part cela, les définitions de la revendication 1 s'appliquent.

**7.** Molécule organique selon une ou plusieurs des revendications 1 à 5, dans laquelle la deuxième fraction chimique comprend une structure de formule IIc :

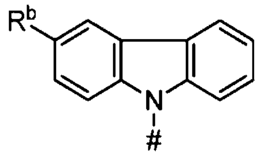

Formule IIc

dans laquelle

$R^b$ est, à chaque occurrence indépendamment d'une autre, choisi dans le groupe consistant en
deutérium, $N(R^5)_2$, $OR^5$, $Si(R^5)_3$, $B(OR^5)_2$, $OSO_2R^5$, $CF_3$, CN, F, Br, I,
alkyle en $C_1$ à $C_{40}$,
qui est facultativement substitué par un ou plusieurs substituants $R^5$ et
dans lequel un ou plusieurs groupes $CH_2$ non adjacents sont facultativement substitués par $R^5C=CR^5$, C≡C, $Si(R^5)_2$, $Ge(R^5)_2$, $Sn(R^5)_2$, C=O, C=S, C=Se, $C=NR^5$, $P(=O)(R^5)$, SO, $SO_2$, $NR^5$, O, S ou $CONR^5$ ;
alcoxy en $C_1$ à $C_{40}$,
qui est facultativement substitué par un ou plusieurs substituants $R^5$ et
dans lequel un ou plusieurs groupes $CH_2$ non adjacents sont facultativement substitués par $R^5C=CR^5$, C≡C, $Si(R^5)_2$, $Ge(R^5)_2$, $Sn(R^5)_2$, C=O, C=S, C=Se, $C=NR^5$, $P(=O)(R^5)$, SO, $SO_2$, $NR^5$, O, S ou $CONR^5$ ;
thioalcoxy en $C_1$ à $C_{40}$,
qui est facultativement substitué par un ou plusieurs substituants $R^5$ et
dans lequel un ou plusieurs groupes $CH_2$ non adjacents sont facultativement substitués par $R^5C=CR^5$, C≡C, $Si(R^5)_2$, $Ge(R^5)_2$, $Sn(R^5)_2$, C=O, C=S, C=Se, $C=NR^5$, $P(=O)(R^5)$, SO, $SO_2$, $NR^5$, O, S ou $CONR^5$ ;
alcényle en $C_2$ à $C_{40}$,
qui est facultativement substitué par un ou plusieurs substituants $R^5$ et
dans lequel un ou plusieurs groupes $CH_2$ non adjacents sont facultativement substitués par $R^5C=CR^5$, C≡C, $Si(R^5)_2$, $Ge(R^5)_2$, $Sn(R^5)_2$, C=O, C=S, C=Se, $C=NR^5$, $P(=O)(R^5)$, SO, $SO_2$, $NR^5$, O, S ou $CONR^5$ ;
alcynyle en $C_2$ à $C_{40}$,
qui est facultativement substitué par un ou plusieurs substituants $R^5$ et
dans lequel un ou plusieurs groupes $CH_2$ non adjacents sont facultativement substitués par $R^5C=CR^5$, C≡C, $Si(R^5)_2$, $Ge(R^5)_2$, $Sn(R^5)_2$, C=O, C=S, C=Se, $C=NR^5$, $P(=O)(R^5)$, SO, $SO_2$, $NR^5$, O, S ou $CONR^5$ ;
aryle en $C_6$ à $C_{60}$,
qui est facultativement substitué par un ou plusieurs substituants $R^5$ ; et
hétéroaryle en $C_3$ à $C_{57}$,
qui est facultativement substitué par un ou plusieurs substituants $R^5$ ;
et dans laquelle à part cela, les définitions de la revendication 1 s'appliquent.

**8.** Molécule organique selon la revendication 6 ou 7, dans laquelle $R^b$ est, à chaque occurrence indépendamment d'une autre, choisi dans le groupe consistant en

- Me, $^i$Pr, $^t$Bu, CN, $CF_3$,
- Ph, qui est facultativement substitué par un ou plusieurs substituants choisis indépendamment les uns des autres dans le groupe consistant en Me, $^i$Pr, $^t$Bu, CN, $CF_3$ et Ph,
- pyridinyle, qui est facultativement substitué par un ou plusieurs substituants choisis indépendamment les uns des autres dans le groupe consistant en Me, $^i$Pr, $^t$Bu, CN, $CF_3$ et Ph ;
- pyrimidinyle, qui est facultativement substitué par un ou plusieurs substituants choisis indépendamment les uns des autres dans le groupe consistant en Me, $^i$Pr, $^t$Bu, CN, $CF_3$ et Ph ;
- carbazolyle, qui est facultativement substitué par un ou plusieurs substituants choisis indépendamment les uns des autres dans le groupe consistant en Me, $^i$Pr, $^t$Bu, CN, $CF_3$ et Ph ;
- triazinyle, qui est facultativement substitué par un ou plusieurs substituants choisis indépendamment les uns des autres dans le groupe consistant en Me, $^i$Pr, $^t$Bu, CN, $CF_3$ et Ph ;

et
- $N(Ph)_2$.

**9.** Utilisation d'une molécule organique selon une ou plusieurs des revendications 1 à 8 en tant qu'émetteur luminescent et/ou matériau hôte et/ou matériau de transport d'électrons et/ou matériau d'injection de trous et/ou matériau de blocage de trous dans un dispositif optoélectronique.

**10.** Utilisation selon la revendication 9, dans laquelle le dispositif optoélectronique est choisi dans le groupe consistant en :

- les diodes électroluminescentes organiques (OLED),
- les cellules électrochimiques électroluminescentes,
- les capteurs OLED,
- les diodes organiques,
- les cellules solaires organiques,
- les transistors organiques,
- les transistors à effet de champ organiques,
- les lasers organiques et
- les éléments de conversion vers le bas.

**11.** Composition, comprenant :

(a) au moins une molécule organique selon une ou plusieurs des revendications 1 à 8, en particulier sous la forme d'un émetteur et/ou d'un hôte, et
(b) un ou plusieurs émetteurs et/ou matériaux hôtes, qui diffèrent de la molécule organique des une ou plusieurs des revendications 1 à 8, et
(c) facultativement, une ou plusieurs teintures et/ou un ou plusieurs solvants.

**12.** Dispositif optoélectronique, comprenant une molécule organique selon une ou plusieurs des revendications 1 à 8 ou une composition selon la revendication 11, en particulier sous la forme d'un dispositif choisi dans le groupe consistant en une diode électroluminescente organique (OLED), une cellule électrochimique électroluminescente, un capteur OLED, une diode organique, une cellule solaire organique, un transistor organique, un transistor à effet de champ organique, un laser organique et un élément de conversion vers le bas.

**13.** Dispositif optoélectronique selon la revendication 12, comprenant

- un substrat,
- une anode et
- une cathode, l'anode ou la cathode étant disposées sur le substrat, et
- au moins une couche électroluminescente, qui est disposée entre l'anode et la cathode et qui comprend une molécule organique selon l'une quelconque des revendications 1 à 8 ou une composition selon la revendication 11.

**14.** Procédé de production d'un dispositif optoélectronique, dans lequel une molécule organique selon l'une quelconque des revendications 1 à 8 ou une composition selon la revendication 11 est utilisée.

**15.** Procédé selon la revendication 14, comprenant le traitement de la molécule organique par un procédé d'évaporation sous vide ou à partir d'une solution.

**Figure 1**

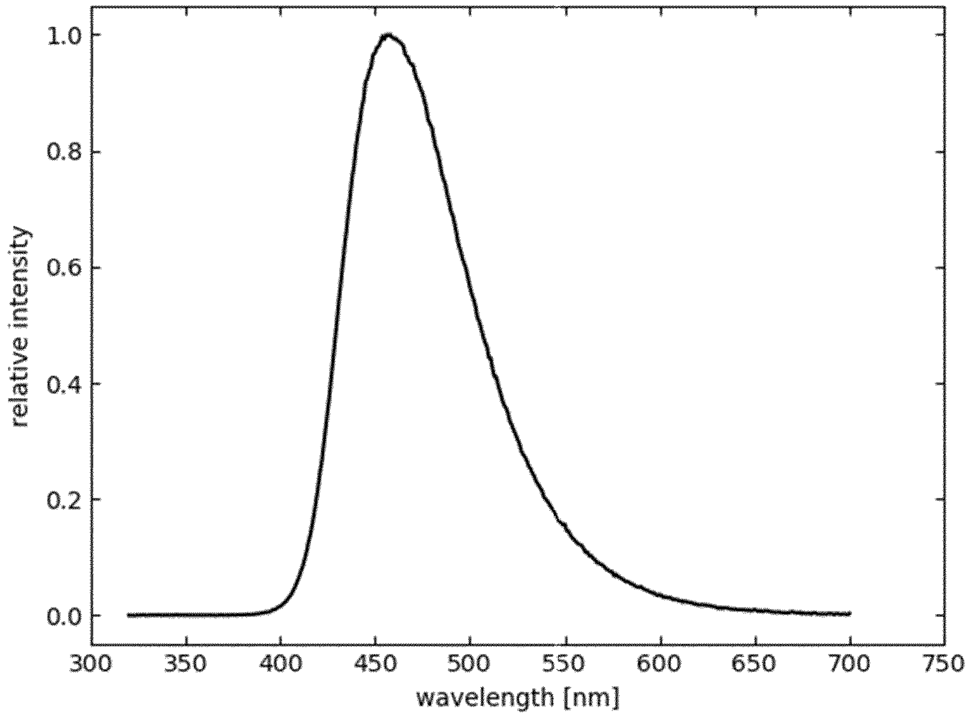


**Figure 2**

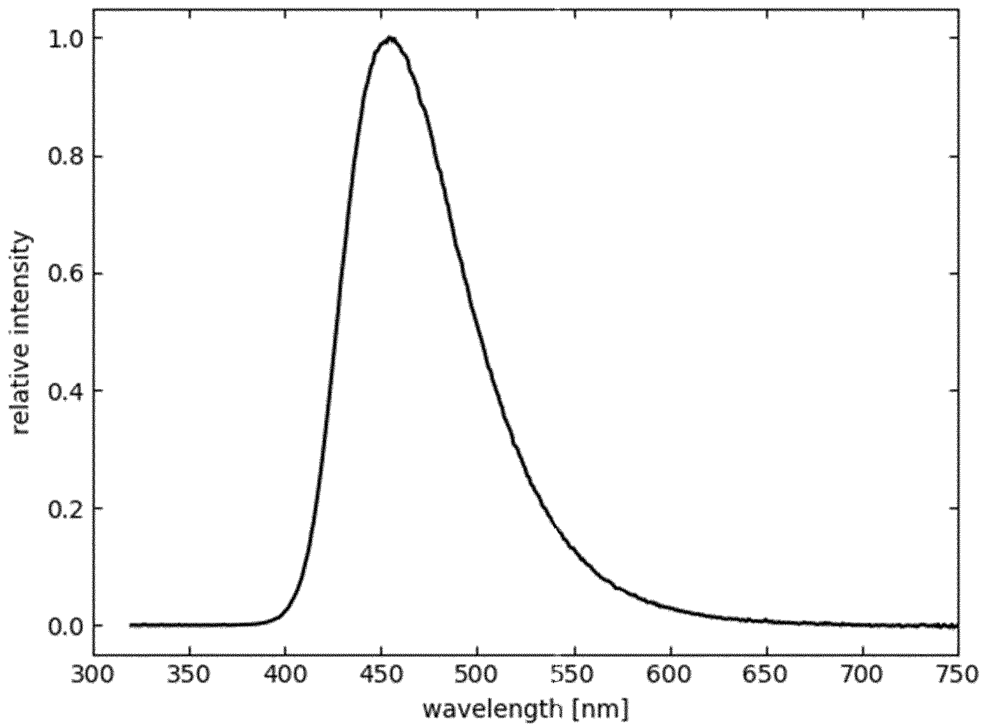

## REFERENCES CITED IN THE DESCRIPTION

*This list of references cited by the applicant is for the reader's convenience only. It does not form part of the European patent document. Even though great care has been taken in compiling the references, errors or omissions cannot be excluded and the EPO disclaims all liability in this regard.*

### Patent documents cited in the description

- WO 2016089080 A1

### Non-patent literature cited in the description

- *CHEMICAL ABSTRACTS*, 73183-34-3
- *CHEMICAL ABSTRACTS*, 51364-51-3
- *CHEMICAL ABSTRACTS*, 564483-18-7
- *CHEMICAL ABSTRACTS*, 36953-42-1
- *CHEMICAL ABSTRACTS*, 402936-15-6